# EUROPEAN PATENT SPECIFICATION

(11) **EP 3 226 932 B1**
(45) Date of publication and mention of the grant of the patent: **27.03.2024**
(21) Application number: 15825663.6
(22) Date of filing: 02.12.2015
(51) Int. Cl.: A61M 5/14, F16B 2/10

(54) **SYSTEM, METHOD, AND APPARATUS FOR CLAMPING**
SYSTEM, VERFAHREN UND VORRICHTUNG ZUR KLEMMUNG
SYSTÈME, PROCÉDÉ ET APPAREIL DE FIXATION

(30) Priority: 02.12.2014 US 201462086356 P
(43) Date of publication of application: 11.10.2017
(73) Proprietor: DEKA Products Limited Partnership, Manchester, NH 03101 (US)
(72) Inventor: JANWAY, Jeffrey M., Hooksett, NH 03106 (US); GRAY, Larry B., Merrimack, NH 03054 (US); GILL, Matthew R., San Francisco, CA 94121 (US); LANIGAN, Richard J., Concord, NH 03301 (US); FRIEDRICH, Thomas A., Loudon, NH 03307 (US); FICHERA, Stephen L., Salem, NH 03079 (US); BODWELL, Jesse T., Manchester, NH 03109 (US); SABIN, Erik N., Manchester, NH 03101 (US)
(74) Representative: Haseltine Lake Kempner LLP
(86) International application number: PCT/US2015/063359
(87) International publication number: WO 2016/089955

(56) References cited:
- EP-A1- 2 532 901
- WO-A1-2010/128929
- WO-A2-2014/100744
- US-A1- 2005 230 444
- US-B1- 8 631 544

## Description

### BACKGROUND

### Field of Disclosure

The present disclosure relates generally to releasably attaching an object to another object (e.g., clamping a medical device onto a pole). More particularly, the present disclosure relates to a system, method, and apparatus for mounting an object onto a pole or other support structure.

### Description of Related Art

Patient care generally involves a number of medical devices and systems that are used to monitor and treat a patient. The specific medical devices required vary with each patient and may change during the course of treatment. Medical devices often require monitoring by health care providers and so need to be easily accessible. They are often expensive, so redundancy is rarely possible, and a given device will often need to be moved to a different patient after a treatment is completed. Given their expense, medical devices need to be firmly and safely attached to a location to prevent either their damage or an interruption to patient care should they come unattached.

Medical devices are typically attached to a vertical pole located near the bedside of their assigned patient. This arrangement facilitates: the attached equipment to be customized according to patient's treatment, convenient monitoring by health care providers, minimizing the length of tubing or other connections between the patient and the device, and moving the pole and the attached equipment to follow movement of the patient. A typical attachment involves a brace fixed to the medical device and a threaded screw that can be tightened to squeeze a section of the support pole positioned between the brace and the screw. Typically, turning the screw clockwise advances the screw into the interior of the brace and attaches the medical device to the pole; counterclockwise rotation retracts the screw and allows the device to be removed. Once the advancing screw contacts the support pole, it exerts a predominantly compression-based force into the pole which holds the medical device in position against the downward pull of gravity. The user manually adjusts the clamp to poles of different diameter by varying the number of screw rotations and rotational direction of screw rotations thus controlling how far into the brace interior the screw is extended. Such positioning and adjustment faces a number of constraints, for example, it can be time consuming, there is risk of cross-threading, there is risk of human error (i.e. not tightening enough) etc.

WO 2010/128929 describes a suspension device for suspending a sign or another article from an elongated object. The suspension device comprises a base part, a holder connected to the base part, and two gripping shanks which are pivotally mounted to the base part and pivotable in relation to each other to and fro between an open position and a closed position. One of the gripping shanks is pivotable in relation to the base part about a first axis of pivotation and the other gripping shank is pivotable in relation to the base part about a second axis of pivotation, which extends in parallel with and at a distance from the first axis of pivotation. The suspension device further comprises at least one spring member acting between the gripping shanks and at least one gear mechanism with a first gear member rigidly connected to one of the gripping shanks and a second gear member rigidly connected to the other gripping shank. The gear members are in engagement with each other and arranged to be turned in relation to each other when the gripping shanks are pivoted in relation to each other. WO2014/100744A2 shows a further clamping assembly according to the preamble of claim 1.

### SUMMARY

The invention is defined in the attached independent claim to which reference should now be made. Further, optional features may be found in the sub-claims appended thereto.

In accordance with an aspect of the invention there is provided a clamp assembly configured for clamping a medical device onto a graspable structure, said clamp assembly comprising: a frame with a top surface and a bottom surface spaced from the top surface so as to form a gap between the top surface and the bottom surface, the top surface comprising a first indentation and the bottom surface comprising a second indentation, the first and second indentations being configured to receive a graspable structure, the top surface and the bottom surface being connected using a back surface, the back surface further comprising an interior face and an opposing exterior face; a first jawed component and a second jawed component, the first and second jawed components each being pivotally retained in the frame such that at least part of the first and second jawed components are disposed between the top surface and the bottom surface in the gap and so as to have an axis of rotation substantially parallel to the back surface, the first and second jawed components each comprising a jawed end and a geared end opposing the jawed end, wherein the geared end of the first jawed component and the geared end of the second jawed component interdigitate; a first actuator and a second actuator coupled to the first and second jawed components, respectively, the first and second actuators each comprising an end, the ends of the first end second actuators being distal to the first and second jawed components, respectively; and at least one bias member biasing the first and second jawed components to first positions in which there is no interference between the first and second indentations and the graspable structure wherein displacement of the first and second actuators causes the displacement of the first and second jawed components to pivot from their first positions to second positions in which the jawed ends of the first and second jawed components are spread apart from each other whereas the geared ends ensure substantially equal and opposite pivotal displacement of the first and second jawed components. The bottom surface further comprises an alignment component configured to be received by complementary alignment component of the graspable structure.

The frame may further comprise a plurality of sections disposed on the interior face of the back surface. The plurality of sections may be configured to accept the first and second jawed components. The first and second jawed components may be pivotally retained via first and second connectors disposed between the top surface and the bottom surface of the frame. The first and second jawed components may pivot around first and second axes of rotation passing through the connectors. The connectors may be hinge pins. The first and second jawed components may further comprise one or more pockets configured to substantially receive the at least one bias member. The at least one bias member may be partially compressed in order to be placed in the one or more pockets of the first and second jawed components and/or be substantially disposed in opposing one or more pockets of the first and second jawed components. The displacement of the first and second actuators may cause displacement of the first and second jawed components which may further compress the at least one bias member. The at least one bias member may be configured to hold the first and second jawed components in their first positions and/or allow the first and second jawed components to be displaced into their second positions and/or allow the first and second jawed components to be displaced, which may cause the jawed ends to grip the graspable structure. The jawed ends of the first and second jawed components may be substantially covered with a layer. The layer may be made of a first material and the first material is an elastomeric material. The layer may be made of a first material having a high friction coefficient with a second material of a holding structure and/or a material having high friction coefficient with the material of a one or more slide-able members disposed on the holding structure, such as rubber. The clamp assembly may further comprise a latch. The frame may further comprise a socket on the back surface. The socket may be configured to pivotally retain the latch via a latch retaining bias member. The latch may be configured to couple the clamp assembly with a clamping device when the latch pivots to a locking position and/or release the clamping device when the latch pivots to an unlocking position. The latch may further comprise a flap portion and a lever portion. The flap portion of the latch may be pivoted towards an interior of the frame to receive one or more pairing members of a clamping device, in the socket. The flap portion of the latch may be pivoted back after completely receiving the one or more pairing members of the clamping device, in the socket. The lever portion may be configured to operatively allow releasing the pairing members from the socket in the back surface of the frame. The frame may further comprise a plurality of tracks configured to receive the first and second jawed components. The jawed ends of the first and second jawed components may be coupled with the frame through first and second connectors, respectively. The first and second jawed components may be coupled with the frame through hinge pins. The first and second jawed components may further comprise sections configured to partially receive the first and second actuators. Each of the first and second actuators may further comprise a pairing member and a paddle member. The sections of the first and second jawed components may be configured to receive the pairing members of the first and second actuators. The displacement of the first and second actuators may cause a desired displacement of the first and second jawed components.

### BRIEF DESCRIPTION OF THE DRAWINGS

These and other aspects will become more apparent from the following detailed description of the various embodiments of the present disclosure with reference to the drawings wherein:
FIGS. 1A-1E show several views of a clamp in accordance with an embodiment of the present disclosure;
FIGS. 2A-2E show several views of a clamp in accordance with an embodiment of the present disclosure;
FIGS. 3A-3E show several views of a clamp in accordance with an embodiment of the present disclosure;
FIGS. 4A-4D show several views of a clamp in accordance with an embodiment of the present disclosure;
FIGS. 5A-5D show several views of a clamp in accordance with an embodiment of the present disclosure;
FIGS. 6A-6G show several views of a clamp in accordance with an embodiment of the present disclosure;
FIGS. 7A-7D show several views of a clamp in accordance with an embodiment of the present disclosure;
FIGS. 8A-8D show several views of a clamp in accordance with an embodiment of the present disclosure;
FIGS. 8E-8F show an alternate embodiment of the clamp shown in FIGS. 8A-8D in accordance with an embodiment of the present disclosure;
FIG. 8G shows an alternate embodiment of a moveable gripper assembly with a housing in accordance with an embodiment of the present disclosure;
FIG. 9A is a perspective view of an exemplary embodiment of a rack apparatus in accordance with an embodiment of the present disclosure;
FIG. 9B is a perspective view of an exemplary embodiment of a device mount, like those depicted in FIG. 9a, wherein the device mount includes a support plate that is adapted to receive a medical device in accordance with an embodiment of the present disclosure;
FIG. 9C is a perspective view of an exemplary embodiment of a joint member that is adapted to couple with the embodiment of a device mount that is depicted in FIG. 9b in accordance with an embodiment of the present disclosure;
FIG. 9D is a perspective view of an exemplary embodiment of a base member that includes a power system that is configured to transmit power to at least one device mount like the embodiment depicted in FIG. 9b in accordance with an embodiment of the present disclosure;
FIG. 9E depicts a perspective view of another exemplary embodiment of a rack apparatus in accordance with an embodiment of the present disclosure;
FIG. 9F is a perspective view of yet another exemplary embodiment of a rack apparatus in accordance with an embodiment of the present disclosure;
FIG. 9G-H are perspective views of an example collar of a rack apparatus in accordance with an embodiment of the present disclosure;
FIG. 9I is a perspective view of an example rack apparatus including a number of mount connectors in accordance with an embodiment of the present disclosure;
FIG. 9J is a perspective view of an example rack apparatus in accordance with an embodiment of the present disclosure;
FIG. 9K is an enlarged view of a portion of the support pole of the example rack apparatus depicted in FIG. 9j in accordance with an embodiment of the present disclosure;
FIG. 10A is a perspective view of an exemplary embodiment of a rack system, wherein the embodiment of a rack depicted in FIG. 9A includes a support pole adapted to couple with the clamp of a medical device in accordance with an embodiment of the present disclosure;
FIG. 10B is a close-up, perspective view of the exemplary embodiment of a rack system depicted in FIG. 10A, wherein the rack embodiment includes a mount connector that may couple to a device connector when the medical device couples with the support pole in accordance with an embodiment of the present disclosure; and
FIG. 10C is another alternate perspective view of the exemplary embodiment of a rack system depicted in FIG. 10A, wherein an embodiment of a medical device includes an embodiment of a device connector that may couple to a mount connector, like the embodiment depicted in FIG. 10B, when the medical device couples with the support pole in accordance with an embodiment of the present disclosure.
FIG. 10D is a perspective view of an exemplary embodiment of a rack system including the embodiment of a rack depicted in FIG. 9e in accordance with an embodiment of the present disclosure;
FIG. 10E is a close-up perspective view of an example infusion pump with a flange in place in the guide trough of a support plate of the example rack embodiment depicted in FIG. 9e in accordance with an embodiment of the present disclosure;
FIG. 10F is a perspective view of an exemplary embodiment of a rack system, wherein the embodiment of a rack depicted in FIG. 9f includes a support pole adapted to couple with the clamp of a medical device in accordance with an embodiment of the present disclosure;
FIG. 10G depicts a perspective view of a support pole of a rack system, wherein the support pole includes a number of mount connectors on a mount connector strip in accordance with an embodiment of the present disclosure;
FIG. 10H depicts a perspective view of a number of mount connectors on a mount connector strip in accordance with an embodiment of the present disclosure;
FIG. 10I depicts a view of a part of an example support pole of an example rack system, wherein the support pole includes a mount connector on a mount connector strip in accordance with an embodiment of the present disclosure;
FIG. 10J shows a side view of an example mount connector coupled to a mount connector strip in accordance with an embodiment of the present disclosure;
FIG. 10K shows a perspective view of an example gripper of a clamp apparatus, wherein the example gripper includes a device connector in accordance with an embodiment of the present disclosure;
FIG. 10L is a close-up perspective view of an exemplary embodiment of a rack system, wherein the embodiment of a rack depicted in FIG. 9F includes a support pole adapted to couple with the clamp of a medical device in accordance with an embodiment of the present disclosure;
FIG. 10M is a perspective view of an exemplary embodiment of a rack system, wherein the embodiment of a rack depicted in FIG. 9J includes a support pole adapted to couple with the clamp of a medical device in accordance with an embodiment of the present disclosure; and
FIG. 10N depicts a close-up perspective view of an exemplary embodiment of a rack system, wherein the embodiment of a rack depicted in FIG. 9J includes a support pole adapted to couple with the clamp of a medical device in accordance with an embodiment of the present disclosure.
FIG. 11A depicts an embodiment of a pivotable cover mechanism in accordance with an embodiment of the present disclosure;
FIG. 11B depicts an actuation spring of an example embodiment of the pivotable-cover mechanism depicted in FIG. 11A acting on the sloped portion of an actuation member, wherein the actuation spring is disposed in an actuation spring pocket in accordance with an embodiment of the present disclosure;
FIG. 11C depicts an example embodiment of the pivotable-cover mechanism wherein a mount connector is covered by a protective member in accordance with an embodiment of the present disclosure;
FIG. 11D depicts an embodiment of a pivotable cover mechanism wherein the protective member is in a non-protective position in accordance with an embodiment of the present disclosure;
FIGS. 11E-I depict several cross-sectional views of an embodiment of a pivotable cover mechanism in accordance with an embodiment of the present disclosure;
FIG. 11J depicts a view of an example embodiment of the pivotable cover mechanism, wherein the protective member is shown in a protective and a non-protective position and wherein the mount connector is of a type having multiple spring contacts in accordance with an embodiment of the present disclosure;
FIG. 11K depicts a cross-sectional view of part of the pivotable cover mechanism, wherein the mount connector is of a type having multiple spring contacts in accordance with an embodiment of the present disclosure;
FIG. 12A depicts a view of an embodiment of a clamshell mechanism wherein the cover member is in a non-protective position in accordance with an embodiment of the present disclosure;
FIG. 12B depicts a view of an embodiment of a clamshell mechanism wherein the cover member is in a protective position in accordance with an embodiment of the present disclosure;
FIG. 12C depicts a close-up perspective view of a part of an embodiment of a clamshell mechanism in accordance with an embodiment of the present disclosure;
FIG. 12D depicts a cross sectional view of a clamshell mechanism wherein the cover member includes a perimeter rib in accordance with an embodiment of the present disclosure;
FIGS. 12E-H depict a number of cross-sectional views of a clamshell mechanism in accordance with an embodiment of the present disclosure;
FIG. 12I-J depict a number of views an exemplary embodiment of a series of the clamshell mechanisms with compliant gasket systems in accordance with an embodiment of the present disclosure;
FIG. 13A depicts an embodiment of an example receivable device in accordance with an embodiment of the present disclosure;
FIG. 13B depicts an example embodiment of a receivable device that includes first and second channels and a latch recess in accordance with an embodiment of the present disclosure;
FIG. 13C depicts a cross-section view of the example pivotable cover mechanism of FIG. 11a wherein a latch member projection is in engagement with a latch recess of an example embodiment of a receivable device in accordance with an embodiment of the present disclosure;
FIG. 13D-G depict a number of cross-section views of an example receivable device and the example clamshell mechanism of FIG. 12a wherein the progression of FIGS. 13d-g demonstrates how receiving a receivable device may cause a clamshell mechanism to automatically reveal a mechanism connector in accordance with an embodiment of the present disclosure;
FIGS. 14A-14E show several views of a clamp in accordance with an embodiment of the present disclosure;
FIGS. 15A-15D show several views of a clamp in accordance with an embodiment of the present disclosure;
FIGS. 16A-16E show several views of a clamp in accordance with an embodiment of the present disclosure;
FIGS. 17A-17E show several views of a clamp in accordance with an embodiment of the present disclosure;
FIG. 18 depicts a representational, side view of an exemplary embodiment of a support system and its sub-components;
FIG. 19 depicts a portion of an exemplary embodiment of the support system and its sub-components shown in FIG. 18;
FIG. 20A depicts an isometric view of an exemplary embodiment of the support system and its sub-components of Figs. 18-19;
FIG. 20B depicts a portion of an exemplary embodiment of the support system and its sub-components shown in FIG. 20A;
FIG. 21A depicts an exploded, top-left, perspective view of an exemplary support system, illustrating the positional relationship between its components;
FIG. 21B depicts a top-down, exploded view of an exemplary support system, illustrating the positional relationship between its sub-components as shown in FIG. 21A;
FIG. 22A depicts an exploded view of exemplary components of a support system, specifically illustrating the positional relationship between a backbone structure, a holding structure and a detachable data and power supply;
FIG. 22B depicts a front view of the exemplary components depicted in FIG. 22A;
FIG. 22C depicts a front, left, representational view of exemplary specific components of a partially assembled support system, illustrating a backbone structure and a holding structure;
FIG 23A depicts an isometric, back view of an example specific component of the support system, illustrating a backbone structure;
FIG. 23B depicts an isometric, front view of an example specific component of the support system, illustrating a backbone structure, as shown in FIG. 23A;
FIG. 24A depicts a perspective view of an exemplary embodiment of a tube-management holder of a support system;
FIG. 24B depicts a perspective view of an exemplary embodiment of a tube-management holder of a support system;
FIG. 24C depicts a perspective view of an exemplary embodiment of a tube-management holder of a support system;
FIG. 25A depicts an assembled back view of example components of the support assembly, specifically illustrating an embodiment of a pairing element on a device and the positional relationship between the device and a detachable data and power supply;
FIG. 25B depicts an assembled isometric view of example components of the support assembly, specifically illustrating an embodiment of a pairing element on a device and the positional relationship between the device and a detachable data and power supply as shown in FIG. 25A;
FIG. 26 depicts a perspective view of a partially-assembled embodiment of a support system, illustrating the positional relationship of a backbone, a holding structure, a clamp assembly and a detachable data and power supply;
FIG. 27 depicts a perspective view of a partially-assembled embodiment of a support system, illustrating the positional relationship of a backbone structure, a holding structure and a clamp assembly;
FIG. 28A depicts a perspective view of an exemplary clamp assembly in a first position, specifically illustrating the assembled sub-components of the clamp assembly;
FIG. 28B depicts a rear view of an exemplary clamp assembly in the first position, illustrating the assembled sub-components of the clamp assembly as shown in FIG. 28A;
FIG. 28C depicts a bottom, left-side perspective view of an exemplary clamp assembly in the first position, illustrating the assembled sub-components of the clamp assembly including a coupling element on a base of the clamp assembly;
FIG. 28D depicts a front view of an exemplary clamp assembly in a second position, illustrating the assembled sub-components of the clamp assembly;
FIG. 28E depicts a front, left-side perspective view of an exemplary clamp assembly in the second position, illustrating the assembled sub-components of the clamp assembly as shown in FIG. 28D;
FIG. 28F is an exploded view of an exemplary clamp assembly, illustrating the positional relationship between the sub-components of the clamp assembly;
FIG. 29A is a cross-sectional, top view of an example of a clamp assembly, illustrating an embodiment of a bias member when the clamp is in the first position;
FIG. 29B is a cross-sectional, isometric view of an exemplary embodiment of a clamp assembly, illustrating an embodiment of a bias member when the clamp is in the first position;
FIG. 29C is a cross-sectional, top view of an example of a clamp assembly, illustrating an embodiment of a bias member when the clamp is in a second position;
FIG. 29D is a cross-sectional, exploded, isometric view of an example of a clamp assembly, illustrating an embodiment of a bias member when the clamp is in a second position;
FIG. 30A is a front view of a partially assembled embodiment of a clamp assembly, illustrating the positional relationship between a frame structure, a bias member, a hinge pin and a latch;
FIG. 30B is an isometric view of a partially assembled embodiment of a clamp assembly, illustrating the positional relationship between a frame structure, a bias member, a hinge pin and a latch;
FIG. 30C is a rear view of a partially assembled embodiment of a clamp assembly, illustrating the positional relationship between a frame structure and a latch;
FIG. 30D is an exploded view of an exemplary specific component of a clamp assembly;
FIG. 31A is a front view of an exemplary frame structure of an embodiment of a clamp assembly;
FIG. 31B is a perspective view of an exemplary frame structure of an embodiment of a clamp assembly;
FIG. 31C is a perspective view of an exemplary frame structure of an embodiment of a clamp assembly;
FIG. 32A is a front view of an exemplary gear plate of an embodiment of a clamp assembly, illustrating a jaw shaped end and a gear shaped end;
FIG. 32B is a side view of the exemplary gear plate of an embodiment of a clamp assembly, illustrating a jaw shaped end and a gear shaped end;
FIG. 32C is a back view of the exemplary gear plate of an embodiment of the clamp assembly, illustrating a set of attachment points therein;
FIG. 33A is a front view of an example actuator of an embodiment of a clamp assembly, illustrating a paddle member and an inserting member therein;
FIG. 33B is a back view of an example actuator of an embodiment of a clamp assembly, illustrating a paddle member, an inserting member and a set of attaching points therein;
FIG. 34A is a perspective, back view of an example latch of an embodiment of a clamp assembly, illustrating a lever member and a flap member of the example latch;
FIG. 34B is a perspective front view of an example latch of an embodiment of a clamp assembly, illustrating a lever member and a flap member of the example latch as shown in FIG 34A;
FIG. 35A is a top, back, right-side, perspective view of an example of a holding structure of an embodiment of a support assembly; and
FIG. 35B is a top, front, left-side, perspective view of an example of a holding structure of an embodiment of a support assembly.

### DETAILED DESCRIPTION

### CLAMP MECHANISMS

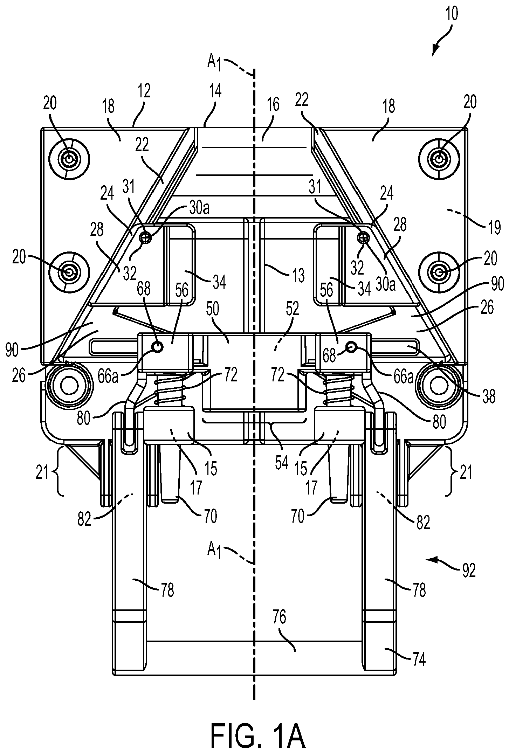

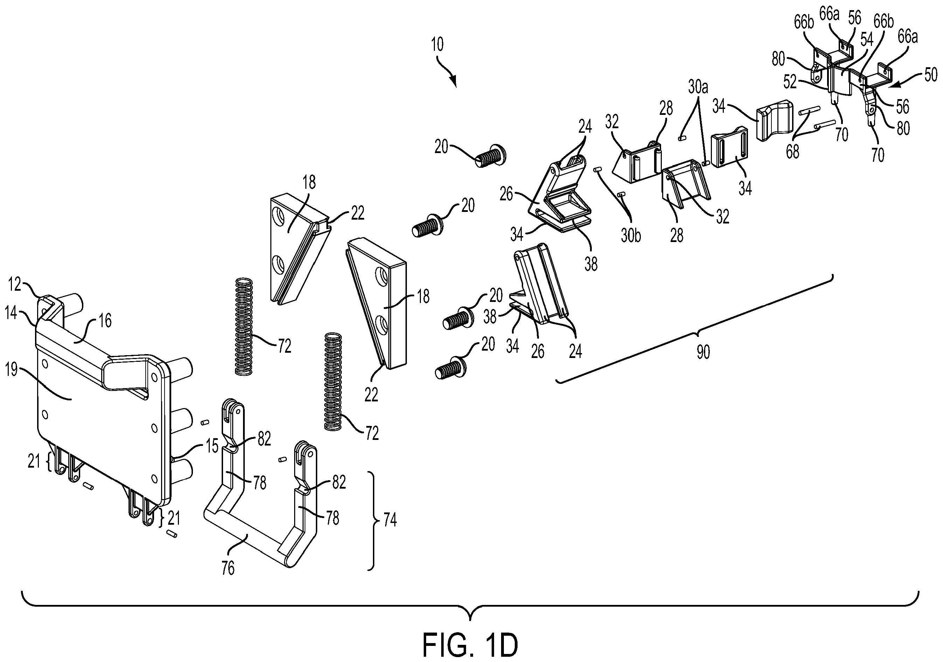

In one example embodiment, as shown in **FIGS. 1A-1E****,** a clamp apparatus **10** is depicted. The clamp apparatus **10** comprises a housing **12.** In the shown embodiment, the housing **12** has a back plate **14,** which is generally planar. On one portion of the back plate **14** is a raised grip **16** extending away from the housing **12.** The grip **16** affords the user ease of movement along a clamped object **100** generally extending along an axis **A1.** The grip **16** is also meant to aid in carrying. The grip **16** may be made of the same material as the rest of the housing **12,** may be made of a different material, or may be made of a combination thereof. Possible materials may include, but are not limited to, rubber, polymer, composite, metal, plastic, foam, etc. Additionally, the grip **16** may comprise ergonomic finger groves, nubs, a ribbed texture, a honeycombed texture, etc.

The rear of the back plate **14** may also feature any of a variety of mechanisms **19** (not shown) to attach a load to the clamp apparatus **10.** Such mechanisms **19** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

On the front portion of the back plate **14,** a groove **13** runs vertically down the centerline (shown as a line of short and long dashes) of the back plate **14.** The groove **13** is further described below. Two compression spring pockets **15** are coupled to the back plate 14 and are raised off the bottom of the front face of the back plate **14.** The compression spring pockets **15** may be generally cylindrical and hollow much like a cup. The compression spring pockets **15** will be elaborated upon later.

Extending off the bottom edge of the back plate **14** toward the bottom of the page are two twin catch structures **21** which are symmetrical around the centerline of the back plate **14.** The catch structures **21** are formed such that a first portion of the structure **21** is a member which extends toward the bottom of page in a manner substantially perpendicular to the bottom edge of the back plate **14.** A second portion of the structure **21** is a member extending toward the bottom of the page in the same manner as the first portion. The first and second portions are offset from each other so as to allow a crosspiece to form a bridge between the first and second portion of the catch structure **21.** The crosspiece of the catch structure **21** runs in a direction substantially parallel to the bottom edge of the back plate **14.** The catch structure **21** will be further elaborated upon later.

In the example embodiment, two blocks **18** are fixedly coupled to the front of the back plate **14** by any variety of means. This could include, but is not to be limited to, screws **20** (as shown), bolts, welds, etc. The back plate **14** and blocks **18** can also be formed as a continuous part during manufacture. The blocks **18** are offset by some distance from the back plate **14.**

The blocks **18** are generally right triangles with their hypotenuses facing **A1.** It should be appreciated, however, that the blocks **18** could take any shape so long as the interior face of the blocks **18** extends in a suitable direction. The blocks **18** also display symmetry around **A1.**

Along the inward facing sides of the blocks **18** there may be tracks **22.** The tracks **22** may engage corresponding protrusions **24** on a surface of a sliding wedge **26.** These components interact in such a way that the sliding wedges **26** are able to traverse the span of the tracks **22.** In the example embodiment, the sliding wedges **26** are approximately "L" shaped, but this should not be construed as limiting the sliding wedges **26** to only an "L" shape. It should also be noted that in place of the protrusions **24** on the sliding wedge **26,** any other type of suitable engagement surfaces, such as ball bearings or rollers, could be employed. In other embodiments, the track **22** may be raised off the blocks **18.** In such embodiments, the protrusions **24** would be replaced by another suitable engagement surface such as a recessed groove, rollers, ball bearings, etc. In yet some additional embodiments, a track **22** comprises the rack portion of a rack and pinion, be the track 22 in a raised or recessed configuration; in place of the protrusions **24,** on the sliding wedge **26,** one or more pinion gears would extend so as to engage the rack track **22,** in this specific embodiment.

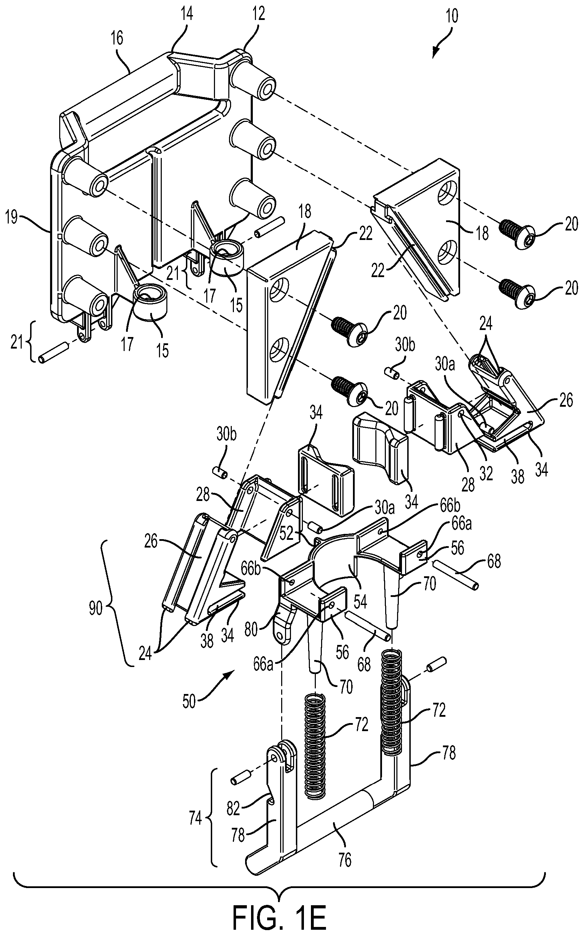

At the top of both the sliding wedges **26,** a pawl **28** may be pivotally coupled. In the embodiment shown in **FIGS. 1A-1E** this is accomplished by means of a pair of pins **30** (though a single pin, hinge, or other suitable arrangement could also be used) running through openings **32** which extend through both the sliding wedge **26** and the pawl **28.** One pin **30a** pivotally couples the pawl to the sliding wedge **26** through the front surfaces of the sliding wedge **26** and the pawl **28.** Likewise, the second **30b** of the pair of pins **30** (best shown in **FIG. 1E**) pivotally couples the pawl **28** to the sliding wedge **26** through the rear surfaces of the pawl **28** and the sliding wedge **26.** Bushings **31** may also be present in some embodiments to provide a bearing surface.

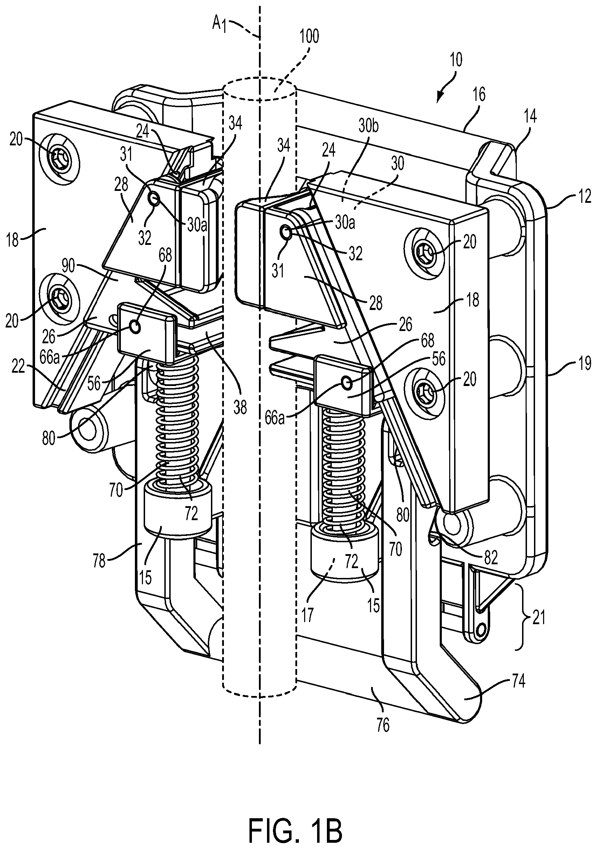

On at least a portion of the pawls **28** there may be a gripping surface **34** which engages the clamped object **100.** This gripping surface **34** consists of a material chosen for its gripping ability. The gripping surface **34** may be made of a high friction material, a compressible material, a material exhibiting both these qualities, or any other suitable material. The gripping surface **34** is made of a material which allows a firm grip without the deformation of a clamped object **100.** Additionally, the gripping surface **34** may be contoured (as is easily seen in **FIG. 1B**).

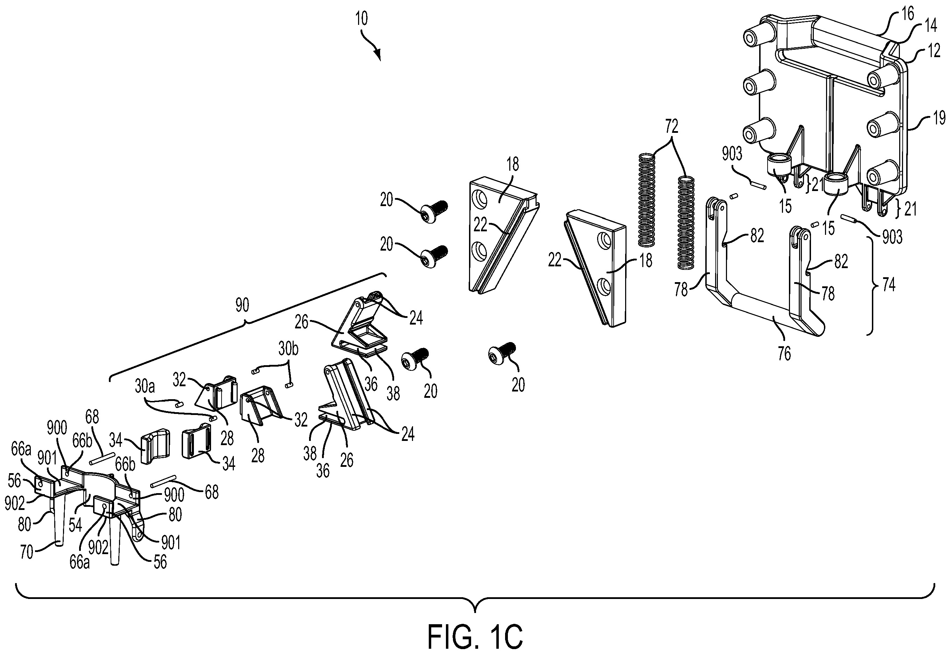

Best shown in the clamp apparatus **10** exploded views in **FIGS. 1C-1E****,** the bottom of the sliding wedge **26** may feature a flange **36.** The flange **36** extends inward, at an angle substantially perpendicular to the axial direction **A1,** from the portion of the sliding wedge **26** which engages the tracks **22.** A slot **38** is cut into the flange **36** and will be elaborated upon later.

Together, the sliding wedge **26,** the pawl **28,** and the gripping surface **34** comprise a sliding wedge-pawl assembly **90.** The sliding wedge-pawl assemblies **90** are capable of movement, together as a unit, up and down the track **22.** This allows the clamp apparatus **10** to adjust to and grip clamped objects **100** of a variety of different girths such that the distance between the gripping surfaces **34** of the sliding wedge-pawl assemblies **90** mimics the diameter of a clamped object **100.**

The clamp apparatus **10,** in this exemplary embodiment, also comprises a second assembly, the spring handle assembly **92.** At the top of the spring handle assembly **92** is a guided lift bar **50.** The rear portion of the guided lift bar **50** has a vertical ridge **52** which engages with the vertical groove **13** in the back plate **14.** This constricts the guided lift bar **50** to movement up and down in the axial direction **A1.**

In the embodiment shown in **FIGS. 1A-1E****,** the center span **54** of the guided lift bar **50** arcs/curves or bends toward the back plate **14.** This allows the guided lift bar **50** to better accommodate the clamped object **100.**

On each the right and left side of the center span **54,** a member **56** may be attached which fits around the flange **36** of the sliding wedge **26.** The member **56** is formed such that a first portion **900** of the member **56** extends off the center span **54** on a plane substantially parallel to the back plate **14.** Extending off the bottom of first portion **900** at an angle substantially perpendicular to the first portion is a second portion **901** of the member **56.** This second portion **901** is formed such that the edge of the second portion **901** distal to **A1** is straight and occupies the same vertical plane extended off the distal edge of the first portion **900.** The edge of the second portion **901** of the member **56** proximal to **A1** tapers toward the distal edge of the second portion **901.** This taper again helps to accommodate the clamped object **100.** The member **56** has a third portion **902** which is attached to the second portion **901** such that the bottom of the third portion **902** is coupled to the front edge of the second portion **901** at an angle that is substantially perpendicular. The third portion **902** extends on a plane parallel to the first portion **900.** The edge of the third portion **902** distal to A1 is straight and occupies the same vertical plane extended off the distal edge of the first portion **900.** The proximal edge of the third portion **902** is flush with the proximal, tapered edge of the second portion **901** and extends upwards from it in a substantially perpendicular manner.

In the example embodiment in **FIGS. 1A-1E****,** the third portion **902** of the member **56** described above has a hole **66a** creating a passage through the third portion **902.** Likewise, the first portion 900 also has a hole **66b** creating a passage through the first portion 900. The centers of both holes 66a, 66b extend along a common axis which is substantially perpendicular to the front face of each the first and third portions 900, 902 of the member **56.** The locations of the holes **66a** and **66b** are selected such that they are in line with the slots **38** in the sliding wedges **26** when the clamp apparatus **10** is assembled. Placing the holes **66a** and **66b** at this location allows the insertion of dowels **68** through each of the holes **66a** and **66b** and their corresponding slots **38,** thus coupling the sliding wedge-pawl assemblies **90** to the spring handle assembly **92.** Though the example embodiments employ the use of a dowel **68** to couple the two assemblies together, other means of coupling the assemblies, such as but not limited to, a bar, rollers, ball bearings, etc. could be implemented.

In the example embodiment, when both assemblies **90** and **92** are coupled together, the guided lift bar **50** functions as a crossbar which ensures that the right and left sliding wedge-pawl assemblies **90** move together in unison along the tracks **22.** This coupling also allows the spring handle assembly **92** to control whether the clamp apparatus **10** is in the open or closed position.

Coupled to the bottom of the second portion 901 of the members **56** a generally cylindrical shape **70** may be extended downward (in additional embodiments, other shapes may be used). As shown in the example embodiments in **FIGS. 1A-1E****,** the generally cylindrical shape **70** may taper slightly in diameter as it extends farther away from the bottom of the second portion 901 of the member **56** toward the bottom of the page. The generally cylindrical shape 70 may be solid or hollow. A coil spring **72** surrounds the generally cylindrical shape **70.** One end of the coil spring **72** abuts the bottom of the second portion 901 of the member **56** from which the generally cylindrical shape **70** extends. The other end of the coil spring **72** seats in the compression spring pocket **15** on the back plate **14** mentioned above. The bottom of the compression spring pocket **15** has a hole **17** through which the generally cylindrical shape **70** may pass as the clamp apparatus **10** is moved to/in the open position. Though the shown embodiments use a coil spring **72,** other embodiments could conceivably employ any other suitable bias member. A wide variety of suitable bias members may be employed. Examples of suitable bias members include, but are not limited to, a gas spring using a bladder, a piston type arrangement, a compression spring made of a compressible, springy material such as rubber, an extension spring, a constant force spring, etc.

In the example embodiment, the coil springs **72** bias the clamp apparatus **10** toward the closed position (as shown in **FIG. 1B**). That is, the coil springs 72 bias the wedges 26 to slide up the tracks 22 such that the pawls 28 approach each other towards the clamped object 100 (e.g., a pole). In the closed position, the sliding wedge-pawl assemblies **90** are sufficiently at the top of the tracks **22** to clamp the pawls 28 onto the clamped object 100 (via attached gripping surfaces 34). The guided lift bar **50** is also at a higher position in the vertical groove **13** in the back plate **14.** Also in this position, the coupling dowel **68,** in relation to **A1,** is located in a more distal end of the slot **38** in the flange **36** of the sliding wedge **26.**

If a clamped object **100** is present in the example embodiment, the coil springs **72** bias the clamping apparatus **10** to clamp down on the object **100.** Depending on the size of the clamped object **100,** the sliding wedge-pawl assemblies' **90** location on the track **22** will vary so that the distance between the sliding wedge-pawl assemblies **90** will mimic the diameter of the clamped object **100.** The larger the clamped object **100** the lower the sliding wedge-pawl assemblies **90** will be on the track **22.** Similarly and consequentially, the location of the guided lift bar **50** along the groove **13** will be lower with larger clamped objects **100.**

The clamping apparatus **10** in the example embodiment is designed in such a way as to utilize the force of gravity to increase the clamping force. As gravity pulls on the clamp, especially when a load is attached to the back plate **14,** a force is exerted on the sliding wedge-pawl assemblies **90.** This force causes the sliding wedge-pawl assemblies **90** to want to ride further up the tracks **22.** Since the clamped object **100** is in the way, the sliding wedge-pawl assemblies **90** cinch up on and exert more clamping force on the clamped object **100.** Additionally, because the pawls **28** are pivotally coupled to the sliding wedge **26,** the pull of gravity causes the point of contact on the pawls **28** to want to swing up and into the clamped object **100.** Since the clamped object **100** is in the way, the pawls **28** cinch up on and exert more clamping force on the clamped object **100.**

In order to move the clamping apparatus **10** to the open position, a pull handle **74** may be pulled down. In the example embodiment, the pull handle **74** comprises a grip **76** and one or more posts **78** extending from the grip **76.** The grip **76** may be made of the same material as the rest of the pull handle **74,** may be made of a different material, or may be made of a combination thereof. Possible materials may include, but are not limited to, rubber, polymer, composite, metal, plastic, foam, etc. Additionally, the handle **74** may comprise ergonomic finger groves, nubs, a ribbed texture, a honeycombed texture, etc.

The one or more posts **78** of the pull handle **74** extend up to a corresponding number of arms **80** on the guided lift bar **50.** The posts **78** are coupled to the arms **80** on the guided lift bar **50** through any of a variety of means. In the example embodiment, coupling is accomplished by means of a pin which runs through both the arm **80** and post **78.** In other embodiments, this coupling may be accomplished in any number of suitable ways including, but not limited to, welds, bolts, screws, etc. The pull handle **74** and guided lift bar **50** could also be made as a single continuous part during manufacture. In some embodiments, the posts **78** extend straight down to the grip **76.** In other embodiments, the posts **78** may be arcuated or have a bend out toward the rear of the page to allow greater ease in grasping the grip **76.** Additionally, in some embodiments, including the example embodiment, the posts **78** have a notch **82** which runs across the back of the posts **78** in a direction substantially parallel to the bottom edge of the back plate **14.**

As aforementioned, to move the clamping apparatus **10** from the closed position to the open position, a pull handle **74** may need to be pulled down. In the example embodiment, as the pull handle **74** is pulled down, the guided lift bar **50** is also pulled down the groove **13** in the back plate **14.** This causes the compression springs **72** to become compressed and causes the generally cylindrical shape **70** to extend through the hole **17** in the compression spring pockets **15.** Pulling down the pull handle **74** also causes the sliding wedge-pawl assemblies **90** to slide down the tracks **22.** Due to the slope of the tracks **22,** moving the clamping apparatus **10** to the open position also causes the location of the coupling dowel **68** within the slot **38** to change. When the clamp is in the fully open position, the coupling dowel **68** is at the most proximal end of the slot **38** in relation to **A1.**

In the example embodiment, to hold the clamping apparatus **10** in the fully open position against the restoring force of the compression springs **72,** the notch **82** in the pull handle **74** may be engaged with the catch structure **21** extending off the back plate **14.** When the clamping apparatus **10** is locked in the open position, the crosspiece 903 of the catch structure **21** is caught by the notch **82** of the pull handle **74** thereby disallowing the compression springs **72** to return the clamping apparatus **10** to the closed position. Other embodiments may employ other types of catch mechanisms in addition to the elbow type catch in the example embodiment. Other suitable catches may include, but are not limited to, a magnetic catch, a ball catch, a latch, a roller catch, etc.

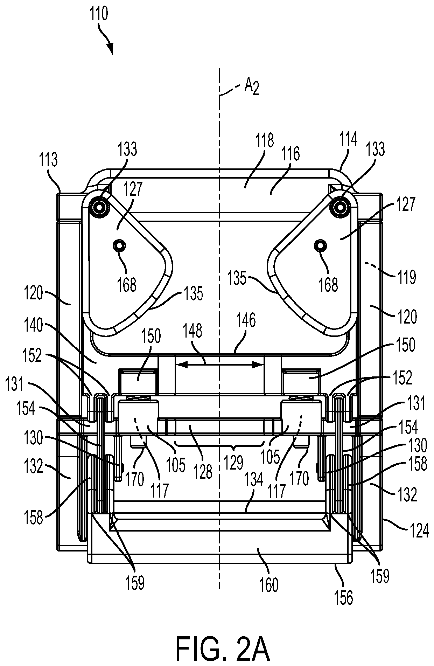

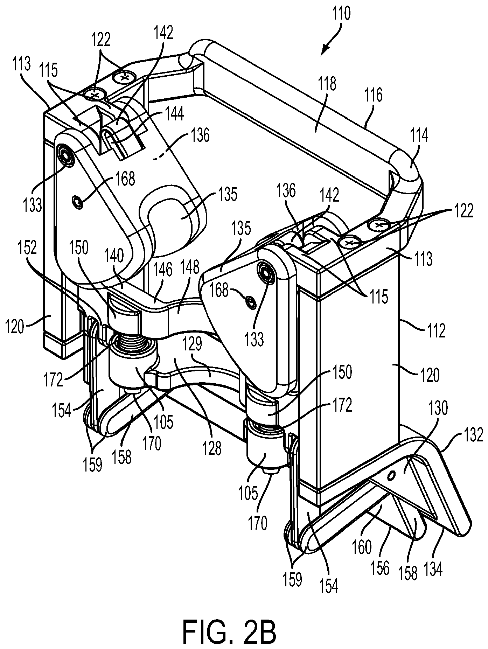

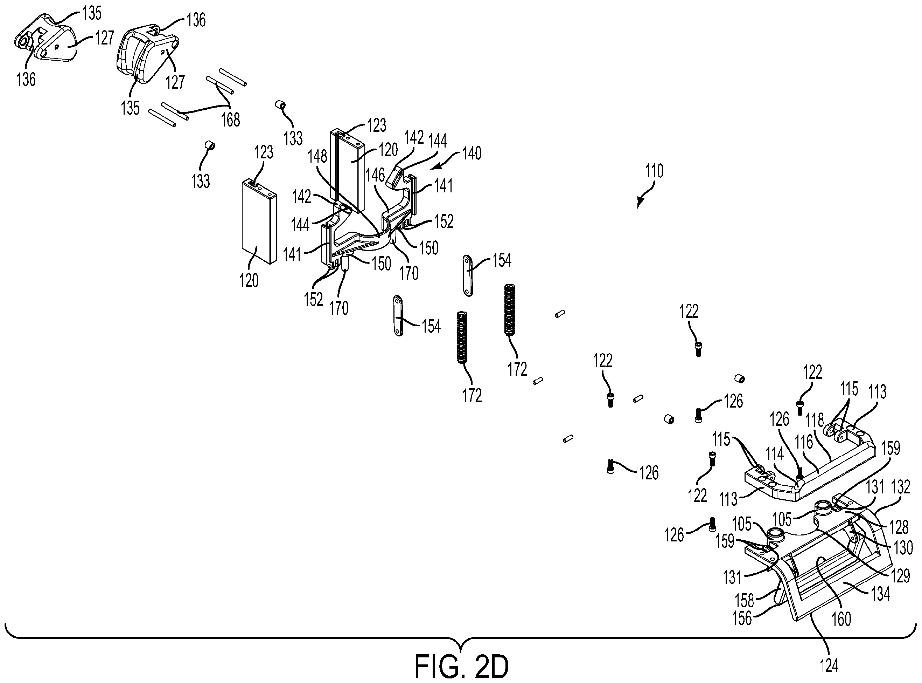

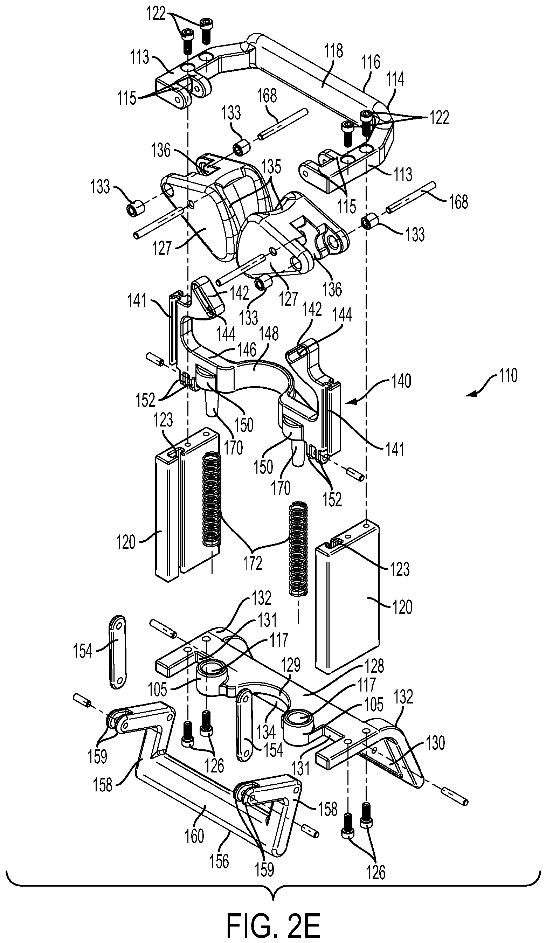

In another embodiment, as shown in **FIGS. 2A-2E****,** a clamp apparatus **110** is depicted. The clamp apparatus **110** comprises a housing **112.** The housing **112** resembles a frame. The housing **112** comprises an upper handle **114** at the top of the housing **112.** In the example embodiment, the upper handle **114** is essentially "U" shaped with the bottom, grip portion **116** of the "U" extended toward the back of the page (directions given in relation to the embodiment depicted in **FIG. 2A**). In other embodiments, the upper handle **114** need not take the shape of a "U", but rather any other desirable form. The grip portion **116** of the upper handle **114** may be cylindrical, planar, or take any other desired form. The grip portion **116** of the upper handle **114** may also have gentle ergonomic finger grooving, nubs, a ribbed texture, a honeycombed texture, etc. **118** (not shown) to increase ease of use. The grip portion **116** may be made of the same material as the rest of the upper handle **114,** may be made of a different material, or may be made of a combination thereof. Possible materials may include, but are not limited to, rubber, polymer, composite, metal, plastic, foam, etc.

In the example embodiment, the uprights **113** of the "U" extend from the grip portion **116** toward the front of the page. The uprights **113** of the "U" each comprise a set of brackets **115** which extend substantially perpendicularly from the faces of the uprights **113** most proximal to **A2** toward **A2.**

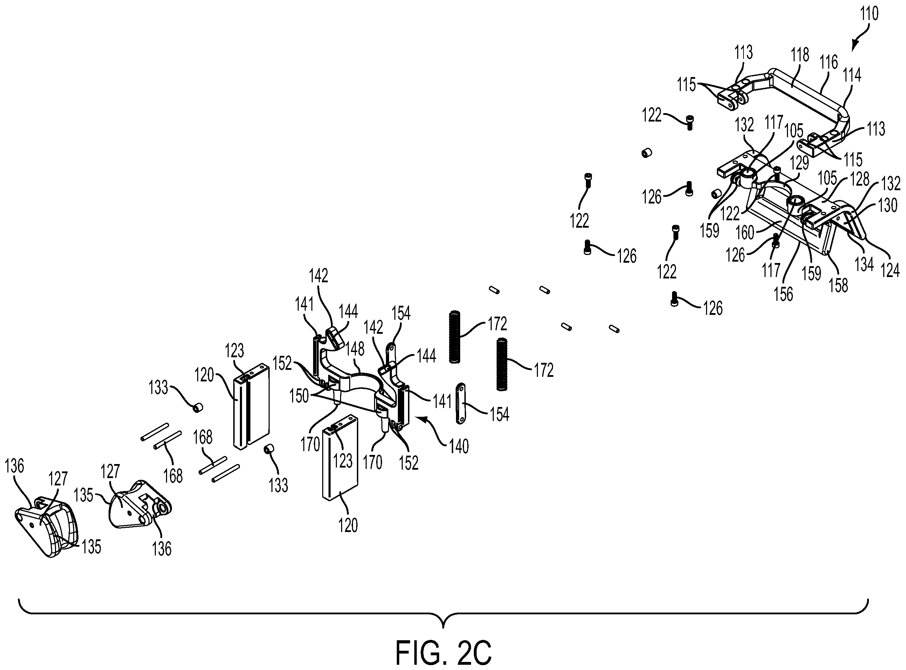

The housing **112** in the example embodiment also comprises one or more members **120** extending from the upper handle **114.** In the embodiment shown in **FIGS. 2A-2E****,** two substantially planar members **120** extend down in parallel fashion from the upper handle **114** at an angle that is generally perpendicular to the bottom surface of the upper handle **114.** The members **120** may be coupled to the upper handle **114** with screws **122**(as shown best in **FIGS. 2C-2E**), bolts, welds, or by any other manner. The upper handle **114** and one or more vertical members **120** may also be formed as a single part during manufacture. The members **120** may also comprise tracks **123** on the faces of the members **120** most proximal to **A2.** In the example embodiment, the tracks **123** run vertically up the face of each member **120** though this need not be true of every embodiment. Additionally, in the example embodiment, the tracks **123** are cut into the members **120.** In other embodiments, the tracks may be raised off the members **120.**

The housing **112** may also comprise a lower handle **124.** In the example embodiment, the lower handle **124** is coupled to the bottom edges of the members **120.** The lower handle **124** may be coupled to the members **120** in any of a variety of ways including screws **126,** bolts, welds, etc (as best shown in **FIGS. 2C-2E**). The lower handle **124** may also be formed with the members **120** as a single continuous part during manufacture. In other embodiments, the upper handle **116,** members **120,** and lower handle **124** are all formed as a continuous part in manufacture. Spanning the distance between the members **120,** the lower handle **124** may comprise a crosspiece **128.** The center span **129** of the crosspiece **128** may arc/curve or bend toward the back of the page to better accommodate a clamped object **100.** The crosspiece **128** also may comprise a pair of compression spring pockets **105.** The compression spring pockets **105** are generally cylindrical and are hollow much like a cup. In the example embodiment, the bottom of the compression spring pockets **105** have an opening **117.** A pair of brackets **130** extend off the bottom of the crosspiece **128** and will be elaborated upon later. The crosspiece **128** may have recessed portions **131** spanning the distance between the distal sides of the compression spring pockets **105** (in relation to **A2**) and the arms **132** of the lower handle **124** (elaborated upon in the following paragraph).

The lower handle **124** extends toward the back of the page in a manner similar to the upper handle **114.** The arms **132** of the lower handle **124** may be arcuated or have a bend which arcs/bends the lower handle **124** toward the bottom of the page. The arms **132** of the lower handle **124** are joined by a grip **134** at the part of the handle closest to the bottom of the page.

The grip **134** may be made of the same material as the rest of the lower handle **124,** may be made of a different material, or may be made of a combination thereof. Possible materials may include, but are not limited to, rubber, polymer, composite, metal, plastic, foam, etc. Additionally, the grip **134** may comprise ergonomic finger groves, nubs, a ribbed texture, a honeycombed texture, etc.

The housing **112** may also feature any of a variety of mechanisms **119** (not shown) to attach a load to the clamp apparatus **110.** Such mechanisms **119** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

The clamping apparatus **110** may further comprise a set of pawls **127** which are pivotally coupled to the brackets **115** of the upper handle **116.** The set of pawls **127** may be coupled to the brackets **115** of the upper handle by any of a variety of ways. Additionally, bushings **133** may be present to provide a bearing surface. The pawls **127** may have a trough **136** cut into them essentially along the center plane of the pawls **127** running parallel to the plane of the grip **116** shown in the example embodiment. The trough **136** will be elaborated upon later.

On at least a portion of the pawls **127** there may be a gripping surface **135** which engages the clamped object **100.** The gripping surface **135** may consist of a material chosen for its gripping ability. The gripping surface **135** could be made of a high friction material, a compressible material, a material exhibiting both those qualities, or any other suitable material. The gripping surface **135** is made of a material which allows a firm grip without the deformation of a clamped object **100.** Additionally, the gripping surface **135** may be contoured. Though the example embodiment includes a single set of pawls **127,** in other embodiments, further sets of pawls **127** may be added to the clamping apparatus **110** to afford the clamping apparatus **110** added stability.

In the example embodiment, the clamping apparatus **110** also comprises a lift bar guide **140.** The lift bar guide **140** comprises a set of protrusions **141** which engage with the tracks **123** in the members **120.** This enables the lift bar guide **140** to travel along the track **123** in the members **120.** In place of protrusions **141** some alternate embodiments employ a variety of different engagement surfaces. These surfaces include, but are not limited to, rollers, ball bearings, etc. In other embodiments, the track **123** may be raised off the members **120.** In such embodiments, the protrusions **141** would be replaced by another suitable engagement surface such as a recessed groove, rollers, ball bearings, etc. It would also be conceivable for some embodiments to use a track **123,** be it raised or recessed, comprising the rack portion of a rack and pinion. In place of the protrusions **141,** on the lift bar guide **140,** one or more pinion gears would extend so as to engage the rack track **123.**

The top portion of the lift bar guide **140** may comprise a set of wings **142** which project inward toward **A2.** The wings **142** are shaped such that they are able to fit within the trough **136** in the pawls **127.** The wings **142** have a slit **144** cut into them (best shown in **FIGS. 2C-2E**) similar to the slot **38** depicted in **FIGS. 1A-1E****.** A coupling dowel **168** couples the pawls **127** to the lift bar guide **140** through the slit **144** in the wings **142.** The lift bar guide **140** has a crossbar **146.** This enables the lift bar guide **140** to cause the pawls **127** to move in unison. The center span **148** of the crossbar **146** may be arced/bent toward the back of the page to better accommodate a clamped object **100.**

On each side of the arced center span **148,** recessed compression spring pockets **150** are recessed into bottom face the lift bar guide **140.** From the centers of the recessed compression spring pockets **150** a generally cylindrical shape **170** extends (though the shape need not be cylindrical in all embodiments) toward the bottom of the page. The generally cylindrical shape **170** may be solid or hollow. The generally cylindrical shape **170** may taper slightly in diameter as it extends farther away from the bottom face of the lift bar guide **140.** The diameter of the generally cylindrical shape **170** is such it occupies much of the center of the recessed compression spring pocket **150,** but leaves a ring surrounding the base of the generally cylindrical shape **170.** One end of a coil spring **172** is seated in the ring surrounding the generally cylindrical shape **170** in the recessed compression spring pocket **150.** The other end of the coil spring **172** abuts the bottom of the compression spring pocket **105** on the lower handle **124** mentioned above. The bottom of the compression spring pocket **105** has a hole **117** through which the generally cylindrical shape **170** may pass as the clamp apparatus **110** is moved to/in the open position. Though the shown embodiments use a coil spring **172,** other embodiments could conceivably employ any other suitable bias member configuration. A wide variety of suitable bias members could be employed. Examples of suitable bias members include, but are not limited to, a gas spring using a bladder, piston type arrangement, a compression spring made of a compressible, springy material such as rubber, an extension spring, constant force spring, spring steel, etc.

In the shown embodiment, more distal from **A2** than the recessed compression spring pockets **150,** a set of brackets **152** extends downward on each side of the bottom face of the lift bar guide **140.** In some embodiments, the placement of the recessed compression spring pockets **150** or other suitable bias structure and the brackets **152** may be switched. Coupled to the brackets **152** on the lift bar guide **140** there may be a link structure **154.** In the example embodiments, the link structure **154** is a generally oblong disc with rounded edges. In other embodiments, the link structure **154** may take other forms and shapes. Examples of link structures **154** in other possible embodiments may include, but are not limited to, prismatic joints, any of a variety or springs, etc. It would also be conceivable to forgo the brackets **152** while coupling a camming surface to the actuator lever handle **156** (introduced in the following paragraph) thus effectively making the lift bar guide **140** a cam follower.

In the example embodiment, the other end of the link structure **154** is coupled to an actuator lever handle **156.** The actuator lever handle **156** has a set of members **158.** One end of the members **158** may be fitted with brackets **159** which allows the members **158** to couple to the link structure **154** as is shown in the example embodiment. From their coupling point to the link structure **154,** the members **158** may extend to and are coupled to the brackets **130** projecting off the bottom face of the crosspiece **128** of the lower handle **124.** In some embodiments, a torsion spring may be employed where the members **158** of the actuator lever handle **156** couple to the crosspiece **128** brackets **130.** The torsion spring may be a substitute for, or used in conjunction with the coil spring **172** or other suitable bias structure. From their coupling point on the crosspiece **128** brackets **130,** the members **158** arc/curve or bend steeply downward. In the example embodiments the members **158** bend at nearly a right angle, though other suitable angles may be used. A gripping portion **160** spans the distance between lowest ends of the members **158.**

The gripping portion **160** may be made of the same material as the rest of the actuator lever handle **156,** may be made of a different material, or may be made of a combination thereof. Possible materials may include, but are not limited to, rubber, polymer, composite, metal, plastic, foam, etc. Additionally, the gripping portion **160** may comprise ergonomic finger grooves, nubs, a ribbed texture, a honeycombed texture, etc.

In the example embodiment, the coil springs **172** bias the clamping apparatus **110** toward the closed position. In the closed position the lift bar guide **140** is at its highest point of travel along the tracks **123** in the members **120.** The pawls **127** are rotated up and inward toward **A2.** Also in the closed position, the coupling dowel **168** is at the bottom of the slit **144** in the wings **142** of the lift bar guide **140.**

If a clamped object **100** is present in the example embodiment, the coil springs **172** bias the clamp apparatus **110** to clamp down on the object **100.** Depending on the size of the clamped object **100,** the lift bar guide's **140** location on the track **123** will vary. The larger the clamped object **100** the lower the lift bar guide **140** will be on the track **123.** Additionally, the pawls **127** will not be fully rotated up and inward toward **A2.** Instead the distance between the gripping surfaces **135** of the pawls **127** will mimic the diameter of the clamped object **100.** This also means that the location of the coupling dowel **168** will be somewhat closer to the top of the slit **144.**

The clamp apparatus **110** in the example embodiment is designed in such a way as to utilize the force of gravity to increase the clamping force. As gravity pulls on the clamp apparatus **110,** especially when a load is attached to the housing **112** the force causes the pawls **127** to want to rotate further in towards **A2.** Since the clamped object **100** is in the way, the pressure of the pawls **127** against the clamped object **100** increases and the clamping apparatus **110** grips the clamped object **100** more vigorously.

To open the clamp apparatus **110** in the example embodiment, a user's hand may reach around the lower handle **124** and grasp the actuator lever handle **156** with their fingers. The user may then pull the actuator lever handle **156** toward the lower handle **124** of the housing **112.** This causes the actuator lever handle **156** to pivot about its coupling to the brackets **130** on the cross piece **128** of the lower handle **124.** This in turn pulls down on the link structure **154** which couples the actuator lever handle **156** to the lift bar guide **140.** As the link structure **154** is pulled downward, the lift bar guide **140** travels down the tracks **123** in the members **120** of the housing **112.** As the lift bar guide **140** travels downward, the compression springs **172** are compressed and the generally cylindrical shape **170** extends through the hole **117** in the compression spring pockets **105** on the crosspiece **128** of the lower handle **124.** The downward travel of the lift bar guide **140** also causes the pawls **127** to rotate downward and away from **A2.** This is caused by the slit **144** in the wings **142** of the lift bar guide **140** sliding over the coupling dowel **168** until the coupling dowel **168** reaches the top of the slit **144.** When the coupling dowel **168** is in this position, the pawls **127** are fully open. The clamp apparatus **110** may then be placed on a clamped object **100.** Once the actuator lever handle **156** is released, the compression springs **172** will bias the clamp apparatus **110** to close and clamp down on the clamped object **100.**

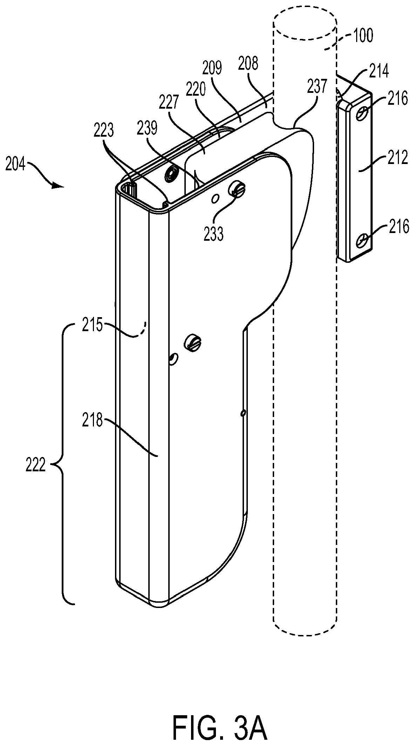

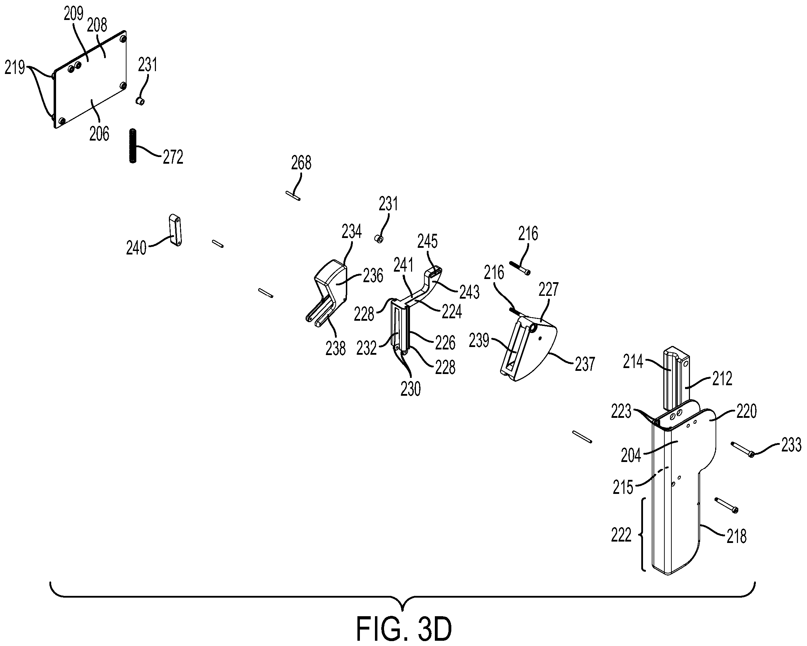

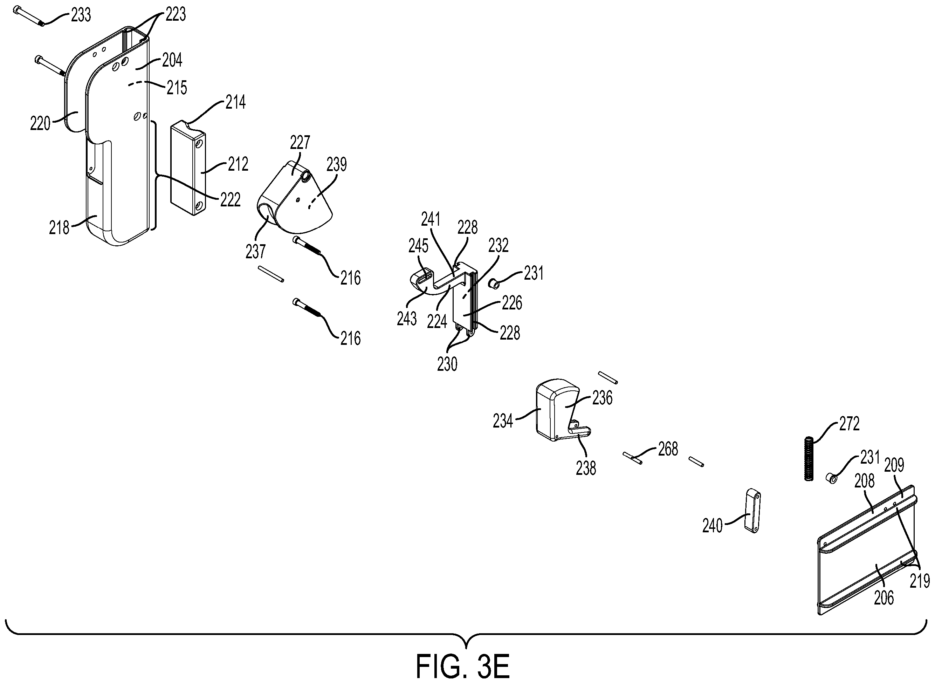

In another embodiment shown in **FIGS. 3A-3E****,** a clamp apparatus **202** is depicted. The clamp apparatus **202** comprises a housing **204.** The housing **204** comprises a number of portions. The first portion of the housing **204** may include a back plate **206.** The back plate **206** may be substantially planar as shown in **FIGS. 3A-3E****.**

The back plate **206** may also include a gripping handle **208** (not shown). The gripping portion **209** of the gripping handle **208** may be made of the same material as the rest of the handle **208,** may be made of a different material, or may be made of a combination thereof. Possible materials may include, but are not limited to, rubber, polymer, composite, metal, plastic, foam, etc. Additionally, the gripping portion **209** of the gripping handle **208** may comprise ergonomic finger groves, nubs, a ribbed texture, a honeycombed texture, etc.

Additionally, the back plate **206** may also feature any of a variety of mechanisms or mounts **219** which allow the user to attach a load to the clamp apparatus **202.** Such mechanisms **219** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

In the example embodiment shown in **FIGS. 3A-3E****,** on the right side of the front face of the back plate **206** a rectangular block **212** projects at an angle substantially perpendicular to the front face of the back plate **206.** The rectangular block **212** need not be rectangular in all embodiments. The rectangular block **212** is coupled to the back plate **206** in any of a variety of ways. The example embodiment employs screws **216,** but bolts, welds or any other suitable means could also be utilized. The back plate **206** and rectangular block **212** could also be formed as a continuous part during manufacture. The rectangular block **212** may be generally planar. The rectangular block **212** may also be arced/curved to better accommodate a clamped object **100.**

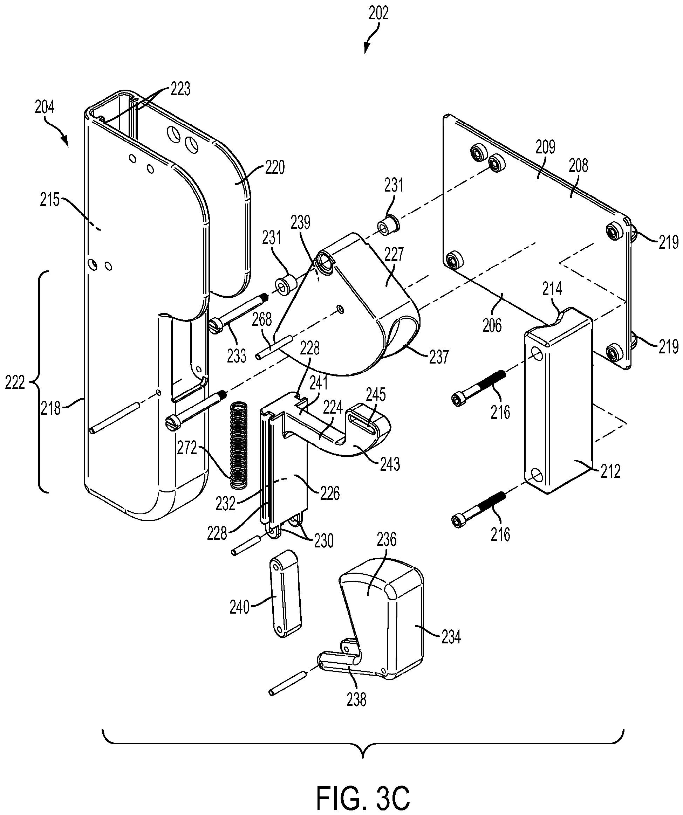

On at least a part of the inward facing side of the rectangular block **212,** a gripping surface **214** may be affixed. The gripping surface **214** can engage the clamped object **100.** This gripping surface **214** consists of a material chosen for its gripping ability. The gripping surface **214** could be made of a high friction material, a compressible material, a material exhibiting both of these qualities, or any other suitable material. The gripping surface **214** is made of a material which allows a firm grip without the deformation of a clamped object **100** Additionally, the gripping surface **214** may be contoured (as shown in **FIGS. 3C-3E**). In order to accommodate the contoured gripping surface **214** the inward face of the rectangular block **212** may also be contoured. Though the example embodiments only have one fixed gripping surface **214,** it would be conceivable to add additional fixed gripping surfaces to the clamping apparatus **202.**

The housing **204** may also comprise a second portion. The second portion of the housing may include a handle sleeve **218.** In the example embodiment, the handle sleeve **218** comprises a body which may be entirely hollow (as shown) or have one or more hollow cavities. In the example embodiment shown in **FIGS. 3A-3E****,** the top and a portion of the right side of the handle sleeve **218** are open to a hollow cavity. In alternate embodiments this need not always be the case. At the top of the handle sleeve **218** two rounded ears **220** project off the front and rear faces of the handle sleeve **218** toward the right of the page.

A portion of the handle sleeve **218** may have grip portion **222** to allow for greater ease of use. The gripping portion **222** may be made of the same material as the rest of the housing **204,** may be made of a different material, or may be made of a combination thereof. Possible materials may include, but are not limited to, rubber, polymer, composite, metal, plastic, foam, etc. Additionally, the gripping portion **222** may comprise ergonomic finger groves, nubs, a ribbed texture, a honeycombed texture, etc.

In the example embodiment, on at least one or both the interior of the front or/and rear faces of the handle sleeve **218** near the left face of the handle sleeve **218** are tracks **223** which extend at least some portion of the length of the handle sleeve **218.** In the embodiment in **FIGS. 3A-3E** the tracks **223** are raised and run vertical. Other embodiments may differ. For example, it would be conceivable to have a track **223** recessed into the sleeve handle. The track(s) **223** may also be cut into or raised out of the interior of the left face of the handle sleeve **218.** In some embodiments, the track **223** may be the rack of a rack and pinion arrangement.

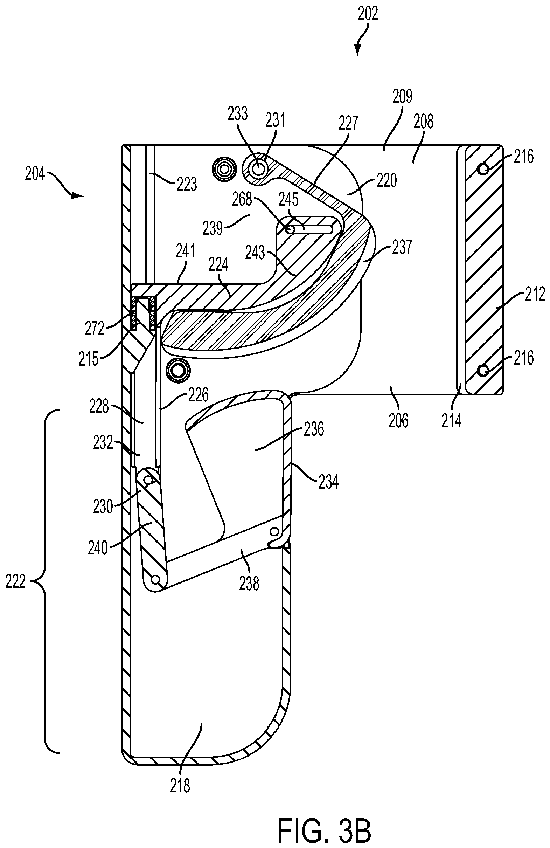

On the left face of the interior cavity, one or more compression spring pocket(s) **215** may be extended out into a hollow cavity as best shown in **FIG. 3B****.** The compression spring pocket(s) **215** may also be extended out from at least one or both the interior of the front or/and rear faces of the handle sleeve **218.** The compression spring pocket (s) **215** will be elaborated upon later.

At the top of the handle sleeve **218** a pawl **227** may be pivotally coupled. The pawl **227** may be pivotally coupled by any of a variety of means such as a screw **233** (as shown), pins, etc. Additionally, bushings **231** may be present to provide a bearing surface. The pawl **227** is able to swing about its pivot axis point within the cavity in the handle sleeve **218.** The pawl **227** is also able to swing about its pivot out towards the fixed gripping surface **214** on the interior face of the rectangular block **212.**

The surface of the pawl **227** facing the fixed gripping surface **214** on the interior face of the rectangular block **212** may be arced as best shown in **FIG. 3C****.** The surface of the pawl **227** facing the fixed gripping surface **214** on the interior face of the rectangular block **212** may further comprise a gripping surface **237.** The gripping surface **237** could be made of a high friction material, a compressible material, a material exhibiting both those qualities, or any other suitable material. The gripping surface **237** is made of a material which allows a firm grip without the deformation of a clamped object **100** Additionally, the gripping surface **237** may be contoured (as shown best in **FIGS. 3A-3E**).

The pawl **227** may be additionally comprised of a trough **239** cut into the pawl **227** essentially along the center plane of the pawl **227** running parallel to the plane of the back plate **206.** The trough **239** is shaped such that it is able to accommodate the shape of a lift bar **241.** As best shown in **FIGS. 3C-3E****,** the lift bar **241** may comprise a first portion comprising a member **224** which projects into the trough **239** in the pawl **227.** The member **224** may be shaped such that at the right end of the member **224** there is a wing like projection **243.** Within the wing like projection **243,** there may be a slit **245.** It should be noted that the slit **144** in **FIGS. 2A-2E** is at an angle and the slit **245** in **FIGS. 3A-3E** is substantially horizontal. Alternate embodiments may employ slits oriented at any angle or may employ arced slits. A coupling dowel **268** runs through the slit **245** and into the pawl **227** coupling the lift bar **241** to the pawl **227.**

The lift bar **241** may also comprise a second portion in which a member **226** extends toward the bottom of the page at an angle that is substantially perpendicular to the member **224** of the first portion. The member **226** of the second portion has an engagement surface **228** which engages with the track **223** on the interior of the handle sleeve **218.** In the shown embodiment, the engagement surface **228** is depicted as a recessed groove. The engagement surface **228** may, however, be raised or take other forms including but not limited to, rollers, ball bearings, etc. In embodiments where the track **223** is the rack of a rack and pinion arrangement, one or more pinion gears capable of engaging the track **223** may be present on the member **226** of the second portion.

The member **226** of the second portion of the lift bar **241** may also have a bracket **230** extending off the bottom surface of the member **226.** The bracket **230** need not extend as shown at an angle substantially perpendicular to the bottom surface of the member **226.**

The member **226** of the second portion of the lift bar **241** may also be comprised of a groove or grooves **232** recessed into the face of the member **226** which abuts the interior surface of the handle sleeve **218** from which the compression spring pocket(s) **215** extend. The groove **232** is of a size and shape sufficient to fit around the compression spring pocket **215** which projects off the interior of the handle sleeve **218.** Additionally, the groove **232** does not run the entire length of the member **226** stopping at least some distance from the top of the member **226.** As shown, the diameter of the groove **232** may taper as it extends toward the top of the member **226.**

A coil spring **272** is placed in the groove **232** such that one end of the coil spring **272** abuts the bottom of the compression spring pocket **215.** The other end of the coil spring **272** abuts the top of the groove **232.** Though the shown embodiments use a coil spring **272,** other embodiments could conceivably employ any other suitable bias member. A wide variety of suitable bias members may be employed. Examples of suitable bias members include, but are not limited to, a gas spring (using a bladder arrangement, piston type arrangement, etc.), a compression spring made of a compressible, springy material such as rubber, an extension spring, constant force spring, and so on.

In the example embodiment, the coil spring **272** biases the clamp apparatus **202** toward the closed position (**FIG. 3A**). In the closed position, the coil spring **272** is not compressed. Additionally, the lift bar **241** is at its highest point of travel along the tracks **223** in the handle sleeve **218** of the housing **112.** Since the lift bar **241** is coupled to the pawl **227** via the coupling dowel **268,** this forces the pawl **227** to be pivoted up and in toward the fixed gripping surface **214.** In the closed position, the coupling dowel **268** abuts the right edge of the slit **245.**

If a clamped object **100** is present in the example embodiment, the coil spring **272** biases the clamp apparatus **202** to clamp down on the object **100.** Depending on the size of the clamped object **100,** the lift bar's **241** location on the track **223** will vary. The larger the clamped object **100** the lower the lift bar **241** will be on the track **223.** Additionally, the pawl **227** will not be fully rotated up and inward toward fixed gripping surface **214.** Instead the distance between the gripping surface **237** of the pawl **227** and the fixed gripping surface **214** will mimic the diameter of the clamped object **100.** This also means that the location of the coupling dowel **268** will be somewhat closer to the left of the slit **245.**

The clamp apparatus **202** in the example embodiment is designed in such a way as to utilize the force of gravity to increase the clamping force. As gravity pulls on the clamp apparatus **202,** especially when a load is attached to the housing **204** the force causes the pawl 227 to want to rotate further up and in towards the fixed gripping surface **214.** Since the clamped object **100** is in the way, the pressure of the pawl **227** against the clamped object **100** increases and the clamping apparatus **202** grips the clamped object **100** more vigorously. Furthermore, the clamped object **100** is pushed against the fixed gripping surface **214** with greater force again causing the clamping apparatus **202** to clamp more vigorously to the clamped object **100.**

This more vigorous clamping force is accomplished by ensuring that the pawl **227** is constructed and shaped in order to ensure the clamp apparatus **202** will be in static equilibrium with a clamped object **100** when the clamp apparatus **202** is clamped onto a clamped object **100.** This may require ensuring that the coefficient of friction of the pawl **227** is greater than the ratio of the vertical distance from the contact point of the pawl **227** on the clamped object **100** to the pivot point of the pawl **227** (said distance hereafter referred to as A) to the horizontal distance from the contact point on the pawl **227** to the pivot point of the pawl **227** (said distance hereafter referred to as B). The compliance and shape of the pawl **227** gripping surface **237** of the pawl **227** also is sufficiently configured.

As shown, the pawl **227** does not have a constant radius from the gripping surface **237** to the pivot point of the pawl **227.** If the radius is constant, and the pawl **227,** gripping surface **237,** or both are relatively compliant, A:B may become less than zero if the pawl **227,** gripping surface **237,** or both become compressed. If the radius of the pawl **227** constantly increases as best shown in **FIG. 3C****,** this cannot occur. The rate of increase in the radius of the pawl **227** may be chosen so that the ratio A:B does not become too large. This may be done to ensure that the coefficient of friction is not inordinately large.

In embodiments of the pawl **227** where the radius of the pawl **227** is constantly increasing and the pawl **227,** gripping surface **237,** or both are compliant, as the downward force of gravity acting on the clamp apparatus **202** increases the ratio A:B decreases. As a result, the normal forces present at the contact point of the pawl **227** on the clamped object **100** increase. The vertical reaction force increases as a result. This may create the more vigorous clamping force described above

To move the clamp apparatus **202** to the open position shown in the embodiment in **FIG. 3B****,** the user must actuate a trigger **234.** The trigger **234** has a button portion **236** which extends at least partially out of the right face of the handle sleeve **218** when the clamp apparatus **202** is in the closed position. Toward the lower right of the button portion **236,** the button portion **236** is pivotally coupled to the handle sleeve **218** by any of a variety of means. The button portion **236** may be hollow or solid. Projecting toward the left of the page of along the bottom plane of the button portion **236** of the trigger **234** may be one or more arms **238.** The one or more arms **238** may be capable of coupling to a linkage structure **240.** The linkage structure **240** also extends up to, and is coupled to, the bracket **230** which extends off the bottom surface of the lift bar **241.** As best shown in **FIG 3C-3E****,** the link structure **240** in the example embodiment is an oblong with rounded edges. In other embodiments, the link structure **240** may take other forms and shapes. Examples of link structures **240** in other possible embodiments may include, but are not limited to, prismatic joints, any of a variety or springs, etc. It would also be conceivable to forgo the brackets **230** while coupling a camming surface to the trigger **234** thus effectively making the lift bar **214** a cam follower.

In the example embodiment, when the trigger **234** is actuated, it acts as a lever pulling the linkage structure **240** and the lift bar **241** toward the bottom of the page. As the lift bar **241** is pulled down the track **223** on the handle sleeve **218** the coil spring **272** gets compressed. The slit **245** in the wing **243** of the lift bar **241** slides over the coupling dowel **268** until the coupling dowel **268** abuts the left most edge of the slit **245.** As a result, the pawl **227** rotates down and away from the fixed gripping surface **214** and into the open position. Releasing the trigger **234** causes the clamping apparatus **202** to return to the closed position as a result of the restoring force of the coil spring **272.** In alternate embodiments, a torsion spring may be employed where the button portion **236** of the trigger **234** is pivotally coupled to the handle sleeve **218.** The torsion spring may be a substitute for or used in conjunction with the coil spring **272** or other suitable bias member configuration.

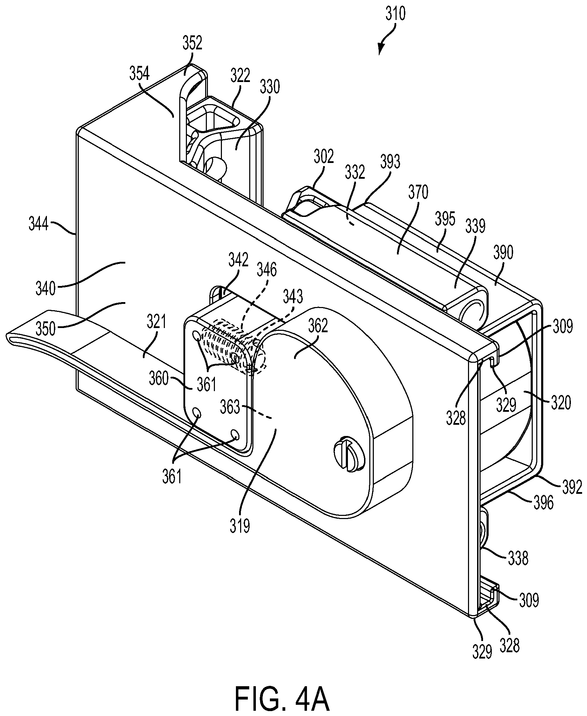

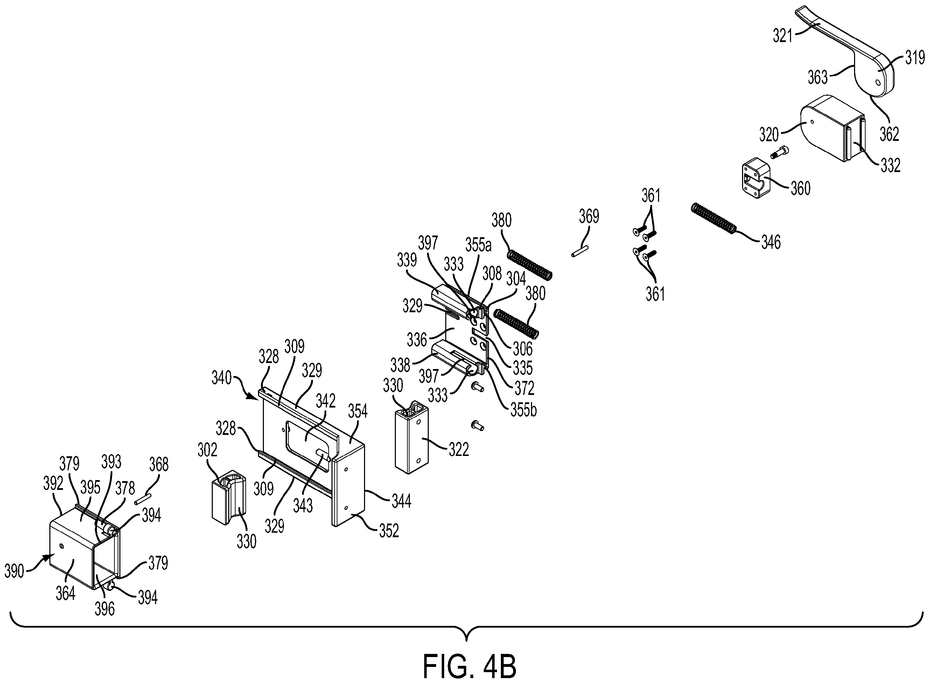

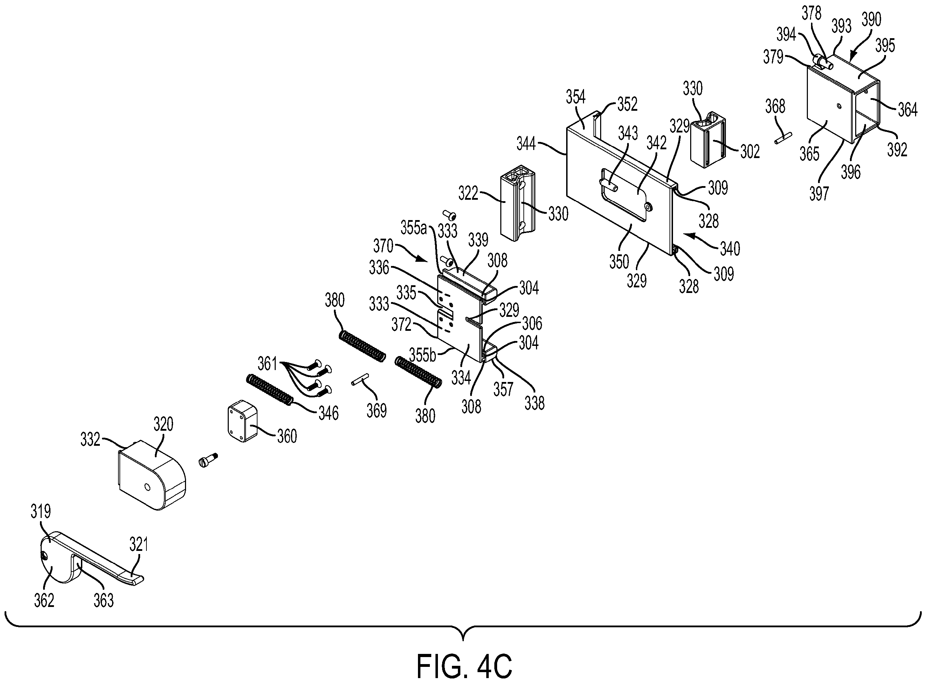

**Fig. 4A** shows a perspective view of a clamp apparatus **310** in the open position according to one embodiment of the present disclosure. A clamped object **100** may be squeezed between a fixed gripper **322** and a sliding gripper **302.** The fixed gripper **322** and sliding gripper **302** may consist of a material chosen for its gripping ability. The fixed gripper **322** and sliding gripper **302** may be made of a material which allows for a firm grip without the deformation of a clamped object **100.** The fixed gripper **322** and sliding gripper **302** may be made of a high friction material, a compressible material, a material exhibiting both these qualities, or any other suitable material. Suitable materials may include any suitable elastomeric or non-deformable substance, including but not limited to plastic, rubber, metal, foam, fabric, gel, etc. At least a portion of the fixed gripper **322** and sliding gripper **302** may comprise a roughly semi-circular depression or contour to accommodate a round clamped object **100** such as a pole.

In some embodiments, the fixed gripper **322** and sliding gripper **302** are formed from a relatively inelastic material, but have caps **330** (not shown) that fit substantially over the fixed gripper **322** and sliding gripper **302.** The cap **330** may be constructed from any suitable material, including but not limited to, elastic materials such as rubber, plastic, gel, foam, fabric, polyurethane, etc. The caps **330** may be replaceable and removably attached to the fixed gripper **322** and sliding gripper **302.**

The fixed gripper **322** may be firmly mounted to the fixed gripper mount end **344** of a guide plate **340.** In some embodiments, a gripper support wall **352** is attached to the fixed gripper mount end **344** of the guide plate **340** and provides additional support for the fixed gripper **322.** The gripper support wall **352** may optionally be supported by one or more buttresses **354** that span from at least a portion of the guide plate **340** to the gripper support wall **352.** In some embodiments, the buttresses **354** may be arched to maximize support.

At least one face of the guide plate **340** may also feature any of a variety of mechanisms **305** (not shown) to attach a load to the clamp apparatus **310.** Such mechanisms **305** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

The sliding gripper **302** is mounted to the sliding gripper mount end **332** of a sliding gripper base **320.** The position of the sliding gripper base **320** is adjustable to accommodate clamped objects **100** of various dimensions and girths. The sliding gripper base **320** will be elaborated upon later.

In an embodiment of the present disclosure shown in **FIG. 4A****,** the clamp apparatus **310** is depicted in the closed position (though a clamped object **100** is not present). To move the clamp apparatus **310** to the closed position, a user must rotate a handle assembly **319,** such that the hand grip **321** of the handle assembly **319** is pointed toward the left of the page as shown in **FIG. 4A****.** This action propels the sliding gripper **302** and all attached structures towards the fixed gripper **322.** If a clamped object **100** is present, the sliding gripper **302** will squeeze the clamped object **100** against the fixed gripper **322,** thus clamping the clamped object **100.**

The handle assembly **319** is rotatably attached to the front face **350** of the guide plate **340.** In the exemplary embodiment shown in **FIGS. 4A-4D****,** the handle assembly **319** is disposed on a plane approximately parallel to the plane of the front face **350** of the guide plate **340** regardless of whether the clamp apparatus **310** is in the open or closed position or in transit between an open and closed position. The handle assembly **319** is comprised of a number of portions. At least a one portion of the handle assembly **319** abuts a cam plate **360,** which is immovably attached to a pressure plate **370** (pressure plate **370** introduced in subsequent paragraphs). In the depicted exemplary embodiment in **FIGS. 4A-4D****,** the handle assembly **319** comprises a cam **362** positioned to contact the cam plate **360.** The rounded, contoured surface of the cam **362** grades into a planate section which spans the length of the hand grip **321.**

In some embodiments, hand grip **321** may be made of the same material as the rest of the handle assembly **319,** may be made of a different material, or may be made of a combination thereof. Possible materials may include, but are not limited to, rubber, polymer, composite, metal, plastic, foam, etc. The hand grip **321** may also comprise ergonomic finger groves, nubs, a ribbed texture, a honeycombed texture, etc. to facilitate ease of grasping.

Additionally, as shown in the example embodiment in **FIGS. 4A-4D** the cam **362** may include at least one flat segment **363.** Clockwise rotation of handle assembly **319** causes the cam **362** to rotate into the cam plate **360.** This displaces the cam plate **360** towards fixed gripper **322.** In the closed position, the cam **362** is fully rotated into the cam plate **360** and the flat segment **363** of the cam **362** abuts the right edge (relative to **FIG. 4A**) of the cam plate **360.** Additionally, in the fully closed position, the planate surface of the hand grip **321** may rest against the bottom edge of the cam plate **360.** The flat segment **363** of the cam **362** prevents the restoring force from a compressed return spring **346** (which spring loads the cam plate **360**) from pushing cam plate **360** back to the open position, and thus may effectively lock the clamp apparatus **310** in the closed position.

To open the clamp apparatus **310,** a user rotates the handle assembly **319** counterclockwise. As the cam **362** releases pressure on the cam plate **360,** the compressed return spring **346** causes the cam plate **360** to automatically return back to the open position as the return spring **346** expands back to a relatively uncompressed state.

In the open position (not shown) the cam plate **360** comes to rest against the right edge (in reference to **FIG. 4A**) of an aperture **342** in the guide plate **340.** The aperture **342** is cut through the guide plate **340** at an angle which is substantially perpendicular to the front face **350** of the guide plate **340.** On the left vertical edge of the aperture **342** a return spring peg **343** may project into the aperture **342** in a direction substantially parallel to the plane of the front face **350** of the guide plate **340.** The return spring peg **343** is slightly smaller in diameter than the return spring **346.** The return spring **346,** may be seated around the return spring peg **343** (as shown in **FIG. 4A**)**.** In the open position, the return spring **346** may be slightly compressed to prevent any "slop" and to keep the cam plate **360** against the right edge of the aperture **342.**

The cam plate **360** is immovably coupled to a pressure plate **370.** In the example embodiment shown in **FIGS. 4A****-4E**, the cam plate is coupled to the pressure plate **370** via screws **361.** In other embodiments, the cam plate **360** and pressure plate **370** may be coupled to each other in any number of ways, including, but not limited to welds, bolts, rivets, etc. In some embodiments, they may be formed as a continuous part during manufacture.

Since the cam plate **360** is attached to the pressure plate **370,** the pressure plate **370** also moves as the cam **362** of the handle assembly **319** displaces the cam plate **360.** When the return spring **346** expands as the clamp apparatus **310** is opened, the pressure plate **370** is also spring loaded to automatically return toward its open orientation. When the clamp apparatus **310** is fully opened, the pressure plate **370** may be approximately flush with the right edge of the guide plate **340** (in reference to **FIG. 4A**). In the example embodiment shown in **FIGS. 4A****-4E**, the pressure plate **370** may not extend out past the right edge of the guide plate **340** because the cam plate **360** to which it is immovably attached is restricted in movement by the right edge of the aperture **342** in the guide plate **340.**

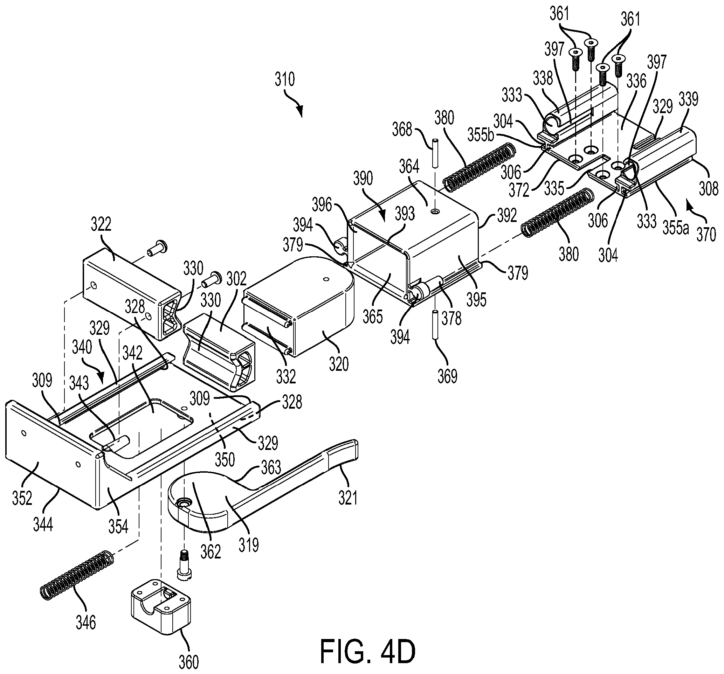

Extending perpendicularly from the center of the left edge **372** of the pressure plate **370** (in reference to **FIG. 4D**) into the pressure plate **370** is a return spring trough **335.** The return spring trough **335** allows the return spring **346** to fit comfortably into the clamp apparatus **310** when the clamp apparatus **310** is fully assembled and operated.

In the example embodiment shown in **FIGS. 4A-4D****,** the pressure plate **370** is slidingly coupled to the guide plate **340** by a tongue-in-groove type association. The top edge **355a** and bottom edge **355b** (relative to **FIGS. 4A-4C**) of the pressure plate **370** function as the tongues. The top edge **355a** and bottom edge **355b** of the guide plate **370** ride along a track **328** which comprises a part of the guide plate **340** structure. In the embodiment depicted, the track **328** is a recessed groove which is cut out of flanges **329** extended off the top and bottom edges of the guide plate **340.** The flanges **329** project toward the back of the page (in relation to **FIG. 4A**) in a direction substantially perpendicular to the plane of the front face **350** of the guide plate **340.** As shown, the tracks **328** may be cut into the flanges **329** such that the tracks **328** run substantially parallel to the plane of the front face **350** of the guide plate **340.**

The clamp apparatus **310** in the illustrated embodiment in **FIGS. 4A-D** also comprises a gripper sled **390.** The gripper sled **390** may also be coupled to the clamp apparatus **310** by one or a number of tongue-in-groove associations. As shown, the gripper sled **390** may be slidably coupled to the pressure plate **370.** Additionally, at least one spring **380** may be disposed between the gripper sled **390** and pressure plate **370** to exert additional clamping force while the clamp apparatus **310** is in the closed position and a clamped object **100** is present.

In an example embodiment, the gripper sled **390** is a generally a hollow, mostly rectangular sleeve open on its right end **392** and left end **393** (relative to **FIG. 4A**). The sliding gripper base **320** may fit into, hollow interior of the sleeve-like gripper sled **390.** Other embodiments may close the left end **393** of the gripper sled **390** and attach the sliding gripper **302** to it such that the left end **393** of the gripper sled **390** performs the function of the sliding gripper base **320.**

In the exemplary embodiment shown in **FIGS. 4A-4D****,** the sliding gripper base **320** is immovably coupled inside the hollow interior of the gripper sled **390.** This may be accomplished in any number of ways. As shown, the sliding gripper base **320** may be coupled into the gripper sled by a first dowel **368** and a second dowel **369.** Other embodiments which employ dowels may use any suitable number of dowels. The first dowel **368** may be inserted through an orifice in the in the back face **364** of the gripper sled **390** into a corresponding orifice in the back face of the sliding gripper base **320** (directions refer to orientation of **FIG. 4A**). The second dowel **369** may be inserted through an orifice in the front face **365** of the gripper sled **390** into a corresponding orifice in the front face of the sliding gripper base **320.**

In the example embodiment shown in **FIGS. 4A-4D****,** the second dowel **369** is not flush with the front face **365** of the gripper sled **390.** Instead, at least a portion of the second dowel **369** projects past the front face **365** of the gripper sled **390.** At least a part of this portion of second dowel **369** rides along a slit **329** which is cut into the edge of the pressure plate **370** opposite the return spring trough **335.** As shown, the slit **329** may be cut into the said edge of the pressure plate **370** at an angle substantially perpendicular to said edge. The interaction of the slit **329** and second dowel **369** effectively restricts the movement of the gripper sled **390.** When the second dowel **369** abuts the left end of the slit **329,** the second dowel **369** and all attached components may travel no further toward the left of the page (in relation to **FIG. 4A**).

The gripper sled **390** may also comprise a set of ears **394.** As shown in the example embodiment in **FIGS. 4A-D**, one of the ears **394** may project off the top face **395** of the gripper sled **390** while the other projects off the bottom face **396** of the gripper sled **390.** In the embodiment illustrated in **FIGS. 4A-4D****,** each ear **394** comprises a post which supports a round cylinder whose elongate section runs in a direction parallel to the plane of the front face **365** of the gripper sled **390.** The ears **394** project off the top face **395** and bottom face **396** of the gripper sled **390** at an angle substantially perpendicular to the top face **395** and bottom face **396** of the gripper sled **390.** In alternate embodiments, the shape, thickness, construction, orientation, etc. of the ears **394** may differ. Additionally, some embodiments may comprise a compression spring peg **378** which projects off each ear **394.** The compression spring pegs **378** are similar to the return spring peg **343.**

In an embodiment of the present disclosure, the top and bottom edges of the front face **365** of the gripper sled **390** may comprise gripper sled tongues **379** which run at least partially along at least one of the top and bottom edges of the front face **365** of the gripper sled **390.** In the example embodiment shown in **FIGS. 4A-4D****,** the gripper sled tongues **379** project off the entire length of the top and bottom edges of the front face **365** of the gripper sled **390** and are extensions of the plane of the front face **365** of the gripper sled **390.**

Extending from the rear face **336** of the pressure plate **370** and oriented approximately parallel to the return spring trough **335** may be a top spring housing **339,** and a bottom spring housing **338.** In an exemplary embodiment shown in **FIGS. 4A-4D****,** the top spring housing **339** and bottom spring housing **338** both comprise a raised ridge **304** and a compression spring pocket **333.** The raised ridge **304** projects off the rear face **336** of the pressure plate **370** at an angle substantially perpendicular to rear face **336** of the pressure plate **370.** The raised ridges **304** run parallel to the top edge **355a** and bottom edge **355b** of the pressure plate **370.** As shown, the raised ridges **304** may span the entire length of the pressure plate **370.** The raised ridges **304** function as a post on which the compression spring pockets **333** of the top spring housing **339** and bottom spring housing **338** are coupled. As shown in the example embodiment in **FIGS. 4A-****4D** the compression spring pockets **333** may be elongated along the entire length of the ridges **304.**

The compression spring pockets **333** overhang the ridges **304** forming "T" type shapes. The portions of the "T" type shapes facing the lateral center line of the pressure plate **370** form the grooves **306** of a tongue-in-groove arrangement in conjunction with the rear face of the pressure plate **370.** The gripper sled tongues **379** are slidably coupled into these grooves **306.**

The opposite portions of the "T" type shapes (those distal to the lateral centerline of the pressure plate **370**) also form the grooves **308** of another tongue-in-groove type arrangement in conjunction with the rear face of the pressure plate **370.** In the embodiment shown in **FIGS. 4A-4D****,** the distal grooves **308** slidably couple around tongues **309** formed by a part of the flanges **329** which are extended off the guide plate **340.**

The compression spring pockets **333** may be hollow so as to allow compression springs **380** to be seated inside the compression spring pockets **333.** In the embodiment shown in **FIGS. 4A-4D****,** the right end (relative to **FIG. 4A**) of the compression spring pockets **333** is closed to provide a surface upon which the compression springs **380** may be compressed against. Additionally, the compression spring pockets **333** each feature a slot **397** (best shown in **FIG. 4D**) which is cut out of the face of the compression spring pockets **333** most proximal to the lateral centerline of the pressure plate **370.**

When assembled, as detailed above, a compression spring **380** may be seated in each of the compression spring pockets **333.** One end of the compression springs **380** abuts the closed ends of the compression spring pockets **333.** The other ends of the compression springs **380** abut the right faces of the ears **394** which protrude off the top face **395** and bottom face **396** of the gripper sled **390.** The compression springs **380** fit around the compression spring pegs **378** which may extend from the ears **394** on the gripper sled **390.** This helps to keep the compression springs **380** firmly in place during operation and use of the clamp apparatus **310.** The compression springs **380** bias the gripper sled **390** and components immovably attached to it (notably sliding gripper **302** and sliding gripper base **320**) to the left of the page (relative to **FIG. 4A**) until the second dowel **369** abuts the left end of the slit **329** and the components may move no further to the left of the page. This ensures that as the handle assembly **319** is actuated, the cam plate **360,** pressure plate **370,** gripper sled **390,** and attached components move together as a unit until the sliding gripper **302** encounters a clamped object **100.**

In the shown embodiment in **FIGS. 4A-4D****,** the diameter of the hollow portions of the compression spring pockets **333** is slightly larger than the diameter of the cylinder portion of the ears **394.** The slot **397** in the compression spring pockets **333** creates a path for the post portion of ears **394** to travel. When a force sufficient to overcome the bias force of the compression springs **380** is applied, the compression springs **380** begin to compress.

Such a force may be generated when a user rotates the handle assembly **319** and a clamped object **100** is present. As mentioned above, in the embodiment shown in **FIGS. 4A-4B****,** the cam plate **360,** pressure plate **370,** gripper sled **390** and attached components move together substantially as a unit until the sliding gripper **302** encounters a clamped object **100.** When the clamped object **100** comes into contact with the sliding gripper **302,** the sliding gripper **302** begins to push the clamped object **100** against the fixed gripper **322.** When the force which the clamped object **100** exerts back against the sliding gripper **302** becomes greater than the bias force of the compression springs **380,** the sliding gripper **302,** sliding gripper base **320,** gripper sled **390** and components immovably coupled to the gripper sled **390** stopping moving. The cam plate **360** and pressure plate **370** continue to move toward their closed orientation as the handle assembly **319** rotates to its closed orientation. This causes the compression springs **380** to begin to compress. As the compression springs **380** are compressed the ears **394** slide progressively further into the hollow portions of the compression spring pockets **333** and along the slots **397** of the compression spring pockets **333** until the clamp apparatus **310** reaches its fully closed orientation.

The force exerted by the compressed compression springs **380** on the clamped object 100 through the gripper sled **390** and sliding gripper **302** helps to create a more vigorous gripping force than could otherwise be achieved. Additionally, the restoring force of the compression springs **380** is complimentary to that provided by the return spring **346** when the clamp apparatus **310** is moved to the open position. The compression spring **380** restoring force causes the gripper sled **390** and immovably attached components to return back to their default orientation along slit **329** in the pressure plate **370.** The force exerted by the compressed compression springs **380** additionally facilitates opening of the clamp apparatus **310.**

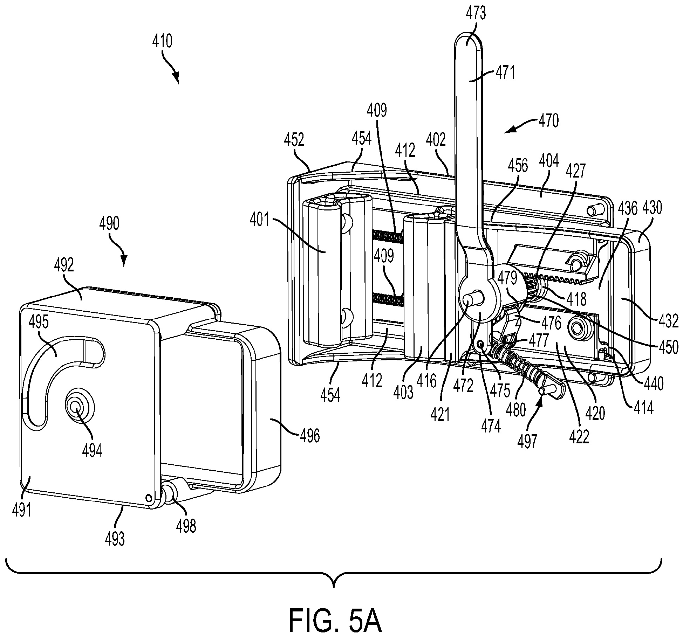

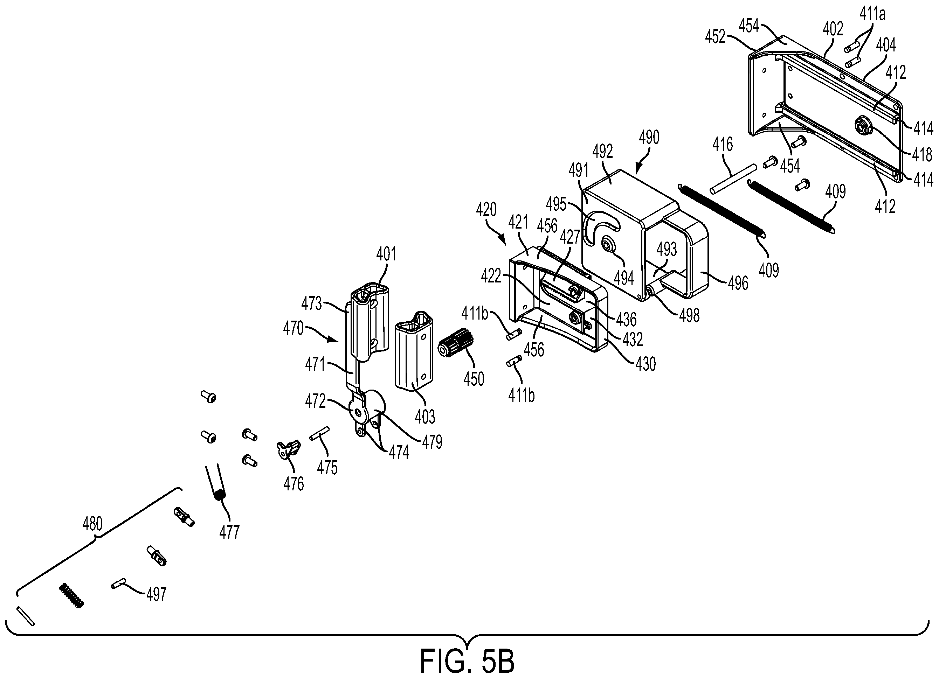

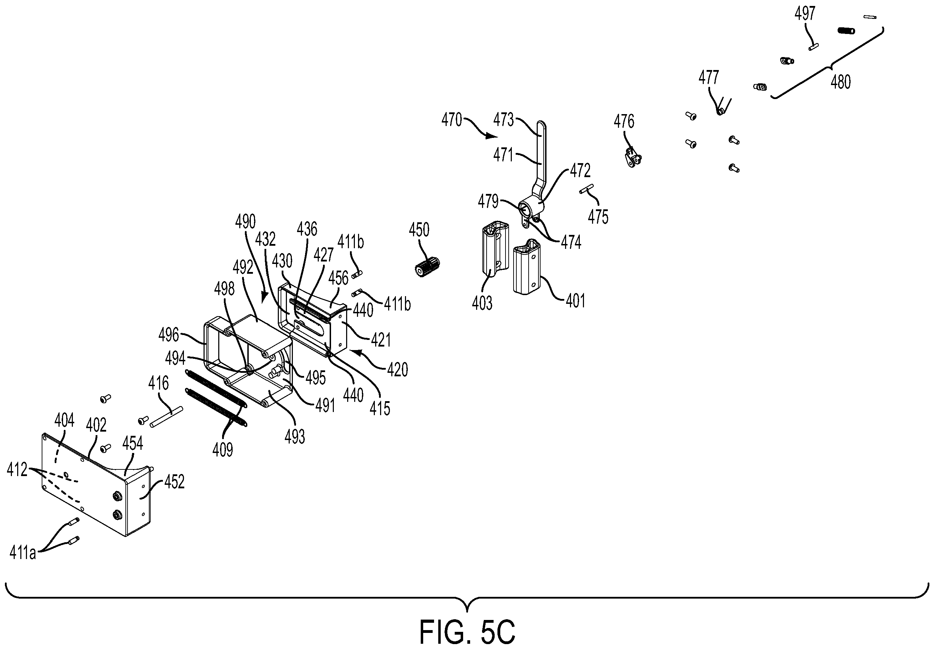

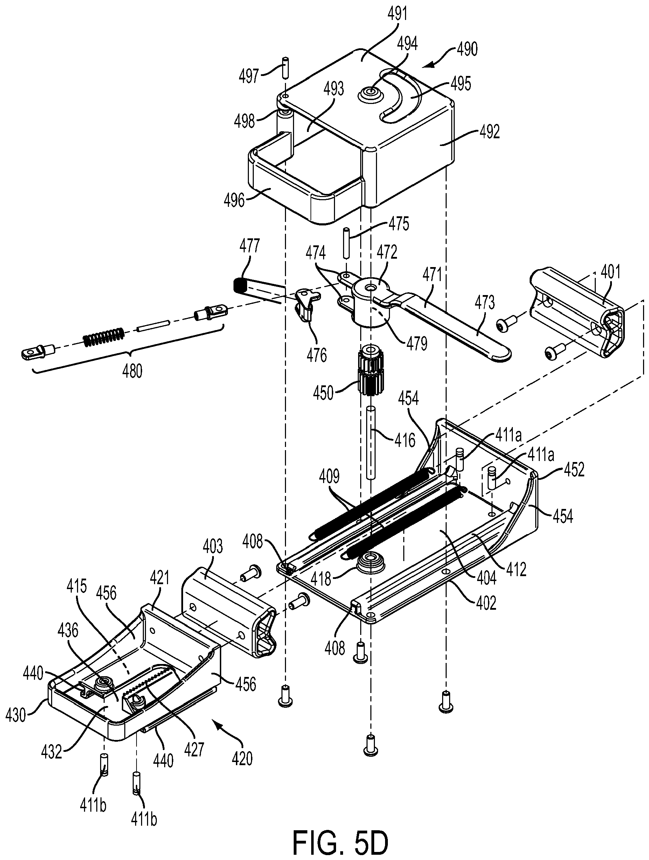

In an embodiment of the present disclosure shown in **FIGS. 5A-5D****,** the restoring force from a pair of tensioned springs **409** acts to clamp a clamped object **100** between a fixed gripper 401 and a sliding gripper **403.** The sliding gripper **403** can then be locked in place by a ratcheting pawl **476,** thus securing clamp apparatus **410** in the clamped position about a clamped object **100.**

In an exemplary embodiment, a fixed gripper **401** may be firmly attached to the front face **404** of an approximately rectangular back plate **402.** The gripping surface of the fixed gripper **401** is oriented perpendicularly to the front face **404** of the back plate **402.** In the embodiment shown in **FIGS. 5A-5D****,** a fixed gripper support wall **452** may be attached to the front face **404** of the back plate **402.** As shown, the fixed gripper support wall **452** may project from the left edge (in relation to **FIG. 5A**) of the back plate **402** in a direction perpendicular to the front face **404** of the back plate **402.** Instead of attaching the fixed gripper **401** to front face **404** of the back plate **402,** the fixed gripper **401** may be fixedly coupled to the right face (in relation to **FIG. 5A**) of the fixed gripper support wall **452.** This is desirable because the fixed gripper support wall **452** is able to provide additional support for the fixed gripper **401.** The fixed gripper support wall **452** may optionally be supported by one or more buttresses **454** that span from at least a portion of the back plate **402** to the fixed gripper support wall **452.** In some embodiments, the buttresses **454** may be arched to maximize support.

The fixed gripper **401** may consist of a material chosen for its gripping ability. The fixed gripper **401** may be made of a high friction material, a compressible material, a material exhibiting both these qualities, or any other suitable material. The fixed gripper **401** may be made of a material which allows a firm grip without the deformation of a clamped object **100.** Suitable materials may include any suitable elastomeric or non-deformable substance, including but not limited to plastic, rubber, metal, foam, fabric, gel, etc. At least a portion of the fixed gripper **401** may comprise a roughly semi-circular depression or contour to accommodate a round clamped object **100** such as a pole.

In some embodiments, the fixed gripper **401** is formed from a relatively inelastic material, but has a cap **458** (not shown) that fits substantially over the fixed gripper **401.** The cap **458** may be constructed from any suitably material, including but not limited to, elastic materials such as rubber, plastic, gel, foam, fabric, polyurethane, etc. The cap **458** may be replaceable and removably attached to the fixed gripper **401.**

In some embodiments, in addition to comprising the mounting site for the fixed gripper **401,** the support plate **402** also includes an attachment site **418** for a gear assembly and a track-way **412** for a rack plate **420.** The gear assembly attachment site **418,** track-way **412,** and rack plate **420** will be elaborated on in subsequent paragraphs.

In an example embodiment, the sliding gripper **403** is firmly attached to the front face **422** of a rack plate **420** such that the gripping surface of the sliding gripper **403** faces the gripping surface of the fixed gripper **401.** As shown in **FIGS. 5A-5D****,** the sliding gripper **403** is coupled to the front face **422** of the rack plate **420** near the edge of the rack plate **420** most proximal to the fixed gripper **401.** In some embodiments, the rack plate **420** may have the shape of a quadrilateral, specifically a rectangle. Some embodiments include a sliding gripper support base **421** which may be similar in varying degrees to the fixed gripper support wall **452.** The sliding gripper support base **421** may optionally have one or more buttresses **456** that span from at least a portion of the rack plate **420** to the sliding gripper support base **421.** In some embodiments, the buttresses **456** may be arched to maximize support.

The sliding gripper **403** may consist of a material chosen for its gripping ability. The sliding gripper **403** may be made of a high friction material, a compressible material, a material exhibiting both these qualities, or any other suitable material. The sliding gripper **403** may be made of a material which allows a firm grip without the deformation of a clamped object **100.** Suitable materials may include any suitable elastomeric or non-deformable substance, including but not limited to plastic, rubber, metal, foam, fabric, gel, etc. At least a portion of the sliding gripper **403** may comprise a roughly semi-circular depression or contour to accommodate a round clamped object **100** such as a pole.

In some embodiments, the sliding gripper **403** is formed from a relatively inelastic material, but has a cap **458** (not shown) that fits substantially over the sliding gripper **403.** The cap **458** may be constructed from any suitably material, including but not limited to, elastic materials such as rubber, plastic, gel, foam, fabric, polyurethane, etc. The cap **458** may be replaceable and removably attached to the fixed gripper **403.**

In the example embodiment shown in **FIGS. 5A-5D****,** the rack plate **420** is roughly rectangular. A handle **430** may project off the edge of the of the rack plate **420** most distal to the fixed gripper **401.** The handle **430** may be a part of a "U" shaped member. As shown, the bottom of the "U" shape and at least a portion of each upright of the "U" shape protrude from rack plate **420** forming a void **432.** The void **432** is defined by the edge of the rack plate **420** and the protruding sections of the "U" shaped handle **430.** A user's finger(s) may easily grip around the bottom of the "U" shape of the handle **430** via this void **432** when a user desires to manipulate the position of the rack plate **420.**

In the example embodiment shown in **FIGS. 5A-5D****,** at least a section of the uprights of the "U" shape of the handle **430** couple the handle **430** to the rack plate **420.** The uprights of the "U" shape of the handle **430** may project off the top and bottom spans (directions relative to orientation in **FIG. 5A**) of the perimeter of the front face **422** of the rack plate **420** toward the front of the page at an angle substantially perpendicular to the front face **422** of the rack plate **420.** The rack plate **420** and handle **430** may be formed as a continuous part during manufacture. Additionally, the top sections of the uprights of the "U" shape of the handle **430** may comprise the buttresses **456** that span from at least a portion of the rack plate **420** to the sliding gripper support base **421.** In alternate embodiments, the handle **430** may be coupled to the rack plate **420** in any of a variety of ways and may take any suitable shape or size.

At least a portion of the handle **430** may be made of a material such as, but not limited to, rubber, polymer, composite, metal, plastic, foam, etc. Additionally, the handle **430** may comprise ergonomic finger groves, nubs, a ribbed texture, a honeycombed texture, etc.

The front face **404** of the back plate **402** may comprise at least one track-way **412** that runs substantially the full length of the width of the back plate **402.** In the embodiment shown in **FIGS. 5A-5B****,** twin track-ways **412** on the front face **404** of the back plate **402** run in parallel fashion from the edge of the back plate **402** on which the fixed gripper **401** is affixed to the opposite edge of the back plate **402.** The twin track-ways **412** run along planes parallel to the top and bottom edges (in reference to **FIG. 5A**) of the back plate **402.** The track-ways **412** may support and guide the rack plate **420** as the clamp apparatus **410** is moved between its clamped and unclamped orientations.

In the exemplary embodiment shown in **FIGS. 5A-5D****,** each of the track-ways **412** comprise a groove **414** which is recessed into each track-way **412.** The groove **414** is recessed into the side of each track-way **412** which faces the other track-way **412.** This causes the track-ways **412** to have an "L" shape. The rear face **415** of the rack plate **420** comprises projections **440** which are dimensioned such that they may be received by the groove **414** in the track-ways **412** on the back plate **402.** This tongue-in-groove type arrangement slidingly and securely couples the back plate **402** and rack plate **420** together.

The clamp apparatus **410** is biased toward the closed position by at least one extension spring **409.** In the embodiment shown in **FIGS. 5A-5D****,** the clamp apparatus **410** comprises two extension springs **409.** One end of each extension spring **409** is hooked around an extension spring peg **411a.** Each extension spring peg **411a** projects toward the front of the page (relative to **FIG. 5A**) from the back plate **402** at an angle perpendicular to the front face **404** of the back plate **402.** The other end of each extension spring **409** is hooked to another extension spring peg **411b.** Each extension spring peg **411b** projects toward the rear of the page (relative to **FIG. 5A**) from the rear face **415** of the rack plate **420** and an angle substantially perpendicular to the rear face **415** of the rack plate **420.**

The extension spring pegs **411a** and **411b** may comprise a feature such as a notch to help ensure the extension springs **409** do not come off the extension spring pegs **411a** and **411b.** In some embodiments, the extension spring pegs **411a** and **411b** may be substituted for by a variety of different attachment means. In some embodiments, hooks, rings, eye bolts, U bolts, or any other arrangement obvious to one skilled in the art may be used. In other embodiments, the clamp apparatus **410** may not use extension springs **409** and instead use any other type of spring such as, but not limited to, a gas spring using a bladder, piston type arrangement, a compression spring, a compression spring made of a compressible, springy material such as rubber, an extension spring, a constant force spring, etc.

In an example embodiment, the non-tensioned length of the extension springs **409** is somewhat smaller than the distance between a set of extension spring pegs **411a** and **411b.** This is desirable because it ensures that the rack plate **420** and attached sliding gripper **403** are always biased against the fixed gripper **401** and that there is no "slop" in the clamp apparatus **410.** Pulling the rack plate **420** and attached sliding gripper **403** away from the fixed gripper **401** (i.e. toward the open position) thus may tension the extension springs **409,** and further spring load the clamp apparatus **410** toward the closed position. When the rack plate **420** is released, the clamp apparatus **410** will automatically default back toward its closed orientation due to the restoring force of the extension springs **409.**

In the exemplary embodiment depicted in **FIGS. 5A-5D****,** a user may open the clamp apparatus **410** by pulling the handle **430** as well as the attached rack plate **420** and sliding gripper **403** away from the fixed gripper **401.** While the clamp apparatus **410** is held in the open position, a clamped object **100** may be placed in the space between the fixed gripper **401** and the sliding gripper **403.** The clamp apparatus **410** may then be allowed to automatically return to the closed position by a user's release of the handle **430.**

Other embodiments, including the embodiment shown in **FIGS. 5A-5D****,** may comprise additional features which provide additional clamping force, make the clamp easier to operate, etc. In addition to the tongue-in-groove type arrangement mentioned above, an embodiment of the present disclosure comprises a lockable ratcheting rack and pinion type connection which may additionally be utilized to inform the movement of the rack plate **420.**

In some embodiments, a gear assembly attachment site **418** may comprise a projection jutting from the front face **404** of the back plate **402.** The gear assembly attachment site **418** is adapted to receive a gear shaft **416.** In an example embodiment, the gear shaft **416** is a rod or dowel made of metal, plastic, or other suitably durable material. The gear shaft **416** may allow a pinion gear **450** to freely rotate about the axis of the gear shaft **416.** In some embodiments, the gear assembly attachment site **418** may take the shape of a raised ring. In embodiments where the gear assembly attachment site **418** is shaped like a raised ring, the center, open section of the ring may have an internal diameter slightly, though not substantially larger than the diameter of gear shaft **416.** The gear shaft **416** may fit securely and non-rotatably within the internal diameter raised ring of the gear assembly attachment site **418.** A pinion gear **450** may be placed on the gear shaft **416.**

The rack plate **420** may comprise a slot that defines a pinion aperture **436** sized to allow the pinion gear **450** to protrude through the aperture **436** toward the front of the page (relative to **FIG. 5A**). As shown in the embodiment in **FIGS. 5A-5D****,** a rack **427** is positioned adjacent the aperture **436** such that the teeth of the rack **427** interdigitate with the teeth of the pinion gear **450.** Since the teeth of the rack **427** and teeth of the pinion gear **450** interdigitate, the pinion gear **450** rotates about the axis of the gear shaft **416** when the rack plate **420** is moved toward or away from the fixed gripper **401.**

The interaction of the teeth of the rack **427** and the teeth of the pinion gear **450** may be exploited via a ratcheting assembly **470** to ratchet the rack plate **420** and attached sliding gripper **403** against a clamped object **100.** This is desirable because it allows a user to generate more clamping force than the extension springs **409** alone are capable of generating. The ratcheting assembly **470** may also enable a user to lock the clamp apparatus **410** against a clamped object 100.

As shown in the exemplary embodiment illustrated in **FIGS. 5A-5D****,** the ratcheting assembly **470** comprises a ratcheting lever **471.** The ratcheting lever **471** comprises a ratcheting lever hub **472.** The ratcheting lever hub **472** may be shaped like a cup which fits over the section of the pinion gear **450** protruding past the rack **427** of the rack plate **420.** The front face (relative to **FIG. 5A**) of the pinion gear **450** may abut the bottom of the cup formed by the ratcheting lever hub **472.** The ratcheting lever hub **472** comprises an orifice which may allow the ratcheting lever hub **472** to be slid onto the gear shaft **416.** In such embodiments, the gear shaft **416** becomes a fulcrum for the ratcheting lever **471.** The ratcheting lever hub **472** may also comprise an opening **479** in the wall of the ratcheting lever hub **472** cup which exposes a number of teeth of the pinion gear **450.**

The ratcheting lever **471** may further comprise a ratcheting lever handle **473.** In the example embodiment in **FIGS. 5A-5D****,** the ratcheting lever handle **473** acts as the input side of the ratcheting lever **471.** The ratcheting lever handle **473** may be grasped by a user and rotated about the axis of the gear shaft **416** to provide an input.

The ratcheting lever handle **473** may be made of the same material as the rest of the ratcheting lever **471,** may be made of a different material, or may be made of a combination thereof. Possible materials may include, but are not limited to, rubber, polymer, composite, metal, plastic, foam, etc. Additionally, the ratcheting lever handle **473** may comprise ergonomic finger groves, nubs, a ribbed texture, a honeycombed texture, etc.

The ratcheting lever **471** may further comprise at least two ratcheting lever posts **474** opposite the ratcheting lever handle **473** which function as the output side of the ratcheting lever **471.** The ratcheting lever posts **474** extend parallel to each other. One ratcheting lever post **474** is extended from the bottom section of the cup of the ratcheting lever hub **472.** The other ratcheting lever post **474** may be extended off the rim section of the cup of the ratcheting lever hub **472.** A ratcheting lever dowel **475** may span the distance between the ratcheting lever posts **474.** A ratcheting pawl **476** and torsion spring **477** may be positioned on the ratcheting lever dowel **475** between the two ratcheting lever posts **474.**

In the exemplary embodiment shown in **FIGS. 5A-5D** a user provides an input to the ratcheting lever lock **471** by rotating the ratcheting lever handle **473** substantially 90° counterclockwise (relative to **FIG. 5A**) from the unlocked position to the locked position. In the unlocked position, the ratcheting lever handle **473** is oriented perpendicular to the top edge (relative to **FIG. 5A**) of the back plate **402** and the ratcheting pawl **476** is retracted away from the teeth of the pinion gear **450.**

As the ratcheting lever handle **473** is rotated to the locked position, the ratcheting pawl **476** rotates into and engages the teeth of the pinion gear **450** through the opening **479** in the ratcheting lever hub **472.** The torsion spring **477** applies a force against the ratcheting pawl **476** which keeps it in engagement with the teeth of the pinion gear **450.** As a user continues to rotate the ratcheting lever handle **473** the ratcheting pawl **476** catches a tooth of the pinion gear **450** and forces the pinion gear **450** to rotate with the ratcheting lever **471.** This rotation of the pinion gear **450** is transmitted to the rack **427** causing the rack **427** and the attached rack plate **420** and sliding gripper **403** to move toward the fixed gripper **401.** If a clamped object **100** is present, this movement squeezes the clamped object **100** against the fixed gripper **401** with more clamping force than the tensioned extension springs **409** alone can generate. The ratcheting pawl **476** additionally locks the clamp apparatus **410** into the ratcheted and closed position because the ratcheting pawl **476** obstructs any rotation of the pinion gear **450** in a direction which would result in movement of the rack **427,** rack plate **420** and attached sliding gripper **403** toward the open position.

In some embodiments, including the embodiment depicted in **FIGS. 5A-5D****,** the clamp apparatus **410** may comprise a cover **490.** In the embodiment shown in **FIGS. 5A-5D****,** the cover **490** has a front plate **491.** Extending perpendicularly off the top and bottom of the rear face (directions refer to orientation in **FIG. 5A**) of the front plate **491** are a top plate **492** and a bottom plate **493.** The rear edges of the top plate **492** and the bottom plate **493,** which run parallel to the plane of the front plate **491,** may be immovably coupled to the cover **490** to the front face **404** of the back plate **403** via screws, or any other suitable fastening method. The right edge (relative to FIG. **5A**) of the bottom plate **493** has a cutout **498.** A dowel **497** may run from the front plate **491** through the cutout **498.**

The front plate **491** of the cover **490** may comprise a second gear assembly attachment site **494.** The second gear assembly attachment site **494** may comprise an orifice which has a diameter slightly, though not substantially larger than the diameter of the gear shaft **416.** The gear shaft may fit securely and non-rotatably into the orifice of the second gear assembly attachment site **494.**

In some embodiments, the front plate **491** may comprise a ratcheting lever handle slot **495** through which the ratcheting lever arm **473** may extend. The ratcheting lever handle slot **495** may arc so as to allow uninhibited travel of the lever handle **473** from the unlocked position to the locked position.

In one embodiment, the cover **490** has a palm support **496.** The palm support **496** may be formed as a U-shaped member projecting from the cover **490** in a manner and direction similar to that of the handle **430** of the rack plate **420.** The palm support 496 is adapted for use as a carrying handle. The palm support 496 may also be utilized to aid in easy, one-handed opening of the clamp apparatus 410. A user may place the palm support 496 in their palm and grasp the handle **430** by placing their finger(s) in the void 432. By clenching their fist, a user may then transition the clamp apparatus 410 to the open position.

The palm support 496 may be made of the same material as the rest of the cover **490,** may be made of a different material, or may be made of a combination thereof. Possible materials may include, but are not limited to, rubber, polymer, composite, metal, plastic, foam, etc. Additionally, the palm support **496** may comprise ergonomic finger groves, nubs, a ribbed texture, a honeycombed texture, etc. to aid in carrying or grasping.

In some embodiments, the clamp apparatus **410** may comprise an over-center linkage **480** to help ensure the ratcheting lever lock **471** stays in a desired position. As shown in the embodiment in **FIGS. 5A-5D****,** the over-center linkage **480** is attached at one end to the dowel **497** running through the cutout **498** in the cover **490.** The other end of the over-center linkage **480** is attached to the ratcheting lever dowel **475** adjacent the ratcheting pawl **476** and torsion spring **477.** The over-center linkage **480** may bias the ratcheting lever lock **471** to stay in either the unlocked position or locked position. When the over-center linkage **480** is in the over center position the clamp apparatus **410** is kept in the locked position. Before the over-center linkage **480** reaches an over-center position, the clamp apparatus **410** is kept in the unlocked position.

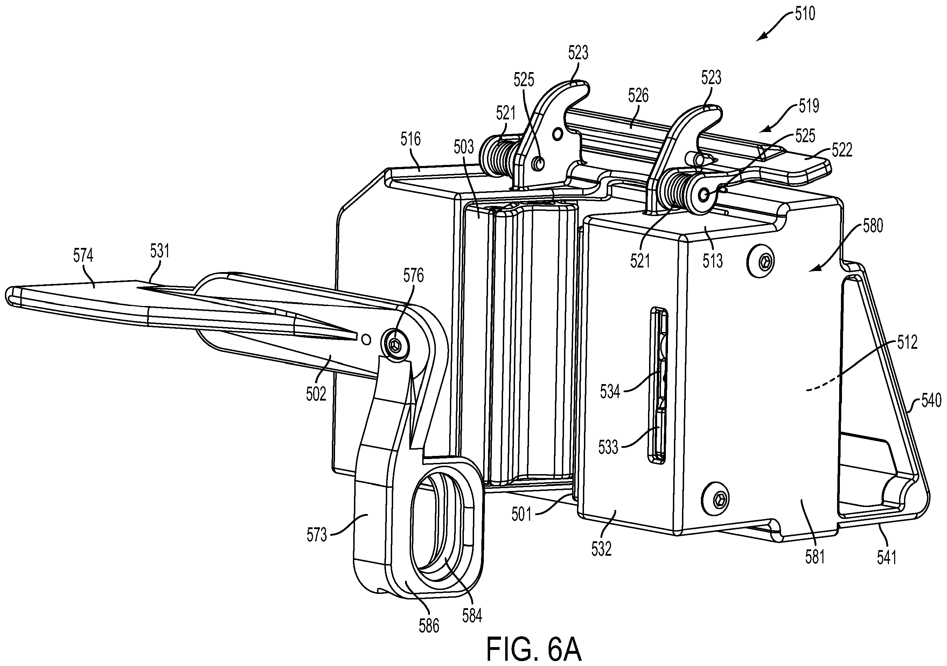

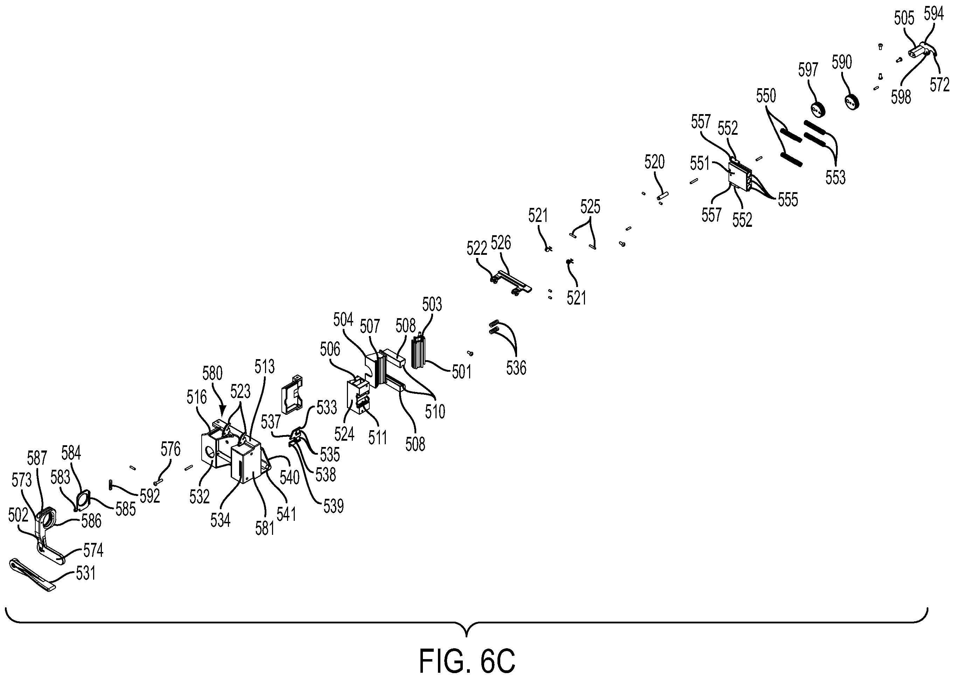

In another example embodiment of the present disclosure shown in **FIG. 6A-6G****,** a sliding gripper **503** is coupled to a sliding gripper base **504** and may be capable of movement towards a fixed gripper **501** mounted on a fixed gripper base **524.** As the sliding gripper **503** is displaced towards the fixed gripper **501,** a clamped object **100** placed between the fixed gripper **501** and sliding gripper **503** may be clamped between the fixed gripper **501** and sliding gripper **503.** As a clamped object **100** is clamped, at least one compression spring **550** compresses. The restoring force of the compressed compression spring **550** supplies additional clamping force as it pushes the sliding gripper **503** against the clamped object **100.** An actuator handle latch **584** locks the clamp apparatus **510** in the closed position, safely securing the clamp apparatus **510** and its attached load (for example, a medical device) to a clamped object **100.**

The fixed gripper **501** and sliding gripper **503** may be comprised of a material chosen for its gripping ability. The fixed gripper **501** and sliding gripper **503** may be made of high friction materials, compressible materials, materials exhibiting both these qualities, or any other suitable material. The fixed gripper **501** and sliding gripper **503** are made of materials which allow for a firm grip without the deformation of a clamped object **100.** Suitable materials may include any suitable elastomeric or non-deformable substance, including but not limited to plastic, rubber, metal, foam, fabric, gel, etc. At least a portion of the fixed gripper **501** and sliding gripper **503** may comprise roughly semi-circular depressions or contours to accommodate a round clamped object **100** such as a pole.

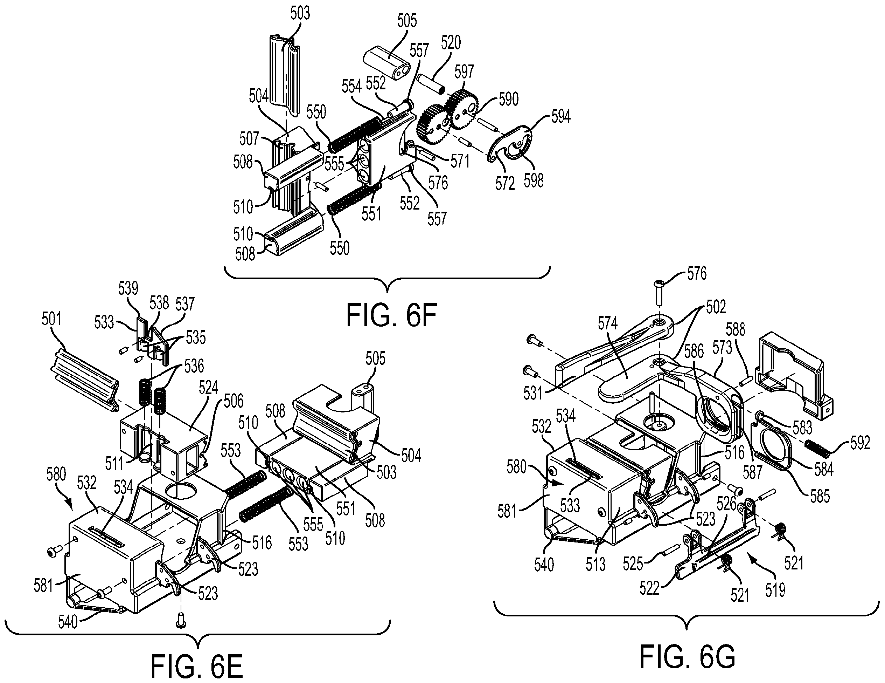

In the example embodiment shown in **FIGS. 6A-6G****,** the fixed gripper **501** is mounted to a fixed gripper base **524.** The fixed gripper base **524** comprises a fixed gripper attachment site **506.** The fixed gripper attachment site **506,** faces the sliding gripper **503.** As best shown in **FIG. 6E****,** the fixed gripper attachment site **506** may be a depression dimensioned to fit the contour of the fixed gripper **501.** In some embodiments, the fixed gripper attachment site **506** may frictionally retain the fixed gripper **501** by means of a friction fit. In alternate embodiments, the fixed gripper **501** may be coupled to the fixed gripper attachment site **506** by any of a variety of means including, but not limited to, screws, bolts, ultrasonic welds, magnets, adhesive, hook and loop tape, or any other suitable coupling means.

The fixed gripper base **524** may be a substantially rectangular block which fits into a cavity of the housing **580** of the clamp apparatus **510.** One side of the fixed gripper base **524** may be fixedly coupled to the right face **581** (relative to **FIG. 6A**) of the housing **580.** The fixed gripper base **524** may be coupled to the right face **581** of the housing **580** by any of a number of means, such as screws, bolts, ultrasonic welds, magnets, adhesive, or any other suitable coupling means. The fixed gripper base **524** may also comprise a strike plate spring bay **511.** The strike plate spring bay **511** will be elaborated upon later.

As best shown in **FIG. 6F****,** the sliding gripper base **504** may comprise both a sliding gripper attachment site **507** and a guide rail **508** to guide movement of the sliding gripper **503.** The sliding gripper attachment site **507** is located on the face of the sliding gripper base **504** which faces the left of the page (relative to **FIG. 6F**). As shown in **FIG. 6F****,** the sliding gripper attachment site **507** may be depression dimensioned to fit the contour of sliding gripper **503.** In some embodiments, including the embodiment in **FIGS. 6A-6G****,** gripper attachment site **507** may frictionally retain the sliding gripper **503** by means of a friction fit. In alternate embodiments, the sliding gripper **503** may be coupled to the sliding gripper attachment site **507** by screws, bolts, ultrasonic welds, magnets, adhesive, or any other suitable coupling means.

Offset from the sliding gripper attachment site **507** may be at least one guide rail **508.** In the example embodiment in **FIG. 6A-6G****,** there are two guide rails **508.** The guide rails **508** are offset from the sliding gripper attachment site **507** toward the front of the page (relative to **FIG. 6F**) and run perpendicular to the face of the sliding gripper base **504** on which the sliding gripper attachment site **507** is disposed. In some embodiments, a guide recess **510** may be defined along/into at least one surface of the guide rail(s) **508.** The at least one guide rail **508** and guide recess **510** will be elaborated upon later.

Some embodiments may also include a slider sled **551.** In some embodiments, the slider sled **551** is involved in four interrelated functions. First, the slider sled **551** provides a predefined track-way for the guide rails **508** of the sliding gripper base **504.** Second, the slider sled **551** may support at least one compression spring **550.** The compression spring(s) **550** may ensure that the slider sled **551,** sliding gripper base **504** and attached components move together as a unit until the sliding gripper **503** abuts a clamped object **100.** When the clamp apparatus **510** is locked in the closed position and the compression spring(s) **550** are compressed, the restoring force exerted by the compressed compression springs **550** provides additional clamping force against a clamped object **100.** Third, the slider sled **551** may comprise at least one return spring pocket **555.** A return spring **553** may be placed in each of the return spring pocket(s) **555.** The return springs **553** may bias the clamp apparatus **510** toward the open position and automatically return the slider sled **551** to the open position when the user actuates the clamp apparatus **510** into the open position. Fourth, the slider sled **551** may comprise a catch **571** which may act as a stop during user actuation of the clamp apparatus **510.**

In relation to the first function, the guide recess **510** is sized to fit a complimentary guide projection **554** located on at least one face of the slider sled **551.** In the embodiment shown in **FIGS. 6A-6G****,** the guide projections **554** run the length of the top face **558** and bottom face **556** of the slider sled **551.** The guide projections **554** may serve as a track-way to direct the slider gripper base **504** as it moves between an open and closed position. In one embodiment, the guide projection(s) **554** are raised ridges running the length of the top face **558** and bottom face **556** and fit into the guide recesses **510** on slider gripper base **504.** Alternatively, the guide projection **554** may be located on slider gripper base **504** or the guide rail(s) **508** of the slider gripper base **504** for movement along a guide groove **510** located on slider sled **551.** Other embodiments may use other guide configurations.

In some embodiments, the guide rail **508** may be hollow and the guide recess **510** may be a slot which is cut through the guide rail **508** and into the hollow portion of the guide rail **508.** The guide rail **508** may be open on one end and a compression spring **550** may be placed into the hollow portion of the guide rail **508** through this opening.

In relation to the second function, at least one of the guide projection(s) **554** on the slider sled **551** may feature a compression spring peg **552** on which one side of a compression spring **550** is seated. In one embodiment, the compression spring peg **552** is an essentially cylindrical structure with an end piece **575** that has a diameter greater than the diameter of its associated compression spring **550.** Movement of slider sled **551** relative to the sliding gripper base **504** compresses the compression spring **550** between the end piece **575** and the end wall of the hollow guide rail **508.** As the compression spring **550** is compressed, the compression spring peg **552** moves into the hollow of the guide rail(s) **508.** Such movement may occur when the clamp apparatus **510** is moved from the open position to the closed position and a clamped object **100** is present. Selection of a compression spring **550** of appropriate elasticity allows the restoring force generated during compression to be sufficient to return the sliding gripper **503** and sliding gripper base **504** to the open position, while at the same time not unduly opposing user actuation of the clamp apparatus **510.**

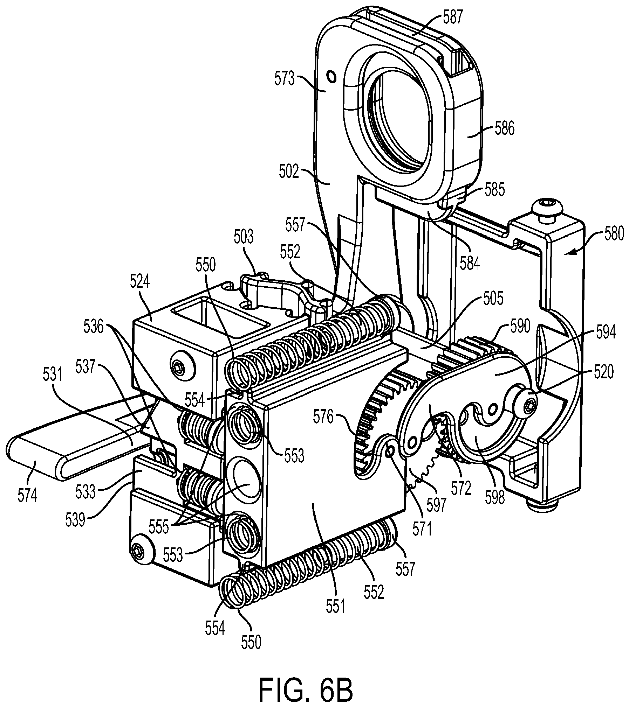

Relative to the third function, in some embodiments, the slider sled **551** may include at least one return spring **553** (best shown in FIG. 6B) which helps to bias the clamp apparatus **510** toward the open position. In the embodiment shown in **FIGS. 6A-6G****,** there are two return springs **553.** Each return spring **553** is seated in a return spring pocket **555** which has a diameter slightly larger than that of the return spring **553.** Each return spring pocket **555** is recessed into the left face (relative to FIG. 6B) of the slider sled **551.** One end of each return spring **553** abuts the bottom of its respective return spring pocket **555.** The opposite end of each return spring **533** abuts the inside of the right face **581** (relative to FIG. 6A) of the housing **580** of the clamp apparatus **510.** As the slider sled **551** is moved toward the right face **581** of the housing **580** when a user actuates the clamp apparatus **510** toward the closed position, the return springs **553** compress between the bottom of the return spring pockets **555** and the inside of the right face **581** of the housing **580.** When a user actuates the clamp apparatus **510** toward the open position, the restoring force exerted by the return springs **553** automatically returns the slider sled **551** to its open orientation.

In the embodiment illustrated in **FIGS. 6A-6G****,** there are three return spring pockets **555** yet only two return springs **553.** In some embodiments, including the illustrated embodiment, a user may add additional return springs **553** to the clamp apparatus **510** if such action is deemed desirable.

The fourth, catch function of the slider sled **551** requires a broader description of how a user may actuate the clamp apparatus **510.** As shown in **FIGS. 6A-6G****,** the clamp apparatus **510** may comprise an actuator handle **502.** User rotation of the actuator handle **502** may generate the force sufficient to actuate the clamp apparatus **510** toward the closed position. The actuator handle **502** is a roughly L-shaped structure comprised of a vertical arm **573** and a horizontal arm **574;** both arms merge at a substantially right angle. The actuator handle **502** comprises at least one means for a rotatably attaching the actuator handle **502** to the clamp apparatus **510.** In the example embodiment depicted in **FIGS. 6A-6G****,** the actuator handle **502** is coupled to a gear shaft **520** with a screw **576.** When the actuator handle **502** is rotated, the gear shaft **520** rotates about its axis.

At rest, the clamp apparatus **510** is biased to the open position. In the open position, the vertical arm 573 of the actuator handle **502** may point toward the bottom of the page as shown in FIG. **6A****.** The horizontal arm **574** may project toward the left of the page in a manner perpendicular to the vertical arm **573** of the actuator handle **502** as shown in FIG. 6A. To actuate the clamp apparatus **510** to the closed orientation, the actuator handle **502** must be rotated clockwise (in relation to FIG. 6A) substantially a full 180°.

In some embodiments, rotation of actuator handle **502** is converted to the linear motion propelling the sliding gripper **503** towards the fixed gripper 501. Thus, rotation of the actuator handle **502** closes the clamp apparatus 510. As mentioned above, rotation of the actuator handle **502** causes the rotation of a gear shaft **520.** In some embodiments, at least one cam gear **590** is driven by the rotation of the gear shaft **520.** Optionally, two or more cam gears **590** may be used to best accommodate the specific space and size needs of a particular embodiment of the clamp apparatus 510.

In the embodiment shown in FIGS. 6A-6G, the cam gear **590** is eccentrically attached to the gear shaft **520** at a distance "r" from the cam gear **590** center. In some embodiments an extension linkage **505** may project toward the center of the cam gear **590** from the gear shaft **520.** The extension linkage **505** may be coupled into the center of the cam gear **590** to help support rotation of the cam gear **590** as the actuator handle **502** is rotated. Over the approximately 180° of rotation of the actuator handle **502,** the cam gear **590** may displace a linear distance of approximately 2"r".

In the exemplary embodiment depicted in FIGS. 6A-6G, linear movement of the cam gear **590** is multiplied and imparted to the sliding gripper **503** through a linkage cam gear 597. The teeth of the linkage cam gear 597 and the teeth of the cam gear **590** interdigitate thus operatively coupling the cam gear **590** to the slider sled 551. In some embodiments, the linkage cam gear 597 is eccentrically coupled to the slider sled 551 at distance "r" from the center of the linkage cam gear 597. In the embodiment shown in FIGS. 6A-6G the linkage cam gear 597 is substantially a mirror image of the cam gear 590. Additionally, the movement of the linkage cam gear 597 mirrors the movement of the cam gear **590.** Consequentially, a 180° rotation of the actuator handle **502** creates a linear displacement of 4"r" in the slider sled 551. This causes the sliding gripper base 504 and sliding gripper **503** to displace toward the fixed gripper 501. If a clamped object 100 is present, the slider sled 551 and sliding gripper base **504** move as a unit only until the sliding gripper 503 contacts the clamped object 100. When the sliding gripper 503 contacts the clamped object 100. The compression springs 550 begin to compress per the above description.

In embodiments where a smaller degree of linear displacement may be desirable, either the cam gear 590 or linkage cam gear 597 may not be eccentrically coupled into the clamp apparatus. This would halve the linear displace of slider sled 551. Alternatively, the distance "r" could be increased or decreased to achieve a greater or lesser degree of displacement of the slider sled 551.

The fourth, stop function of the slider sled 551 may prevent the actuator handle 502 from being rotated past the fully open orientation. As best shown in FIG. 6B the slider sled 551 features a catch 571. The catch 571 may be a nub which projects into a claw shaped cutout 576 in the slider sled 551. Other suitable shaped cutouts may alternatively be used. The catch 571 catches a claw shaped prong 572 which extends off a thin disc 594 which is coupled to the center of the cam gear 590. The thin disc 594 may be coupled to the center of the linkage cam gear 597. The thin disc 594 may feature a semi-circle track 598 which the gear shaft 520 may extend through. As the actuator handle **502** is rotated the thin disc 594 and attached prong 572 follow the eccentric motion of the cam gear **590.** The position of the gear shaft **520** along the semi-circle track 598 also changes. In the closed position, the gear shaft **520** may be located at the right end of the semi-circle track 598 (relative to FIG. 6B). Also in the closed position, the prong 572 may not intrude into the catch 571 cutout. After 90° of actuator handle **502** rotation toward the open position, the gear shaft **520** is located at the lowest point in the arc of the semi-circle track 598. Consequently, the thin disc 594 and attached prong 572 are at the highest point in their travel and the prong 572 has entered the claw shaped cutout 576 above the nub catch 571. In the fully open position, the gear shaft **520** may be located at the left end of the semi-circle track 598. The prong 572 may fully protrude into the claw shaped cutout 576 and hook around the nub catch 571. In this position, the actuator handle **502** may not be further rotated toward the open direction because the catch 571 blocks any further movement of the prong 572. Additionally, further rotation of the actuator handle **502** is prohibited because the gear shaft **520** is at the end of the semi-circle track 598 and the thin disc 594 blocks any further travel.

In some embodiments, an actuator handle latch 584 functions to operatively prevent the actuator handle **502** from being rotated out of the locked position. The actuator handle latch 584 (best shown in FIG. 6G) may be a roughly rectangular, planar structure. There may be a hole through roughly the center of the actuator handle latch 584. The hole may be large enough to comfortably accommodate a user's finger. Relative to FIG. 6G, the top edge of the actuator handle latch 584 may comprise a latch compression spring peg 583 on which an actuator handle spring 592 may be seated. The bottom edge may comprise projections 585.

In some embodiments, the vertical arm 573 of the actuator handle **502** comprises a latch housing 586. As shown best in FIG. 6G, the latch housing 586 extends perpendicularly from the vertical arm 573 and over the top face 513 of the clamp apparatus 510. The latch housing 586 may comprise a channel 587 sized to fit the actuator handle latch 584, latch compression spring peg 583 actuator handle spring 592 and the projections 585. The channel 587 may be cut along the central plane of the latch housing 586 running perpendicular to the vertical arm 573. The channel 587 guides movement of the actuator handle latch 584. There may be a hole through roughly the center of the actuator latch housing 586 which is large enough to accommodate a user's finger.

The actuator handle latch 584 projects out of the actuator latch housing 586 and against the top face 513 of the housing **580.** A dowel 588 may run through the channel 587 above the actuator handle spring 592. The dowel 588 is disposed such that the actuator handle spring 592 may bias the actuator handle latch 584 against the top face of the housing **580.**

In the path of the actuator handle latch 584 a ramp 516 is disposed. As the actuator handle **502** is rotated toward the closed position, the actuator handle latch 584 abuts the ramp 516. As the actuator handle **502** continues to rotate toward the closed position, the actuator handle latch 584 rides up the ramp 516. This causes the actuator handle latch 584 to be pushed up the channel 587 and into the actuator latch housing 586 which in turn compresses the actuator handle spring 592 between the dowel 588 and the latch compression spring peg 583. When the actuator handle **502** is in the fully closed position, the actuator handle latch 584 clears the ramp 516 and the restoring force of the spring causes the actuator handle latch 584 to spring back against the top face 513 of the housing **580.** This locks the clamp apparatus 510 in the closed position as any movement toward the open position is prevented by the actuator handle latch 584 catching on the lip of the ramp 516. To release the clamp apparatus 510 from the locked position, a user may insert a finger into the hole in the actuator handle latch 584 and latch housing 586 and pull the actuator handle latch 584 back inside the actuator latch housing 586. This allows the actuator handle latch **584** to clear the lip of the ramp **516** thus allowing rotation of the actuator handle **502** toward the open position.

In some embodiments, the horizontal arm **574** of the actuator handle **502,** may also comprise a lock/latch feature **531.** This lock/latch feature **531** may be present in conjunction with or as a substitute for the actuator latch **584.** In embodiments where the horizontal arm **574** comprises a lock/latch feature **531,** the front face **532** of the clamp apparatus **510** housing **580** may comprise a slot **534** through which a spring loaded strike plate **533** protrudes. The strike plate **533** (best shown in **FIG. 6E****)** may be roughly planar. The bottom of the strike plate **533** (relative to **FIG. 6E****)** may comprise at least one strike plate spring peg **535** on which a strike plate spring **536** is seated. In the embodiment depicted in **FIGS. 6A-6G****,** there are two strike plate spring pegs **535** and two accompanying strike plate springs **536.** The strike plate springs **536** fit inside the strike plate spring bay **511** recessed into the fixed gripper base **524.** In some embodiments, the top edge of the strike plate **533** (relative to **FIG. 6E****)** may comprise a ramp portion **537,** a trough portion **538,** and a post portion **539.** The strike plate **533** protrudes from the slot **534.** The strike plate **533** may be pushed into the slot **534,** in the front face **532** of the housing **580** such that it does not protrude past the surface of the front face **532** of the housing **580.** In this position, the strike plate springs **536** are compressed between a portion of the strike plate spring bay **511** and the strike plate spring pegs **535.** This spring loads the strike plate **533** to automatically return to its protruding orientation.

As the actuator handle **502** is rotated to the closed position, the horizontal arm **574** of the actuator handle **502** contacts the ramp portion **537** of the strike plate **533.** As the horizontal arm **574** is further rotated, it moves to a more elevated section of the ramp portion **537.** Since the strike plate springs **536** are not strong enough to cause the horizontal arm **574** to deflect, the strike plate springs **536** compress and the strike plate **533** is pushed into the slot **534** to its non-protruding position. When the horizontal arm **574** passes the top of the ramp portion **537,** the restoring force of the strike plate springs **536,** causes the strike plate **533** to be pushed back toward its protruding position with the trough portion **538** abutting the horizontal arm **574.** This locks the clamp apparatus **510** in the closed position. In this locked position, the horizontal arm **574** cannot be further rotated toward the closed position because the post portion **539** of the strike plate **533** blocks such movement. Additionally, the horizontal arm may not progress toward the open position because it will abut and be restricted in movement by the lip of the ramp portion **537.** To unlock the clamp apparatus **510,** a user must depress the post portion of the strike plate **533** into the slot **534** and compress the strike plate springs **536.** This allows the horizontal arm **574** to clear the lip of the ramp **537** as a user rotates the actuator handle **502** toward the open position.

In some embodiments of the present disclosure, a quick release clip **519** may be used to secure a medical device or other object to the clamp apparatus **510.** The quick release clip **519** may comprise a torsion clip **522** and a latch hook **523.** In some embodiments of the present disclosure, at least one torsion spring **521** may be used to clip a load for the clamp apparatus **510** between the torsion clip **522** and the latch hook **523.** In the example embodiment shown in **FIGS. 6A-6G****,** two latch hooks **523** are firmly attached to the top face **513** of the housing **580.** The latch hooks **523** are offset from each other. The hook portions of the latch hooks **523** project toward the back of the page (relative to **FIG. 6A**)**.** The torsion clip **522** is pivotally attached to the latch hook **523** by a fastening means **525,** which may for example be a pin, dowel, cotter pin, bolt, hex bolt, screw, or other means known to one skilled in the art. As shown in **FIGS. 6A-6G****,** the torsion clip **522** may be a relatively planar member which spans the distance between the two latch hooks **523.** In some embodiments, at least one surface of torsion clip **522** may comprise a catch **526.** The catch **526** may act as a stop for a receiving structure on a medical device or other object. The torsion spring(s) **521** may supplement the catch **526** by biasing the receiving structure into contact with the latch hooks **523.** The latch hooks **523** may also couple to a receiving structure on a medical device or other object. Rotation of the torsion clip **522** downwards spring loads each torsion spring **521** so that the torsion clip **522** will automatic pivot to the closed position when released. This is desirable because it causes the quick release clip **519** to automatically adjust to a load, such as medical device or other object, regardless of the size of the receiving structure.

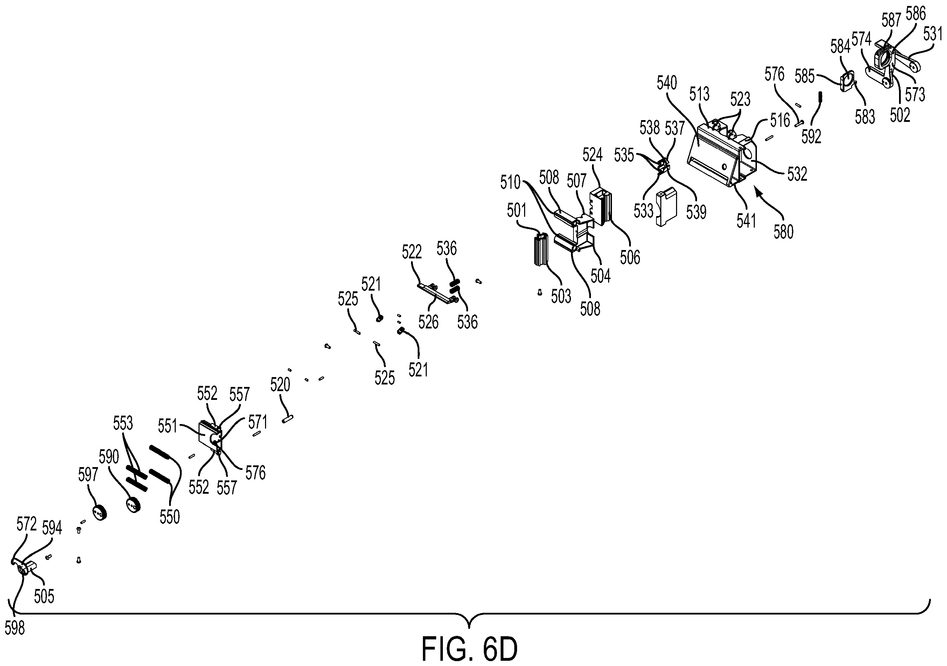

As best shown in **FIG. 6D****,** some embodiments may comprise a rest **540** for a medical device or other object which may be coupled to the clamp apparatus via the quick release clip **519.** As shown, the rest **540** may project at an angle from the top face **513** of the housing **580.** Extending perpendicularly from the bottom edge of the back face **512** of the housing **580** may be a rest support **541** for the rest **540.** The rest support **541** couples the back face **512** of the housing **580** to the rest **540.** Additionally, the rest **540** may have various features which help to hold the medical device or other object in place on the rest **540.**

The housing **580** or rest **540** may also feature any of a variety of mechanisms **515** (not shown) to attach a load to the clamp apparatus **510.** Such mechanisms **515** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

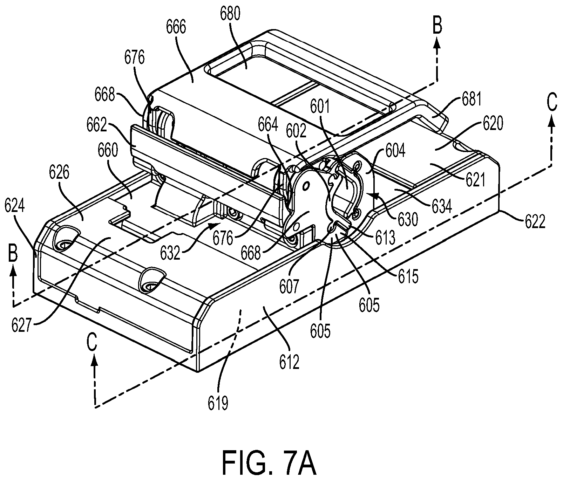

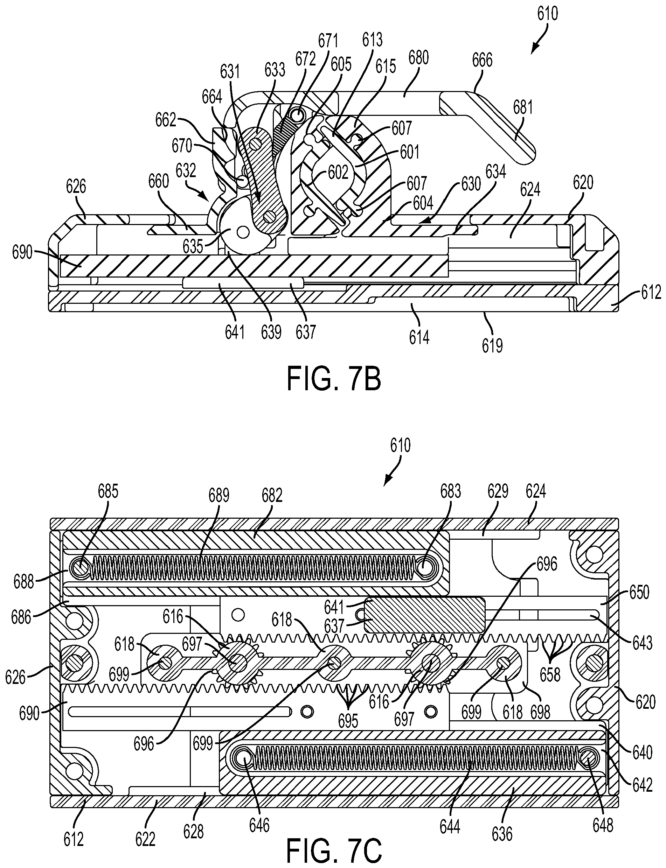

**FIGS. 7A-7D** show another embodiment of a clamp apparatus **610.** The clamp apparatus **610** comprises a first moving jaw **630** and a second moving jaw **632,** coupled to move in unison. A clamped object **100** may be clamped between the first moving jaw **630** and the second moving jaw **632** and clamped by the clamp apparatus **610.**

In some embodiments, the clamp apparatus **610** includes a housing **612.** As shown in **FIGS. 7A-7D****,** the housing **612** may be shaped like a rectangular tray. The bottom face **614** of the housing **612,** may be substantially planar. In some embodiments, the bottom face **614** of the housing **612** may have one or more gear attachment sites **616.** The bottom face may also have one or more raised posts **618.** The raised posts may comprise a hole sunk substantially into the center of the posts **618.** The hole may additionally be tapped to receive the thread of a screw. As shown in **FIG. 7A****,** the gear attachment sites **616** and the raised posts **618** may all be in line with each other. Also as shown, the gear attachment sites **616** and the raised posts **618,** may run along the center line of the bottom face **614** running parallel to the front wall **622** and back wall **624** of the housing **612.** The gear attachment sites **616** and raised posts **618** will be further elaborated upon later.

At least a portion of the housing **612** may also feature any of a variety of mechanisms **619** (not shown) to attach a load to the clamp apparatus **610.** Such mechanisms **619** may include, but are not limited to, brackets, magnets, straps, suction cups, hooks, screws or bolts, a friction fit, etc. This load could be any number of things, especially a medical device (such as an infusion pump, or peristaltic infusion pump), I.V. bag, etc.

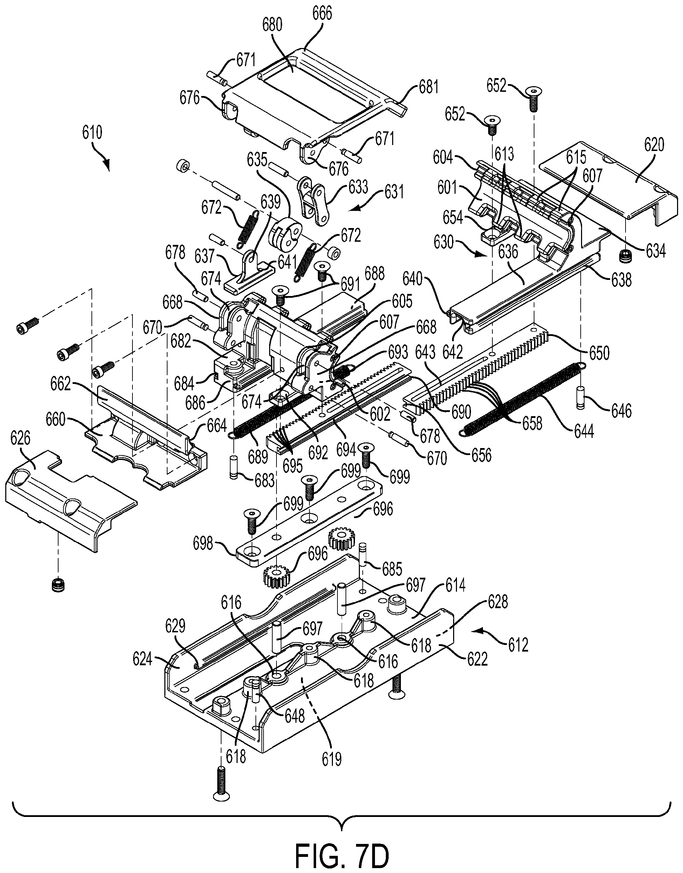

As mentioned above, the housing **612** may comprise a front wall **622** and a back wall **624.** Relative to **FIG. 7D****,** the front wall **622** projects toward the top of the page from the edge of the bottom face **614** which faces the front of the page. The front wall **622** projects substantially perpendicularly to the plane of the bottom face **614** of the housing **612.** The interior face of the front wall **622** may comprise a projecting track section **628** which runs parallel to the top and bottom edges of the front wall **622.** The back wall **624** projects toward the top of the page from the edge of the bottom face **614** of the housing **612** which faces the back of the page. The back wall **624** projects perpendicularly to the bottom face **614** of the housing **612.** The interior face of the back wall **624** may comprise a projecting track section **629** which runs parallel to the top and bottom edges of the back wall **624.**

In the embodiment shown in **FIGS. 7A-7D****,** the right side **620** and left side **626** of the housing **612** are detachable end caps. The right side **620** and left side **626** of the housing **612** may be coupled to the bottom face **614** of the housing **612** via screws, bolts, welds, or any other suitable means. In other embodiments, the right side **620** and left side **626** may be formed as a continuous part of the housing **612** during manufacture. The right side **620** of the housing **612** may have an overhanging flange **621** which overhangs a portion of the bottom face **614** of the housing **612.** Similarly, the left side **626** of the housing **612** may have an overhanging flange **627** which overhangs a portion of the bottom face **614** of the housing **612.**

In some embodiments, a first gripper **601** and a second gripper **602** are firmly attached to a first bracket **604** and a second bracket **606** respectively. The first bracket **604** and second bracket **606** respectively comprise a part of the first moving jaw **630** and second moving jaw **632.** In the example embodiment depicted in **FIG. 7A-7D****,** each of the first bracket **604** and second bracket **606** comprise friction fit features **607.** The friction fit features **607** allow the respective grippers **601** and **602** to be coupled to the first bracket **604** and second bracket **606.** In other embodiments, the grippers **601** and **602** may be coupled to the first bracket **604** and second bracket **606** by any number of coupling means including, but not limited to, screws, bolts, ultrasonic welds, magnets, adhesive, etc.

The first gripper **601** and second gripper **602** consists of a material chosen for its gripping ability. The first gripper **601** and second gripper **602** may be made of a high friction material, a compressible material, a material exhibiting both these qualities, or any other suitable material. The first gripper **601** and second gripper **602** are made of a material which allows a firm grip without the deformation of a clamped object **100.** Suitable materials may include any suitable elastomeric or non-deformable substance, including but not limited to plastic, rubber, metal, foam, fabric, gel, polyurethane, etc. At least a portion of the first gripper **601** and second gripper **602** may comprise roughly semi-circular depressions or contours to accommodate a round clamped object **100** such as a pole. The first gripper **601** and second gripper **602** may be replaceable.

In some embodiments, the first gripper **601** and second gripper **602** may comprise gripper teeth **613** which project from the top and bottom edges of the first gripper **601** and second gripper **602.** The gripper teeth **613** may be disposed about the first gripper **601** and second gripper **602** such that they may interdigitate with each other when the clamp apparatus **610** is in the closed position. The gripper teeth **613** allow the first gripper **601** and second gripper **602** to better encompass and hold a clamped object **100** when the clamp apparatus **610** in the closed position. The first bracket **604** and second bracket **606** may comprise bracket teeth **615** which support the gripper teeth **613** on the first gripper **601** and second gripper **602.** The bracket teeth **615** may be disposed about the first bracket **604** and second bracket **606** such that they interdigitate with each other similarly to the gripper teeth **613.**

The first bracket **604** may have a flange **634** which extends perpendicularly off the face of the first bracket **604** opposite the face to which the first gripper **601** is attached. The flange **634** is shaped and disposed such that it may slide under the overhanging flange **621** of the right side **620** of the housing **612.** A polygonal block **636** may be fixedly coupled to the bottom face of the first bracket **604** (relative to **FIG. 7D**). In the example embodiment depicted in **FIGS. 7A-7D****,** the polygonal block **636** is specifically a long, rectangular block. The short, right and left ends of the long, rectangular block run parallel to the right edge of the flange **634** of the first bracket **604.** The long sides of the rectangular block in the example embodiment shown in **FIGS. 7A-7D****,** extend for roughly seventy-five percent of the length of the front wall **622** of the housing **612.** This may differ in alternate embodiments. The first bracket **604,** first gripper **601,** flange **634,** and polygonal block **636** collectively may comprise the first moving jaw **630.**

One side of the polygonal block **636** may abut the interior face of the front wall **622.** The side of the polygonal block **636** which abuts the interior face of the front wall **622** may include a recessed groove **638** which accepts the projecting track section **628** on the interior face of the front wall **622.** The projecting track section **628** operatively functions as a guide to inform the movement of the first moving jaw **630.**

The side of the polygonal block **636** opposite the recessed groove **638** may include a projecting jaw track section **640.** The projecting jaw track section **640** runs substantially parallel to the recessed groove **638.** The bottom of the polygonal block **636** may comprise an extension spring trough **642** which is sunk into the bottom face of the polygonal block **636.** The extension spring trough **642** also runs parallel to both the recessed groove **638** and projecting jaw track section **640.** The bottom of the polygonal block **636** may abut the bottom face **614** of the housing **612.**

A first extension spring **644** may be placed in the extension spring trough **642.** As shown in the embodiment in **FIGS. 7A-7D****,** the right end (relative to **FIG. 7D**) of the extension spring **644** may be coupled into the extension spring trough **642** by a first extension spring peg **646.** The left end of the extension spring **644** may be coupled to the bottom face **614** of the housing **612** by a second extension spring peg **648.** The first extension spring **644** biases the first moving jaw **630** toward the closed position. Moving the first moving jaw **630** from the closed position to the open position extends the first extension spring **644.** The restoring force from the first extension spring **644** will automatically cause the first moving jaw **630** to return to the closed position. When a clamped object **100** is present, the restoring force of the first extension spring **644** will cause the first moving jaw **630** to press the first gripper **601** into the clamped object **100,** automatically adjusting to the size of girth of the clamped object **100.**

In some embodiments, including the embodiment shown in **FIGS. 7A-7D****,** a first rack **650** may additionally be coupled to the bottom of the first moving jaw **630.** As shown, the first rack **650** is coupled to the first moving jaw **630** via two screws **652.** One screw **652** couples the first rack **650** to the first moving jaw **630** via a screw hole in the flange **634.** As shown, the first moving jaw **630** may further comprise a coupling ledge **654** which projects along the plane of the bottom of the first bracket **604.** The coupling ledge **654** projects toward the left of the page relative to **FIG. 7D****.** The second screw **652** couples the first rack **650** to the first moving jaw **630** through a screw hole in the coupling ledge **654.**

As shown in **FIGS. 7A-7D****,** the first rack **650** has a rack groove **656** recessed into the face of the first rack **650** which faces the back of the page relative to **FIG. 7D****.** The face opposite the rack groove **656** comprises a number of rack teeth **658.**

The second moving jaw **632** may be generally similar to the first moving jaw **630.** In the embodiment shown in **FIGS. 7A-7D****,** the second moving jaw **632** is similar to the first moving jaw **630** although it comprises some additional or different components. The second bracket **606** may comprise a second flange **660** which extends perpendicularly off the face of the second bracket **606** opposite the face to which the second gripper **603** is attached. As shown in **FIGS. 7A-7D****,** the second flange **660** may be detachable. In embodiments where the second flange **660** may be detachable, the second flange **660** may be coupled to the second bracket **606** via screws, bolts, magnets, adhesive, etc.

The second flange **660** may comprise a handle mechanism cover **662.** The handle mechanism cover **662** may be raised off the second flange **660** toward the top of the page. At least one section of the handle mechanism cover **662** may comprise an arcuated segment **664** which faces a pivoting handle **666.** The arcuated segment **664** allows the pivoting handle **666** to rotate. The handle mechanism cover **662** helps to keep foreign material and debris from getting inside the clamp apparatus **610.** The handle mechanism cover **662** does not abut the second bracket **606.** The handle mechanism cover **662** is offset from the second bracket **606** toward the left of the page relative to **FIG. 7D****.** The void created between the second bracket **606** and the handle mechanism cover **662** allows various linkages to couple the pivoting handle **666** to the inner workings of the clamp apparatus **610.**

The second bracket **606** may additionally comprise wings **668** which project off the front and back edges of the second bracket **606** toward the handle mechanism cover **662.** In the embodiment shown in **FIGS. 7A-7D****,** the wings **668** are not coupled to the handle mechanism cover **662.** A handle spring peg **670** extends through the bottom of each wing **668.** The handle spring pegs **670** protrude into the void between the second bracket **606** and the handle mechanism cover **662.** One end of a handle extension spring **672** may be placed around each handle spring peg **670.**

As shown in the embodiment depicted in **FIGS. 7A-7D** a slit **674** is recessed into the each wing **668** on a plane parallel to the front wall **622** and back wall **624** of the housing **612.** The slit **672** may effectively make the top portion of each wing **668** into a coupling bracket to which fins **676** projecting off the pivoting handle **666** may be inserted. A dowel **678** may run through each wing **668** into the slits **674** and through the fins **676** of the pivoting handle **666.** The dowels **678** pivotally couple the pivoting handle **666** to the wings **668** of the second bracket **606.** The dowels **678** act as the pivot axis for the pivoting handle **666.**

The fins **676** of the pivoting handle **666** may also comprise a hole through which a second set of handle spring pegs **671** may extend. The second set of handle spring pegs **671** may protrude into the void between the second bracket **606** and the handle mechanism cover **662.** The end of each handle extension spring **672** not connected to the first set of handle spring pegs **670** is connected to the second set of handle spring pegs **671.** The handle extension spring **672** thus acts as an over-center linkage and helps keep the pivoting handle **666** in the closed position if the pivoting handle **666** is in the closed position and helps keep the pivoting handle **666** in the open position if the pivoting handle **666** is in the open position.

In the example embodiments shown in **FIGS. 7A-7D****,** the pivoting handle **666** extends toward the right of the page. In some embodiments, including those displayed in **FIGS. 7A-7D****,** the pivoting handle **666** comprises an open section **680** through which a user may place their fingers. The open section **680** of the pivoting handle may be included to allow a user to grasp the pivoting handle **666** more easily. The pivoting handle may also comprise a bent or arced section **681.** Again, the bent or arced section of the pivoting handle **666** may make it easier for a user to grasp the pivoting handle **666.**

A portion of the bent or arced section **681** of the pivoting handle **666** may be made of the same material as the rest of the pivoting handle **666,** may be made of a different material, or may be made of a combination thereof. Possible materials may include, but are not limited to, rubber, polymer, composite, metal, plastic, foam, etc. Additionally, the bent or arced section **681** may comprise ergonomic finger groves, nubs, a ribbed texture, a honeycombed texture, etc. to afford a user greater ease of use.

The second moving jaw **632** may additionally comprise a second polygonal block **682.** The second polygonal block **682** may be fixedly coupled to the bottom face of the second bracket **606** (relative to **FIG. 7D**)**.** In the example embodiment depicted in **FIGS. 7A-7D****,** the second polygonal block **682** is specifically a long, rectangular block. The short, right and left ends of the long, rectangular block run perpendicular to the planes of the front wall **622** and back wall **624** of the housing **612.** The long sides of the rectangular block in the example embodiment shown in **FIGS. 7A-7D****,** extend for roughly seventy-five percent of the length of the back wall **624** of the housing **612.** This may differ in alternate embodiments.

One side of the second polygonal block **682** may abut the interior face of the back wall **624.** The side of the second polygonal block **682** which abuts the interior face of the back wall **624** may include a recessed groove **684** which accepts the projecting track section **629** on the interior face of the back wall **624.** The projecting track section **629** operatively functions as a guide to inform the movement of the second moving jaw **632.**

The side of the second polygonal block **682** opposite the recessed groove **684** may include a projecting second jaw track section **686.** The projecting second jaw track section **686** runs substantially parallel to the recessed groove **684.** The bottom of the second polygonal block **682** may comprise a second extension spring trough **688** which is sunk into the bottom face of the second polygonal block **682.** The extension spring trough **688** also runs parallel to both the recessed groove **684** and projecting second jaw track section **686.** The bottom of the second polygonal block **682** may abut the bottom face **614** of the housing **612.**

A second extension spring **689** may be placed in the extension spring trough **688.** As shown in the embodiment in **FIGS. 7A-7D****,** the left end (relative to **FIG. 7D****)** of the second extension spring **689** may be coupled into the extension spring trough **688** by a third extension spring peg **683.** The right end of the extension spring **689** may be coupled to the bottom face **614** of the housing **612** by a fourth extension spring peg **685.** The second extension spring **689** biases the second moving jaw **632** toward the closed position. Moving the second moving jaw **632** from the closed position to the open position extends the second extension spring **689.** The restoring force from the second extension spring **689** will automatically cause the second moving jaw **632** to return to the closed position. When a clamped object **100** is present, the restoring force of the second extension spring **689** will cause the second moving jaw **632** to press the second gripper **603** into the clamped object **100,** automatically adjusting to the size or girth of the clamped object **100.**

In some embodiments, including the embodiment shown in **FIGS. 7A-7D****,** a second rack **690** may additionally be coupled to the bottom of the second moving jaw **632.** As shown, the second rack **690** is coupled to the second moving jaw **632** via two screws **691.** One screw **691** couples the second rack **690** to the second moving jaw **632** via a screw hole in a ledge **692** which projects under the second flange **660.** As shown, the second moving jaw **630** may further comprise an additional ledge **693** which projects along the plane of the bottom of the second bracket **606.** The additional ledge **693** projects toward the right of the page relative to **FIG. 7D****.** The second screw **691** couples the second rack **690** to the second moving jaw **632** through a screw hole in the additional ledge **693.**

As shown in **FIGS. 7A-7D****,** the second rack **690** has a second rack groove **694** recessed into the face of the second rack **690** which faces the front of the page relative to **FIG. 7D****.** The face opposite the second rack groove **694** comprises a number of second rack teeth **695.**

When the clamp apparatus **610** is assembled, the second rack groove **694** fits around and is guided by the projecting jaw track section **640** coupled to the first moving jaw **630.** Similarly the rack groove **656** fits around and is guided by the projecting second jaw track section **686.** The first rack teeth **658** and the second rack teeth **695** face each other. The first rack **650** and second rack **690** run substantially parallel to each other. The first rack teeth **568** and second rack teeth **695** mesh with teeth on opposite sides of at least one pinion gear **696.** The at least one pinion gear **696** may be placed on a gear shaft **697** which runs into the at least one gear attachment site **616** described earlier in the specification. In the embodiment depicted in **FIGS. 7A-7D****,** two pinion gears **696** are present. Each pinion gear **696** is placed on its own gear shaft **697** which in turn runs into its own gear attachment site **616** located on the bottom face **614** of the housing **612.** To ensure the pinion gears **696** do not stray off their associated gear shafts **697,** the pinion gears **696** may be sandwiched against the back face **614** of the housing **612** by a bar-like plate **698.** The bar-like plate **698** is coupled to the raised posts **618** which project off the back face **614** of the housing **612** via screws **699.**

Since both the first rack **650** and the second rack **690** mesh with the same pinion gear(s) **696** on opposite sides of said pinion gear(s) **696,** any movement of either the first moving jaw **630** or the second moving jaw **632** necessitates movement of the other moving jaw in the opposite direction. If one moving jaw is pulled to the open position, the other moving jaw must then also move to the open position. If one moving jaw retracts toward the closed position, the other moving jaw must then also retract toward the closed position.

The clamp apparatus **610** additionally comprises a tightening/locking mechanism **631.** The tightening/locking mechanism **631** may comprise a number of components. In the embodiment depicted in **FIGS. 7A-7D****,** the tightening/locking mechanism **631** comprises a linkage **633,** a cam **635,** and a cincher **637.** The cincher **637** may comprise a post **639** and a flat plate **641.** The tightening/locking mechanism **631** may be disposed in the void between the handle mechanism cover **662** and the gripper bracket **605.** The linkage **633** is pivotally coupled on one end to the pivoting handle **666.** The linkage **633** may be pivotally coupled to the pivoting handle **666** by any means known to one skilled in the art. The other end of the linkage **633** is pivotally coupled to an end of the cam **635.** The other end of the cam **635** may comprise a slot which accepts the post **639** of the cincher **637.** The cam **635** may be pivotally coupled to the post **639** of the cincher **637** by any means known to one skilled in the art. In the example embodiment, the post **639** of the cincher **637** projects perpendicularly from the flat plate **641** of the cincher **637.**

The flat plate **641** of the cincher **637** is disposed under the rack **650** of the first moving gripper **630** when the clamp apparatus **610** is fully assembled. The post **639** of the cincher **637** projects up through a channel **643** which is cut out of the rack **650.** The channel **643** may not run the entire length of the rack **650.**

In the embodiment shown in **FIGS. 7A-7D****,** as the pivoting handle **666** is pivoted from the open position to the closed position, the linkage **633** also moves. Movement of the linkage **633** causes the cam **635** to rotate. Rotation of the cam **635** causes the cincher **637** to experience linear displacement along the channel **643** of the rack **650.** Since the channel **643** does not run the entire length of the rack **650,** the post **639** of the cincher **637** abuts the end of the channel **643** and begins to cause linear displacement of the rack **650.** Linear displacement of the rack **650** causes both the first moving jaw **630** and second moving jaw **632** to move, cinch down on, and clamp harder on a clamped object **100.** In the embodiment shown in **FIGS. 7A-7D****,** the linkage **633** is also an over-center linkage. When the pivoting handle **666** moves all the way to the closed position, the linkage **633** assumes an over-center position. When the linkage **633** assumes this over-center position, the clamp apparatus **610** is effectively locked.

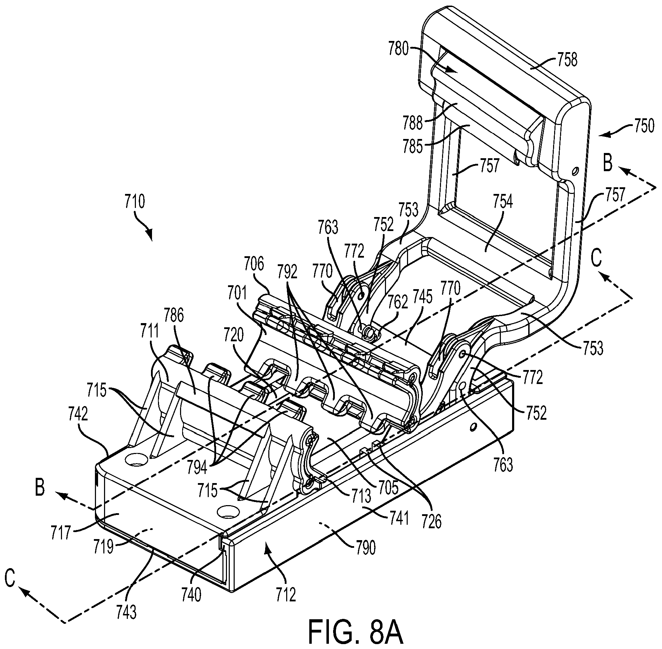

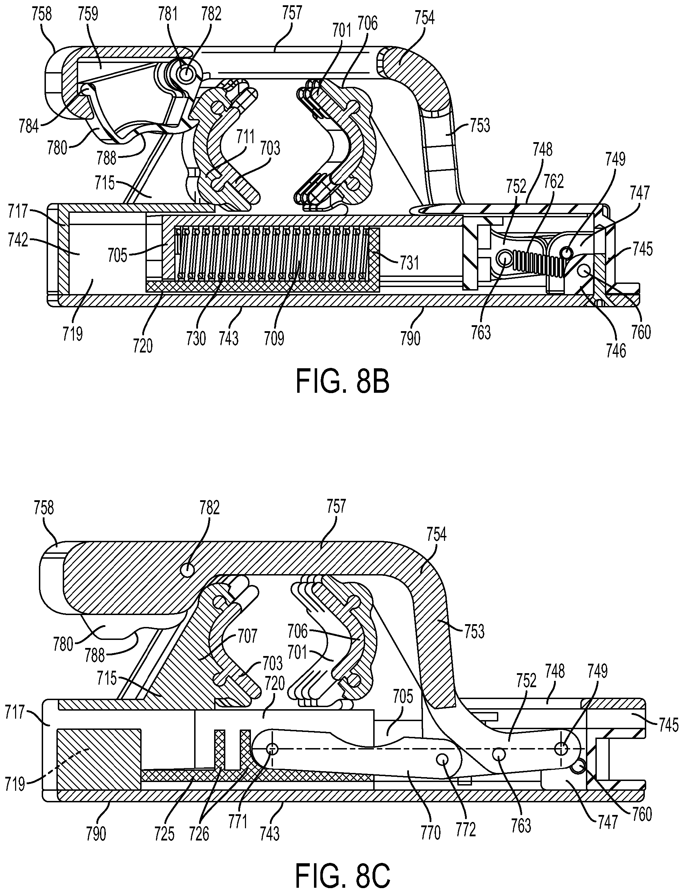

**FIGS. 8A-8D** show another example embodiment of a clamp apparatus **710.** In the clamp apparatus **710** shown in **FIGS. 8A-8D****,** a user rotates a toggle handle **750** to provide the force needed to propel a movable gripper assembly **704** towards a fixed gripper assembly **703** via at least one linkage **770** which may be an over-center linkage.

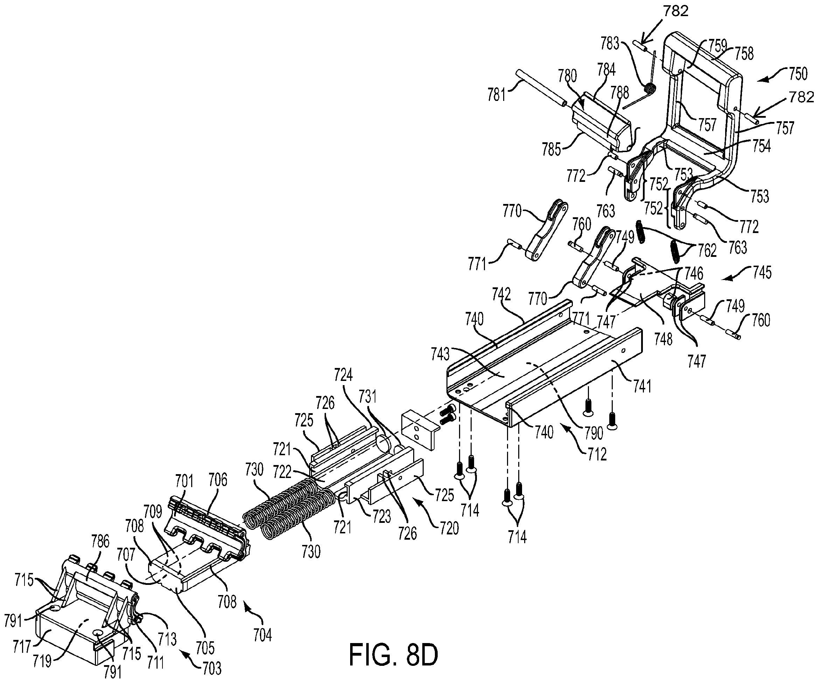

In some embodiments, such as the embodiment shown in **FIGS. 8A-8D****,** the fixed gripper assembly **703** comprises a fixed gripper cradle **711,** a fixed gripper **713,** and a fixed gripper base **717.** The fixed gripper cradle **711** extends off the top face of the fixed gripper base **717.** More specifically, the fixed gripper cradle **711** extends from the right edge (relative to **FIG. 8D****)** of the fixed gripper base **717** at an angle roughly perpendicular to the top face of the fixed gripper base **717** and is fixedly coupled to the fixed gripper base **717.**

A fixed gripper **713** is coupled to the face of the fixed gripper cradle **711** which faces the movable gripper assembly **704.** The fixed gripper **713** may be coupled to the fixed gripper cradle **711** by any of a variety of coupling means including, but not limited to, screws, bolts, magnets, adhesive, ultrasonic welds, snap fit, friction fit. In some embodiments the fixed gripper **713** may be overmolded onto the fixed gripper cradle **711.**

The fixed gripper base **717** may be a roughly rectangular block as shown in **FIGS. 8A-****8D.** The fixed gripper base **717** may comprise a cavity **719** which is dimensioned to fit and surround the gripper sled **705** when the clamp apparatus **710** is in the closed orientation. The fixed gripper base **717** may also comprise at least one buttress **715** which helps to support the fixed gripper cradle **711.** The fixed gripper base **717** may comprise one or a number of threaded holes **791.** In the embodiment depicted in **FIGS. 8A-8D****,** four screws **714** run through the housing **712** of the clamp apparatus **710** and into corresponding threaded holes **791** in bottom of the fixed gripper base **717.** The four screws **714** couple the fixed gripper base **717** to the housing 712. In alternate embodiments, different coupling methods may be employed including, bolts, welds, magnets, adhesive, and any other coupling method known to one skilled in the art. The fixed gripper base **717** may alternatively be a continuous part of the housing **712.**

In some embodiments, including the embodiment shown in **FIGS. 8A-8D****,** the movable gripper assembly **704** comprises a movable gripper cradle **706,** movable gripper **701,** and a gripper sled **705.** As shown in **FIGS. 8A-8D****,** the movable gripper cradle **706** extends off the top face of a gripper sled **705.** More specifically, the movable gripper cradle **706** extends from the right edge (relative to **FIG. 8D****)** of the gripper sled **705** at an angle roughly perpendicular to the top face of the gripper sled **705** and is fixedly coupled to the gripper sled **705.** This may differ in alternate embodiments.

A movable gripper **701** is coupled to the face of the movable gripper cradle **706** which faces the fixed gripper assembly **703.** The movable gripper **701** may be coupled to the movable gripper cradle **706** by any of a variety of coupling means including, but not limited to, screws, bolts, magnets, adhesive, ultrasonic welds, snap fit, friction fit.

The movable gripper **701** and fixed gripper **713** may consist of a material chosen for its gripping ability. The movable gripper **701** and fixed gripper **713** may be made of a high friction material, a compressible material, a material exhibiting both these qualities, or any other suitable material. The movable gripper **701** and fixed gripper **713** are made of a material which allows for a firm grip without the deformation of a clamped object **100.** Suitable materials may include any suitable elastomeric or non-deformable substance, including but not limited to plastic, rubber, metal, foam, fabric, gel, etc. At least a portion of the movable gripper **701** and fixed gripper **713** may comprise roughly semi-circular depressions or contours to accommodate a round clamped object **100** such as a pole. The movable gripper **701** and fixed gripper **713** may be replaceable.

In some embodiments, the movable gripper **701** and fixed gripper **713** may comprise gripper teeth **792** (As shown in **FIG. 8A****)** which project from the top and bottom edges of the movable gripper **701** and fixed gripper **713.** The gripper teeth **792** may be disposed about the movable gripper **701** and fixed gripper **713** such that they may interdigitate with each other when the clamp apparatus **710** is in the closed position. The gripper teeth **792** allow the movable gripper **701** and fixed gripper **713** to hold an increased range of clamped object **100** when the clamp apparatus **710** is in the closed position. By disposing the gripper teeth **794** such that they may interdigitate, the movable gripper **701** may move further toward the closed position. The movable gripper cradle **706** and the fixed gripper cradle **711** may comprise cradle teeth **794** which support the gripper teeth **792** on the movable gripper **701** and fixed gripper **713.** The cradle teeth **794** may be disposed about the movable gripper cradle **706** and the fixed gripper cradle **711** such that they interdigitate with each other similarly to the gripper teeth **792.**

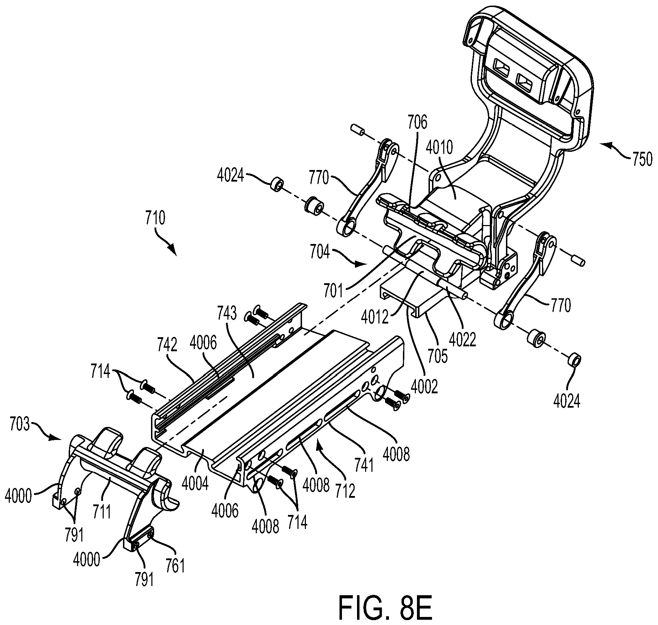

As illustrated in the example embodiment in **FIGS. 8A-8D****,** the gripper sled **705** may be roughly rectangular. The gripper sled **705** may be substantially hollow and open to the hollow on one end. In **FIGS. 8A-8D****,** the gripper sled **705** is hollow, except for a dividing wall **707** (relative to **FIG. 8D****)** which extends from the interior bottom face of the hollow to the interior top face of the hollow. The dividing wall **707** divides the hollow portion of the gripper sled **705** into two spring bays **709** which are roughly equally dimensioned. The gripper sled **705** in **FIGS. 8A-8D** is open to the hollow on its right end (relative to **FIG. 8D****).** In the embodiment shown in **FIGS. 8A-8D** a spring **730** is seated in each of the spring bays **709.** The spring **730** is a compression spring **730.** In a preferred embodiment, the clamp apparatus **710** may be adapted to fit at least one constant force spring **4012** instead of or in addition to the compression spring **730.** Constant force springs **4012** may be used in other embodiments such as but not limited to those detailed above. Using a constant force spring **4012** is preferable because it may make the clamp apparatus **710** easier to operate, especially when it is being used to clamp a large/thick object. It may also allow the clamp apparatus **710** to be made more compactly. An alternative embodiment comprising a constant force spring **4012** is shown in **FIGS. 8E-8F****.**

The gripper sled **705** may also comprise sled projecting tracks **708** on its front and back faces (relative to orientation in **FIG. 8D****).** The sled projecting tracks **708** fit into guide grooves **721** on a driven member **720.** In the example embodiment shown in **FIGS. 8A-8D****,** the driven member **720** is roughly "U" shaped. The bottom face **722** of the driven member **720** comprises the bottom span of the "U" shape. Projecting perpendicularly from front and back edges (relative to **FIG. 8D****)** of the bottom face **722** of the driven member **720** toward the top of the page are a front upright wall **723** and a back upright wall **724.** The front upright wall **723** and back upright wall **724** comprise the upright spans of the "U" shape. The guide grooves **721** run along the surfaces of the front upright wall **723** and back upright wall **724** which face each other.

In some embodiments, the driven member **720** may comprise at least one appendage **725** which extends from either the front upright wall **723** or back upright wall **724.** In the exemplary embodiment illustrated in **FIGS. 8A-8D****,** the driven member **720,** includes two appendages **725.** One appendage **725** extends from the face of the front upright wall **723** opposite the face on which the guide groove **721** of the front upright wall **723** is disposed. The other appendage extends from the face of the back upright wall **724** opposite the face on which the guide groove **721** of the back upright wall **724** is disposed.

The appendages **725** are roughly "L" shaped. One portion of each appendage **725** projects from its corresponding front upright wall **723** or back upright wall **724** at an angle substantially perpendicular to the front upright wall **723** and back upright wall **724.** This portion of each appendage **725** comprises the horizontal span of the "L" shape. The vertical span of the "L" shape is formed by a second portion of the appendage **725** which projects toward the top of the page from the distal end of the first portion of the appendage **725** at an angle substantially perpendicular to the first portion of the appendage **725.** As shown in **FIGS. 8A-8D** the one or more appendages may be buttressed by at least one support piece **726.** In some embodiments, including the embodiment shown in **FIGS. 8A-8D****,** the one or more appendages may not span the entire length of the front upright wall **723** and back upright wall **724** of the driven member **720.** In the shown embodiment, the appendages stop short of the left edge (relative to **FIG. 8D****)** of the driven member **720.**

The appendages **725** or a portion of the appendages **725** may fit into and slide along a grooved track **740** on front wall **741** and back wall **742** the housing **712.** The bottom of the driven member **720** may ride along the bottom face **743** of the housing **712.**

When the clamp apparatus **710** is assembled, the gripper sled **705** fits in the driven member **720** between the front upright wall **723** and back upright wall **724.** When the clamp apparatus **710** is not clamped around a clamped object **100** the gripper sled **705** fits in the driven member **720** such that the right and left faces (relative to **FIG. 8D****)** of the gripper sled **705** are flush with the right and left edges of the driven member **720.** One end of each compression spring **730** abuts the interior left face (relative to **FIG. 8D****)** of the hollow portion of the gripper sled **705.** The other end of each compression spring **730** abuts a compression spring disc **731** which projects toward the top of the page from the right edge (relative to **FIG. 8D****)** of the driven member **720.** The compression springs **730** bias the gripper sled **705** to the unclamped position where the gripper sled **705** is flush with the right and left edges (relative to **FIG. 8D****)** of the driven member **720.**

When the clamp apparatus **710** is actuated from the open position to a clamped position the driven member **720** moves toward the fixed gripper assembly **703** and the appendages **725** of the driven member **720** slide along the grooved tracks **740** on the housing **712.** In turn, this displaces the movable gripper assembly **704** toward the fixed gripper **703** assembly. Until the movable gripper **701** contacts a clamped object **100,** the driven member **720** and movable gripper assembly **703** move as a unit. When the movable gripper **701** comes into contact with a clamped object **100,** the movable gripper assembly **704** can make no further progress toward the fixed gripper assembly **703** because the clamped object **100** is in the way. The driven member **720** continues to move toward the fixed gripper assembly **703** compressing the compression springs **730** between the interior left wall (relative to **FIG. 8D****)** of the hollow portion of the gripper sled **705** and the compression spring discs **731.** The restoring force of the compression springs **730** causes the movable gripper assembly **704** to exert a more vigorous clamping force on the clamped object **100.**

When the clamp apparatus **710** is moved from a clamped position toward an open position, the restoring force of the compression springs **730** may automatically spring the clamp apparatus **710** back to the unclamped and open position.

The clamp apparatus **710** may be moved from the open position to the closed position by user actuation of a toggle handle **750.** One end of the toggle handle **750** may be pivotally coupled to the housing **712** of the clamp apparatus **710.** In the embodiment shown in **FIGS 8A-****8D,** the toggle handle **750** attaches to the right (relative to **FIG. 8D****)** end cap **745** of the housing **712.** As shown, the right end cap **745** projects perpendicularly from the bottom face **743** of the housing **712** toward the top of the page. The right end cap **745** may be fixedly coupled to the housing **712** via screws, bolts, welds, etc. or may be molded as a continuous part of the housing **712.**

The right end cap **745** may comprise a number of other features. As shown in **FIGS. 8A-8D****,** the right end cap **745** may comprise a pair of projections **746** which project toward the fixed gripper assembly **703.** The projection **746** may extend parallel to the front wall **741** and back wall **742** of the housing **712.** Extension spring pegs **760** may protrude from each of the pair of projections **746.** In the embodiment depicted in **FIGS. 8A-8D****,** each of the extension spring pegs **760** project substantially perpendicularly from one of the pair of projections **746.** One end of an extension spring **762** is placed around each extension spring peg **760.** The extension springs **762** will be elaborated upon later.

Extending from the top edge of the right end cap **745** toward the fixed gripper assembly **703** may be a guide piece **748.** The guide piece **748** may extend parallel to the plane of the bottom face **743** of the housing **712.** The guide piece **748** may overhang the bottom face **743** of the housing **712.** As shown, the guide piece **748** in **FIGS. 8A-8D****,** may only extend from the medial section of the top edge of the right end cap **745.**

The right end cap **745** may also comprise a pair of U-brackets **747.** In the embodiment shown, the U-brackets **747** are disposed on the right end cap **745** such that the uprights of each U-bracket **747** project in the same direction and plane as the pair of projections **746.** One of the upright sections of one U-bracket **747** may be flush with the front edge of the right end cap **745** and abut the interior face of the front wall when the clamp apparatus **710** is assembled. One of the upright sections of the other U-bracket **747** may be flush with the back edge of the right end cap **745** and abut the interior face of the back wall **742** of the housing **712** when the clamp apparatus **710** is assembled. The other upright of each U-bracket **747** may be offset from the first upright of each U-bracket **747** such that it nearly abuts the extension spring pegs **760.** The bottom span of the U-bracket **747** may be formed by a face of the right end cap **745.** In alternate embodiments, the number, location, and orientation of projections **746,** U-brackets **747,** extension spring pegs **760,** and extension springs **762** may differ.

In the embodiment shown in **FIGS. 8A-8D****,** the toggle handle **750** is pivotally coupled into the U-brackets **747.** As shown, this is accomplished by means of dowel pins **749** which run through the U-brackets **747** and into the coupling spans **752** of the toggle handle **750.** The toggle handle **750** in the exemplary embodiment may be divided up into a number of sections. As indicated above, the toggle clamp may have one or more coupling spans **752** to which other components of the clamp apparatus **710** may be coupled. Relative to **FIG. 8D****,** the coupling spans **752** are two vertical spans. As shown, the coupling spans **752** are offset from each other. Extending toward the right of the page from the each coupling span **752** at an angle roughly perpendicular to each coupling span **752** may be a horizontal span **753.** The horizontal spans **753** may be joined by a strut **754.** In some embodiments, the strut **754** may complete the toggle handle **750.** In the illustrated embodiment in **FIGS. 8A-8D****,** the toggle handle **750** comprises additional sections. Projecting off the strut **754** vertically toward the top of the page (relative to **FIG. 8D****)** are two extension spans **757.** The extension spans **757** may be connected together by a handle grip **758** which a user may grasp when actuating the toggle handle **750.**

At least a portion of the handle grip **758** may be made of the same material as the rest of the toggle handle **750,** may be made of a different material, or may be made of a combination thereof. Possible materials may include, but are not limited to, rubber, polymer, composite, metal, plastic, foam, fabric, etc. Additionally, the handle grip **758** may comprise ergonomic finger groves, nubs, a ribbed texture, a honeycombed texture, etc. to facilitate ease of grasping and gripping.

In addition to the coupling spans **752** coupling the toggle handle **750** to the clamp apparatus **710,** the coupling spans **752** may also comprise a pair of handle extension spring pegs **763.** In the example embodiment shown in **FIGS. 8A-8D****,** one of the pair of handle extension spring pegs **763** projects perpendicularly from each coupling span **752** of the toggle handle **750.** In the example embodiment in **FIGS. 8A-8D****,** the handle extension spring pegs **763** project from the surface of each coupling span **752** which faces the opposite coupling span **752.** The end of the each extension spring **762** not seated on the first pair of extension spring pegs **760** is seated around one of the pair of handle extension spring pegs **763.** In the example embodiment in **FIGS. 8A-8D****,** the extension springs **762** act as over-center springs. When the toggle handle **750** is in the open position, the extension springs **762** bias the toggle handle **750** to stay in the open position. When the toggle handle **750** is in the closed position, the extension springs **762** move to an over-center position and bias the toggle handle **750** to stay in the closed position.

The coupling spans **752** of the toggle handle **750** may additionally couple to linkages **770.** In the example embodiment in **FIG. 8A-8D****,** one end of each linkage **770** is pivotally coupled to the driven member **720.** As shown, one linkage **770** is pivotally coupled between the front upright wall **723** of the driven member **720** and the vertical span of the appendage 725 which extends off the front upright wall **723** of the driven member **720.** Also as shown in **FIGS. 8A-8D****,** the other linkage **770** is pivotally coupled between the back upright wall **724** of the driven member **720** and the vertical span of the appendage **725** which extends off the back upright wall **724** of the driven member **720.** In the example embodiment in **FIGS. 8A-8D****,** a dowel **771** is used to pivotally couple the linkages **770** to the driven member **720.**

The other end of each linkage **770** pivotally couples to the top of one of the coupling spans **752** of the toggle handle **750.** The linkage **770** and coupling spans **752** may be pivotally coupled by means of a coupling dowel pin **772.** Any other suitable coupling means may also be used.

When the clamp apparatus **710** is actuated, the coupling span **752** of the toggle handle **750** and the linkages **770** collectively may act as an over-center linkage. To actuate the toggle handle **750** a user may grasp the handle grip **758** of toggle handle **750.** The user may then rotate the toggle handle **750** substantially a full 90° counter-clockwise from the orientation of the handle toggle handle **750** shown in **FIG. 8A****.** In some embodiments, the sufficient degree of rotation may be larger or smaller (e.g. 95°). As the toggle handle **750** is rotated, the linkage **770** and coupling span **752** which comprise the over-center linkage move toward the center position. This pushes the driven member **720** and movable gripper assembly **704** as detailed above. Slightly before the toggle handle **750** has been rotated a full 90° counter-clockwise, the linkage **770** and coupling span **752** comprising the over-center linkage reach the center position. When the linkage and coupling span **752** comprising the over-center linkage reach the center position a large force is generated on the moveable gripper assembly 704 by applying only a small force to the toggle handle **750.** When the toggle handle **750** is rotated the full 90° counter-clockwise, the linkage **770** and the coupling span **752** comprising the over-center linkage reach an over-center position which keeps the toggle handle **750** and clamp apparatus **710** in the closed and clamped position and acts as a passive latch. This clamping action makes actuation of the clamp apparatus **710** easy for the user while also providing a sufficiently strong clamping force.

In some embodiments, the toggle handle **750** comprises a toggle handle latch **780** that operatively secures the toggle handle **750** and clamp apparatus **710** in the closed and clamped position. The toggle handle latch **780** may be disposed on the handle grip **758** of the toggle handle **750** such that it fits in a concavity **759** in the handle grip **758.** The toggle handle latch **780** may be pivotally coupled to the handle grip **758** and may be pivotable between an advanced and a retracted position. In some embodiments a pivot pin bearing **781** runs the length of the toggle handle latch **780.** In the embodiment shown in **FIGS. 8A-8D****,** the pivot pin bearing **781** runs along the bottom edge of the toggle handle latch **780.** A pivot pin **782** may pivotally couple the toggle handle latch **780** to the handle grip **758** by running through the pivot pin bearing **781** and into at least part of the handle grip **758.**

In some embodiments, including the embodiment illustrated in **FIGS. 8A-8D****,** the toggle handle latch **780** may be adapted such that a torsion spring **783** may be slid over at least a portion of the pivot pin bearing **781.** The torsion spring **783** may bias the toggle handle latch **780** to the advanced position. When the toggle handle latch **780** is pivoted toward the retracted position, the torsion spring **783** is spring loaded such that the restoring force of the torsion spring 783 causes the toggle handle latch **780** to automatically pivot back to the advanced position. In the advanced position, the toggle handle latch **780** is in its most protruding position. In the retracted position, the toggle handle latch **780** is pushed into the concavity **759** such that it protrudes minimally from the handle grip **758.**

In some embodiments, the toggle handle latch **780** may comprise a stop surface **784** along at a part of at least one face of the toggle handle latch **780.** The stop surface **784** catches on a part of the concavity **759** in the handle grip **758** and ensures the torsion spring **783** cannot eject the toggle handle latch **780** out of the concavity **759.**

The toggle handle latch **780** may also comprise a latch projection **785.** The latch projection **785** in the example embodiment depicted in **FIGS. 8A-8D** runs substantially the full length of the toggle handle latch **780** and projects off the toggle handle latch **780** toward the bottom of the page (relative to **FIG. 8D****).** This may differ in alternative embodiments.

In some embodiments, the left face (relative to **FIG. 8D****)** of the fixed gripper cradle 711 comprises a ramp catch **786** for the latch projection **785** of the toggle handle latch **780.** The catch **786** in alternative embodiments need not comprise a ramp. The catch **786** may take any other suitable form.

In the example embodiment in **FIGS. 8A-8D****,** as the toggle handle **750** and toggle handle latch **780** are rotated toward the closed position, the latch projection **785** of the toggle handle latch **780** abuts the catch **786** ramp. As the toggle handle **750** continues to rotate toward the closed position, the latch projection **785** of the toggle handle latch **780** rides up the catch **786** ramp. This causes the toggle handle latch **780** to be pivoted into the retracted position, i.e. into the concavity **759** of the handle grip **758.** In turn, this twists the torsion spring **783** and stores mechanical energy in the torsion spring **783.** When the toggle handle **750** is in the fully closed position, the latch projection **785** of the toggle handle latch **780** clears the catch **786** ramp and the restoring force of the torsion spring **783** causes the toggle handle latch **780** to spring back to the advanced position. This locks the clamp apparatus **710** in the closed position as any movement toward the open position is prevented by the latch projection **785** of the toggle handle latch **780** catching on the lip of the catch **786** ramp.

To rotate the toggle handle **750** back toward the open position and/or unclamp the clamp apparatus **710,** a user must manually push in the toggle handle latch **780** to the retracted position. This allows the latch projection **785** of the toggle handle latch **780** to clear the lip of the catch **786** ramp, thus allowing rotation of the toggle handle **750** toward the open position.

In some embodiments, the toggle handle latch **780** may have various contours which provide an ergonomic benefit to the user as a user tries to depress the toggle handle latch **780** to the retracted position when opening the clamp apparatus **710.** In the embodiment shown in **FIGS 8A-8D****,** the toggle handle latch **780** comprises a valley **788** which may better accommodate a user's fingertips as they pivot the toggle handle latch **780** into the retracted position. In other embodiments there may be additional ergonomic contours which supplement or replace the valley **788.**

In some embodiments including a toggle handle **750** or actuator similar to the toggle handle **750,** the toggle handle **750** or toggle handle latch **780** may include an anti-pinch feature (not shown) to preclude a user from pinching a finger when rotating the toggle handle **750** to the closed position. In some embodiments, the anti-pinch feature may be a guard protrusion. In other embodiments, the anti-pinch feature may be an extended gripping portion on the toggle handle **750** which distances a users fingers from the latch projection **785** and the catch **786.**

In some embodiments, the housing **712** of the clamp apparatus **710** may also feature any of a variety of mechanisms **790** (not shown) to attach a load to the clamp apparatus **710.** Such mechanisms may include, but are not limited to, brackets, magnets, straps, suction cups, hooks, screws, bolts, a friction fit, etc. This load could be any number of things, especially a medical device (such as an infusion pump, or peristaltic infusion pump), I.V. bag, etc.

In some embodiments, the clamp apparatus **710** may be adapted such that the fixed gripper assembly and **703** movable gripper assembly **704** may be oriented obliquely to the right and left ends (relative to **FIG. 8D****)** of the housing **712.** In embodiments where the gripper assemblies **703** and **704** are oriented obliquely, any load attached to the clamp apparatus **710** by any of the variety of mechanisms **790** detailed above would be at an angle oblique to a clamped object **100** clamped in the clamp apparatus **710.** Such an orientation may be helpful in accommodating the needs of a load attached to the clamp apparatus **710** through any of the variety of mechanisms **790** described in the preceding paragraph.

**FIGS. 8E-8F** show an alternative embodiment of the example clamp apparatus **710** shown in **FIGS. 8A-8D****.** As shown, the alternative embodiment of the clamp apparatus **710** shown in **FIG. 8E** comprises a fixed gripper assembly **703** similar to the fixed gripper assembly **703** shown in **FIGS 8A-8D****.** The fixed gripper assembly **703** in **FIG. 8E** is somewhat simplified and allows the clamp apparatus **710** to have a more open concept which facilitates ease of cleaning. The fixed gripper assembly **703** in **FIG. 8E** does not include a fixed gripper base **717** as it does in **FIGS. 8A-8D****.** The fixed gripper assembly in **FIG. 8E** features two support legs **4000.** Each support leg **4000** may be coupled to the left (relative to **FIG. 8E****)** face of the fixed gripper cradle **711.** The support legs **4000** may be coupled to the fixed gripper cradle **711** at an angle which is substantially perpendicular to the left face of the fixed gripper cradle **711.** In some embodiments, including the embodiment shown in **FIG. 8E****,** the support legs **4000** may be formed as a continuous part of the fixed gripper cradle **711b.**

One support leg **4000** may coupled to the fixed gripper cradle **711** near the front edge of the fixed gripper cradle **711.** The second support leg **4000** may be coupled to the fixed gripper cradle **711** near the back edge of the fixed gripper cradle **711.** The support legs **4000** are slightly arched in the example embodiment shown in **FIG. 8E****.** As shown, the width of the support legs **4000** may gradually decrease as the support legs **4000** extend toward the bottom of the page. The bottom of the support legs **4000** may be substantially parallel to the direction of elongation of the housing **712.**

As shown in the example embodiment in **FIGS. 8E****,** the threaded holes **791** which are located in the fixed gripper base **717** in **FIGS. 8A-8D** may be disposed at the bottom of the support legs **4000.** The threaded holes **791** may extend through the bottom of the support legs **4000** in a direction substantially perpendicular to the front and back faces of each support leg **4000.** As shown, four screws **714** may run through the housing **712** of the clamp apparatus **710** and into the corresponding threaded holes **791** in the bottom of the support legs **4000** thereby coupling the fixed gripper assembly **703** to the housing **712.**

As shown, the alternative embodiment of the clamp apparatus **710** shown in **FIGS. 8E-8F** comprises a movable gripper assembly **704** similar to the movable gripper assembly **704** shown in **FIGS 8A-8D****.** As shown, the movable gripper assembly **704** comprises a gripper sled **705.** The gripper sled **705** may be roughly planate and rectangular. The gripper sled **705** in **FIGS. 8E-8F** is roughly planate and rectangular though one end of the rectangular gripper sled **705** is rounded. The gripper sled **705** may comprise a dovetail cutout **4002** as shown in **FIG. 8E****.** The gripper sled **705** may be extruded.

The dovetail cutout **4002** of the gripper sled **705** may be sized to accommodate and slide along a dovetail projection **4004** on the housing **712** of the clamp apparatus **710.** As shown in the example embodiment in **FIG. 8E****,** the dovetail projection **4004** in the housing **712** may run roughly parallel with the front wall **741** and back wall **742** of the housing **712.** The dovetail projection **4004** on the housing **712** may run along the medial portion of the bottom face **743** of the housing **712.**

As shown in **FIG. 8E****,** the housing **712** may include roller tracks **4006.** As shown, the roller tracks **4006** are similar to the grooved tracks **740** shown in **FIGS. 8A-8D****.** The roller tracks of the housing **712** will be further elaborated on later. The housing **712** may also include any number of housing voids **4008.** The housing voids **4008** may be cut into the housing **712** or may be created during manufacture of the housing **712.** The housing voids **4008** help to keep debris and unwanted matter from accumulating in and on the clamp apparatus **710.** The housing voids **4008** may also aid in making the clamp apparatus **710** easier to clean. In some embodiments, the housing **712** may be extruded. In such embodiments, the clamp **710** may be extruded from any suitable material.

The movable gripper assembly **704** may comprise a number of additional components in addition to the gripper sled **705.** Projecting perpendicularly from the top face of the gripper sled **705** on the right (relative to **FIG. 8E****)** of the gripper sled **705** there may be a spring housing **4010.** The spring housing **4010** may project in a direction that is substantially perpendicular to the top face of the gripper sled **705.** The spring housing **4010** may be dimensioned such that the sides of the spring housing **4010** are flush with the edges of the gripper sled **705.** The spring housing **4010** may be coupled to the gripper sled **705** by any of a variety of fastening means.

In some embodiments, the movable gripper cradle **706** may be coupled to the left side (relative to **FIG. 8E****)** of the spring housing **4010.** In such embodiments, the movable gripper cradle **706** may be coupled to the spring housing **4010** by any suitable fastener. In the example embodiment, the movable gripper cradle **706** is made as a continuous part of the spring housing **4010.** As shown, the movable gripper cradle **706** is disposed on the spring housing **4010** such that it is at substantially the same height as the fixed gripper cradle **703.**

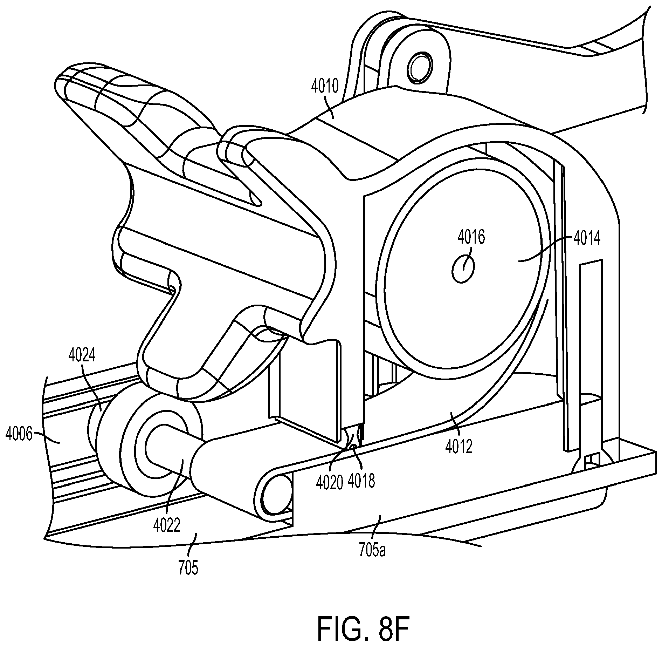

As shown in the cross section of the clamp apparatus **710** in **FIG 8F****,** the spring housing **4010** is substantially hollow. Within the hollow portion of the spring housing **4010** a constant force spring **4012** is housed. In some embodiments, there may be more than one constant force spring **4012** housed in the spring housing **4010.** The constant force spring **4012** in some example embodiments may be a rolled ribbon of spring steel. The constant force spring **4012** may be a laminar spring. In some embodiments, the constant force spring **4012** may be a triple laminar spring. In some embodiments, the constant force spring **4012** may be an approximately19lb constant force spring **4012.** Use of a constant force spring **4012** provides many benefits over other varieties of bias members as detailed above.

As shown, the constant force spring **4012** may be disposed about a mandrel **4014** which is capable of rotating about the axis of an axle **4016.** In the example embodiment, the mandrel **4014** is a solid spindle. In other embodiments, the mandrel **4014** may not be solid. In some embodiments, the mandrel **4014** may be a hollow cylinder. In some embodiments, the mandrel **4014** may be mostly hollow and comprise a number of supporting spokes. The axle **4016** may span across the hollow section of the spring housing **4010.** The axle **4016** may extend in a direction substantially perpendicular to the front wall **741** and back wall **742** of the housing **712** shown in **FIG. 8E****.**

In the example embodiment in **FIG. 8F****,** the gripper sled **705** features a raised section **705a.** The raised section **705a** of the gripper sled **705** projects off the gripper sled **705** toward the top of the page in manner substantially perpendicular to the rest of the gripper sled **705.** As shown, a small gap **4018** may be left between the top of the raised portion **705a** of the gripper sled **705** and the bottom of the left side of the spring housing **4010.** The constant force spring **4012** may extend out of the spring housing **4010** through the small gap **4018.**

To help keep debris and other matter from entering the spring housing **4010,** spring housing sealing member **4020** may be placed at the bottom of the left side of the spring housing **4010.** As shown in the example embodiment in **FIG. 8F****,** a part of the spring housing sealing member **4020** may be seated in a cavity recessed into the bottom face of the left side of the spring housing **4010.** The spring housing sealing member **4020** may be made of a deformable material. As the constant force spring **4012** is advanced and retracted out of and back into the spring housing **4010** during operation of the clamp apparatus **710,** the spring housing sealing member **4020** blocks any debris or other matter on the constant force spring **4012** from being pulled into the spring housing **4010** as the constant force spring **4012** retracts back into the spring housing **4010.**

One end of the constant force spring **4012** may be located exterior to the spring housing **4010** at all times. The end of the constant force spring **4012** located exterior to the spring housing **4010** may be fixedly coupled to a roller axle **4022.** By pulling the roller axle **4022** toward the left of the page (relative to **FIG. 8F****)** the constant force spring **4012** is unwound and spooled out of the spring housing **4010.** If the roller axle **4022** is released, the restoring force of the constant force spring **4012** will cause the roller axle **4022** to be biased back to the position shown in **FIG. 8F****.** The constant force spring **4012** will also retract back into the spring housing **4010.**

A roller **4024** may be seated on each end of the roller axle **4022.** One of the rollers **4024** is visible in **FIG. 8F****.** The rollers **4024** are capable of rotation about the axis of the roller axle **4022.** As shown in **FIGS. 8E-8F****,** the rollers **4024** may ride and roll along the roller tracks **4006** on the front wall **741** and back wall **742** of the housing **712.**

Referring back to **FIG. 8E****,** the linkages **770** extending from the toggle handle **750** may be coupled onto the roller axle **4022.** As such, the roller axle **4022** functions similarly to the driven member **720** in **FIGS. 8A-8D** and may be referred to as an alternative driven member. When the clamp apparatus **710** is actuated from the open position to a clamped position via rotation of the toggle handle **750,** the roller axle **4022** moves toward the fixed gripper assembly **703** and the rollers **4024** on the roller axle **4022** slide along the roller tracks **4006** on the housing **712.** In turn, this displaces the movable gripper assembly **704** toward the fixed gripper **703** assembly. Until the movable gripper **701** contacts a clamped object **100,** the roller axle **4022** and movable gripper assembly **703** move as a unit. When the movable gripper **701** comes into contact with a clamped object **100,** the movable gripper assembly **704** can make no further progress toward the fixed gripper assembly **703** because the clamped object **100** is in the way. The roller axle **4022** continues to move toward the fixed gripper assembly **703.** This causes the constant force spring **4012** to be pulled out of the spring housing **4010.** The restoring force of the constant force spring **4012** causes the movable gripper assembly **704** to exert a more vigorous clamping force on the clamped object **100.**

When the clamp apparatus **710** is moved from a clamped position toward an open position by rotation of the toggle handle **750,** the restoring force of the constant force spring **4012** may automatically spring the clamp apparatus **710** back to the unclamped and open position.

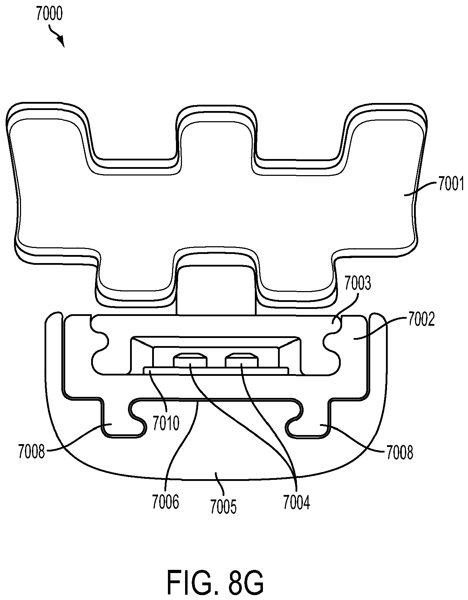

**Fig. 8G** shows an alternate embodiment of a moveable gripper assembly **7000** with a housing **7005** in accordance with an embodiment of the present disclosure. The moveable gripper assembly **7000** may be similar to the moveable gripper assembly shown **in** **Fig. 8F****.** The moveable gripper assembly **7000** includes a moveable gripper **7001,** a driven member **7002,** and a gripper sled **7003** with a housing **7005.** The driven member **7002** is guided via guide members **7008** along a track **7006.** Note that the contact force spring **7010** (e.g., spring **4012** as shown in **Fig. 8F****)** is secured to the driven member **7002** by fasteners **7004.**

### A Rack Apparatus

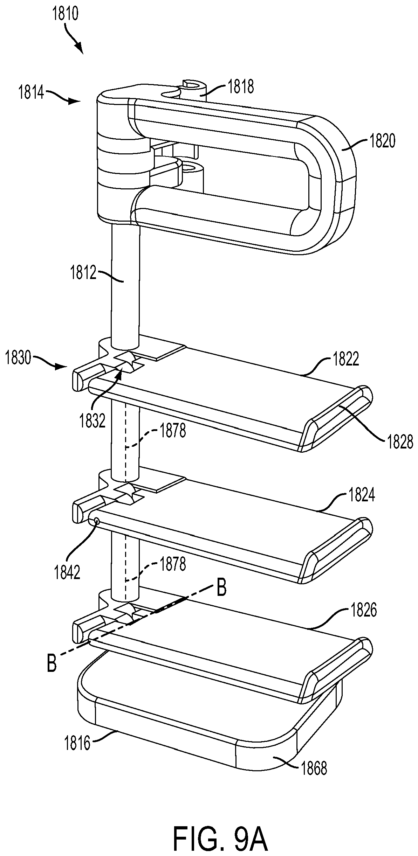

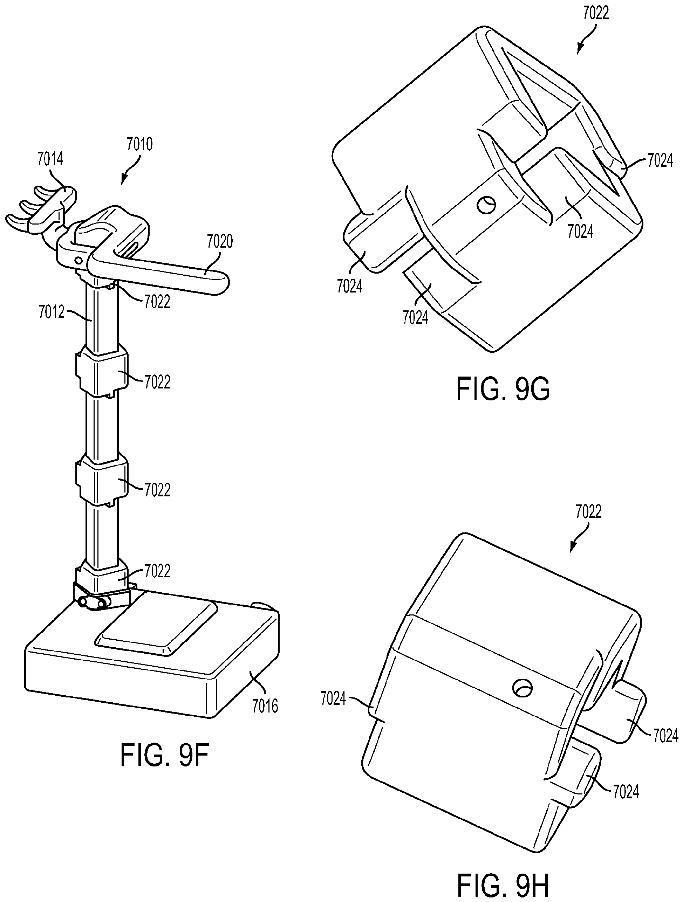

**FIG. 9a** depicts one exemplary embodiment of a rack **1810.** The rack **1810** includes a cylindrically-shaped support pole **1812.** A clamp assembly **1814** may be attached to a first end portion of the support pole **1812.** The clamp assembly **1814** may further include a clamp mechanism **1818** and an elongated, U-shaped handle **1820** that may be oriented perpendicularly to the longitudinal axis of the support pole **1812.** The clamp assembly **1814** and the clamp mechanism 1818 may be configured to removably couple with a support structure such as an IV pole. As should be appreciated by those having ordinary skill in the art, any number of clamp mechanisms may be used to accomplish this objective, including the clamp mechanisms described below and above. The handle **1820** enables the rack **1810** and any received medical devices to be carried as unit from one location to another. In certain embodiments, the handle **1820** may serve as a means to actuate the clamp mechanism **1818.** One such embodiment could include a handle **1820** that shares an axis of rotation with a clamp mechanism **1818,** wherein the clamp mechanism **1818** includes at least one fixed gripper and at least one mobile gripper that may be coupled to the handle **1820.** Actuation of the clamp mechanism **1818** may be achieved by rotating the handle **1820** in a first direction such that the at least one mobile gripper rotates towards the at least one fixed gripper and a support structure therebetween. The at least one mobile gripper and the at least one fixed gripper may be secured in a clamped position by a latch or any other means known in the relevant art when the aforementioned grippers exert a sufficient clamping force on the support structure. Rotating the handle **1820** in a second, opposite direction may rotate the at least one mobile gripper away from the at least one fixed gripper, and the clamp mechanism **1818** may be decoupled from the support structure when the at least one mobile gripper is sufficiently far from the support structure.

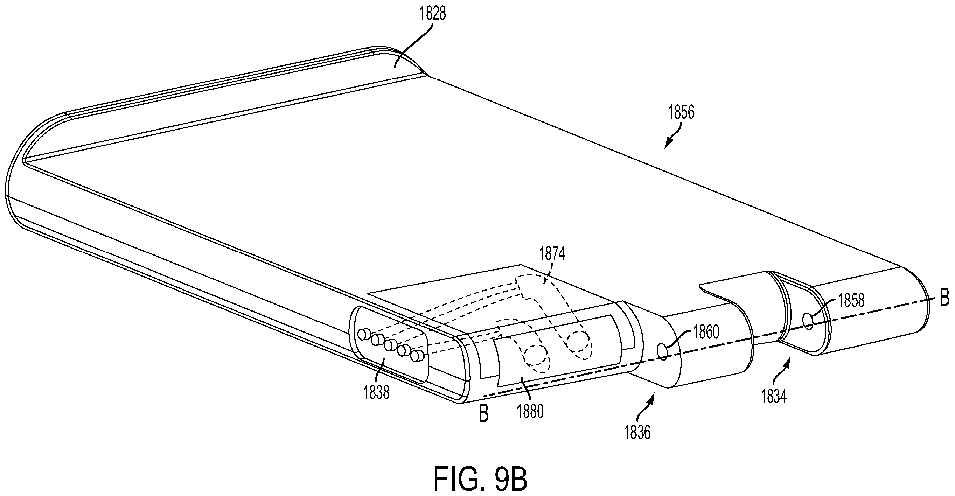

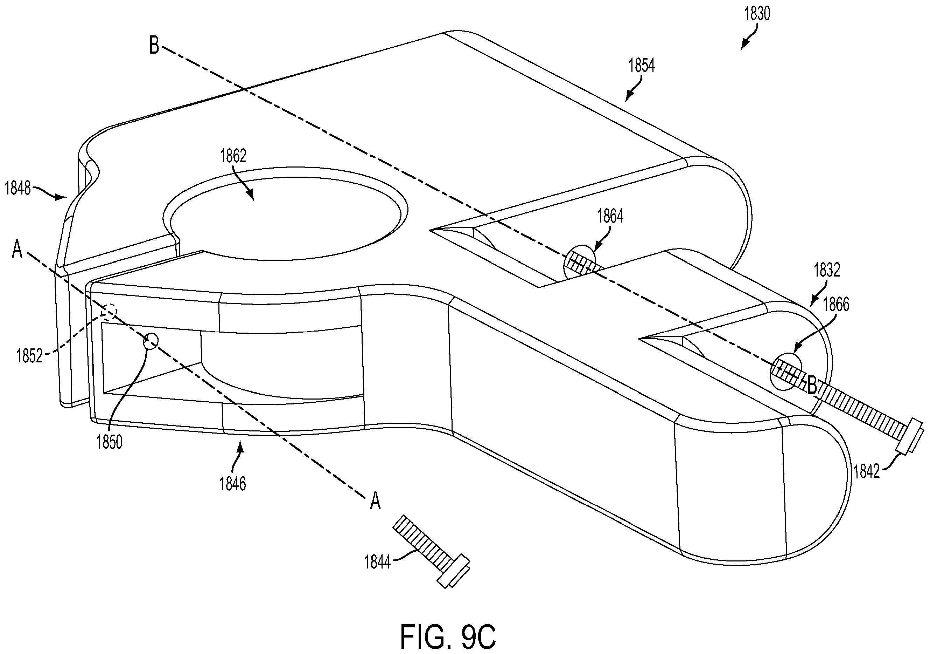

A variety of medical device mounts may be disposed between the first end and a second end of the support pole **1812.** **FIGS. 9a** and **9b** depict an exemplary embodiment where the mounts may be elongated support plates that extend perpendicularly to the support pole **1812.** **FIG. 9a** depicts a rack **1810** having a first support plate **1822,** a second support plate **1824,** and a third support plate **1826.** **FIG. 9b** depicts an embodiment of an individual support plate **1856.** The support plate **1856** may be sized to receive and support a medical device. Examples of medical devices that may be received by the support plate **1856** include syringe pumps, infusion pumps, dialysis machines, pill dispensers, and chemotherapy devices. A first end portion of the support plate **1856** may be coupled to the support pole **1812** using a joint member **1830.** The support plate **1856** may include a first support plate projection **1834** and a second support plate projection **1836** that may interface with the joint member **1830** (see Fig. 9C) to facilitate coupling. To more securely receive and retain a medical device, the support plate **1856** may include a flange **1828** that extends upwardly from a second end portion of the support plate **1856.**

To reduce the need to run power cables from electrical outlets to each individual medical device, each support plate **1856** may include a mount connector **1838** that may be adapted to transmit electrical power to a received medical device. In certain embodiments, the mount connector **1838** may also be adapted to enable signals to be communicated between two or more medical devices and thus provide each medical device with a network connection.

In the embodiment depicted in **FIG. 9a****,** a corresponding number of joint members **1830** couple each of the support plates **1822, 1824, 1826** to the support pole **1812.** Each joint member **1830** may be configured to receive a support plate **1856** such that the joint member **1830** enables the received support plate **1856** to rotate around a longitudinal axis of the support pole **1812.** **FIG. 9c** depicts an exemplary joint member **1830** that permits rotation around a longitudinal axis of the support pole **1812.** The joint member **1830** may include a joint member aperture **1862** that is sized to receive the support pole **1812.** The joint member **1830** may be rotated and re-secured to the support pole **1812** by loosening a threaded screw **1844,** rotating the exemplary joint member **1830** and a received support plate **1856** to the desired position, and retightening the threaded screw **1844.**

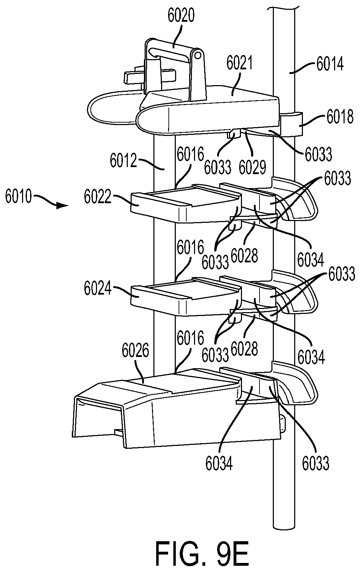

As depicted in **FIG. 9e****,** the exemplary joint member **1830** may include a first clamping arm **1846** and a second clamping arm **1848,** each having an inner surface that forms a portion of the joint member aperture **1862.** The first and the second clamping arms **1846, 1848** may further include a first threaded aperture **1850** and a second threaded aperture **1852** respectively. The first threaded aperture **1850** and the second threaded aperture **1852** may be aligned along a line A-A and each may be sized to receive the threaded screw **1844.** As will be understood by persons having ordinary skill in the art, rotating the threaded screw **1844** in a first direction, generally clockwise, may pull the first and the second clamping arms **1846, 1848** towards one another and enable the joint member aperture **1862** to exert a predominantly horizontal force against a received support pole **1812** such that the received support pole **1812** may support, against the force of gravity, the weight of the joint member **1830,** the received support plate **1856,** and any received medical devices. Turning the threaded screw **1844** in a second, opposite, and generally counter-clockwise direction may push the first and the second clamping arms **1846, 1848** apart and may reduce the force applied to the support pole **1812** by the joint member aperture **1862** and may enable the joint member **1830** to be rotated about the support pole **1812.**

In addition, the joint member **1830** may be hingably coupled with a received support plate **1856,** and the joint member **1830** may be placed in one of a vertical or a horizontal orientation such that the received support plate **1856** (eg. **1822, 1824, 1826** in **FIG. 9a****)** can rotate in a transverse plane or a longitudinal plane of the support pole **1812.** **FIGs. 9b** and **9c** respectively depict an embodiment of the present disclosure wherein a support plate **1856** and a joint member **1830** are configured to be hingably coupled, and wherein the resulting hinged joint may be placed in a substantially horizontal orientation such that the support plate **1856** may rotate in a longitudinal plane of the support pole **1812.** **FIG. 9a** depicts an embodiment wherein the rack **1810** includes three of this type of coupling mechanism. Alternatively, a support plate **1856** or other type of medical device mount may be fixedly and rigidly coupled to the support pole **1812** in different embodiments.

In the embodiment depicted in **FIG. 9b****,** the support plate **1856** may include a first support plate projection **1834** and a second support plate projection **1836** that extend in substantially parallel directions from a first end portion of the support plate **1856.** The first support plate projection **1834** and the second support plate projection **1836** respectively include a first support plate aperture **1858** and a second support plate aperture **1862** that may be aligned along a line **B-B,** and wherein each is sized to receive a pin **1842.**

In the embodiment depicted in **FIG. 9c****,** the joint member **1830** may include a first joint member projection **1832** and a second joint member projection **1856** that extend in substantially parallel directions. The first joint member projection **1832** and the second joint member projection **1856** may respectively include a first joint member aperture **1864** and a second joint member aperture **1866** that may be aligned along a line **B-B,** and wherein each is sized to receive a pin **1842.**

To hingably couple the support plate **1856** to the joint member **1830** as depicted in **FIG. 9a****,** the first and the second support plate projections **1834, 1836** and the first and the second joint member projections **1832, 1856** (referring now also to **FIGS. 9b****-c)** may be respectively sized and disposed on the support plate **1856** and joint member **1830** such that the respective projections **1832, 1834, 1836, 1856** are capable of interleaving. The apertures **1864, 1866** of the joint member **1830** are configured to align with the apertures **1860, 1858** of the support plate **1856** such that all four apertures **1858, 1862, 1864, 1866** will align along the line **B-B** when the four projections **1832, 1834, 1836, 1856** are interleaved. When properly aligned, a pin **1842** may be inserted through and retained in the four apertures **1858, 1862, 1864, 1866** such that the joint member **1830** retains the support plate **1856.** As will be understood by persons having ordinary skill in the art, a number of methods are available to maintain the position of the support plate **1856** about the pin **1842.** In certain embodiments, the friction between the interleaved projections **1832, 1834, 1836, 1856** and/or the friction between the pin **1842** and the four apertures **1858, 1862, 1864, 1866** in which the pin **1842** is disposed may be sufficient to maintain the position of the support plate **1856** about the pin **1842.** Any other structure may secure the joint member **1830** to the support plate **1856** known to one of ordinary skill in the relevant art.

In other embodiments, the position of the support plate **1856** about the pin **1842** may be maintained at one of several predefined positions by a detent pin (not shown) that is capable of engaging one of several detents (not shown) in an inner joint member projection. The detents may be annularly inscribed at several positions about the pin **1842.** In embodiments having such detents, a detent pin aperture may retain the detent pin and be disposed in an outer support plate projection so as to enable the detent pin to selectively engage any one of the detents in the inner joint member projection. Once a healthcare provider engages the detent pin with the appropriate detent, the detent and the detent pin can prevent the support plate **1856** from rotating out of the selected position.

In particular embodiments, like the embodiment depicted in **FIG. 9a****,** the weight of multiple received medical devices may cause the rack **1810** to become unbalanced and begin to rotate about the point where the clamp mechanism **1818** couples with a support structure like an IV pole. To mitigate this type of rotation, a base member **1816** may be employed that exerts a stabilizing force on the support structure. As depicted in **FIG. 9a****,** the base member **1816** may comprise an elongated housing **1868** that is coupled to a second end portion of the support pole **1812** and that extends perpendicularly to the support pole **1812.** The base member **1816** may include a rounded notch **1840** that is configured to abut a substantially cylindrical support structure. The notch **1840** may be disposed on the elongated housing **1868** such that the base member **1816** and the clamp mechanism **1818** position the support pole **1812** in spaced relation to and substantially parallel to an elongated, cylindrical support structure like an IV pole. In other embodiments, the base member may comprise a second clamp assembly like the clamp assembly **1814** that may be coupled to the first end of the support pole **1812.**

An advantage of the exemplary embodiment depicted in **FIG. 9a****,** is that the base member **1816** and the elongated housing **1868** can serve other functions in addition to providing a counterbalancing force to the rack **1810.** For example, the elongated housing **1868** may serve as a bedside surface on which a healthcare provider may temporarily store items that are needed to care for a patient. In another embodiment, the elongated housing **1868** could also be configured to receive a medical device and include the same features as a support plate **1856,** such as a mount connector **1838** that is configured to provide one or both of electrical power and a network connection to a received medical device. In embodiments where the base member **1816** does not include an elongated housing **1868,** the base member **1816** may nevertheless be configured to receive, power, and provide a network connection to an additional medical device.

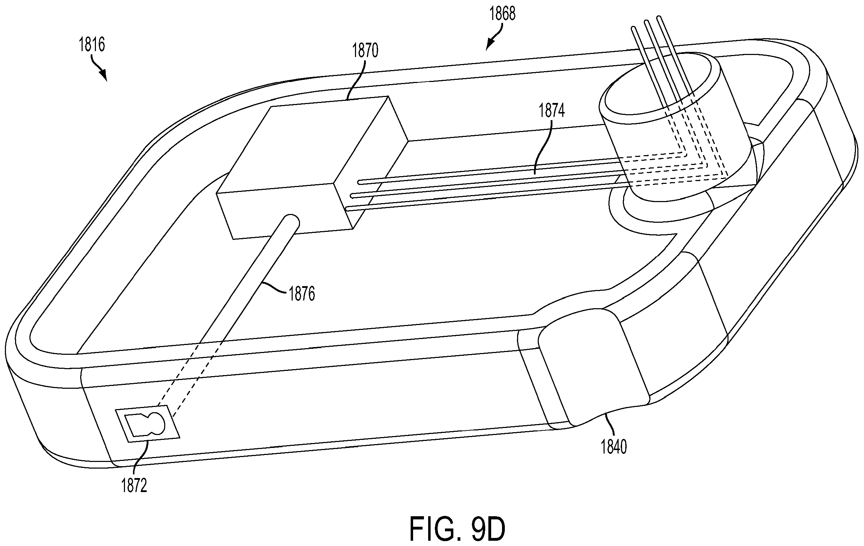

Another advantage of the exemplary embodiment depicted in **FIG. 9a** and the exemplary base member **1816** depicted in **FIG. 9d** is that the elongated housing **1868** may provide space to contain certain elements of a power system. **FIG. 9d** depicts an exemplary power system that includes a power supply **1870,** a power connector **1872,** power transmission cables **1874,** and a main power cable **1876.** As discussed above, embodiments that include a power system may have the advantage of reducing the number of cables that are needed to power the received medical devices. Rather than having to run a separate power cable from an electrical outlet to each medical device, a single power cable may be connected from an electrical outlet to a power connector **1872** that is preferably located on the elongated housing **1868** of the base member **1816.** A main power cable **1876** may then deliver power to a power supply **1870.** The power supply **1870** may be configured to convert balanced or unbalanced AC current to direct current and provide the desired voltage and amperage for any received medical devices. A respective power transmission cable **1874** may be used to transmit electrical power from the power supply **1870** to a respective mount connector **1838** and a received medical device. The power transmission cables **1874** may provide one more DC voltages for use by any received medical devices. In certain embodiments, the respective power transmission cable **1874** may operatively run from a power supply **1870,** up through a hollow support pole **1812,** and may be operatively distributed to the respective mount connector **1838.** Each of the support plates **1856** may include a mount connector **1838** and receive a respective power transmission cable **1874** that enables the mount connector **1838** to supply electrical power to a received medical device. In some embodiments, a common power bus may be positioned within a hollow support pole **1812** that receives power from the power transmission cables **1874;** each mount connector **1838** may be electrically coupled to the power bus.

In addition to supplying power to a received medical device, the exemplary mount connector **1838** depicted in **FIG. 9b** may be configured to provide a network connection to a received medical device. In embodiments that are capable of receiving two or more medical devices, it may be advantageous to enable the received medical devices to communicate with one another. For example, a patient may require a regime of several different drugs that are administered by respective syringe pumps. In other instances, it may be desirable to arrange are relay infusion of the same drug using two or more pumps. Enabling the rack **1810** to transmit signals between network-capable syringe pumps may allow for each syringe pump to know how much of which drugs were delivered by the other syringe pumps in the rack **1810** network. To achieve this objective, exemplary embodiments like the embodiment depicted in **FIG. 9a** may include a central bus **1878** that is operatively coupled to the support pole **1812.** Each of the support plates **1856** may include a support-plate bus **1880** that operatively interfaces with the central bus **1878** and that is coupled to a mount connector **1838.**

In some embodiments, each received medical device may broadcast its data over the central bus **1878.** In other embodiments a turn-based communication scheme may be used by the received medical devices to communicate with each other using the central bus **1878.** In yet additional embodiments, a carrier-sense, multiple-access with optional collision avoidance communication scheme may be used by the medical devices when communicating via the central bus **1878.**

Yet another advantage of the exemplary embodiment of the rack **1810** depicted in **FIG. 9a** and the exemplary base member **1816** depicted in **FIG. 9d** is that the elongated housing **1868** may optionally include provisions, such as casters and the like, for coupling with two or more wheels. In addition to the handle **1820,** wheels may allow the rack **1810** to be more easily moved from one location to another, particularly when transporting multiple received medical devices. As should be understood by persons having ordinary skill in the art, wheels may be coupled to the elongated housing **1868** by any number of well-known means. In addition, two or more wheels may be coupled to a wheel assembly structure that enables the wheels to be coupled to or decoupled from the elongated housing **1868** as a group. In other exemplary embodiments, the support pole **1812** may include provisions for mounting two or more wheels or a wheel assembly.

**FIG. 9e** depicts one exemplary embodiment of a rack **6010.** The rack **6010** includes a cylindrically-shaped support pole **6012.** A clamp mechanism **6018** and a U-shaped handle **6020** on a handle plate **6021** may be oriented perpendicularly to the longitudinal axis of the support pole **6012.** The clamp mechanism **6018** may be configured to removably couple with a support structure **6014** such as an IV pole. As should be appreciated by those having ordinary skill in the art, any number of clamp mechanisms **6018** may be used to accomplish this objective, including the clamp mechanisms described below and above. The handle **6020** enables the rack **6010** and any received medical devices to be carried as unit from one location to another. In certain embodiments, the handle **6020** may serve as a means to actuate the clamp mechanism **6018.** One such embodiment could include a handle **6020** that shares an axis of rotation with a clamp mechanism **6018,** wherein the clamp mechanism **6018** includes at least one fixed gripper and at least one mobile gripper that may be coupled to the handle **6020.** Actuation of the clamp mechanism **6018** may be achieved by rotating the handle **6020** in a first direction such that the at least one mobile gripper rotates towards the at least one fixed gripper and a support structure **6014** therebetween. The at least one mobile gripper and the at least one fixed gripper may be secured in a clamped position by a latch or any other means known in the relevant art when the aforementioned grippers exert a sufficient clamping force on the support structure **6014.** Rotating the handle **6020** in a second, opposite direction may rotate the at least one mobile gripper away from the at least one fixed gripper, and the clamp mechanism **6018** may be decoupled from the support structure **6014** when the at least one mobile gripper is sufficiently far from the support structure **6014.**

A variety of medical device mounts may be disposed between the first end and a second end of the support pole **6012.** **FIG. 9e** depicts an exemplary embodiment where the mounts may be elongated support plates that extend perpendicularly to the support pole **6012.** **FIG. 9e** depicts a rack **6010** having a first support plate **6022,** a second support plate **6024,** and a third support plate **6026.** The first support plate **6022,** second support plate **6024,** and third support plate **6026** may be sized to receive and support a medical device such as any of those described above. One end portion of each of the first support plate **6022,** a second support plate **6024,** and a third support plate **6026** may be coupled to the support pole **6012** via a joint member **6016.** The joint member **6016** may be similar to the joint member **1830** described above.

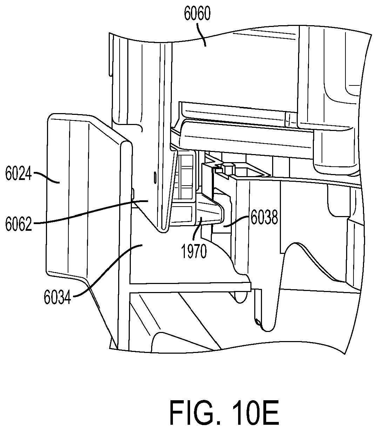

The third support plate **6026** may perform the same function as the base member **1816** and elongate housing **1868** in **FIGS. 9a****-d.** In some embodiments, the third support plate **6026** may also house elements of a power system like the power system described above and may include a mount connector **6038** (best shown in **FIG. 10E****)** that is configured to provide one or both of electrical power and a network connection to a received medical device. As described above in relation to **FIGS. 9a****-d** the first support plate **6022,** second support plate **6024,** and third support plate **6026** may each include a mount connector **6038.**

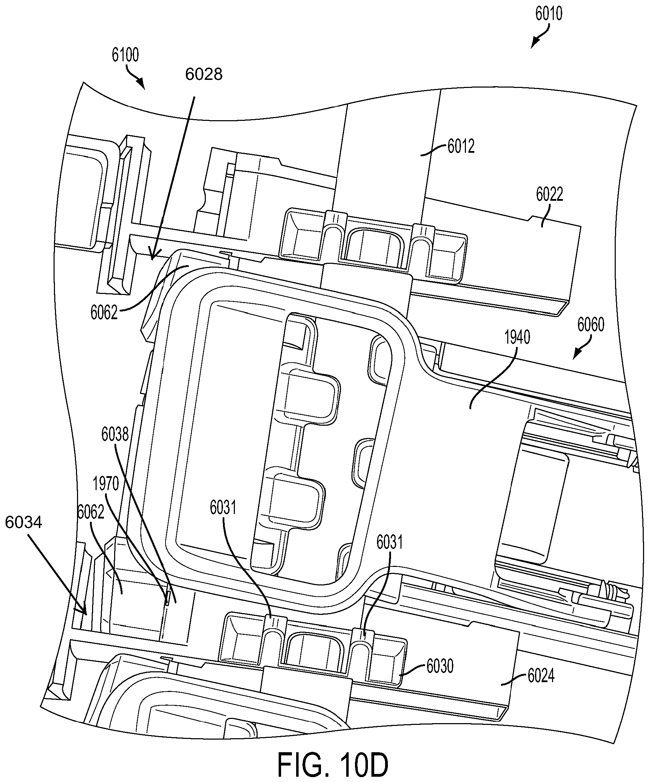

As shown in **FIG. 9e****,** the first support plate **6022,** a second support plate **6024,** and a third support plate **6026** each may include a first guide trough **6034** and a second guide trough **6028.** As shown, the third support plate **6026** only includes a first guide trough **6034.** As shown, the handle plate **6021** also includes a handle plate guide trough **6029.** The guide troughs **6026, 6034, 6029** may also include guide rails **6033.** The guide rails **6033** may be the sides of the guide troughs **6026, 6034, 6029,** or may be projections which project off the first support plate **6022,** second support plate **6024,** and third support plate **6026** or handle plate **6021.** In embodiments where the guide rails **6033** are projections, the guide rails **6033** may define the sides of the guide troughs **6026, 6034, 6029.** As in the exemplary embodiment in **FIG. 9e****,** the ends of the guide rails **6033** may bow out or angle out away from the longitudinal axis of the guide troughs **6026, 6034, 6029.** This may allow a medical device to be easily and sightlessly slid into the guide troughs **6026, 6034, 6029** and docked on the supports plates **6022, 6024, 6026.** In some embodiments, the medical device may include a feature or features such as a flanges **6062** (see **FIG. 10d****)** which may be sized to fit within the guide troughs **6026, 6034, 6029.**

**FIG. 9f** depicts one exemplary embodiment of a rack **7010.** The rack **7010** includes a support pole **7012.** A clamp mechanism or assembly (not shown) may be attached to a first end portion of the support pole **7012.** The rack **7010** may include handle **7020** that may be oriented perpendicularly to the longitudinal axis of the support pole **7012.** The clamp mechanism or assembly may be configured to removably couple with a support structure such as an IV pole. As should be appreciated by those having ordinary skill in the art, any number of clamp mechanisms or assemblies may be used to accomplish this objective, including the clamp mechanisms and assemblies described herein. The handle **7020** enables the rack **7010** and any received medical devices to be carried as unit from one location to another. In certain embodiments, the handle **7020** may serve to actuate the clamp mechanism the clamp mechanism or assembly. The example rack **7010** shown in **FIG. 9f** also includes a hanger feature **7014** which may for example be used to hang IV bags, IV lines, etc.

The rack **7010** may include a base member **7016** similar to that described in **FIG. 9d****.** In such embodiments, the base member **7016** may include, for example, certain elements of a power system. The base member **7016** may also include certain components of a communication system. The base member **7016** may include wheels to aid in transporting the rack **7010** and any attached medical devices.

The rack **7010** depicted in **FIG. 9f** may optionally include, in yet additional embodiments, support plates like those embodiments depicted in **FIGS. 9a****-e.** The rack **7010** includes a number of collars **7022** which help to assure that a medical device coupled to the rack **7010** is correctly and securely coupled to the rack **7010.** The collars **7022** may be coupled to the support pole **7012** at suitable locations. In some embodiments, the collars **7022** may be spaced apart from one another at equal intervals. The example embodiment depicted in **FIG. 9f** includes four collars **7022.**

Referring now also to **FIGS. 9g****-h,** the collars **7022** may include a number of alignment features **7024.** **FIG. 9g** depicts a front perspective view of an example collar **7022** and **FIG. 9f** depicts a back perspective view of the same example collar **7022.** The alignment features **7024** may be one of or any combination of protuberances, recesses, steps, cutouts, pegs, posts, or any other suitable feature in various embodiments. The alignment features **7024** may be dimensioned such that any medical device which is to be attached to the rack **7010** can only be attached in a correct orientation. In some embodiments, the alignment features **7024** may not be included on a collar **7022** but rather on the support pole **7012** itself. Such embodiments may not include collars **7022.**

In the example embodiment in **FIGS. 9f****-h,** the alignment features **7024** include a number of protuberances and cutouts. The alignment features **7024** are dimensioned such that a clamp apparatus (for example, the clamp apparatus **710** in **FIGS. 8a****-f)** is prevented from clamping closed on the support pole **7012** in all but a correct orientation. In turn, this causes a medical device which is attached to the clamp apparatus to be correctly oriented on the rack **7010.**

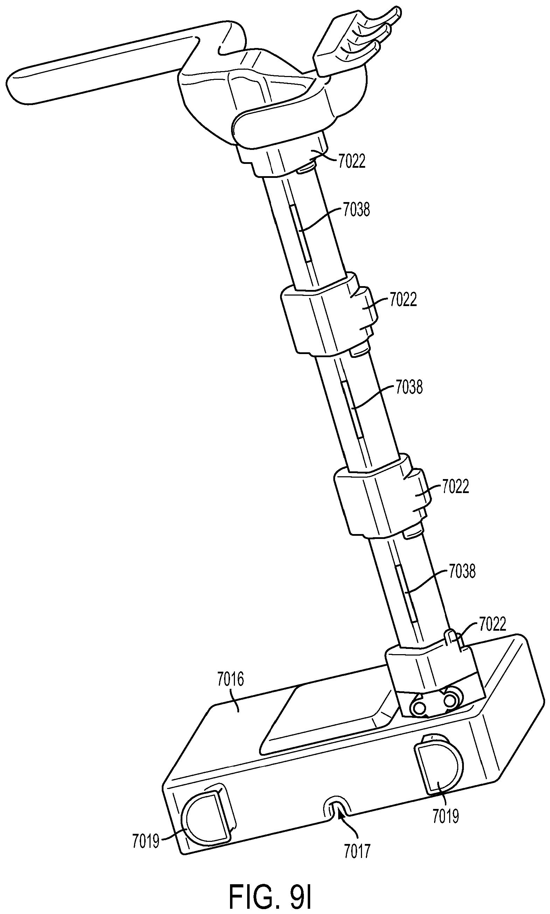

**FIG. 9i** shows a view of the back of the example rack **7010** in **FIG. 9f****.** As shown, the base member **7016** includes an opening **7017** to allow for a power cable, communications cable, etc. to enter the interior of the base member **7016.** The base member **7016** may also includes a number of projections **7019** which may be used to wrap a power cable, communications cable, etc. around when the entire length of the cable is not needed or the cable is not in use. In some embodiments, the rack **7010** may include at least one plug or receptacle which may be configured to receive a power cable, communications cable, etc.

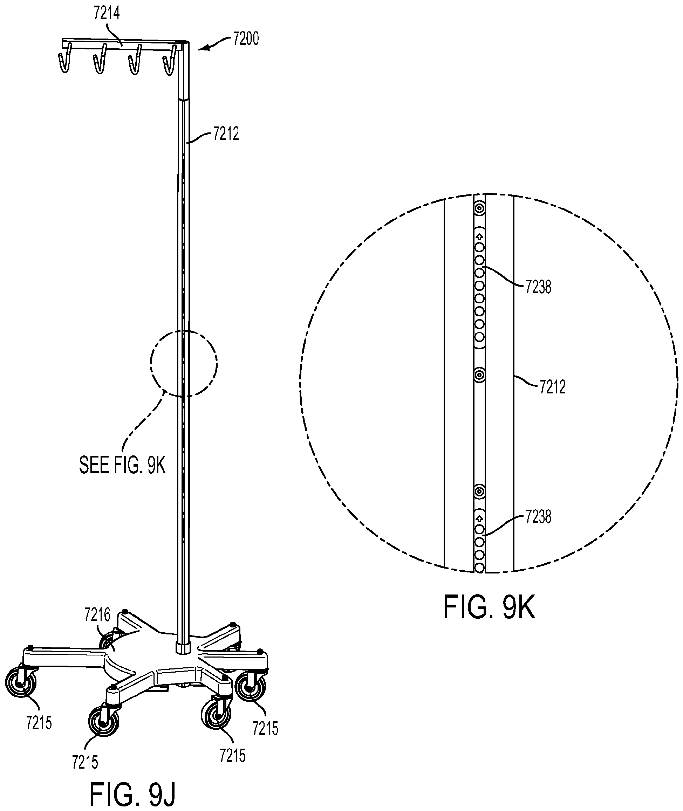

As shown in **FIG. 9i** the rack **7010** may include one or a number of mount connectors **7038.** The mount connectors **7038** may be configured to provide one or both of electrical power and a network connection to a received medical device. In some embodiments, the mount connectors **7038** may be configured to allow a received medical device to communicate over a CANbus and/or over USB. In other embodiments, the mount connectors **7038** may allow for communication using other communication schemes, such as, for example, any of those described above in relation to **FIGS. 9a****-d.** The rack **7010** may include a mount connector **7038** for each attached medical device. In the example embodiment, the rack **7010** includes three mount connectors **7038.** As shown, the mount connectors **7038** are included as a part of the support pole **7012** and are located on the back of the support pole **7012.** In other embodiments, the mount connectors **7038** may be located elsewhere on the rack **7010.** In embodiments which include mount connectors **7038,** the collars **7022** may assure that a medical device can only be received by the support pole **7012** in a manner in which it operatively engages a respective mount connector **7038**.Referring now to **FIG. 9j****,** another example embodiment of a rack **7200** is shown. The rack **7200** may be an IV pole, as shown. The rack **7200** may include a support pole **7212** to which a number of medical devices may be coupled. In some embodiments of the rack **7200,** the rack **7200** may include collars and/or alignment features similar to those described in reference to **FIGS. 9f****-i.** The collars and/or alignment features may help to ensure that medical devices are attached to the rack **7200** in a correct and secure manner. In other embodiments, the rack **7200** may include one or more support plates such as any of those described in reference to **FIGS. 9a****-e.** In some embodiments, the rack **7200** may include a combination of collars alignment features and support plates.

A hanger feature **7214** may also be included on the rack **7200.** In the example embodiment, a hanger feature **7214** is attached to the top end of the support pole **7212.** In alternate embodiments, the hanger feature **7214** may be located elsewhere on the rack **7200.** The hanger feature **7214** may be used to hang IV bags, IV lines, etc.

The bottom of the support pole **7212** of the rack **7200** may couple into a base member **7216** as it does in **FIG. 9j****.** The base member **7216** may include a number of wheels or casters **7215** which may allow the rack **7200** and any attached devices to be easily moved around a care facility. The base member **7216** may be similar to that described in **FIG. 9d****.** For example, the base member **7216** may include certain elements of a power system as described in relation to **FIG. 9d****.** In other embodiments, the base member **7216** may also include certain elements of a communication system.

Referring now also to **FIG. 9k****,** the support pole **7212** may be similar to that depicted in **FIGS. 9f****-i.** The support pole **7212** may include a number of mount connectors **7238.** The mount connectors **7238** may be configured to provide one or both of electrical power and a network connection to a received medical device. In some embodiments, the mount connectors **7238** may allow a received medical device to communicate over a CANbus and/or over USB. In other embodiments, the mount connectors **7238** may allow for communication using other communication schemes, such as, for example, any of those described above in relation to **FIGS. 9a****-d.** The rack **7200** may include a mount connector **7238** for each attached medical device. In the example embodiment, the rack **7200** may, for example, include up to nine mount connectors **7238** and be capable of receiving nine medical devices. In other embodiments, the number of mount connectors **7238** and number of medical devices which can be received may differ. In embodiments which include mount connectors **7038,** collars and/or alignment features (such as the collars **7022** and alignment features **7024** shown in **FIG. 9i****)** may be included. The collars and/or alignment features may assure that a medical device can only be received by the support pole **7212** in a manner in which it operatively engages a respective mount connector **7238.**

As will be understood by persons having ordinary skill in the art, the racks **1810, 6010, 7010, 7200** and their components can be made from a variety of rigid, engineering materials. Possible materials include aluminum alloys, stainless steel alloys, steel alloys, and engineering polymers. In addition, a variety of coatings may be applied to the racks **1810, 6010, 7010, 7200** and their components. Many of the possible coatings may provide a means of reducing the likelihood of cross-contamination. Cross-contamination may pose a serious health risk to young and old patients and patients with weakened immune systems. Optionally, an antibacterial, an antiviral, or an antimicrobial coating may be applied to the structural components of the racks **1810, 6010, 7010, 7200** to kill or inhibit the growth bacteria, viruses, fungi, and various other microorganisms. Exemplary coatings may include copper, copper particles, silver, silver particles, or other materials that have antibacterial, antiviral, or antimicrobial properties.

### Rack Systems

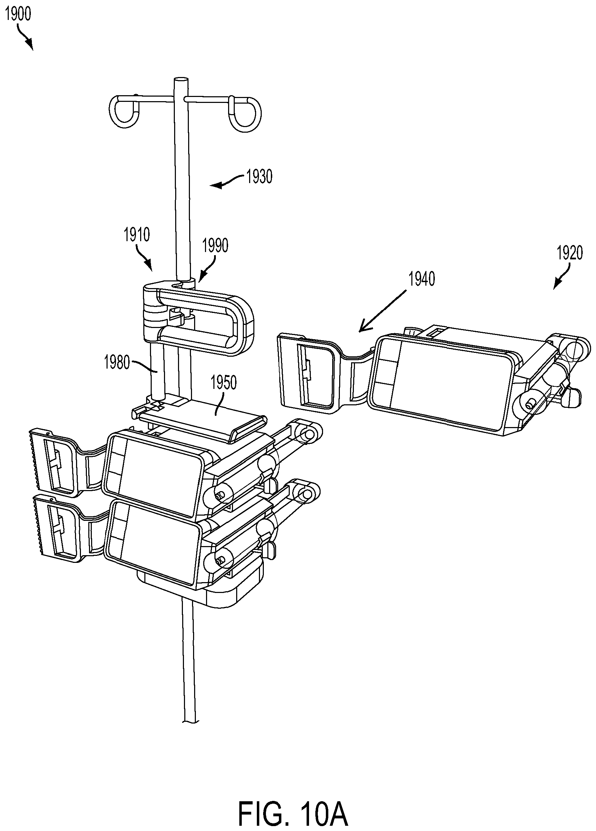

**Fig. 10a** shows an exemplary rack system **1900.** The exemplary embodiment of a rack **1810** depicted in **FIG. 9a** may be one element of a rack system **1900** shown in **Fig. 10a****.** Another element of the rack system **1900** may be a device that includes a mounting mechanism that is configured to couple with the rack, such as a clamp mechanism like any one of those described above. It should be understood that the exemplary embodiment depicted in **FIG. 9a** is but one embodiment of a rack that may be used in a rack system, and alternative embodiments of the rack and mounting mechanism may depart, perhaps substantially, from the exemplary embodiments described herein.

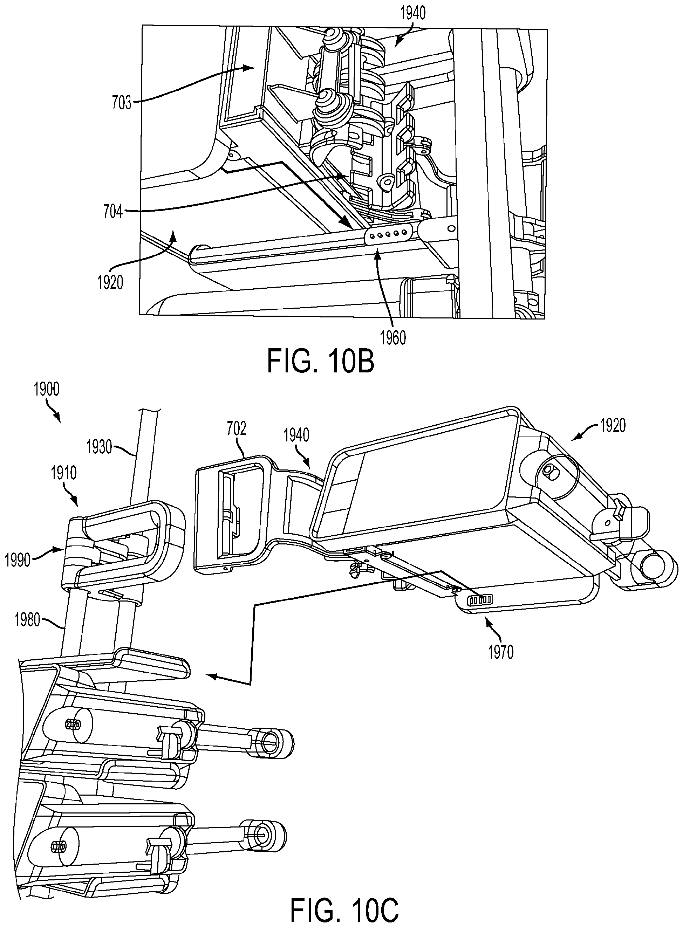

**FIG. 10a** depicts an embodiment of a rack system **1900** comprising a rack **1910** that is substantially the same as the rack **1810** embodiment described above and depicted in **FIGs. 9a****-d,** a medical device **1920** that may be received by a support plate **1950** of the rack **1910,** and a clamp mechanism **1940** that is coupled to a first side of a medical device **1920** and that is adapted to securely couple the medical device **1920** to the rack **1910.** **FIG. 10b** depicts the same embodiment as **FIG. 10a** but from a different perspective. **FIG. 10b** includes a view of the clamp mechanism **1940,** described in detail below, and a mount connector **1960** that is disposed on the support plate **1950.** **FIG. 10c** is yet another perspective of the embodiment depicted in **FIGs. 10a** and **10b** and includes a view of a device connector **1970** that is disposed on the medical device **1920.** The mount connector **1960** and the device connector **1970** are preferably disposed on the support plate **1950** and the medical device **1920** respectively so that they are operatively aligned and capable of coming into contact when the clamp mechanism **1940** couples the medical device **1920** to the rack **1910.**

In a preferred embodiment of the rack system **1900,** the clamp mechanism **1940** may be a mechanism like the embodiment depicted in **FIGs. 8a-8d** or described in relevant portions of the specification above. The clamp mechanism **1940** may latch onto the support pole **1980** depicted in **FIGs. 10a-10c****.**

As should be evident from the description of the above embodiments of a clamp mechanism **1940,** actuating the clamp mechanism **1940** to couple an attached medical device **1920** to a support pole **1980** may have a first phase and a second phase. Refer now to **FIGs. 8a-****8d** and **FIGs. 10a-10C****.** In the first phase, user rotation of the handle **702** may move the driven member **710** and the slidably attached mobile gripper **704** towards the fixed gripper **703** until the girth of the support pole **1980** arrests the movement of the mobile gripper **704.** Thus, the first phase ends when the fixed gripper **703** and the mobile gripper **704** contact the support pole **1980.** In the second phase, continued rotation of the handle **702** may continue to drive the driven member **710** towards the fixed gripper **703** and bias the compression spring **730** (or other bias member) because the driven member **710** may continue to move independently of the mobile gripper **704.** Therefore, the second phase enables the user to increase the clamping force and ensure that the medical device **1920** is securely coupled to the rack **1910.**

In some embodiments, the medical device may be a monitoring client (e.g., a tablet computer) to monitor the operation of the other medical devices (e.g., via wireless communications such as WiFi or Bluetooth, for example). The monitoring client may have a serial interface to connect to the mount connector **1960** (see Fig. **10B****).** Additionally, as mentioned above, the monitoring client may couple to a clamp such as any of those described herein. The clamp may then be used to secure the monitoring client to a rack **1910.**

Referring now to **Figs. 10a-10c****,** the rack system **1900** may be best employed where a patient requires treatment with a coordinated regime of drugs, particularly where the drugs are to be administered by syringe pumps. Because syringe pumps are capable of continuously or discretely delivering precise quantities of fluid over a period of time, syringe pumps are well-suited to administering a regime of different drugs at predefined times. Computerized and networked syringe pumps may allow such a regime to be administered automatically. Embodiments of the present disclosure, like the embodiment of a rack system **1900** depicted in **FIGs. 10a****-c,** may enable a healthcare provider to quickly setup a group of networked syringe pumps to administer such a regime of drugs. Additionally, embodiments of the present disclosure, like the embodiment of a rack system **1900** depicted in **FIGS. 10a****-c,** may help to minimize the number of cords and cables which would otherwise be present when a group of pumps is setup.

For example, a healthcare provider may quickly couple the clamp assembly **1990** to a support structure **1930,** such as an IV pole, and connect the rack **1910** to a source of electrical power. If no syringe pumps or other devices are already coupled to the rack **1910,** the healthcare provider may proceed to couple the required syringe pumps to the rack **1910** one at a time. The healthcare provider may couple each syringe pump to the rack **1910** by placing a portion of each syringe pump on one of the support plates **1950** such that the support plate **1950** bares at least a portion of the weight of the syringe pump, allowing the healthcare provider to more easily maneuver the syringe pump into position. Once the support pole **1980** is positioned between the fixed gripper **703** (see **FIGs. 8a-8d****)** and the mobile gripper **702** (see **FIGs. 8a-8d****)** and once the mount connector **1960** and the device connector **1970** are in general alignment, the healthcare provider may rotate the handle **702** through the first phase of operation. During the first phase of operation, the device clamp-mechanism **1940** may automatically adjust to the size of the support pole **1980** and the mount connector **1960** and the device connector **1970** may be brought into contact with one another. The healthcare provider may secure the syringe pump to the rack **1910** by continuing to rotate the handle **702** through the second phase of operation, and the healthcare provider may repeat the procedure for as many syringe pumps as may be desired. Thus, the healthcare provider may provide each syringe pump with electrical power and a network connection to other syringe pumps via the mount connector **1960** and device connector **1970** without having to run multiple power and network cables that may complicate the setup procedure and clutter the environment around the patient. Moreover, any one of the syringe pumps may be decoupled from the rack **1910,** or another syringe pump may be coupled to the rack **1910,** without having to detach or attach any additional cables. When treatment is complete, certain syringe pumps may remain coupled to the rack and continue to treat the patient while others may be decoupled, again without having to detach any additional cables, and used to treat a different patient. Alternatively, a healthcare provider could transport the entire rack system **1900** and any syringe pumps coupled thereto by decoupling the rack **1910** from the support structure **1930.** A rack **1910** that includes a handle **1820** and/or wheels may make transporting the rack system **1900** and medical devices **1920** easier in this scenario.

**FIG. 10d** shows a view of another embodiment of a rack system **6100** including the rack **6010** embodiment shown in **FIG. 9e****.** A medical device, which for exemplary purposes is shown as an infusion pump **6060,** is in place on the rack **6010.** A medical device may also, for example, be a monitoring client, PCA, physiological monitor, etc. As shown, the flanges **6062** of the infusion pump **6060** are disposed within the guide troughs **6028, 6034** of the first support plate **6022** and second support plate **6024,** respectively. A clamp mechanism **1940** is included on the infusion pump **6060** and is shown clamped around the support pole **6012.** Additionally, as shown in **FIG. 10d****,** the joint members **6030** of the support plates **6022, 6024, 6026** may include functional protrusions **6031.** In the example embodiment, the functional protrusions **6031** are hooks or hangers. The functional protrusions **6031** may, for example, be used to hang various cabling, lines, or IV bags.

**FIG. 10e** depicts an infusion pump **6060** with a flange **6062** which is in place within a guide trough **6034** of the second support plate **6024.** As shown, the infusion pump **6060** includes a device connector **1970** and the second support plate **6024** includes a mount connector **6038.** The mount connector **6038** may be included in the side wall of the guide trough **6034.** The device connector **1970** and the mount connector **6038** are not in contact with one another in **FIG. 10e****.** Additionally, the flange **6062** of the infusion pump **6060** is relatively loose within the guide trough **6034.** As mentioned above, any or all of the support plates **6022, 6024, 6026** may include mount connectors **6038** which may or may not be similarly disposed.

Referring now back to **FIG. 10d****,** the device connector **1970** and the mount connector **6038** are shown in contact with one another. Additionally, the flanges **6062** of the infusion pump **6060** are well retained within the guide troughs **6034, 6028** of the support plates **6022, 6024.** In order to bring the device connector **1970** and the mount connector **6038** into contact and firmly retain the flanges **6062** within the guide troughs **6034, 6028** it may be needed , in some specific embodiments, to actuate the clamp mechanism **1940** to the clamped position.

In **FIG. 10e****,** the clamp mechanism **1940** (not shown) is not in the clamped position. When the clamp mechanism **1940** is not in the clamped position, the flanges **6062** of the infusion pump **6060** may be easily moved around within the guide troughs **6034, 6028.** This may be helpful in inserting the infusion pump **6060** and in aligning the device connector **1970** and mount connector **6038.** The clamp mechanism **1940** may then be actuated as described above into the clamped position. This action may drive the device connector **1970** and the mount connector **6038** into contact and cause the flanges **6062** to cinch up against a side wall of the guide troughs 6028, 6034 thus retaining the infusion pump **6060** on the rack **6010.**

The healthcare provider may repeat the procedure for as many infusion pumps **6060** or medical devices as may be desired. Thus, the healthcare provider may provide each infusion pump **6060** or medical device with electrical power and a network connection to other infusion pumps **6060** or medical devices via the mount connector **6038** and the device connector **1970** without having to run multiple power and network cables that may complicate the setup procedure and clutter the environment around the patient. Moreover, any one of the infusion pumps **6060** or medical devices may be decoupled from the rack **6010,** of another infusion pump **6060** or medical device may be coupled to the rack **6010,** without having to detach or attach any additional cables. When treatment is complete, certain infusion pumps **6060** or medical devices may remain coupled to the rack **6010** and continue to treat the patient while others may be decoupled, again without having to detach any additional cables, and used to treat a different patient. Alternatively, a healthcare provider could transport the entire rack **6010** and any infusion pump **6060** or medical devices coupled thereto by decoupling the rack **6010** from a support structure **6014** (see **FIG. 9e****).** A rack **6010** that includes a handle **6020** and/or wheels may make transporting the rack **6010** and infusion pumps **6060** or medical devices easier in this scenario.

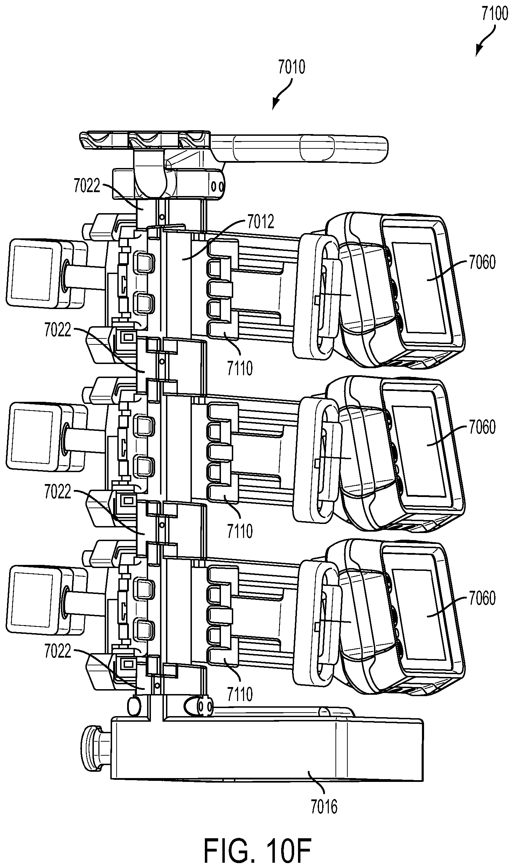

**FIG. 10f** shows yet another embodiment of a rack system **7100** which includes the example rack **7010** shown in **FIGS. 9f****-i.** The rack system **7100** may allow for a number of medical devices to be coupled onto the rack **7010.** The rack system **7100** may also be configured to provide power and/or a network connection to any medical devices coupled to the rack **7010.** Some embodiments of the rack system **7100** may differ, perhaps substantially, from the embodiment shown herein.

A number medical devices, which for exemplary purposes, are shown as an infusion pumps **7060,** are in place on the rack **7010** in **FIG. 10f****.** A medical device may also, for example, be a monitoring client, a PCA, physiological monitor, etc. As shown, a clamp apparatus **7110** is coupled to each of the infusion pumps **7060.** The clamp apparatuses **7110** shown are similar to those in the embodiments depicted above in **FIGS. 8a-8g****.** In other embodiments, the clamp apparatuses **7110** may be any suitable clamp described herein. The clamp apparatuses **7110** are shown in the open position in **FIG. 10f****.** The infusion pumps **7060** may be securely coupled to the rack **7010** by actuating the example clamp apparatuses **7110** to the closed position as described above in reference to **FIGS. 8a-8f****.** The collars **7022** ensure that as the clamp apparatuses **7110** are closed, the infusion pumps **7060** are in the proper orientation on the support pole **7012** of the rack **7010.**

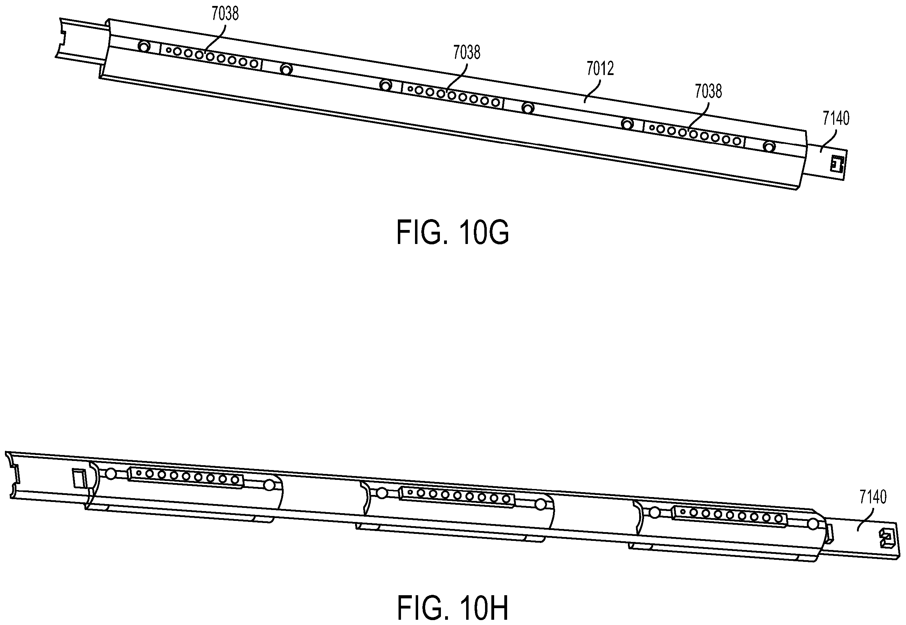

Referring now also to **FIG. 10g****,** a view of the support pole **7012** of the rack system **7100** is shown. The collars **7022** have been removed from the support pole **7012** in **FIG. 10g****.** As depicted also in **FIG. 9i****,** the support pole **7012** includes a number of mount connectors **7038.** At least a part of the mount connectors **7038** project through openings in and are proud of the tube which forms the support pole **7012** in the example embodiment. As mentioned above, the mount connectors **7038** may provide power to received medical devices. As also mentioned above, the mount connectors **7038** may be configured to enable a received medical device to communicate over CANbus and/or over a USB.

Referring now also to **FIG. 10h** the mount connectors **7038** may be included on a mount connector strip **7140.** The mount connector strip **7140** may help to facilitate assembly. When assembled, the mount connectors **7038** may be snap fit onto the mount connector strip **7140.** The mount connector strip **7140** may then be placed into the tube which forms the support pole **7012.** This ensures that the mount connectors **7038** are easily lined up with the openings in the tube of the support pole **7012.** Fasteners may then be used to fixedly couple the mount connectors **7038** on the mount connector strip **7140** to the support pole **7012.**

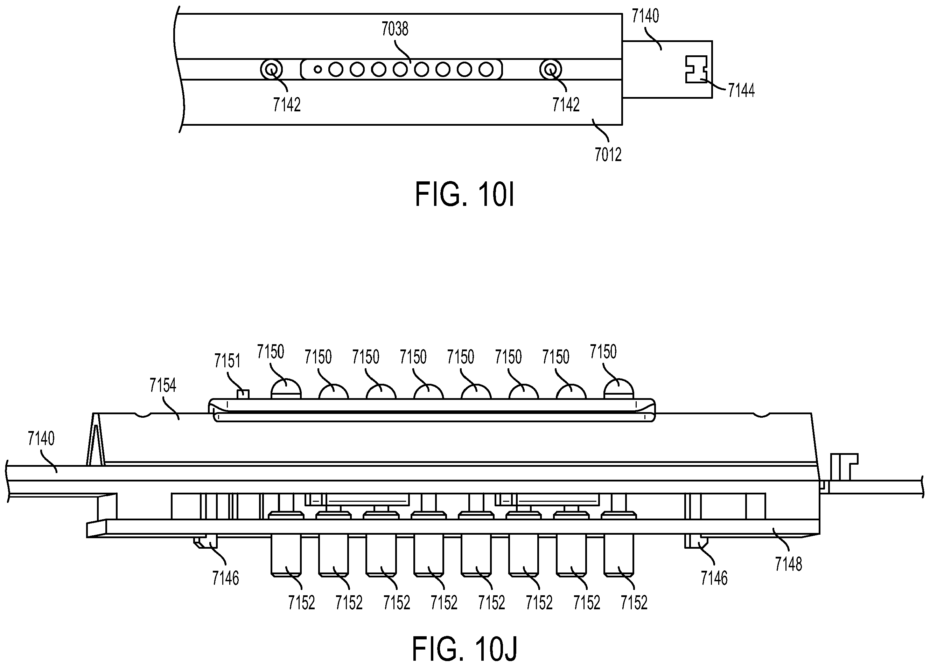

**FIG. 10i** depicts a close up view of a portion of the support pole **7012** for the rack system **7100.** As shown, an example mount connector **7038** is disposed in its assembled location. Holes **7142** are included in the support pole **7012.** Fasteners (not shown) may be inserted into the holes **7142** to fixedly couple the mount connectors **7038** on the mount connector strip **7140** to the support pole **7012.** An end of the mount connector strip **7140** is also shown protruding from the end of the support pole **7012.**

As shown, in some embodiments, the end of the mount connector strip **7140** may include a coupling feature **7144.** The coupling feature **7144** may be configured to receive a coupling feature **7144** on another mount connector strip **7140.** This may be useful in assembly of alternative embodiments of rack systems which are designed to receive a large number of pumps. In such embodiments, multiple mount connector strips **7140** may, for example, be coupled together and fed into a longer tube of a longer support pole **7012.** In embodiments a coupling feature **7144** on a mount connector strip **7140** may couple into the end cap of a support pole **7012.**

**FIG. 10j** depicts a side view of an example mount connector **7038** which has been snap fit into place on an example mount connector strip **7140.** The example mount connector **7038** includes a bottom portion **7148,** a top portion **7154,** connector pins **7150,** and sockets **7152.** Two snap fit features **7146** are visible and are holding the bottom portion **7148** in place on the mount connector strip **7140.** The top portion may also be held in place on the mount connector strip **7140** by snap fit features **7146.** In the embodiment depicted in **FIG. 10j****,** the snap fit features **7146** holding the top portion **7154** in place are not visible.

The connector pins **7150** may be biased to project off the top portion **7154** of the mount connector **7038.** In such embodiments, compression springs (not shown) may provide the biasing force. Also as shown, the two outside connector pins **7150** are prouder of the top portion **7154** than all other connector pins **7150.** These two connector pins **7150** may be connected to ground in order to ensure a ground connection is made before other connections. As shown, the connector pins **7150** may always be engaged in the sockets **7152.** This may be done to ensure that a received medical device will always be provided with electrical power and/or a network connection via the mount connector **7038.** In some embodiments, a different number of connector pins **7150** and sockets **7152** may be included.

The example mount connector **7038** also includes a hall sensor **7151.** The hall sensor **7151** may be tripped by a magnet on a received medical device or a clamp on a medical device, for example. When tripped, the hall sensor **7151** may create a delay of predetermined duration before power is supplied to the received medical device.

In the example embodiment, the bottom portion **7148** of the mount connector **7140** is a PCB. The PCB may be configured and populated such that it may allow a received medical device to communicate over a CANbus and over USB. In such embodiments, and referring now also to **FIG.** 10f, the base member **7016** of the example rack **7010** may include a USB port (not shown) to allow for connection to a computer. Such a computer may be used, for example, by trained personnel to update, access data or logs from, perform diagnostics on, etc. an attached medical device.

In order to reduce cost, in some embodiments, the PCB making up the bottom portion **7148** may not be entirely populated. For example, some PCBs may not include the components which would enable USB communication. A care facility, such as a hospital, thus may only have a few rack systems **7100** which are CANbus and USB configured. These may be used, for example, as special diagnostic rack systems **7100** when needed while less expensive, non-USB capable rack systems **7100,** may be used to provide everyday patient care.

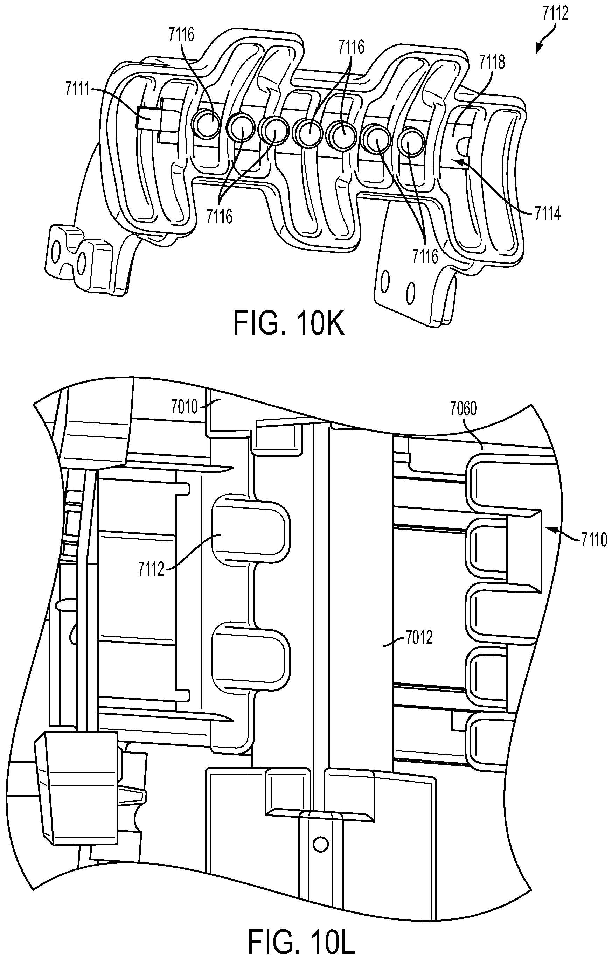

Referring now **FIG. 10k****,** an example embodiment of a gripper **7112** of a clamp apparatus **7110** is shown. The gripper **7112** is similar to the fixed gripper assembly **703** shown in **FIG. 8e****.** The gripper **7112** includes a device connector **7114.** As shown, the device connector **7114** includes a number of connector pins **7116** attached to a PCB **7118.** A high friction, compliant gripper material (not shown) may be attached to the gripping face of the gripper **7112.** In such embodiments, the gripper material may be overmolded onto the gripper **7112.** The gripper material, may include holes through which the connector pins **7116** may interface with a mount connector **7038** on a support pole **7012.** In some embodiments the gripper material may have a thickness such that the connector pins **7116** do not protrude out of the gripper material.

As shown, the gripper **7112** may also include a magnet **7111.** In such embodiments, the magnet may or may not be covered by the overmolded gripper material. The magnet **7111** may trip a hall sensor **7151** on a mount connector **7038** (see **FIG. 10j****)** as the gripper **7112** comes into close proximity of the mount connector **7038.** Tripping the hall sensor **7151** may cause a delay of predetermined duration before power is supplied from the mount connector **7038** to the device connector **7114** in the gripper **7112.**

Referring now also to **FIG. 10l**, a clamp apparatus **7110** attached to an infusion pump **7060** is depicted as it is being attached to the support pole **7012** of a rack **7010.** The gripper **7112** is in contact with the support pole **7012.** The device connector **7114** on the gripper **7112** is in contact with a mount connector **7038** (see, for example, **FIG. 10g****)** on the support pole **7012.** The clamp apparatus **7110** may be actuated to the clamped position to couple the infusion pump **7060** onto the rack **7010** and keep the device connector **7114** in contact with the mount connector **7038.** A connection from the device connector **7114** to the infusion pump **7060** (not shown) may be included to provide power and/or a network connection to the infusion pump **7060.** In some embodiments, a connector on the on infusion pump **7060** may be disposed such that the infusion pump **7060** may be provided power and/or a network connection via another connector on the clamp apparatus **7110** when the clamp apparatus **7110** and infusion pump **7060** are coupled together.

Once an infusion pump **7060** is attached, the healthcare provider may repeat the procedure for as many infusion pumps **7060** or medical devices as may be desired. Thus, the healthcare provider may provide each infusion pump **7060** or medical device with electrical power and a network connection to other infusion pumps **7060** or medical devices via the mount connector **7038** and the device connector **7114** without having to run multiple power and network cables that may complicate the setup procedure and clutter the environment around the patient. Moreover, any one of the infusion pumps **7060** or medical devices may be decoupled from the rack **7010,** or another infusion pump **7060** or medical device may be coupled to the rack **7010,** without having to detach or attach any additional cables. When treatment is complete, certain infusion pumps **7060** or medical devices may remain coupled to the rack **7010** and continue to treat the patient while others may be decoupled, again without having to detach any additional cables, and used to treat a different patient. Alternatively, a healthcare provider could transport the entire rack **7010** and any infusion pump **7060** or medical devices coupled thereto by decoupling the rack **7010** from a support structure such as an IV pole. A rack **7010** that includes a handle **7020** (best shown in **FIG. 9f****η** and/or wheels may make transporting the rack **7010** and infusion pumps **7060** or medical devices easier in this scenario.

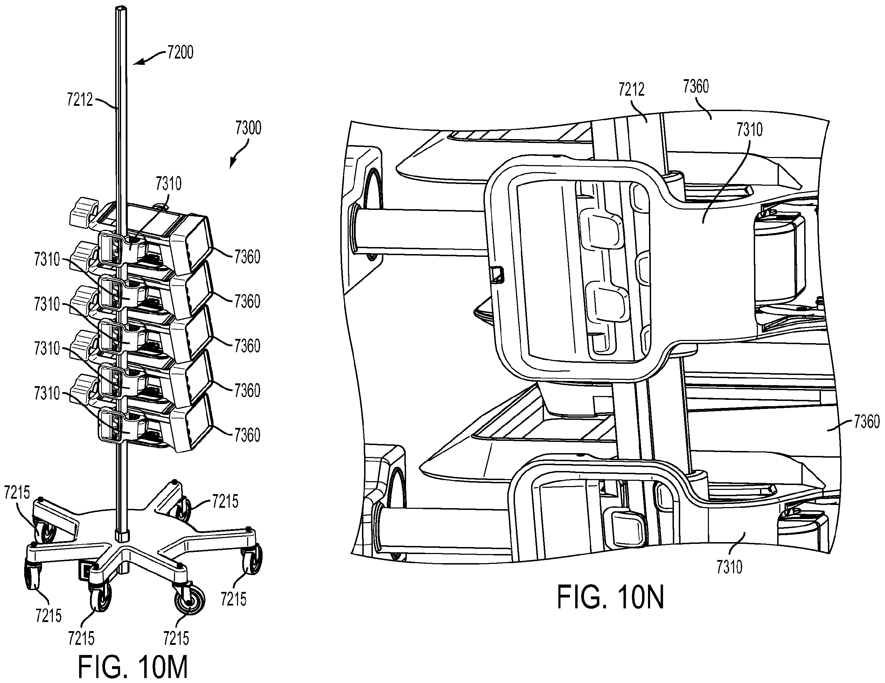

**FIG. 10m** depicts yet another embodiment of an example rack system **7300** including the example rack **7200** shown in **FIGS. 9j****-k.** The rack system **7300** may allow for a number of medical devices to be coupled onto the rack **7200.** The rack system **7300** may also be configured to provide power and/or a network connection to any medical devices coupled to the rack **7200.** Some embodiments of the rack system **7300** may differ from the embodiment shown herein.

As shown, the example rack system **7300** also comprises a number of medical devices **7360** and a number of clamps **7310** which are coupling the medicals devices **7360** to the rack **7200.** In the example embodiment, the medical devices **7360** are depicted as infusion pumps. Other medical devices **7360,** for example, a monitoring client, PCA, physiological monitor, etc. may also be coupled to the rack **7200.** Also as shown, the clamps **7310** are similar to the clamp apparatus **710** embodiments depicted in relation to **FIGS. 8a****-g.** In other embodiments, the clamps **7310** may differ. For example, the clamps **7310** may be, but are not limited to, other embodiments of clamps described herein.

As shown, only five medical devices **7360** are coupled to the rack **7200.** In the example embodiment shown in **FIG. 10m****,** there is space above the five coupled medical devices **7360** for additional medical devices **7360** to be coupled to the support pole **7212** of the rack **7200** if necessary. In some embodiments, a user may couple another rack (eg. any of racks **1810, 1910, 6010, 7010** shown and described in relation to **FIGS. 10a****-l)** to the rack system **7300.**

A clamp **7310** attached to a medical device **7360** may be actuated to a closed and clamped position around the support pole **7212** of the rack **7200** in order to couple a medical device **7360** to the rack **7200.** Referring now also to **FIG. 10n****,** two medical devices **7360** are shown coupled to the support pole **7212.** The clamps **7310** holding the medical devices **7360** in place on the support pole **7212** are shown actuated to the closed position. As mentioned above in relation to **FIG. 9j****,** one or more collars and/or alignment features may be included to help ensure that medical devices **7360** are coupled to the support pole **7212** in a correct and secure manner.

As mentioned in reference to **FIG. 9k****,** the example rack **7200** of the rack system **7300** may include a number of mount connectors **7238** which may provide power and/or a network connection to attached medical devices **7360.** In such embodiments of the rack system **7300,** the mount connectors **7238** may, for example, be similar to any those described above in relation to **FIGS. 10f-1****.** Additionally, the clamps **7310** of the example rack system **7300** may include a device connector which is similar to the device connector **7114** described above in relation to **FIGS. 10k-1****.** When a medical device **7360** is coupled to a support pole **7212** as shown in **FIGS. 10m****-n,** the medical device **7360,** a respective mount connector **7238** and respective device connector may operatively engage and provide power and/or a network connection to the medical device **7360.** In embodiments where the rack system **7300** is configured to allow other racks to couple into the rack system **7300,** the rack system **7300** may provide power and/or a network connection to the other racks in a similar manner.

Once a medical device **7360** is coupled to the rack **7200,** the healthcare provider may repeat the procedure for as many infusion pumps or medical devices **7360** as may be desired. Thus, the healthcare provider may provide each infusion pump or medical device **7360** with electrical power and a network connection to other infusion pumps or medical devices **7360** via the mount connector **7238** and a device connector without having to run multiple power and network cables that may complicate the setup procedure and clutter the environment around the patient. Moreover, any one of the infusion pumps or medical devices **7360** may be decoupled from the rack **7200,** or another infusion pump or medical device **7360** may be coupled to the rack **7200,** without having to detach or attach any additional cables. When treatment is complete, certain infusion pumps or medical devices **7360** may remain coupled to the rack **7200** and continue to treat the patient while others may be decoupled, again without having to detach any additional cables, and used to treat a different patient. Alternatively, a healthcare provider could transport the entire rack **7200** and any infusion pump or medical devices **7360** coupled thereto. A rack **7200** that includes a handle and/or wheels **7215** may make transporting the rack **7200** and infusion pumps or medical devices **7360** easier in this scenario.

### PROTECTIVE MECHANISMS

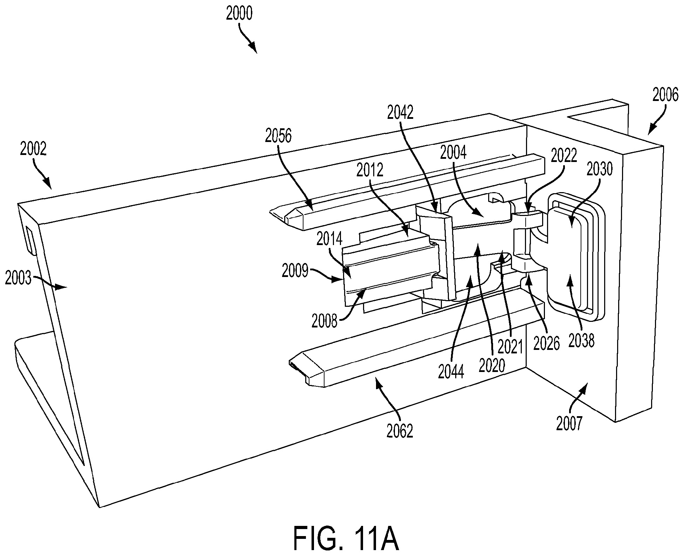

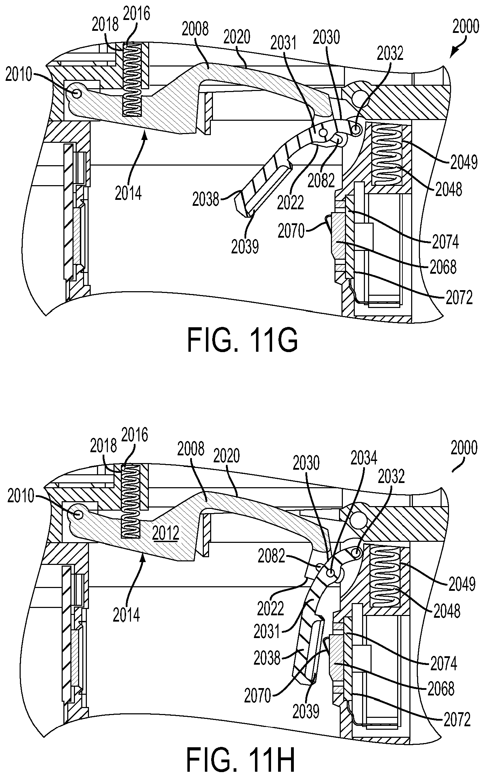

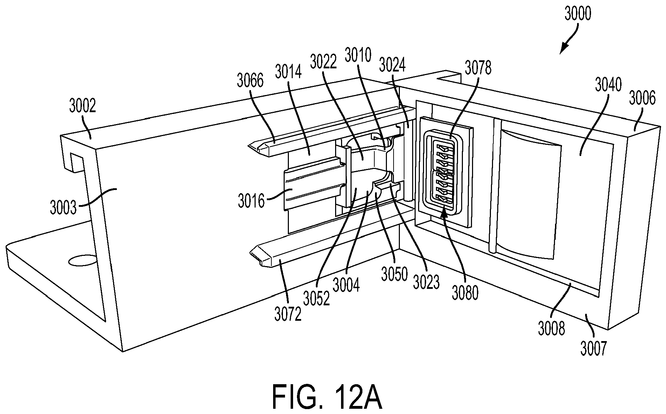

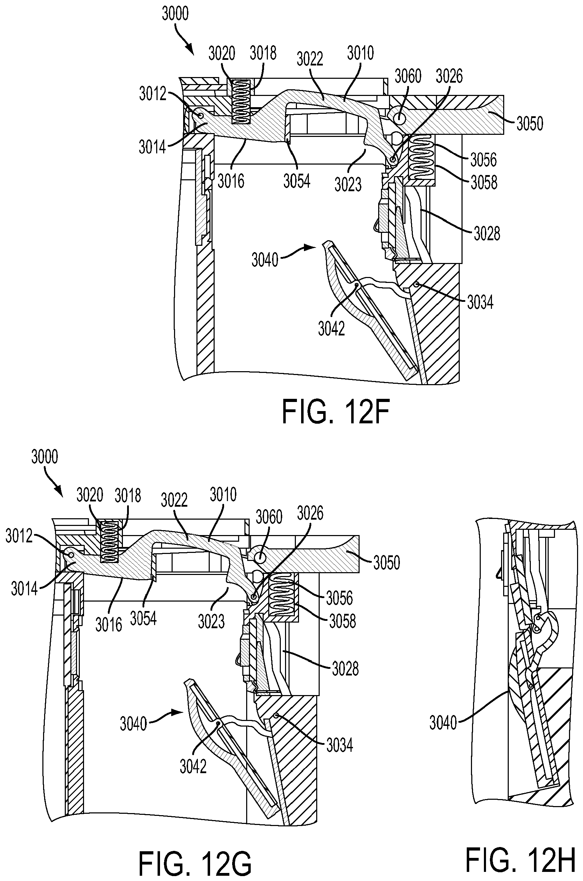

In addition, the medical device mounts of the rack **1810, 1910, 6010, 7010, 7200** may each include a protective mechanism that may protect the mount connector when not in use and during cleaning. For example, the mount connector **2068, 3078** may be covered by a pivotable-cover mechanism **2000** or a clamshell mechanism **3000.** Such protective mechanisms may also be used in other systems, for example, in medical systems beyond the rack embodiments described herein. **FIG. 11a** depicts an embodiment of a pivotable-cover mechanism **2000.** **FIG. 12a** depicts an embodiment of a clamshell mechanism **3000.** In the embodiments depicted in **FIG. 11a** and **12a** of the pivotable-cover and clamshell mechanisms **2000, 3000** respectively, coupling an electronic device, such as syringe pump, to the mechanism may automatically reveal the mount connector **2068, 3078** and allow the mount connector **2068, 3078,** to interface with the connector of the received electronic device.

### Pivotable-Cover Mechanism & Clamshell Mechanism: Common Components

In the embodiments of the present disclosure depicted in **FIGs. 11a** and **12a****,** the pivotable-cover mechanism **2000** and the clamshell mechanism **3000** may utilize similar components to receive an electronic device and initiate steps to reveal the respective mount connector **2068, 3078.** However, the two mechanisms **2000, 3000** are only two of many possible embodiments to complete the process of revealing the mount connector **2068, 3078.** The two example mechanisms **2000, 3000** may respectively include a guide member **2002, 3002,** a first rail projection **2056, 3066** and a second rail projection **2062, 3072.** The first rail projection **2056, 3066** and the second rail projection **2062, 3072** may be disposed on a guide member face **2003, 3003,** and run in parallel to one another along a longitudinal axis of the guide member **2002, 3002** such that the first and the second rail projections **2056, 3066, 2062, 3072** are capable of aligning the connector of a received device with the mount connector of the protective mechanisms **2068, 3078.**

The pivotable-cover mechanism **2000** and the clamshell mechanism **3000** may also each include a backstop member **2006, 3006** having a backstop member face **2007, 3007** to which the mount connector **2068, 3078** may be coupled. The backstop member face **2007, 3007** may be approximately perpendicular to the guide member face **2003, 3003.** The first and the second rail projections **2056, 3066, 2062, 3072** may extend to the backstop member face **2007, 3007** such that the first and the second rail projections **2056, 3066, 2062, 3072** are long enough to support and stabilize a received electronic device. The mount connector **2068, 3078** may be coupled to the backstop member face **2007, 3007** such that the mount connector **2068, 3078** is operatively positioned to interface with the connector of a received electronic device. The protective mechanisms **2000, 3000** may also include a bus **2072, 3082** that is operatively coupled to the respective mount connector **2068, 3078.** In preferred embodiments of the two protective mechanisms **2000, 3000,** the mount connector **2068, 3078** may be coupled to the backstop member face **2007, 3007** such that the mount connector **2068, 3078** is positioned between the first and the second rail projections **2056, 3066, 2062, 3072.**

To provide a mechanism for initiating the process of revealing the mount connector **2068, 3078,** the pivotable-cover mechanism **2000** and the clamshell mechanism **3000** may respectively include an actuation member **2008, 3010** that is pivotally coupled to the guide member **2002, 3002** at a first, stationary pivot **2010, 3012,** wherein the first, stationary pivot **2010, 3012** may be disposed between the backstop member face **2007, 3007** and a first end of the guide member **2002, 3002.** The actuation member **2008, 3010** may include a sloped portion **2012, 3014** that extends from the first, stationary pivot point **2010, 3012** towards the mount connector **2068, 3078.** The sloped portion **2012, 3014** may further include a sloped face **2014, 3016,** wherein the sloped face **2014, 3016** may be configured such that it may slope upwardly and out of the plane of the guide member face **2003, 3003** from the first, stationary pivot **2010, 3012** towards the mount connector **2068, 3078.** Thus, the sloped portion **2012, 3014** may protrude from the plane of the guide member **2002, 3002** when the actuation member **2008, 3010** is in a first position and the mount connector **2068, 3078** is covered. To reveal the mount connector **2068, 3078,** the actuation member **2008, 3010,** the sloped portion **2012, 3014,** and the sloped face **2014, 3016** may pivot about the first, stationary pivot **2010, 3012** in a first direction from the first position to a second position. When the actuation member **2008, 3010** is in the second position and the mount connector **2068, 3078** is uncovered, the sloped face **2014, 3016** may lie substantially in the plane of the guide member **2002, 3002.**

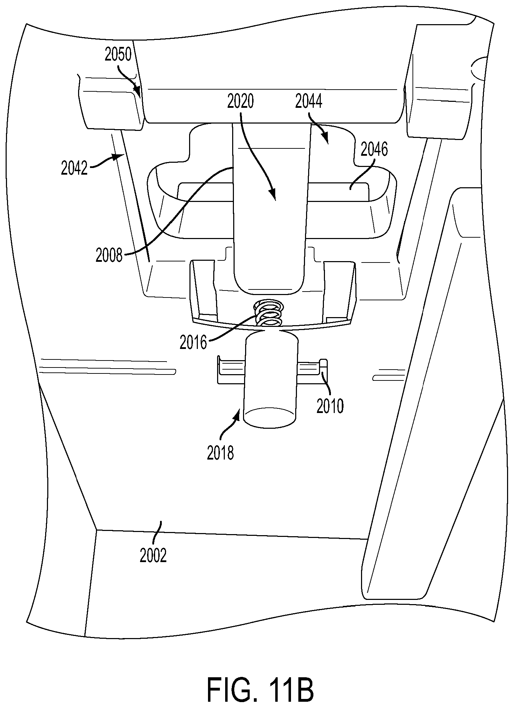

In addition, actuation springs **2016, 3018** may provide a mechanism for biasing the actuation members **2008, 3010** such that the actuation members **2008, 3010** may automatically return to the first position. To house the actuation springs **2016, 3018,** the guide members **2002, 3002** may include an actuation spring pocket **2018, 3020.** In certain embodiments, the actuation springs **2016, 3018** and the actuation spring pockets **2018, 3020** may be disposed on the guide members **2002, 3002** such that the actuation springs **2016, 3018** may be coupled to and exert a biasing force on the sloped portion **2012, 3014** of the actuation member **2008, 3010.** **FIG. 11b** depicts the actuation spring **2016** of an embodiment of the pivotable-cover mechanism **2000** acting on the sloped portion **2012** of the actuation member **2008,** wherein the actuation spring **2016** is disposed in the actuation spring pocket **2018.** The clamshell mechanism **3000** may be similarly configured.

The pivotable-cover mechanism **2000** and the clamshell mechanism **3000** may also respectively include a latch member **2042, 3050** that may be configured to latch onto a received electronic device when the latch member **2042, 3050** is in a latched position. The latch member **2042, 3050** may be disposed within an aperture or a guide member recess **2004, 3004** defined by the guide member **2002, 3002.** In addition, the guide member recess **2004, 3004** and the latch member **2042, 3050** may be adapted to extend below a portion of the backstop member **2006, 3006** such that a first end portion of the latch member **2042, 3050** may be adjacent to the sloped portion **2012, 3014** of the actuation member **2008, 3010,** and a second, opposite end portion of the latch member **2042, 3050** may be disposed within the guide member recess **2004, 3004** on the opposite side of the backstop member **2006, 3006.**

To allow a user to selectively engage or disengage the latch member **2042, 3050,** the latch member **2042, 3050** may be adapted to pivot about a third, stationary pivot **2050, 3060.** To latch onto a received electronic device, the latch member **2042, 3050** may include a latch projection **2046, 3054** disposed on a first end portion of the latch member **2042, 3050** and adapted to protrude from the guide member face **2003, 3003** when the latch member **2042, 3050** is in the latched position. The latch member **2042, 3050** may also include a latch member aperture **2044, 3052** through which the actuation member **2008, 3010** may pass, and thus, the latch member aperture **2044, 3052** may be sized and adapted to allow the actuation member **2008, 3010** to pivot through its full range of motion without interference from the latch member **2042, 3050.**

Like the actuation member **2008, 3010,** the latch member **2042, 3050** may be adapted such that a biasing force automatically returns the latch member **2042, 3050** to the latched position. Thus, a latch member spring **2048, 3056** may be disposed within a latch member spring pocket **2049, 3058** operatively defined by the guide member **2002, 3002** or the backstop member **2006, 3006.** In a preferred embodiment, the backstop member **2006, 3006** defines the latch member spring pocket **2049, 3058** such that the latch member spring **2048, 3056** may exert a downward force on the latch member **2042, 3050** between the third, stationary pivot **2050, 3060** and the second end portion of the latch member **2042, 3050.** To position the latch member projection **2046, 3054** at a desired height above the guide member face **2003, 3003,** the backstop member **2006, 3006** may be operatively sized and disposed on the guide member **2002, 3002** such that the backstop member **2006, 3006** may arrest pivotal movement of the latch member **2042, 3050** at the correct position relative to the guide member face **2003, 3003.** An alternative mechanism may include at least one arrester projection operatively disposed within the guide member recess **2004, 3004** and adapted to do the same. In a preferred embodiment, a first arrester projection **2052, 3062** (not shown) and a second arrester projection **2054, 3064** (not shown) may be disposed on opposite sides of the guide member recess **2004, 3004** between the third, stationary pivot point **2050, 3060** and the second end portion of the latch member **2042, 3050,** wherein the first and second arrester projections **2052, 2054, 3062, 3064** are capable of exerting a normal moment of force to counteract the moment of force that the latch member spring provides **2048, 3056.**

As will be understood by persons having ordinary skill in the art, the pivotable-cover and clamshell mechanisms **2000, 3000** and their components can be made from a variety of rigid, engineering materials. Possible materials include aluminum alloys, stainless steel alloys, steel alloys, and engineering polymers. In addition, a variety of coatings may be applied to the mechanisms **2000, 3000** and their components. Many of the possible coatings provide a means of reducing the likelihood of cross-contamination. Cross-contamination may pose a serious health risks to young and old patients and patients with weakened immune systems. Optionally, one or more of an antibacterial, an antiviral, or an antimicrobial coating may be applied to the structural components of the pivotable-cover and clamshell mechanisms **2000, 3000** to kill or inhibit the growth bacteria, viruses, fungi, and various other microorganisms. Exemplary coatings may include copper, copper particles, silver, silver particles, or other materials that have antibacterial, antiviral, or antimicrobial properties.

### A Pivotable-Cover Mechanism

While both the pivotable-cover mechanism **2000** and the clamshell mechanism **3000** may include the aforementioned elements to receive an electronic device and initiate the steps to reveal the mount connector **2068, 3078,** the pivotable-cover mechanism **2000** and the clamshell mechanism **3000** employ additional, different mechanical linkages to complete the task.

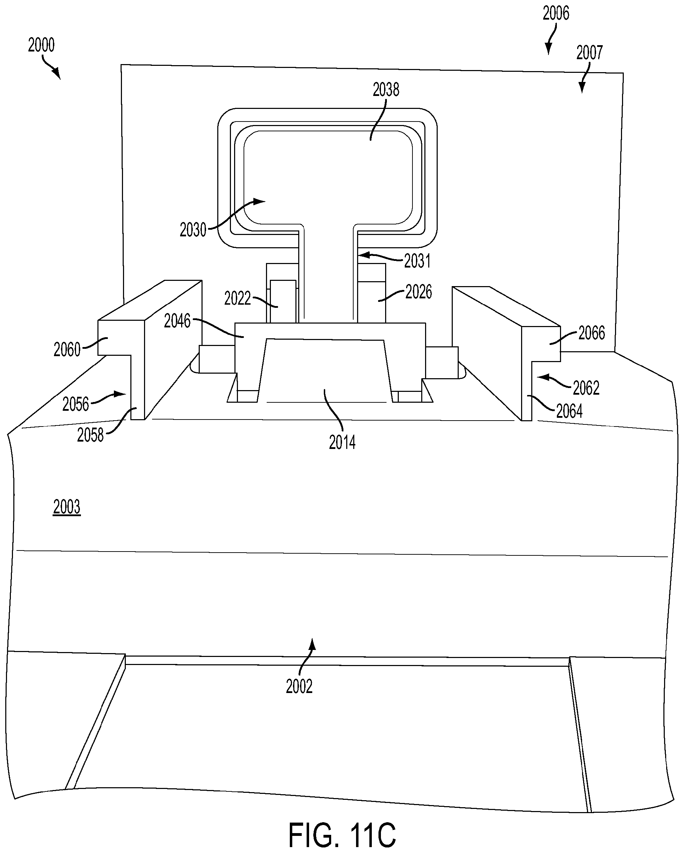

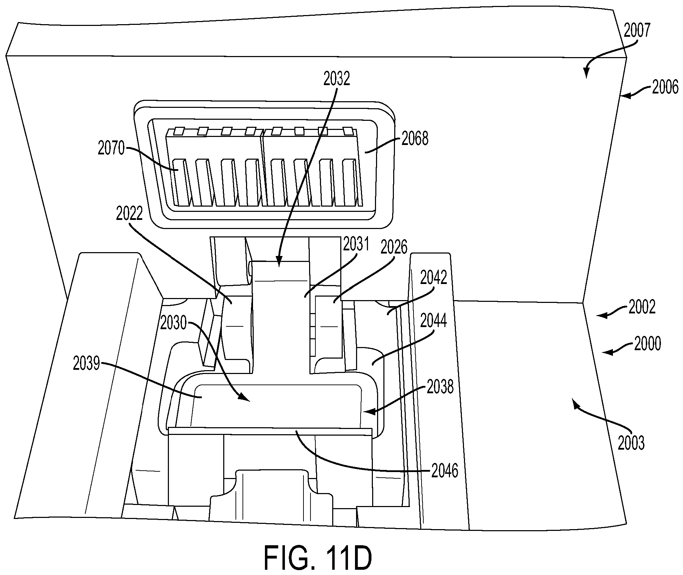

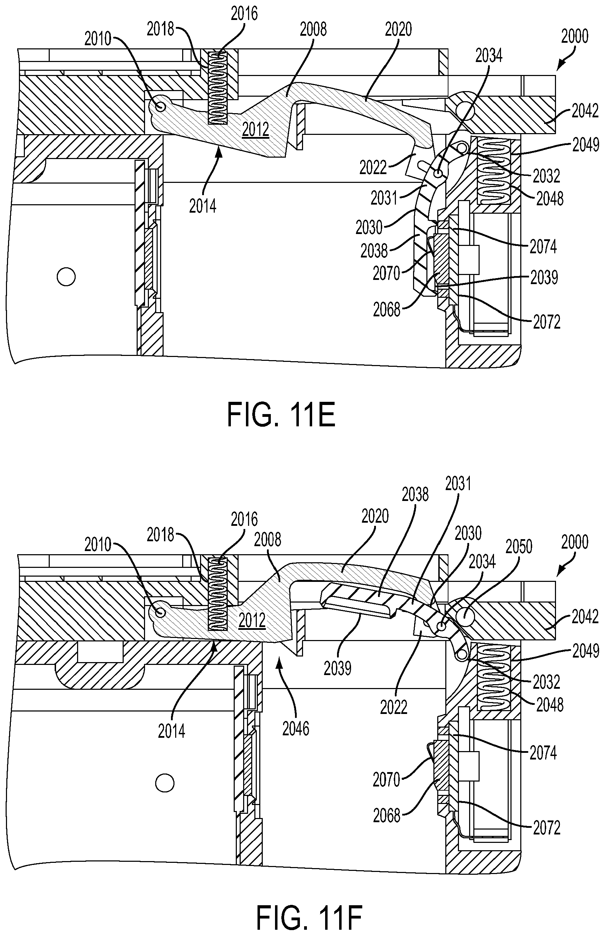

**FIGS. 11a-11i** depict an exemplary embodiment of the pivotable-cover mechanism **2000.** **FIGS. 11a** and **11c** depict an embodiment of the pivotable-cover mechanism **2000** wherein the mount connector **2068** is covered by a protective member **2030.** **FIG. 11e** depicts a cross-section of the pivotable-cover mechanism **2000** wherein the mechanism has received an electronic device, the actuation member **2008** is in the second position, and the mount connector **2068** is uncovered. In addition, **FIG. 11e** depicts a number portions that may comprise the actuation member **2008,** including: the sloped portion **2012,** a bridge portion **2020,** a first channeled projection **2022,** and a second channeled projection **2026.** **FIG. 11d** more clearly shows the first and second channeled projections **2022, 2026** and the gap between them.

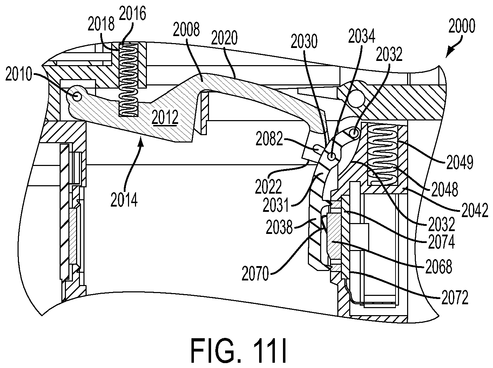

The protective member **2030** of the pivotable-cover mechanism **2000** may be pivotally coupled to either the backstop member **2006** or the guide member **2002** at a second, stationary pivot **2032.** As depicted in **FIG. 11f****,** the protective member **2030** is coupled to the backstop member **2006** in this particular embodiment. The third, stationary pivot **2050** lies on the guide member **2002** immediately above the second, stationary pivot **2032.** Additionally, the protective member **2030** may comprise a cover portion **2038** and a stem portion **2031** that couples the cover portion **2038** to the backstop member **2006.** The cover portion **2038** may be configured to cover the mount connector **2068.** The protective member **2030** may also include first and second actuation projections **2034, 2036** (not shown) wherein the first and second actuation projections **2034, 2036** are adapted to respectively engage the first channel **2024** and the second channel **2028** of the first and second channeled projections **2022, 2026** of the actuation member **2008.** Therefore, the first and second channeled projections **2022, 2026** may be shaped and sized such that the pivotal moment of the actuation member **2008** from the first position to the second position may pivot the protective member **2030** from a protective position, wherein the cover portion **2038** covers the mount connector **2068,** to a non-protective position, wherein the protective member **2030** is disposed within the guide member recess **2004.** Consequently, the bridge portion **2020** of the actuation member **2008** may be curved between the sloped portion **2012** and the first and second channeled projections **2022, 2026** to allow the protective member **2030** to nest within the actuation member **2008** when the protective member **2030** is in the non-protective position. **FIGS. 11e-11i** depict the positions of the actuation member **2008** and the protective member **2030** as they move from their respective positions when the mount connector **2068** is uncovered and in contact with the connector of an electronic device **(****FIG. 11e****)** to their respective positions when the mount connector **2068** is covered **(****FIG. 11i****).**

In addition, the pivotable-cover mechanism **2000** may include a latch member **2042** having a latch member aperture **2044** adapted to operatively receive a portion of the protective member **2030** when the protective member **2030** is in the non-protective position. **FIG. 11d** depicts the protective member **2030** in the non-protective position and shows the latch member aperture **2044** receiving the cover portion **2038** of the protective member **2030.** **FIG. 11d** also depicts the stem portion **2031** of the protective member **2030** disposed between the first and second channeled projections **2022, 2026.**

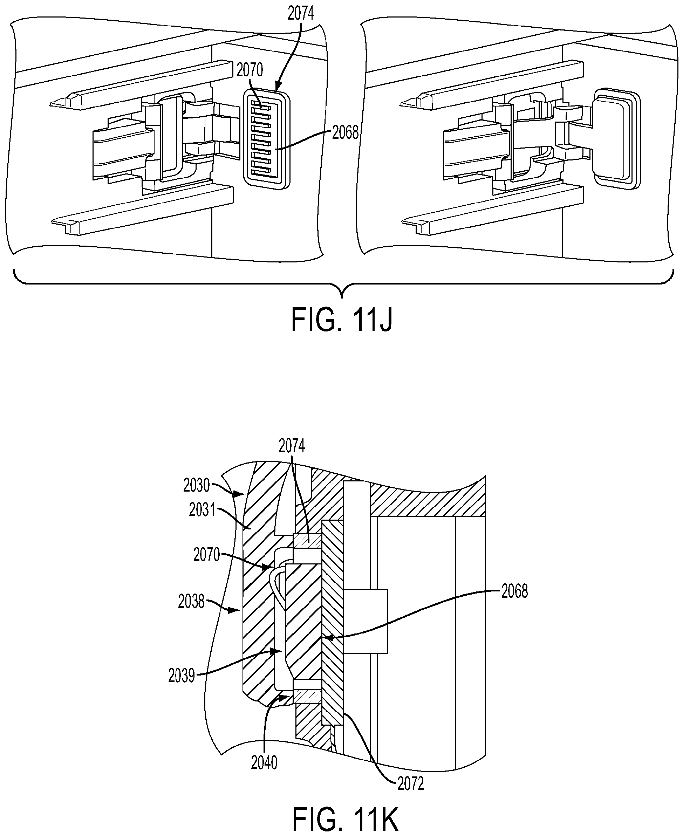

To protect the mount connector **2068** during cleaning and normal maintenance, the pivotable-cover mechanism **2000** may include a compliant gasket **2074** configured to, when the protective member **2030** is in the protective position, mechanically seal the mount connector **2068** within a cover portion recess **2039** defined by a perimeter rib **2040** of the cover portion **2038.** In an exemplary embodiment depicted in **FIG. 11k****,** the compliant gasket **2074** may encompass and abut the mount connector **2068.** In a preferred embodiment, the actuation spring **2016,** acting through the actuation member **2008,** may bias the protective member **2030** such that the protective member **2030** may automatically return to the protective position. Consequently, the spring force of the actuation spring **2016** may enable the perimeter rib **2040** to contact and compress the compliant gasket **2074** when the protective member **2030** is the protective position. Thus, the perimeter rib **2040** may create mechanical seal with the compliant gasket **2074.** **FIG. 11k** depicts an exemplary embodiment of the pivotable-cover mechanism **2000** wherein the perimeter rib **2040** has created a mechanical seal with the compliant gasket **2074.**

As will be appreciated by persons having ordinary skill in the art, the compliant gasket **2074** may be made of any suitably compliant material; such materials may include, but are not limited to, isobutylene, natural rubber, neoprene, styrene butadiene, and silicone. In addition, the compliant gasket material may be chosen so that the compliant gasket **2074** is capable of resisting corrosion from solvents ordinarily used for cleaning device surfaces.

**FIGS. 11j** and **11k** depict an embodiment of the present disclosure, wherein the mount connector **2068** is of a type having multiple spring contacts **2070.** To protect the spring contacts **2070** when the cover portion **2038** covers the mount connector **2068,** the cover portion recess **2039** may include a compressible material, including but not limited to a polyurethane foam, disposed within the cover portion recess **2039** and adapted to receive the spring contacts **2070.**

### A Clamshell Mechanism

Whereas the protective member **2030** of the pivotable-cover mechanism **2000** is capable of pivoting towards the guide member **2002** to expose the mount connector **2068,** the clamshell mechanism **3000** includes a cover member **3040** that is capable of pivotally sliding across the backstop member face **3007** to expose the mount connector **3078.**

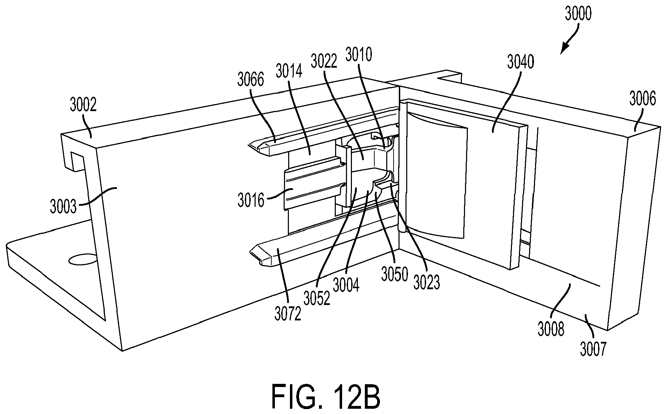

**FIG. 12a** depicts an exemplary embodiment of the clamshell mechanism **3000,** wherein the cover member **3040** is in a non-protective position and the mount connector **3078** is exposed. **FIG. 12b** depicts the same embodiment of the clamshell mechanism **3000,** wherein the cover member **2040** is in a protective position and the mount connector **3078** is covered.

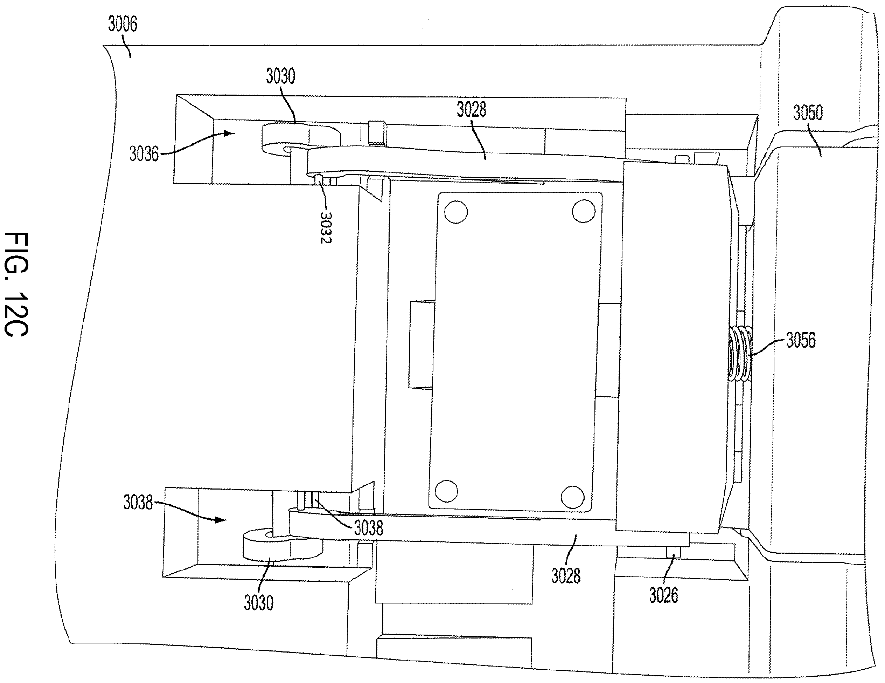

To pivotally slide the cover member **3040** across the backstop member face **3007,** the clamshell mechanism **3000** may include at least one first link-member **3028** and at least one second link-member **3030** to enable movement of the actuation member **3010** to pivotally slide the cover member **3040.** In the embodiment of the clamshell mechanism **3000** depicted in **FIG. 12a****,** the actuation member **3010** includes a sleeve portion **3024** that is coupled to the bridge portion **3022** and that is opposite to the sloped portion **3014.** **FIG. 12c** depicts an embodiment having a pair of first-link members **3028** that are pivotally coupled at respective first end portions to the sleeve portion **3024** of the actuation member **3010** at a first moveable pivot **3026.** In addition, the pair of first link-members **3028** of the embodiment depicted in **FIG. 12c** are pivotally coupled at respective second end portions to respective first end portions of a pair of second link-members **3030** at a second moveable pivot **3032.** As should be understood by persons having ordinary skill in the art, the backstop member **3006** is adapted to and coupled to the guide member **3002** so as to enable the at least one first link-member **3028** to couple with the actuation member **3010** and pass behind the backstop member face **3007.** Moreover, the backstop member **3006** may be adapted to enable the at least one first and second link-members **3028, 3030** to move through their respective ranges of motion without interference from the backstop member **3006.**

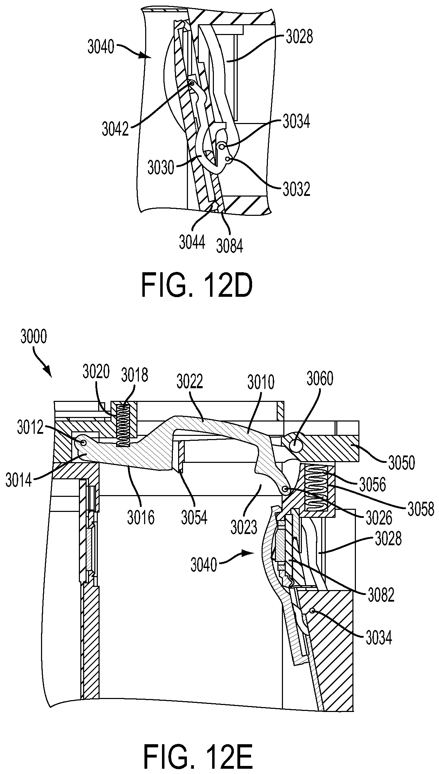

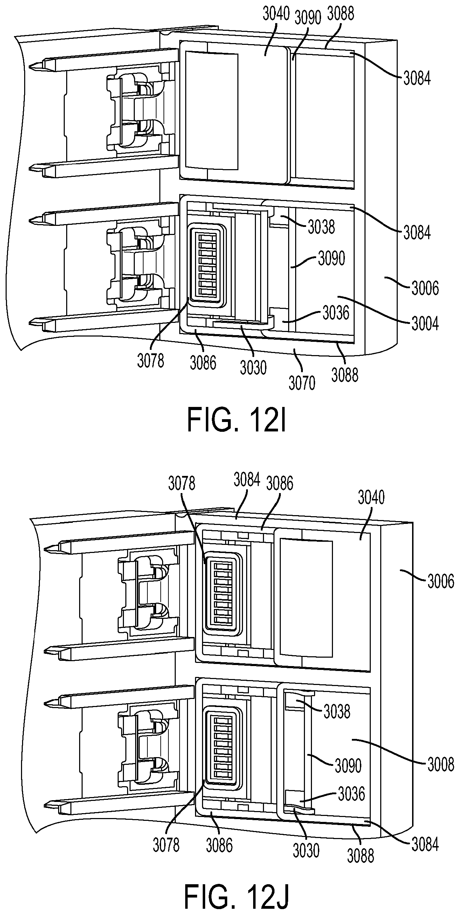

By way of movement of the at least one second link-member **3030,** the cover member **3040** may pivotally slide across the backstop member face **3007** to reveal the mount connector **3078.** In the embodiment of the clamshell mechanism **3000** depicted in **FIGS. 12d-12h****,** the respective first end portions of the pair of second link-members **3030** are coupled to respective second end portions of the pair of first link-members **3028** at the second moveable pivot **3032** and to the backstop member **3006** at a stationary clamshell pivot **3034.** **FIGS. 12d-12h** also depict the pair of second link-members **3030,** wherein respective second end portions of the pair of second link-members **3030** are coupled to the cover member **3040** at a third moveable pivot **3042.** In addition, the backstop member **3006** defines first and second pass-thru apertures **3036, 3038,** depicted in **FIGS. 12i** and **12j****,** through which the pair of second-link members **3030** respectively pass to couple with the cover member **3040.** The first and second pass-thru apertures **3036, 3038** may be respectively shaped and sized so as to enable the pair of second link-members **3030** to move through their respective ranges of motion without interference from the backstop member **3006.**

As the clamshell mechanism **3000** progresses through the stages of uncovering the mount connector **3078** depicted in **FIGS. 12e-12h****,** movement of the actuation member **3010** in a first direction from a first position to a second position may cause the cover member **3040** to move from a protective position, wherein the mount connector **3078** is covered, to a non-protective position, wherein the mount connector **3078** is uncovered. During the uncovering process, pivotal movement of the actuation member **3010** in the first direction may cause the pair of first link-members **3028** to pivot slightly about the first moveable pivot **3026** and to move in a substantially translational direction towards the guide member **3002.** In turn, the substantially translational movement of the pair of first link-members **3026** may cause the second moveable pivot **3032** to transit a plane that is parallel to the guide member face **3003** and that passes through the stationary clamshell pivot **3034.** In doing so, the second moveable pivot **3032** may move from a first position to a second position, wherein the second moveable pivot **3032** is closer to the guide member **3002** in the second position than in the first position. Movement of the second moveable pivot **3032** from the first position to the second position may thereby cause the pair of second link-members **3030** to pivot about the stationary clamshell pivot **3034** and pivotally slide the cover member **3040** from the protective position to the non-protective position. The reverse process, depicted in the progression of figures from **FIG. 12h** to **FIG. 12e****,** may be used to cover the mount connector **3078.**

Like the pivotable-cover mechanism **2000,** an actuation spring **3018** may be used to bias the clamshell mechanism **3000** so that the actuation member **3010** automatically returns to the first position under the force of the actuation spring **3018,** and acting through the at least one first link-member **3028** and the at least one second link-member **3030,** the actuation member **3010** may thereby cause the cover member **3040** to automatically return to the protective position.

To house the cover member **3040** when it is in the non-protective position, the backstop member may define a backstop member recess **3008** that the cover member **3040** may pivotally slide into as it pivotally slides across the backstop member face **3007** and exposes the mount connector **3078.** **FIGS. 12e-12h** depict the progression of the cover member **3040** as it uncovers the mount connector **3078** and slides into the backstop member recess **3008.** As depicted in **FIGS. 12e-12h****,** the deepest portion of the backstop member recess **3008** may be an end portion that is furthest from the guide member **3002,** and the backstop member recess **3008** may slope from the deepest portion towards the backstop member face **3007.** The backstop member recess **3008** may be shaped and sized such that the cover member **3040** lies below the plane of the backstop member face **3007**when the cover member **3040** is in the non-protective position. In addition, the backstop member recess **3008** may be shaped and sized such that the cover member **3040** may surround and cover the mount connector **3078** when the cover member is in the protective position.

The clamshell mechanism **3000** may also include a compliant gasket system **3084** designed to protect the mount connector **3078,** wherein the compliant gasket system **3084** includes a first gasket portion **3086,** a second gasket portion **3088,** and a transitional gasket portion **3090.** The second gasket portion **3088** may mirror the first gasket portion **3086** and may be disposed on an opposite side of the transitional gasket portion **3090.** **FIGS. 12i** and **12j** depict an exemplary embodiment of a series of the clamshell mechanisms **3000** with compliant gasket systems **3084.** **FIG. 12i** depicts the cover member **3040** in relation to the compliant gasket system **3084** when the cover member **3040** is in the protective position. In contrast, **FIG. 12j** depicts the cover member **3040** in relation to the compliant gasket system **3084** when the cover member **3040** is in the non-protective position. As depicted in **FIG. 12d****,** the cover member **3040** may include a perimeter rib **3044** that may be shaped and sized such that, when cover member **3040** is in the protective position, the perimeter rib **3044** may compress the first gasket portion **3086** and a portion of the transitional gasket portion **3090.** Likewise, **FIG. 12j** depicts the mechanical seal created by the perimeter rib **3044** (see **FIG. 12d**) the second gasket portion **3088,** and a portion of the transitional gasket portion **3090.** In both of **FIGS. 12i** and **12j****,** the perimeter rib **3044** compresses respective portions of the transitional gasket portion **3090** such that the first and second pass-thru apertures **3036, 3038** are within the mechanical seal created by the perimeter rib **3044** and the compliant gasket system **3084.** The first and second pass-thru apertures **3036, 3038** may be contained with the mechanical seal to protect at least the first and second pairs of link-members **3028, 3030** against the threat of contamination from foreign matter, particularly during cleaning of the clamshell mechanism **3000.**

Like the compliant gasket **2074** of the pivotable-cover mechanism **2000,** the compliant gasket system **3084** of the clamshell mechanism **3000** may be made of any suitably compliant material; such materials may include, but are not limited to, isobutylene, natural rubber, neoprene, styrene butadiene, and silicone. In addition, the compliant gasket material may be chosen so that the compliant gasket system **3084** is capable of resisting corrosion from solvents ordinarily used for cleaning device surfaces.

Additionally, and like the pivotable-cover mechanism **2000,** the mount connector **3078** of the embodiment depicted in **FIGS. 12a-12j** may be of a type having multiple spring contacts **3080.** Moreover, the cover member **3040** may likewise include a compliant material, such as but not limited to a polyurethane foam, that may be shaped and sized to receive and protect the spring contacts **3080** when the cover member **3040** is in the protective position.

### A SYSTEM FOR RECEIVING A DEVICE

The aforementioned pivotable-cover or clamshell mechanisms **2000, 3000** may be an embodiment of a protective mechanism **5002** that is a first element of a system for receiving a device **5000.** A second element of the system for receiving a device **5000** may be a receivable device **5020** that may include a device connector **5022** and a means for being received by the protective mechanism **5002,** such as the pivotable-cover or clamshell mechanisms **2000, 3000.**

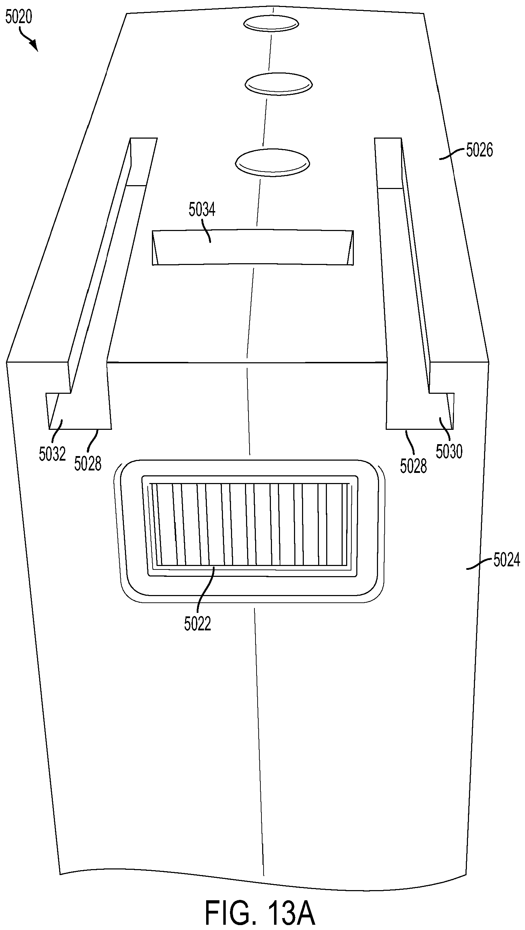

**FIG. 13a** depicts an exemplary embodiment of a receivable device **5020,** wherein the receivable device includes a device connector **5022** that is disposed on a first face **5024** of the receivable device **5020** such that the device connector **5022** is adapted to interface with a mechanism connector **5004** like the respective mount connectors **2068, 3078** of the pivotable-cover and clamshell mechanisms **2000, 3000.**

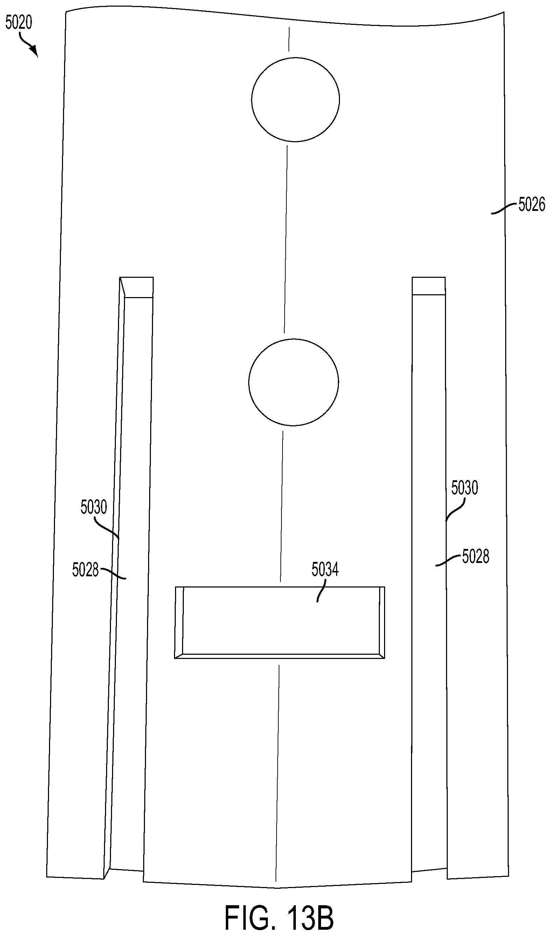

To receive the receivable device **5020,** the protective mechanism **5002** may include at least one rigid member **5008** disposed on a guide member **5006.** The at least one rigid member **5008** may be similar to the respective first and second rail projections **2056, 3066, 2062, 3072** of the pivotable-cover and clamshell mechanisms **2000, 3000** as described herein. The receivable device **5020** may include at least one channel **5028** defined by a second face **5026** of the receivable device **5020** and each of the at least one channel **5028** may be adapted to receive a respective at least one rigid member **5008** of the protective mechanism **5002.** In embodiments of the protective mechanism **5002** that include respective first and second rail projections **2056, 3066, 2062, 3072** like those of the pivotable-cover and clamshell mechanisms **2000, 3000,** the at least one channel **5028** may comprise a first channel **5030** adapted to receive the respective first rail projection **2056, 3066** and a second channel **5032** adapted to receive the respective second rail projection **2062, 3072.** **FIGS. 13a** and **13b** depict an embodiment of the receivable device **5020** that includes the aforementioned first and second channels **5030, 5032** that are adapted to receive the respective first and second rail projections **2056, 3066, 2062, 3072** of the pivotable-cover and clamshell mechanisms **2000, 3000.**

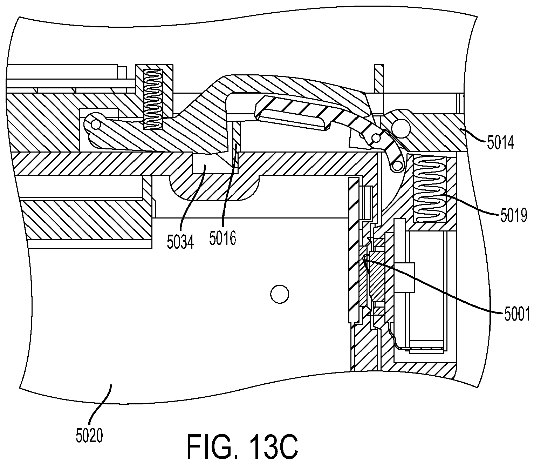

To secure the receivable device **5020** in place after the protective mechanism **5002** receives the receivable device **5020,** the protective mechanism **5002** may include a latch member **5014** having a latch member projection **5016** that engages a latch recess **5034** defined by the second face **5026** of the receivable device **5020.** **FIGS. 13a** and **13b** depict an exemplary embodiment having a latch recess **5034,** and **FIG. 13c** depicts how the latch member projection **5016** may engage the latch recess **5034** to secure a received receivable device **5020.** Additionally, the protective mechanism **5002** may include any of the features discussed above with respect to the pivotable-cover and clamshell mechanisms **2000, 3000;** such features may include, but are not limited to, a latch member spring **2048, 3056** and latch member aperture **2044, 3052,** for example.

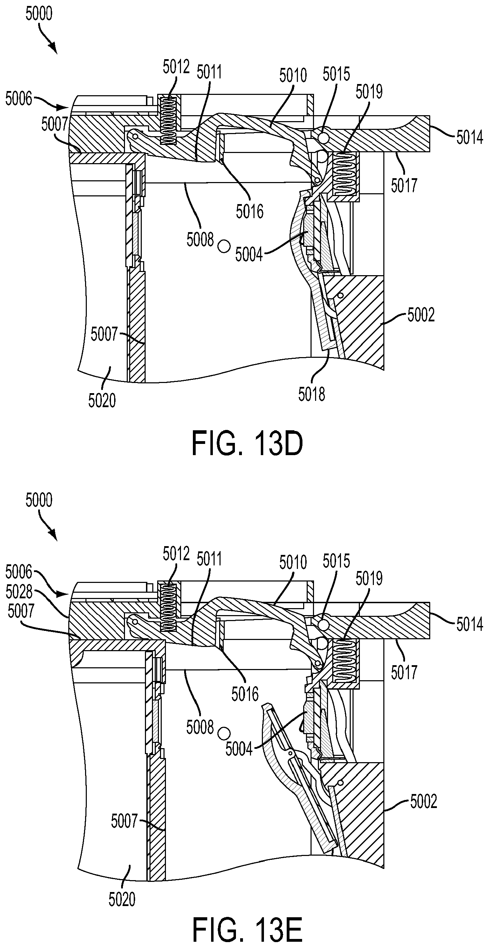

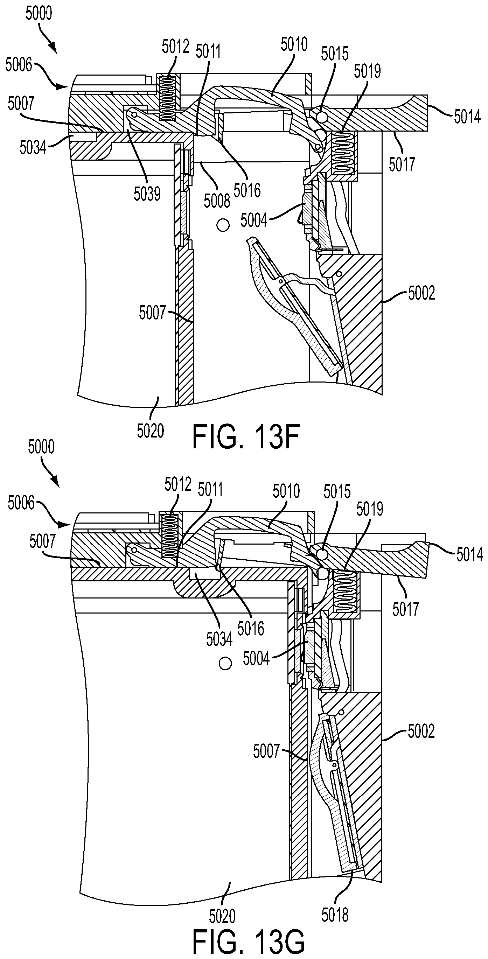

When used in combination, the receivable device **5020** may cause the protective mechanism **5002** to automatically reveal the mechanism connector **5004** as the protective mechanism **5002** receives the receivable device **5020,** thereby allowing the mechanism connector **5004** and the device connector **5022** to interface with each other. For example, the progression of **FIGS. 13d-13g** demonstrates how receiving a receivable device **5020** may cause the clamshell mechanism **3000** to automatically reveal a mechanism connector **5004.** As each of the at least one rigid member **5010** of the a respective protective mechanism slides within a corresponding at least one channel **5028** of the receivable device **5020,** the receivable device **5020** engages the sloped face **5011** of the actuation member **5010** as it slides towards the backstop member face **5007** and mechanism connector **5004** (**FIG. 13d**). As the receivable device **5020** continues to slide toward the backstop member face **5007,** the receivable device **5020** may begin to pivot the actuation member **5010** in a first direction from a first position to a second position (**FIGS. 13e** and **13f**). As described above with respect to the pivotable-cover and clamshell mechanisms **2000, 3000,** or other embodiment of the protective mechanism **5002,** this pivotal movement of the actuation member **5010** may cause the cover member **5018** to reveal the mechanism connector **5004.** The receivable device **5020** may slide toward the backstop member face **5007** until it is in a received position where it contacts the backstop member face **5007** and the device connector **5022** interfaces with the mechanism connector **5004** (**FIG. 13g**).

The progression from **FIG. 13g** to **FIG. 13d** depicts the reverse process wherein decoupling the receivable device **5020** from the protective mechanism **5002** may cause the actuation member **5010** to pivot from the second position to the first position under the force of an actuation spring **5012** and thereby cover the mechanism connector **5004.**

As should be understood by persons having ordinary skill in the art, the at least one protective mechanism **5002** may be designed such that the cover member **5020** is capable of pivoting from the protective position to the non-protective position as the receivable device **5020** slides towards the backstop member face **5007** and the mechanism connector **5004.** The pivotable-cover and clamshell mechanisms **2000, 3000** described above are but two exemplary embodiments wherein the mechanical linkages and the constituent components are shaped and sized so as to pivot or otherwise move through their respective ranges of motion while the receivable device **5020** causes the actuation member **5010** to pivot as the receivable device **5020** coupled or decouples with a protective mechanism **5002.**

When the receivable device **5020** is in the received position, the latch member **5014** of the respective protective mechanism **5002** may pivot to a latched position such that the latch member projection **5016** engages the latch recess **5034** defined by a second face of the receivable device **5020.** In a preferred embodiment of the system for receiving a device **5000,** the latch recess **5034** may be disposed on the first face of the receivable device **5020** such that it is between the first channel **5030** and the second channel **5032.** Like the sloped face **5011,** the latch member projection **5016** may slope away from the plane of the guide member **5006** and towards the backstop member face **5007** such that the receivable device **5020** may cause the latch member projection **5016** and latch member **5014** to pivot out of the way as the receivable device **5020** slides towards the backstop member face **5007.** As described above with respect to the pivotable-cover and clamshell mechanisms **2000, 3000** a latch spring **5019** may be used to automatically restore the latch member **5014** to the latched position when the receivable device **5020** is in the received position.

To decouple the receivable device **5020** from the protective mechanism **5002,** pivoting the latch member **5014** away from the latched position may cause the latch member projection **5016** to disengage from the latch recess **5034** and allow the receivable device **5020** to slide in the opposite direction away from the backstop member face **5007** and the mechanism connector **5004.** Pivoting the latch member **5014** away from the latched position may be achieved by manually pulling on a latch member release tab **5017** that is disposed on an opposite end portion of the latch member **5014** with respect to the latch member projection **5016.** Where the latch member release tab **5017** is on an opposite side of the latch member pivot point **5015** with respect to the latch member projection **5016,** pulling towards the mechanism connector **5004** causes the latch member projection **5016** to disengage from the latch recess **5034.**

Various alternatives and modifications can be devised by those skilled in the art without departing from the disclosure. Accordingly, the present disclosure is intended to embrace all such alternatives, modifications and variances. Additionally, while several embodiments of the present disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. And, those skilled in the art will envision other modifications within the scope and spirit of the claims appended hereto. Other elements, steps, methods and techniques that are insubstantially different from those described above and/or in the appended claims are also intended to be within the scope of the disclosure.

The embodiments shown in drawings are presented only to demonstrate certain examples of the disclosure. And, the drawings described are only illustrative and are nonlimiting. In the drawings, for illustrative purposes, the size of some of the elements may be exaggerated and not drawn to a particular scale. Additionally, elements shown within the drawings that have the same numbers may be identical elements or may be similar elements, depending on the context.

Where the term "comprising" is used in the present description and claims, it does not exclude other elements or steps. Where an indefinite or definite article is used when referring to a singular noun, e.g. "a" "an" or "the", this includes a plural of that noun unless something otherwise is specifically stated. Hence, the term "comprising" should not be interpreted as being restricted to the items listed thereafter; it does not exclude other elements or steps, and so the scope of the expression "a device comprising items A and B" should not be limited to devices consisting only of components A and B. This expression signifies that, with respect to the present disclosure, the only relevant components of the device are A and B.

Furthermore, the terms "first", "second", "third" and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the embodiments of the disclosure described herein are capable of operation in other sequences and/or arrangements than are described or illustrated herein.

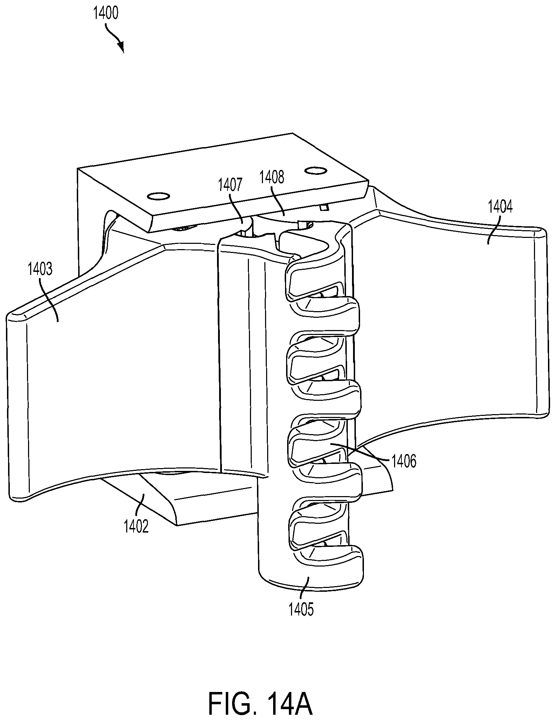

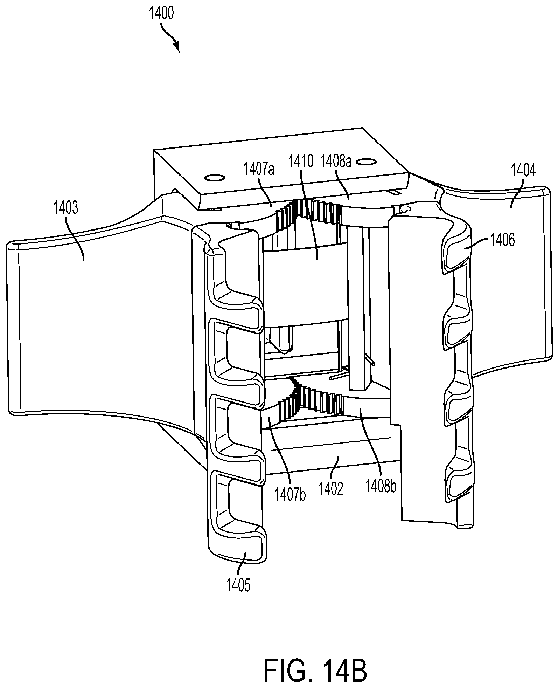

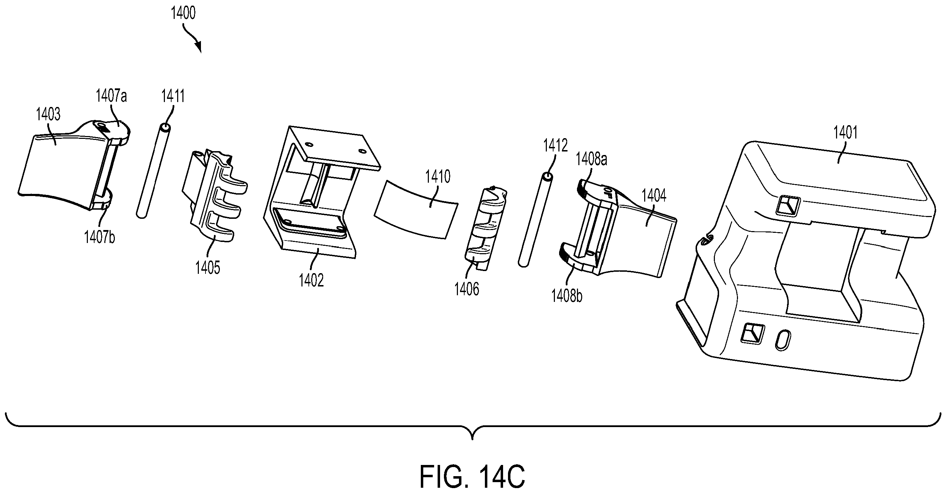

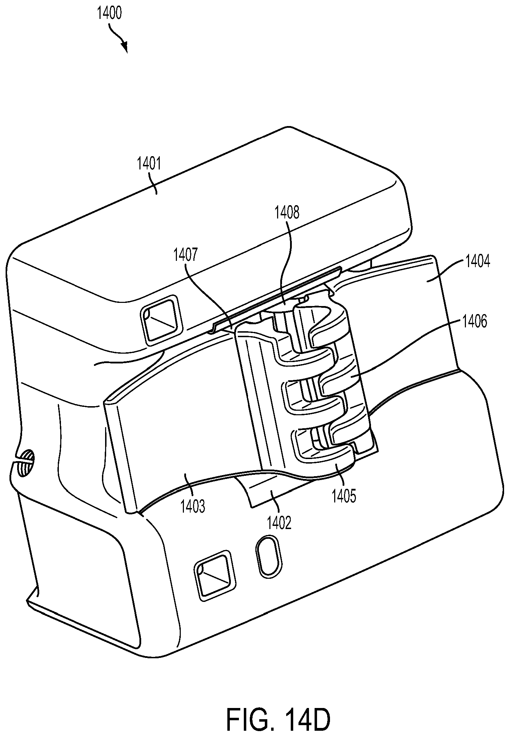

In one example embodiment, as shown in **FIGS. 14A-14E**, a clamp apparatus **1400** is depicted. The clamp apparatus **1400** comprises a body **1402.** In the shown embodiment, the clamp apparatus **1400** has a first handle **1403** and a second handle **1404.** The first handle **1403** and the second handle **1404** may be operatively coupled to the body **1402.** The clamp apparatus also includes a first movable gripper **1405** and a second movable gripper **1406.** The first movable gripper **1405** and the second movable gripper **1406** are coupled to the first handle **1403** and the second handle **1404,** respectively. In one example embodiment, the body **1402** is positioned intermediately between the handles and the grippers. The first handle **1403** and the second handle **1404** are fixedly coupled to the first movable gripper **1405** and the second movable gripper **1406,** respectively, thereby controlling the movement of the first movable gripper **1405** and the second movable gripper **1406.** The clamp apparatus **1400** also includes a first gear set **1407** and a second gear set **1408** that are operatively coupled to the first handle **1403** and the second handle **1404,** respectively, as well as the first movable gripper **1405** and the second movable gripper **1406,** respectively, and are also rotatably coupled to the body. The first gear set **1407** and the second gear set **1408** are configured to operatively engage one another. In one example embodiment, the first gear set **1407** may include an upper first gear **1407a,** and a lower first gear **1407b** that is fixedly coupled to the upper first gear **1407a,** such that the upper first gear **1407a** and the lower first gear **1407b** move together in unison. Similarly, the second gear set **1408** may include an upper second gear **1408a,** and a lower second gear **1408b** that is fixedly coupled to the upper second gear **1408a,** such that the upper second gear **1408a** and the lower second gear **1408b** move together in unison. The upper first gear **1407a** and the lower first gear **1407b** may be configured to operatively engage the upper second gear **1408a** and the lower second gear **1408b,** respectively.

The clamp apparatus **1400** also includes at least one bias member **1410** operatively engaged with the first handle **1403** and the second handle **1404,** such that the handles are configured for operation by a user so as to overcome the at least one bias member 1410. The at least one bias member **1410** is configured to bias the first handle **1403** and the second handle **1404** toward a first position. The first movable gripper **1405** and the second movable gripper **1406** are engaged with one another, defining a clamped position, when the first handle **1403** and the second handle **1404** are in the first position. The first handle **1403** and the second handle **1404** are configured to thereby move, under actuation, to a second position, whereby the first movable gripper **1405** and the second movable gripper **1406** are disengaged from one another, defining an unclamped position.

In some embodiments, the clamp apparatus further comprises a gripping surface on the first movable gripper **1405** and the second movable gripper **1406,** configured to engage a clamped object. In some embodiments, the grippers are configured to clamp onto a pole. In one example embodiment, the clamp apparatus **1400** is for use with medical devices and medical accessories. In one example embodiment, the clamp apparatus **1400** is configured to couple a medical device **1401** to a support pole. The pole may be an IV pole. The medical device **1401** may be a monitor comprising a tablet computer. In one example embodiment, the clamp apparatus **1400** is configured to couple an infusion pump to a support pole. The infusion pump may be a peristaltic infusion pump. In one example embodiment, the clamp apparatus **1400** is capable of automatically mimicking the girth of a variety of different clamped objects.

In one example embodiment, at least part of at least one of the first movable gripper **1405** and the second movable gripper **1406** may be comprised of a material which will firmly grip, but not deform, a clamped object. In some embodiments, at least a part of at least one of the first movable gripper **1405** and the second movable gripper **1406** may be comprised of polyurethane. In some embodiments, at least part of at least one of the grippers may be comprised of rubber, or may be coated in a rubbery, gripping material. In some embodiments, at least one of the first movable gripper **1405** and the second movable gripper **1406** may be at least partially covered by a removable surface. In some embodiments, at least one of the first movable gripper **1405** and the second movable gripper **1406** may comprise at least one approximately arcuate, semi-circular, or contoured face at least on the gripping surface.

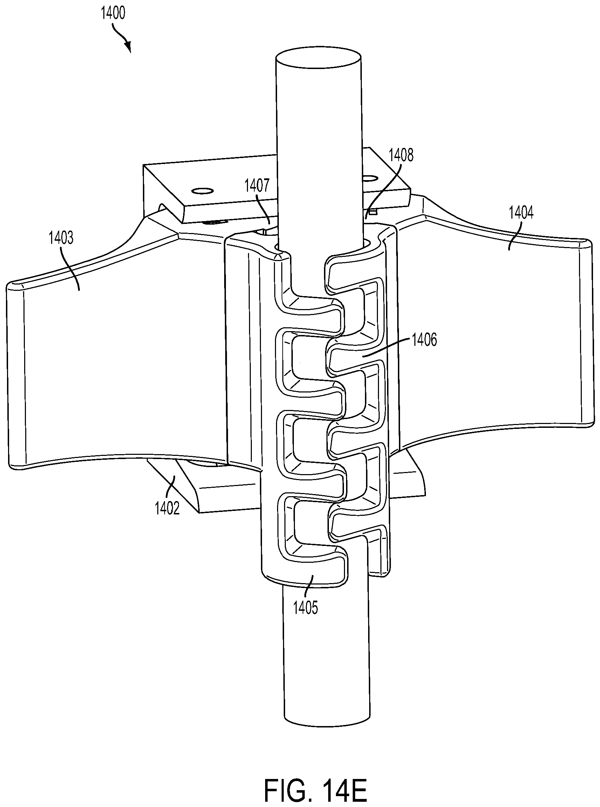

In one example embodiment, at least a part of at least one of the first movable gripper **1405** and the second movable gripper **1406** has fingers. In one example embodiment, the first movable gripper **1405** and the second movable gripper **1406** both have fingers. As shown in **FIG. 14A****,** the fingers of the first movable gripper **1405** and the second movable gripper **1406** are interdigitated when the grippers are engaged with one another, corresponding to the handles being in the first position. As shown in **FIG. 14E****,** the fingers of each gripper are partially interdigitated due to partial engagement of the grippers with one another, corresponding to the handles being in an intermediate position between the first and second positions.

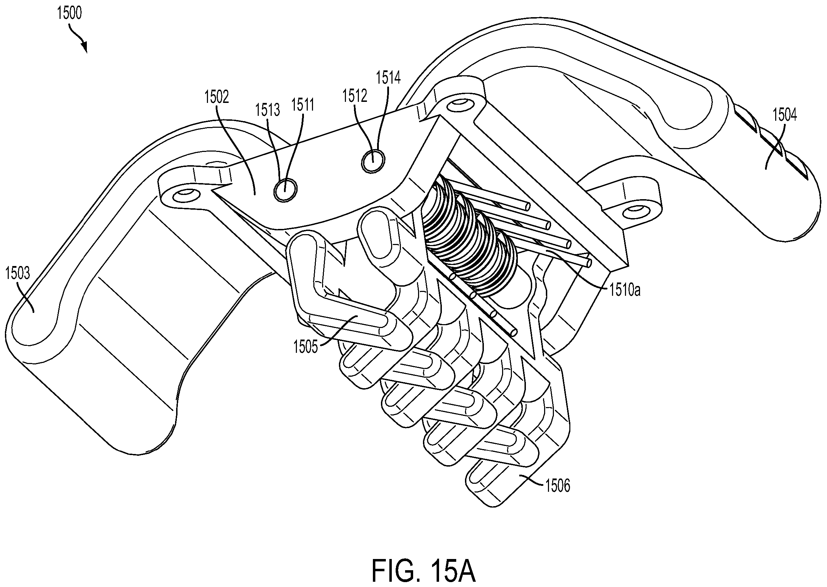

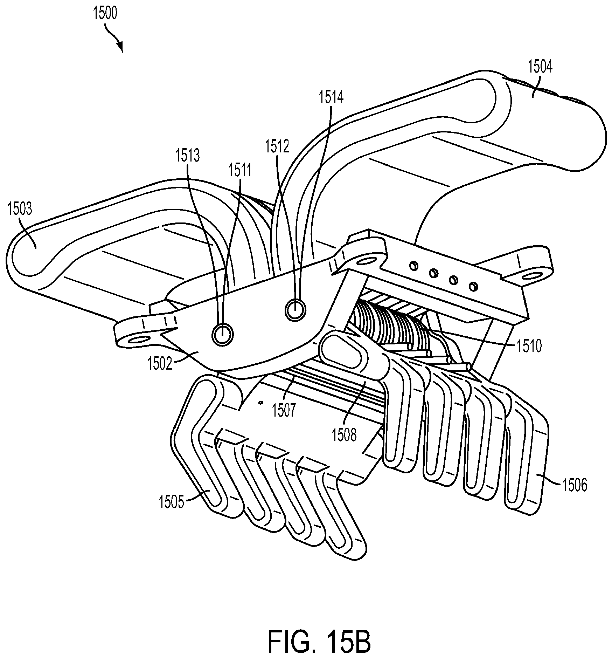

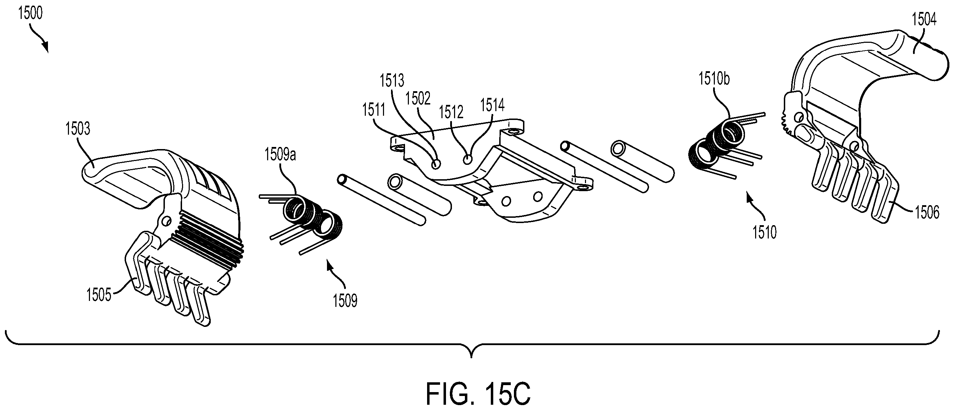

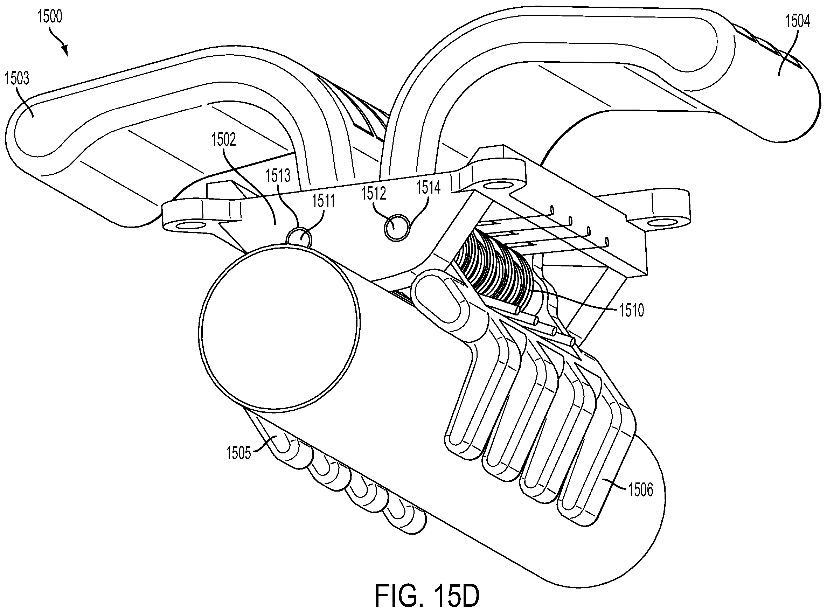

In some embodiments, the at least one bias member **1410** is a spring. Further, the at least one bias member **1410** may be a flat spring. The at least one bias member **1410** may also be a leaf spring. In one example embodiment, the at least one bias member may be at least one array of multiple bias members. Further, the at least one bias member may be an array of multiple flat springs arranged in a layered configuration. In one example embodiment, the at least one bias member **1410** may be made of a flexible, compressible material. In some embodiments, as shown in **FIGS. 15A-15D**, the at least one bias member **1410** may comprise a first bias member and a second bias member. In one embodiment, the first bias member may be a first bias member array **1509,** including multiple individual bias members **1509a,** and the second bias member may be a second bias member array **1510,** including multiple individual bias members **1510a.** In one example embodiment, the first and second bias members may each include a single bias member. Additionally, the first and second bias members or the individual bias members **1509a** and **1510a** may be springs, or, in one example embodiment, may be torsion springs.

In one example embodiment, the first handle **1503** and the second handle **1504** may be paddles. In one example embodiment, the handles may be concave shaped away from or towards the body **1502,** the handles being actuatable. The handles may be configured to be pulled by a user from a first side, or pushed by the user from a second side, in order to move the grippers from the first position to the second position. In some embodiments, the first handle **1503** and the second handle **1504** may further comprise a palm support. The member adapted as a palm support may be U-shaped. In one example embodiment, the first handle **1503** and a second handle **1504** may provide a pair of pull handles configured for operation by a user so as to actuate the first movable gripper **1505** and the second movable gripper **1506** from the first position to the second position.

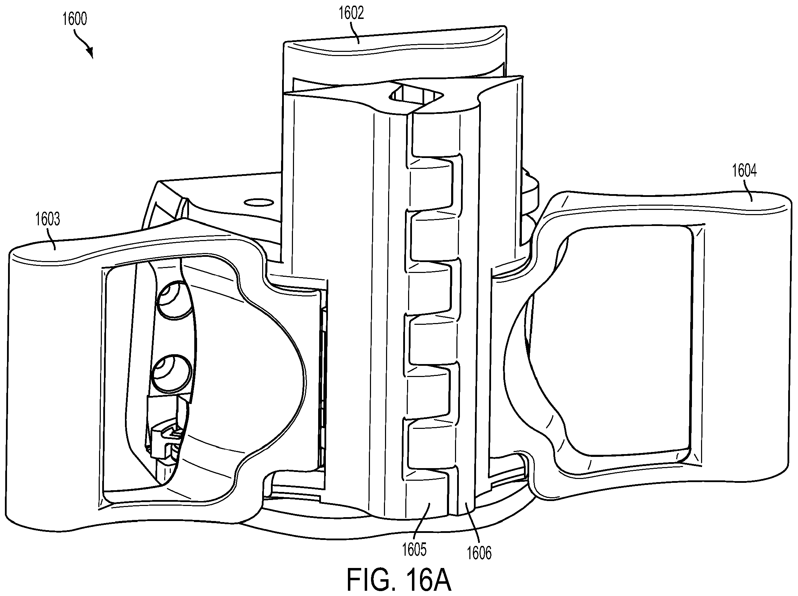

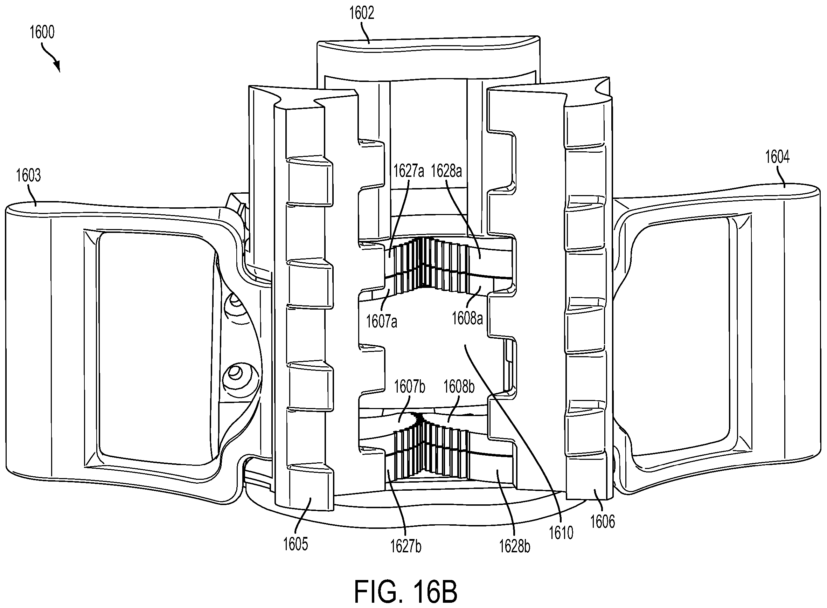

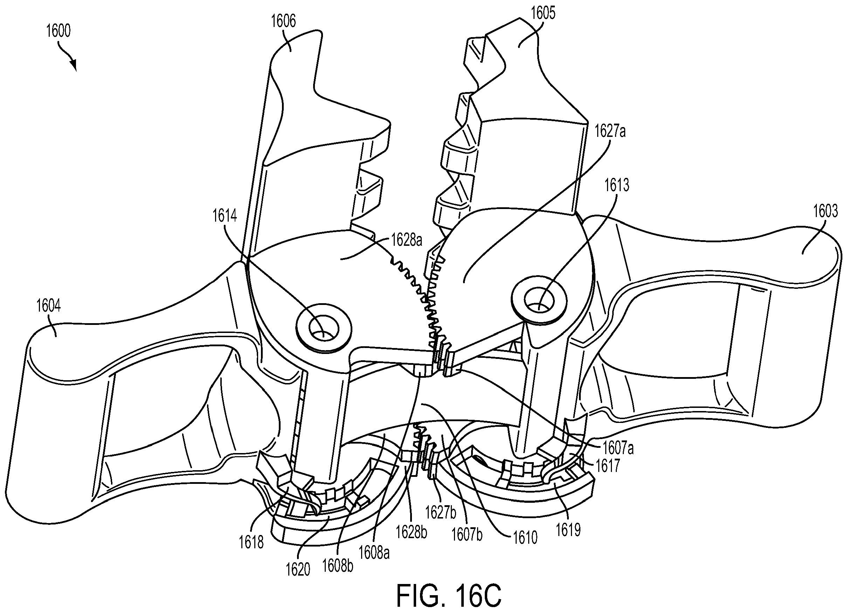

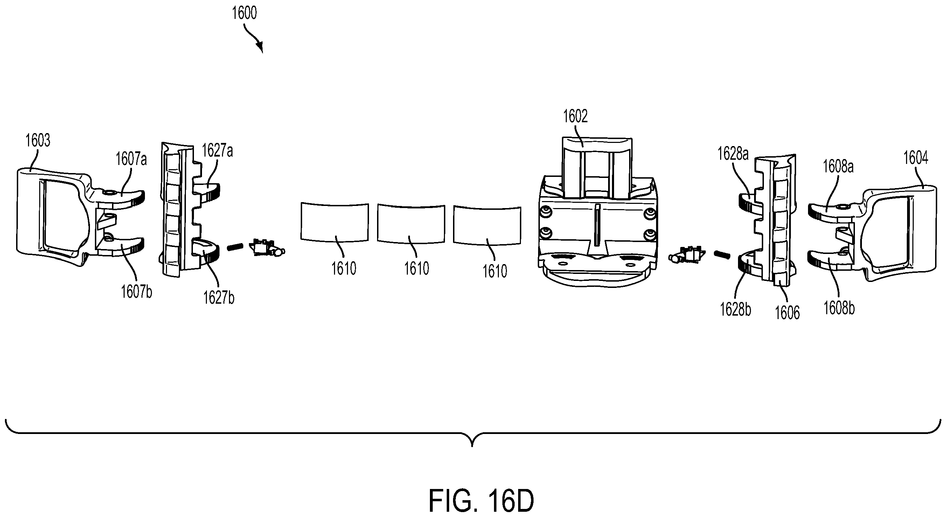

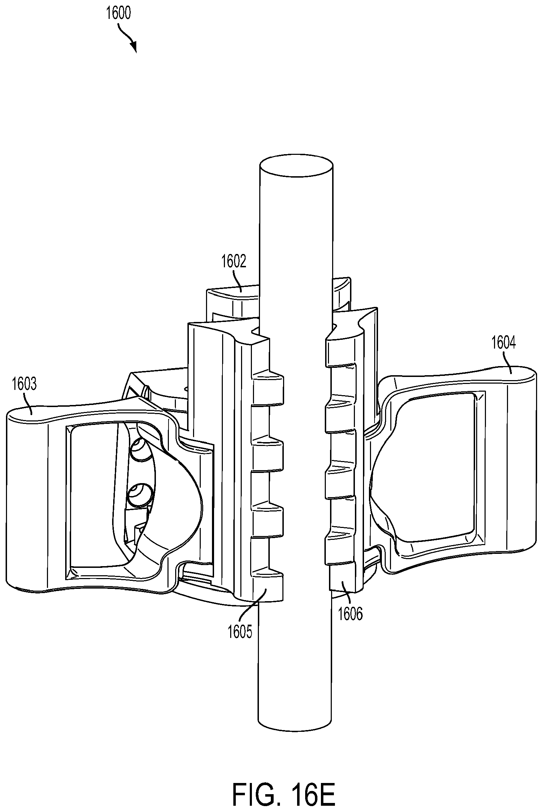

In one example embodiment, as shown in **FIGS. 16A-16E****,** a clamp apparatus **1600** is depicted. The clamp apparatus **1600** comprises a third gear set **1627** and a fourth gear set **1628,** the gear sets operatively coupled to the first handle **1603** and the second handle **1604,** respectively, and rotatably coupled to the body **1602.** In one example embodiment, the third gear set **1627** and fourth gear set **1628** may share an axis of rotation with the first gear set **1607** and the second gear set **1608,** respectively. The third gear set **1627** and the fourth gear set **1628** may be operatively coupled to the first movable gripper **1605** and the second movable gripper **1606,** respectively. The third and fourth gear sets may be configured to operatively engage a locking mechanism in association with the handles. The locking mechanism comprises a first hook **1617,** a first catch **1619,** a second hook **1618,** and a second catch **1620.** In one example embodiment, the third and fourth gear sets may be operatively engaged with one another.

In one example embodiment, the handles and third and fourth gear sets may be configured to permit slight initial rotational movement of the handles in advance of subsequent rotational movement of the grippers, when moving the first and second handles from the first position to the second position. Similarly, the handles and gears may be configured to permit slight additional rotational movement of the handles after the grippers stop their rotational movement, when moving the first and second handles from the second position back to the first position. The initial slight rotational movement of the handles may perform an unlocking function, freeing the grippers to move, while the additional slight rotational movement of the handles after the grippers stop moving may perform a locking function, preventing the grippers from moving.

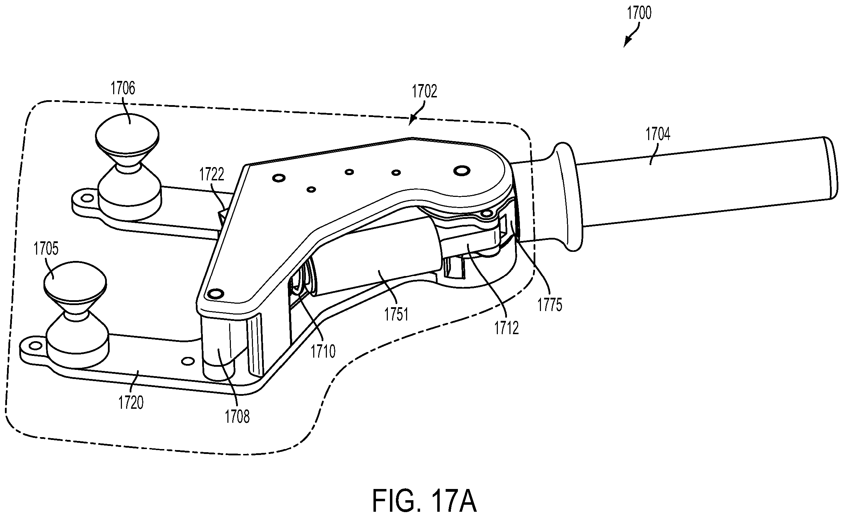

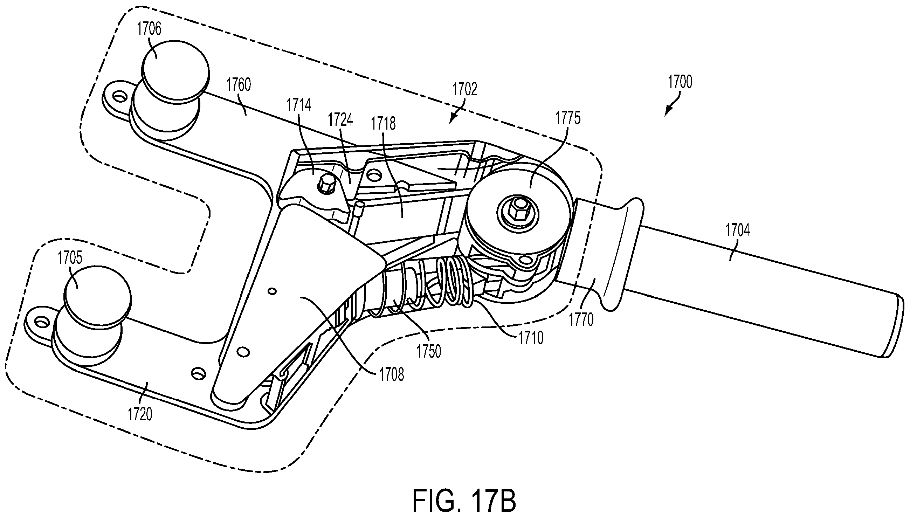

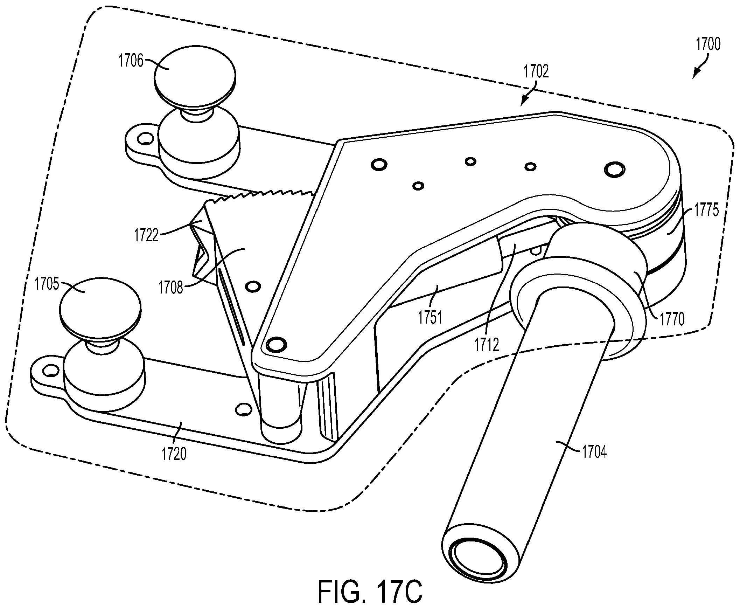

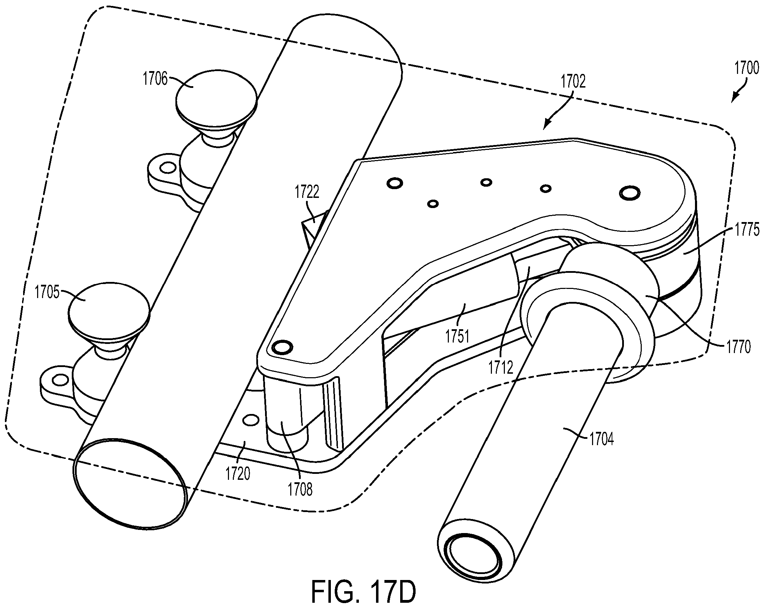

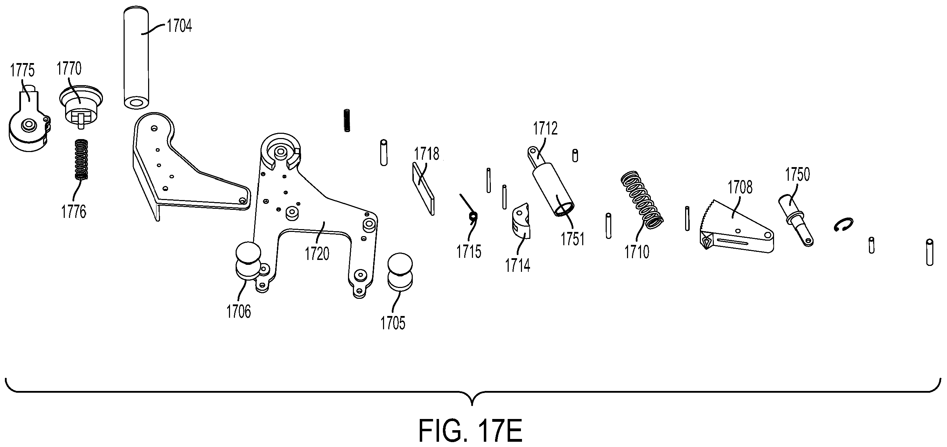

In one example embodiment, as shown in **FIGS. 17A-17E****,** a clamp apparatus **1700** is depicted. The clamp apparatus **1700** comprises a body **1702,** the body **1702** having a first end and a second end. The clamp also includes a lever **1704,** the lever **1704** operatively coupled to the first end of the body **1702.** The clamp apparatus **1700** also includes a movable gripper **1708.** The movable gripper **1708** is coupled to an intermediate portion of the body **1702,** between the first end and second end. The clamp apparatus **1700** includes a first fixed gripper **1706** and a second fixed gripper **1705.** The fixed grippers are positioned at the second end of the body **1702.** The fixed grippers are configured to approximately oppose the movable gripper **1708** such as to secure a pole from opposing sides. The clamp apparatus **1700** also includes a connector member **1712.** The connector member **1712** has a first end operatively coupled to the lever **1704** and a second end operatively coupled to the movable gripper **1708.**

In one example embodiment, the movable gripper **1708** is rotatable about a coupling point of the intermediate portion of the body **1702.** The movable gripper **1708** is approximately wedge-shaped, having a narrow end and a wide end. The narrow end of the movable gripper **1708** is coupled to the body **1702,** and the movable gripper **1708** is rotatable about the narrow end. The wide end of the movable gripper **1708** may have a ridged surface. Further, the ridged surface may extend along the wide end of the wedge-shaped movable gripper **1708.** The wide end of the movable gripper **1708** may have a contoured face **1722** opposing the at least two fixed grippers. In some embodiments, the contoured face **1722** may be a semi-circular or wedge-shaped face. The face **1722** of the wide end of the movable gripper **1708** may be configured to complement the shape of a pole.

In one example embodiment, the grippers further comprise gripping surfaces configured to engage a clamped object. The gripping surfaces may be made of a material which will firmly grip, but not deform, a clamped object. In one example embodiment, the grippers are configured to close onto a pole. In one example embodiment, the grippers are rubber. In another example embodiment, the grippers are coated in a rubbery, gripping material.

In one example embodiment, the body **1702** may comprise a back plate **1720** to which the first fixed gripper **1706** and the second fixed gripper **1705** are fixed.

In one example embodiment, the movable gripper **1708,** the first fixed gripper **1706** and the second fixed gripper **1705** may be configured such that the movable gripper **1708** and the fixed grippers are substantially opposite and are capable of automatically mimicking the girth of a clamped object.

In one example embodiment, the connector member **1712** is configured to rotate the movable gripper **1708** about the narrow end upon actuation of the connector member **1712.**

In one example embodiment, the connector member **1712** includes a bias member **1710.** In one example embodiment, the bias member **1710** is a spring, and in some embodiments the spring is a compression spring. In other embodiments, the bias member **1710** may be a compressible or expandable spring. In some embodiments, the connector member **1712** includes a piston. The piston may be a spring-biased piston. The bias member **1710** is oriented such that movement of the connector member **1712** towards the movable gripper **1708** stores mechanical energy in the bias member **1710.**

In one example embodiment, the connector member **1712** is rotatably connected to the lever **1704** at the first end of the connector member **1712.** The connector member **1712** is coupled to a lever joint **1775,** the lever joint **1775** positioned at, and also operatively coupled to, an end of the lever **1704.** The connector member **1712** may also be rotatably connected to the movable gripper **1708** at the second end of the connector member **1712.** The connector member **1712** is configured to, under actuation of the lever **1704,** extend towards the movable gripper **1708,** thereby rotating the movable gripper **1708** towards the fixed grippers.

In one example embodiment, the clamp apparatus **1700** further comprises a bias member support **1750** coupled to the connector member **1712.** The bias member support **1750** has a portion with a diameter less than a diameter of the bias member **1710.** The portion of the bias member support **1750** is positioned to fit inside the diameter of the bias member **1710.**

In one example embodiment, the clamp apparatus **1700** further comprises a bias member housing **1751** coupled to the connector member **1712.** The bias member housing **1751** is hollow and has a sealed end. The bias member housing **1751** has a diameter greater than the diameter of the bias member **1710.** In one example embodiment, the lever **1704** of the clamp apparatus **1700** is a handle. The lever **1704** is configured to, under actuation, rotate towards the body **1702.** The lever **1704** is configured to move the connector member **1712** toward a first position and thereby move the movable gripper **1708** toward the fixed grippers. The lever **1704** is further configured to move the connector member **1712** toward a second position and thereby move the movable gripper **1708** away from the fixed grippers. In one example embodiment, the lever joint **1775** is a cam, such that when the lever **1704** is moved to the first position, the cam pushes the connector member **1712,** thereby pushing the movable gripper **1708** closer to the fixed grippers. In one example embodiment, the clamp apparatus **1700** is configured to allow the moveable gripper **1708** to stop when abutting against an object while allowing the connector member **1712** to continue to move as the lever **1704** is further actuated.

In one example embodiment, the lever **1704** includes a slideable ring **1770** coaxially aligned with and surrounding the top end of the lever **1704,** the top end being nearest the lever joint **1775.** The slideable ring **1770** is configured to free the lever **1704** from a locked position. The slideable ring **1770** is configured to slide out of a notch in the lever joint **1775,** thereby unlocking the lever **1704** and freeing the lever **1704** to rotate. The slideable ring **1770** includes a ring bias member **1776,** the ring bias member **1776** configured to bias the slideable ring **1770** to a notched position. In one example embodiment, the ring bias member **1776** is a compression spring, while in another embodiment the ring bias member **1776** is an expansion spring.

In one example embodiment, the clamp apparatus **1700** further comprises a locking assembly, the locking assembly configured to interact with the movable gripper **1708.** The locking assembly includes a pawl **1714,** and the pawl **1714** includes a pawl bias member **1715.** In one example embodiment, the pawl bias member **1715** is a spring, and in some embodiments the pawl bias member **1715** is a torsion spring. The pawl **1714** is rotatably coupled to the locking assembly, the pawl **1714** configured to rotate into contact with an upper ridged surface of the movable gripper **1708,** locking the gripper in place.

In one example embodiment, the locking assembly further comprises a slideable member **1718,** and the pawl **1714** positioned in contact with the slideable member **1718.** The slideable member **1718** has a first end in contact with the lever joint **1775.** The slideable member **1718** contacts an outer surface of the lever joint **1775,** the outer surface having a depressed portion. The locking assembly is configured to move the slideable member **1718** into the depressed portion of the lever joint **1775,** allowing the pawl **1714** to rotate into contact with the movable gripper **1708** and thereby locking the movable gripper **1708** in place.

In one example embodiment, the clamp apparatus **1700** is configured for use with medical devices and medical accessories. In one example embodiment, the body **1702** includes a means of coupling the clamp to a load. In one example embodiment, the load is a medical device. In some embodiments, the medical device is a peristaltic infusion pump or syringe infusion pump. In one example embodiment, the clamp apparatus **1700** is configured to couple a medical device to a support pole. In one embodiment, the pole may be an IV pole. In one embodiment, the medical device is a monitor comprising a tablet computer.

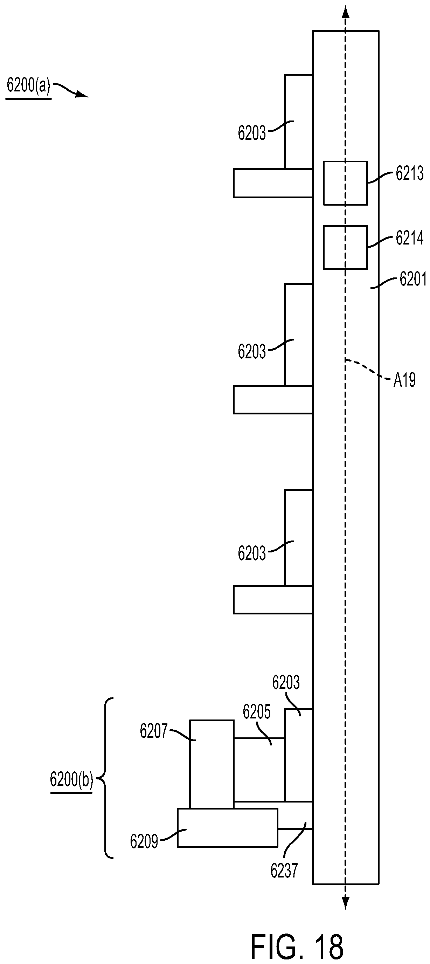

FIG. 18 depicts a prospective, side view illustration of an example support system 6200(a) in accordance with an embodiment of the present disclosure. FIG. 18 further provides a representational view of the support system 6200(a) such that various embodiments of the support system 6200(a) may be illustrated under this view. The support system 6200(a) may serve to ensure an easy and efficient clamping of a device 6207 or a plurality of similar or dissimilar devices 6207 to another component.

The present disclosure may provide one or more engagement mechanisms between a component that is to be clamped and a component on which clamping takes place. Such an engagement mechanism may serve to facilitate safe, easy and robust clamping of the clamped device. Additional engagement mechanisms may be configured to be in conjunction with, in addition to, or independent of the primary engagement mechanism. Such additional engagement mechanisms may provide further stability for the clamped device 6207 and the clamp arrangement 6200(b), as a whole. Various embodiments of the present disclosure mainly illustrate the clamping of medical devices in a hospital facility or a medical setting. Such devices may include, but are not limited to infusion pumps such as: syringe pumps, peristaltic pumps, cassette-based pumps, etc. Additionally, other devices such as physiological monitors, gravity-fed drug delivery devices and any other suitable medical equipment may be clamped as well. In some embodiments, the support system 6200(a) may be configured to allow clamping of a portable computing device such as a smart phone, tablet, or other similar devices with a keypad, a touch screen, or any other sufficient user interface. Though the present disclosure is described for use in a hospital facility or a medical setting, the support system 6200(a) embodiment or its exemplary components described herein may also be used as a combination or independently, in any other setting. Additionally, various components of the embodiments described herein need not be used exclusively within the context of the embodiments described herein. For example, the clamping devices 6207 described in relation to the embodiments disclosed herein may be used by themselves to clamp to an object or any other suitable support such as a pole (e.g. IV pole), rod, or the like.

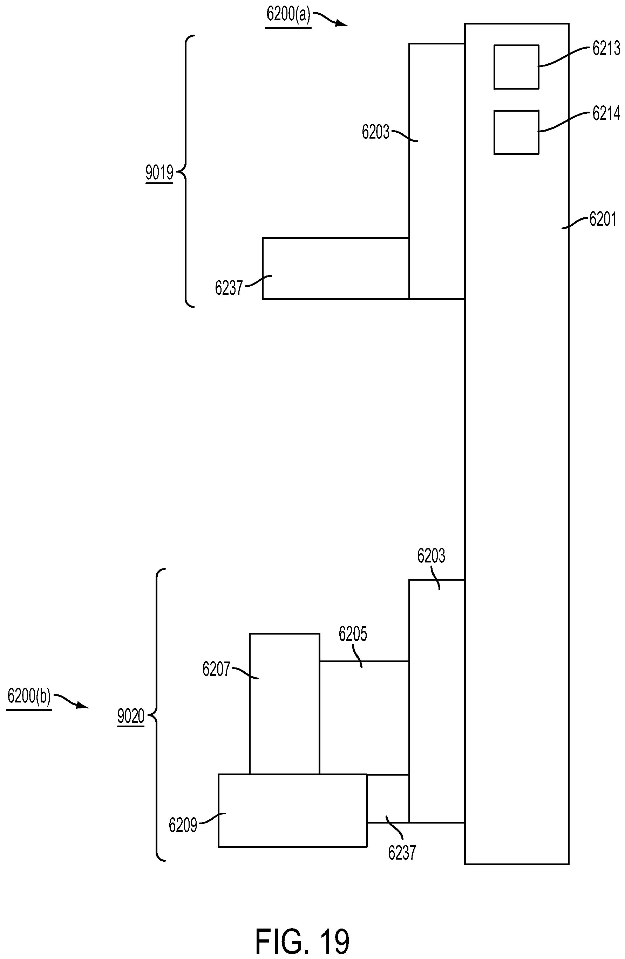

A support system 6200(a) may comprise components that may be assembled in a pre-defined manner to allow clamping of a device or devices 6207 to other components. The exemplary support system 6200(a) in FIG. 18 may include a backbone structure 6201 that may be secured to a pole, such as an IV pole or may independently position itself, such that a vertical axis A19 (shown in dotted line in FIG.19A) may pass through it. In some embodiments the backbone structure 6201 may include a sturdy base component that may allow the independent positioning of the support system 6200(a) on a flat surface such as flat ground, floor or a table top. Such a base component may be removably attached or permanently engineered to the backbone structure 6201. To facilitate such independent positioning, some embodiments may include a base structure which may be attached to a bottom of the backbone structure 6201. Optionally, the base structure may also provide wheels for mobility.

The backbone structure 6201 may be hollow and may further provide one or more data communication ports 6213 and power supply inlets 6214 to facilitate information exchange between one or more devices 6207 clamped on the backbone structure 6201 and allow charging of a clamped device or devices 6207. The hollow feature of the backbone structure 6201 may facilitate housing internal circuitry related to data communication and power supply circuits. In some specific embodiments, other circuitry may also or instead be housed in the hollow interior of the backbone structure 6201.

The backbone structure 6201 may be made of a variety of suitable materials. It may be desirable that the material chosen are low density and therefore low weight, high strength, superior malleability, easy machining, corrosion resistance, rust resistance, especially from air and moisture, and good thermal and electrical conductivity etc. However, other materials with similar qualities or different qualities may also be used. In some specific embodiments, metal alloys like stainless steel or metals like aluminum may be used. Additionally, alternatively, or optionally, plastics possessing similar qualities may be used. In other embodiments, a combination of different materials, providing such features, may be used. Additionally, the backbone structure 6201 may be of a pre-defined breadth and length to engage a known number of holding structures 6203 with sufficient clearance between a plurality of attachable devices 6207. Though multiple holding structures 6203 are depicted in the example embodiment, in some specific embodiments, a single holding structure 6203 may be used.

A holding structure 6203 may serve to engage another component of the support system 6200(a) with the backbone structure 6201. A first set of fixation points may be provided on the backbone structure 6201. A second set of complementary set of fixation points may also be provided on the holding structure 6203. The holding structure 6203 may be configured to provide a substantially graspable feature such that when the holding structure 6203 is engaged with the backbone structure 6201, the substantially graspable feature may stand generally parallel to the axis A19 of the backbone structure 6201. Additionally, the holding structure 6203 may be engaged with the backbone structure 6201 in such a fashion as to provide electrical communication with the backbone structure 6201 via a base portion 6237, as depicted in FIG. 18. The plurality of holding structures 6203 may be disposed at necessary intervals on the backbone structure 6201. The general view in FIG. 18, shows four sequential holding structures 6203.

The holding structure 6203 in a clamping arrangement 6200(b) according to the invention is equipped with other components to illustrate the complete clamping arrangement 6200(b) in the support system 6200(a). Some of the following drawings show supplementary, explicit views of a specific embodiment of the clamping arrangement 6200(b) on a single holding structure 6203. The drawings also depict exemplary embodiments of individual components and their inter-relation to form the clamping arrangement 6200(b).

The support system 6200(a) may further provide an intermediate clamp assembly 6205. A first face of the clamp assembly 6205 may engage with a device 6207 which is to be clamped, and the opposing face may be engaged with the holding structure 6203. Hence, the clamp assembly 6205 may serve to clamp the device 6207 on the holding structure 6203. A first side of the device 6207, which is distal from the clamp assembly 6205 may be a work station or interface for the user of the device 6207 in various embodiments.

A clamping arrangement 6200(b) may also provide a power supply pack 6209 configured to detachably engage with a device 6207. The pack 6209 may function as an alternative power supply source when the device 6207 is not clamped on the holding structure 6203. The detachable power supply pack 6209 may be further configured to clamp along with the device 6207 and positionaly adjust to facilitate an uninterrupted engagement between the clamp assembly 6205 and the holding structure 6203. The detachable power supply pack 6209 may be configured so as to receive a base portion 6237 of the holding structure 6203 into a housing provided on the detachable power supply pack 6209. The power supply pack 6209 may be removably coupled with the device 6207 via a separate engagement mechanism. Additionally, once attached to the device 6207, the detachable power supply pack 6209 may not require any supplementary efforts for clamping it to the holding structure 6203, other than the ones employed by the user for clamping the device 6207 to the holding structure 6203. Thus, the power supply pack 6209 may be clamped on the holding structure 6203 via the engagement mechanisms between device 6207 and the clamping assembly 6205 and a second engagement between the clamp assembly 6205 and the holding structure 6203.

FIG. 19 depicts a proximal, side view illustration of an exemplary support system 6200(a), as shown in FIG. 18. Two holding structures assemblies, 9019 and 9020 are shown in FIG. 19. A complete clamping arrangement 6200(b) is shown on holding structure assembly 9020, suggesting that an identical arrangement may be provided on the holding structure assembly 9019, if required. Additionally, if other holding structures are present, identical arrangements may be provided on them. Alternatively, one or more holding structures 6203 included in the support system 6200(a) may be configured such that its complete clamping arrangement 6200(b) differs from others in the support system 6200(a). For example, embodiments allowing clamping of a portable computing device such as a tablet, smart-phone, etc., as well as an infusion pump, the complete clamping arrangement 6200(b) for each, may differ. A clamping arrangement 6200(b) is shown in section 9020 of FIG. 19. A similar assembly or arrangement may be achieved for section 9019 of FIG. 19.

A data communication port 6213 and a power supply source 6214 provided on the backbone structure 6201, may be used to facilitate data communication between a device or devices 6207 and supply power to all the devices 6207, respectively, clamped on a single backbone structure 6201. The data communication and power supply may be achieved through internal circuitry within the backbone structure 6201. This internal circuitry for the data communication and power supply may be provided wholly or partially within an internal hollow cavity inside the backbone structure 6201. The power supply source 6214 on the backbone structure 6207 may be a plug-in from the main power source (e.g., AC mains, for example).

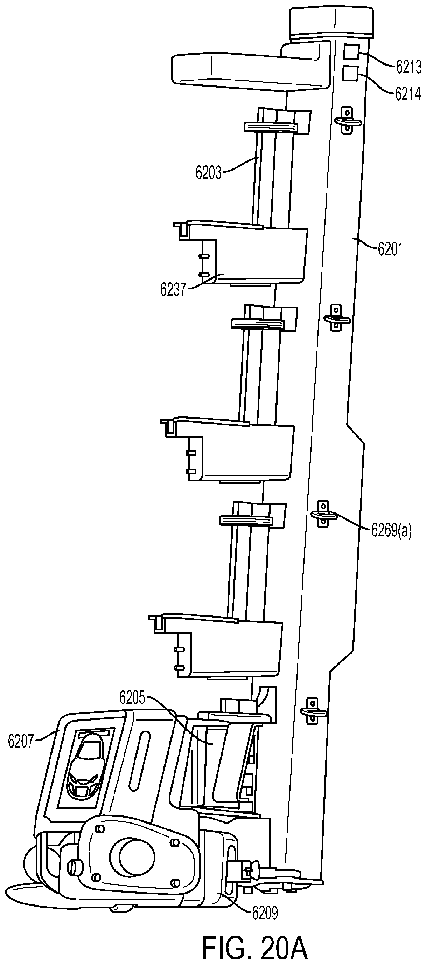

Various components of a specific embodiment of a support system 6200(a) are also shown in the isometric view depicted in FIG. 20A. The support system 6200(a) depicted in FIG. 20A may be one of many specific examples of the generic embodiment depicted in FIG. 18. FIG. 20A shows a backbone structure 6201, which may be a longitudinal, hollow, substantially rectangular structure. Additionally, the backbone structure 6201 may serve as a principal support structure for the complete support system 6200(a). The backbone structure 6201 may also include one or more protrusion which in the example embodiment is shown as a number of tube management members 6269(a). The tube management members 6269(a) shown in FIG. 20A may collectively serve as a tube management system for managing a plurality of tubes coming out or going into a device or devices 6207 that may be clamped on the backbone structure 6201. Various embodiments of the tube management members will be described later in further detail herein. It should also be noted that in some embodiments, one or more protrusion, such as the side protrusions or tube management members 6269(a) depicted in FIG. 20A, may be used to hang various medical supplies. Such supplies may include, for instance, an IV bag or a gravity-fed drug infusion set-up.

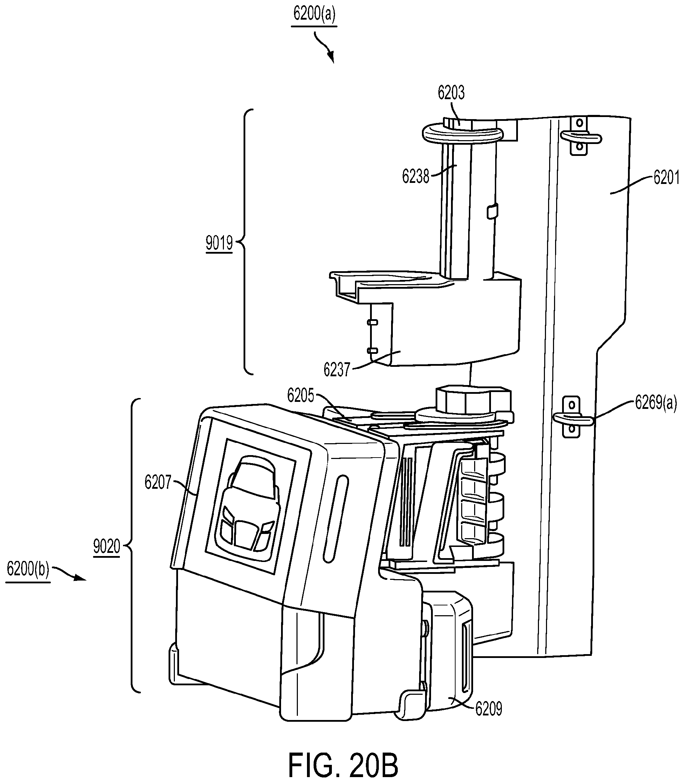

Components of the support system 6200(a) are also shown in the isometric view depicted in FIG. 20B. The embodiment depicted in FIG. 20B may be one of many specific examples of the generic embodiment depicted in FIG. 19. The holding structure 6203 shown in FIG. 20B includes a rod portion 6238 and a base portion 6237 which is an exemplary embodiment of the holding structures 6203. Section 9019 and section 9020 each include a holding structure 6203. An example of a completely assembled clamping arrangement 6200(b) is shown in section 9020 of FIG.19B and FIG. 20B to illustrate that a similar arrangement may be achieved for section 9019 of FIG. 19 and FIG. 20B. Alternatively, in embodiments where a wide variety of devices 6207 may be attached to a support system, the arrangement in section 9020 may differ.

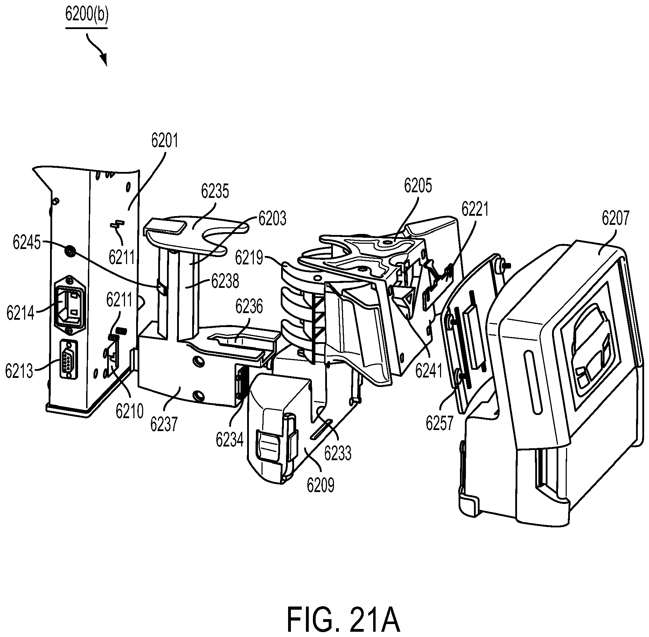

FIG. 21A depicts an exploded, isometric view of an example embodiment of a complete clamping arrangement 6200(b) and depicts a positional relationship between components of a clamping arrangement 6200(b)). The clamping arrangement 6200(b) may comprise a backbone structure 6201 that may be generally planar. The backbone structure 6201 may further comprise a first set of fixation points 6211 configured to engage or attach a holding structure 6203 to the backbone structure 6201. The holding structure 6203 may be engaged such that a rear face of the holding structure 6203 may be configured to mate with a front surface of the backbone structure 6201. As shown, the front face of the holding structure may provide pre-determined one or more mechanisms for engaging additional components that are described in-depth later in the present disclosure. The holding structure 6203 may comprise a top portion 6235. The top portion 6235 may be configured to show or visually indicate a clear distinction between holding structure 6203 placed longitudinally adjacent to another holding structure 6203 on the backbone structure 6201. This distinction may be useful since a single plate of the backbone structure 6201 may provide attachment sites for a plurality of holding structures 6203.

The holding structure 6203 may further comprise a base portion 6237 that provides a passage 6234 for a data communication and a power supply circuitry that may extend from a hollow interior of a backbone 6201 to the device 6207. The passage 6234 may receive the data communication and power supply circuitry from a data communication port 6213 and power supply outlet 6214 on the backbone structure 6201 which extends to the device 6207 via the passage 6234. The base portion 6237 may further provide a coupling element for engaging a clamp assembly 6205. One embodiment of the coupling element may be a recess 6236 that receives a complementary coupling element that may be provided on the clamp assembly 6205. The coupling element or recess 6236 may serve as an additional engagement mechanism between the holding structure 6203 and the clamp assembly 6205. This additional engagement mechanism may also serve as an alignment mechanism to bring together the holding structure 6203, the clamp assembly 6205 and the device 6207, in line with one another. Moreover, the engagement of the coupling element or recess 6236 on the holding structure 6203 with a complimentary coupling element on the clamp structure 6205 may serve to increase the robustness of the connection between the holding structure 6203 and the clamp structure 6205. The holding structure 6203 may further provide an intermediate rod portion 6238 between the top portion 6235 and the base portion 6237.

FIG. 21A further depicts an intermediate rod portion 6238 that may be a graspable structure and may serve as an important element to provide the primary engagement between a holding structure 6203 and a clamp assembly 6205. An embodiment of the intermediate rod 6238 portion may generally be a cylindrical structure. In other embodiments, the intermediate rod portion 6238 may have a different yet graspable structure. For example, the intermediate rod structure 6238 may have any number of suitable cross-sectional shapes. This intermediate rod portion 6238 may be disposed at a minimal distance from the backbone structure 6201 in order to avoid any interference when griping this portion. A top portion 6235 and a base portion 6237 may be engaged with the backbone structure 6201 via a first set of fixation points 6211 that may be provided on the backbone structure 6201. The minimum distance between the intermediate rod portion 6238 and the backbone structure 6201 may be dependent upon the dimensions of the top portion 6235 and the base portion 6237. In the example embodiment, the intermediate rod portion 6238 may extend in a manner substantially parallel to the longitudinal direction of the backbone structure 6201. In some other embodiments the intermediate rod shaped portion 6238 may extend from the base portion 6237 at an angle with respect to the longitudinal direction of the backbone structure 6201 and the intermediate rod portion 6238 may form skew lines which may lie in substantially parallel planes. This may be desirable to allow a device or devices 6207 to be oriented at a particular angle when clamped on the backbone structure 6201. The holding structure 6203 may be made of hard and light weight materials including but not limited to thermosetting plastics like PVC or uPVC, etc. the holding structure may also be made of metal alloys with similar qualities. Regardless, the material for making the holding structure 6203 may be equivalent or lighter than the material for making the backbone structure 6201.

An intermediate rod portion 6238 may be gripped by a clamp assembly 6205. The clamp assembly 6205 may provide a griping means that may be configured to secure the intermediate rod portion 6238. A rear portion of the clamp assembly 6205 may provide an additional engagement mechanism for receiving one or more devices 6207 that is to be clamped to the holding structure 6203. Thus, the clamp assembly 6205 may serve as a liaison between the holding structure 6203 and the device 6207. Additionally, the clamp assembly 6205 may provide operable means that may be employed by a user for controlling the clamping and un-clamping of the device 6207. The operable means may be configured to control the movement of the gripping means provided on the clamp assembly 6205. These sub-components are disused in further detail hereinafter.

An engaging mechanism between a clamp assembly 6205 and a device 6207 may include the use of a latch 6221 that may assist in locking or unlocking the engagement between the device 6207 and the clamp structure 6205. The latch 6221 may be received in a socket or bay 6241 that may be provided on a rear face of the clamp assembly 6205. The latch 6221 may be further disposed in the socket 6241 via a flexible member that may be configured to facilitate a pivoting movement of the latch 6221. In one embodiment of the present disclosure, the flexible member may be a spring.

FIG. 21A further depicts a device 6207 with an alternative detachable power supply pack 6209. The detachable power supply pack 6209 may be engaged with a device 6207 such that it does not interfere when the device 6207 is clamped to a holding structure 6203. The detachable power supply pack 6209 may be further configured to accommodate a base portion 6237 of the holding structure 6203 when the device 6207 along with the detachable power supply pack 6209 is clamped to the intermediate rod portion 6238 of the holding structure 6203 through the clamp assembly 6205. The detachable power supply pack 6209 may be used when the device 6207 is unclamped from the holding structure 6203. The device 6207 may further include a back plate 6257 that may be configured to pair the device 6207 with the clamp assembly 6205.

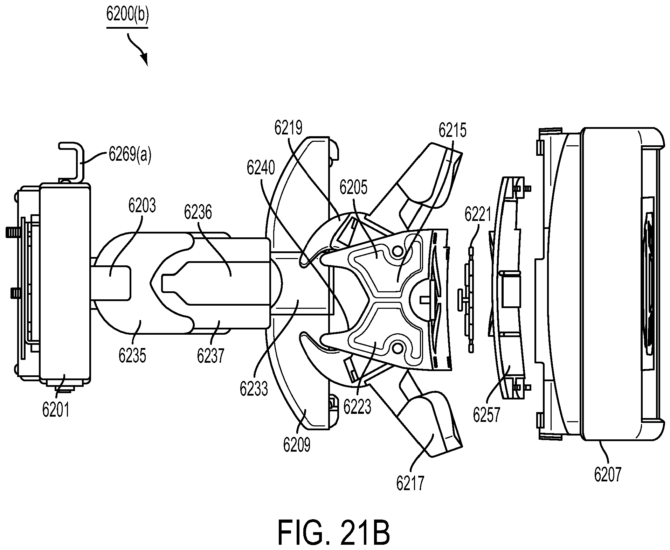

FIG. 21B depicts an exploded, top view of a clamping arrangement 6200(b). The FIG. 21B further illustrates the alignment of the components that make up the clamping arrangement 6200(b). The top view of the clamping arrangement 6200(b) depicts a top end of a backbone structure 6201 along with a side protrusion 6269(a) that may serve as a tube management member. A single plate of backbone structure 6201 may comprise a plurality of side protrusions 6269(a) that may be placed successively and made of similar dimensions for efficient tube management. The top portion of the holding structure 6235 may be a flat formation with a generally-crescent shape at the end of the formation. The generally crescent shape may serve to guide a user about the manner of engaging the clamp assembly 6205 with the holding structure 6203, since the top planar surface 6223 of the frame structure 6215 may provide an opposing crescent shape (i.e., complementary shape) at the end of the top planar surface 6223.

With reference to FIG. 21B, a base portion 6237 of the holding structure 6203 may be in sequence with a top portion 6235. The base portion 6237 may be further elongated than the top portion 6235 of the holding structure 6203. Such dimensional relation between the two sub-components may be conducive to the purpose of the holding structure 6203 which is to provide a graspable means for engaging a clamp assembly 6205 of the clamping arrangement 6200(b). However, the inter-relation between the dimensions of the top potion 6235 and the base portion 6237 of the holding structure 6203 may differ in various embodiments.

Further description of the base portion 6237 may be provided by referring to both FIG.21A and FIG. 21B, in combination. A passage 6234 may be provided in the base portion 6237. The passage 6234 (referring to FIG. 21A) may be configured to primarily house a data communication and power supply circuitry extending from the backbone structure 6201 to a device 6207. Besides the data communication and power supply circuitry, the base portion may also provide an alignment component or recess 6236 .The recess 6236 may serve to enhance the stability of the clamping arrangement 6200(b).

Referring again to FIG. 21B, one or more devices 6207 may provide a detachable power supply pack 6209. The detachable power supply pack 6209 may be configured to receive a base portion 6237 of a holding structure 6203. The base portion 6237 may be received in a depression or housing 6233 that may be provided on the detachable power supply pack 6209. Thus the detachable power supply pack 6209 may be positionaly accommodated below the clamp assembly 6205 as the base portion 6237 of the holding structure 6203 is received by the housing or depression 6233 on the detachable power supply pack 6209.

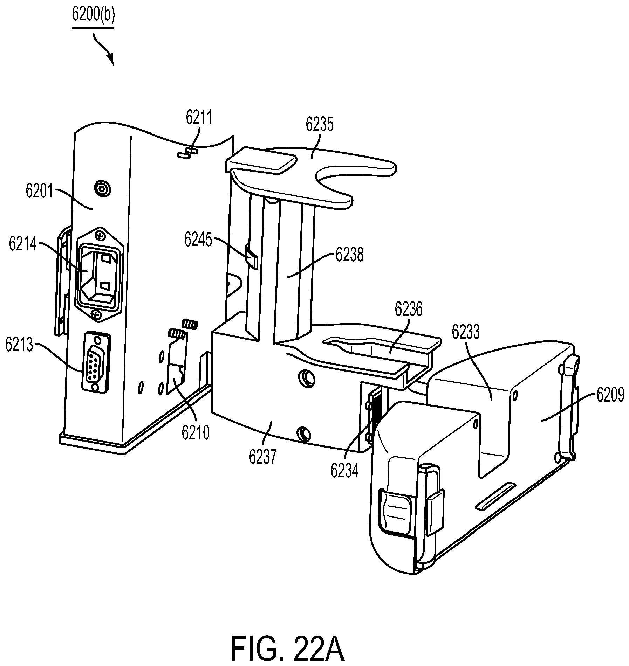

FIG. 22A depicts an exploded view of a part of the clamping arrangement 6200(b), illustrating the positional relationship between a backbone structure 6201, a holding structure 6203 and a detachable power supply pack 6209. This isometric view of the backbone structure 6201 illustrates one or more data communication ports 6213 and a power supply inlet 6214 on a side of the backbone structure 6201. The power supply inlet 6214 may serve as a plug in point for a power supply source that may be received from the main power supply. Alternatively, the data communication ports 6213 and power supply inlet 6214 may be disposed at any other convenient site on the backbone structure, depending upon a desired configuration of the clamping arrangement 6200(b). The backbone structure 6201 may further provide a first set of fixation points 6211 that may facilitate engaging the holding structure 6203 or like, with the backbone structure 6201. The first set of fixation points 6211 may provide an attachment mechanism in form of set screws, pins, splin, or the like. In some embodiments the holding structure 6203 may be permanently fastened to the backbone structure 6201.

FIG. 21A further depicts a backbone structure 6201 which may further provide a power supply outlet 6210. The outlet 6210 may serve to extend the power supply circuitry from the backbone structure 6201 to the base portion 6237 of the holding structure 6203. A passage 6234 in the base portion of the holding structure 6237 may facilitate a connection between the power supply outlet 6210 and the device 6207. There may be additional embodiments that may provide a different configuration to facilitate electrical communication between the backbone structure 6201 and the device 6207. Some of these embodiments may not require the holding structure 6203 as an intermediary component.

The holding structure 6203 may further provide a rib portion 6245). The rib portion 6245 may serve as a grip enhancing means on the intermediate rod portion 6238 of the holding structure 6203. In some specific embodiments, the rib portion 6245 is an outcropping on the intermediate rod portion 6238. Additionally or optionally, the rib portion 6245 may be made of an identical material as the holding structure 6203 or a distinct material therefrom. The intermediate rod portion 6238 may be provided with a single or a plurality of rib portions 6245. The number of rib portions 6245 may depend on the degree of grip-enhancement required by an embodiment.

An exemplary embodiment of a detachable power supply pack 6209 is also depicted in the FIG. 22A. The detachable power supply pack 6209 may be engaged with a device 6207 that is be clamped on the backbone structure 6201. The detachable power supply pack 6209 may mate with the device 6207 from one side such that its opposing side may extend towards the holding structure 6203. The detachable power supply pack 6209 may further provide an opening, housing or depression 6233. The depression 6233 may serve to accommodate, receive or house the base portion 6237 of the holding structure 6203, e.g., partially or completely. The engagement between the holding structure 6203 and the backbone structure 6201 may accomplish more than one purpose. For example, one purpose may be to mechanically fasten the holding structure 6203 to the backbone structure 6201. Another purpose of this engagement may be to provide an electrical contact between the power supply outlets 6210 and the base portion 6237 of the holding structure 6203. Continuous circuitry may extend from the power supply outlet 6210 to the base portion 6237 of the holding structure 6203.

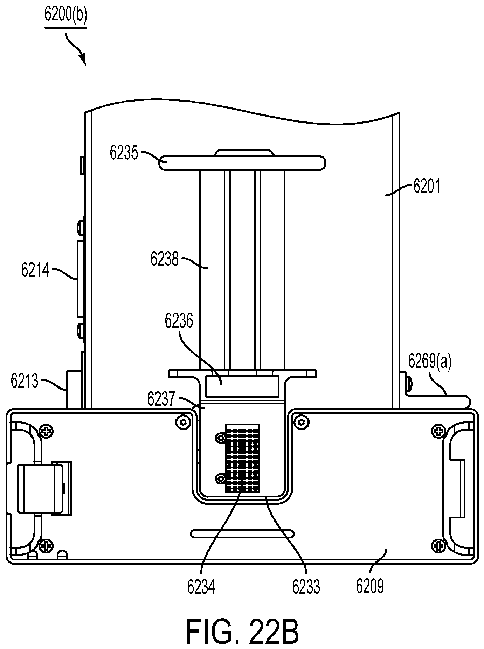

FIG. 22B depicts a front view of an example embodiment of a positional relationship between a backbone structure 6201, a holding structure 6203, and a detachable power supply pack 6209. A depression or housing 6233 on the detachable power supply pack 6209 may accommodate a base portion 6237 of the holding structure 6203. The base portion 6237 of the holding structure 6203 may comprise a recess 6236 and a passage 6234 between the backbone structure 6201 and the device 6207. The passage 6234 may comprise power supply circuitry. Further, the passage 6234 of the base portion 6237 of the holding structure 6203 may be received by a depression or housing 6233 in the detachable power supply pack 6209. Another embodiment of the detachable power supply pack 6209 may provide a different non-interfering feature. The non-interfering feature of a different embodiment may serve the same purpose as the depression or housing 6233 in the detachable power supply pack 6209 of the present disclosure. The design or shape of the detachable power supply pack 6209 may vary in-order to provide the non-interfering feature therein.

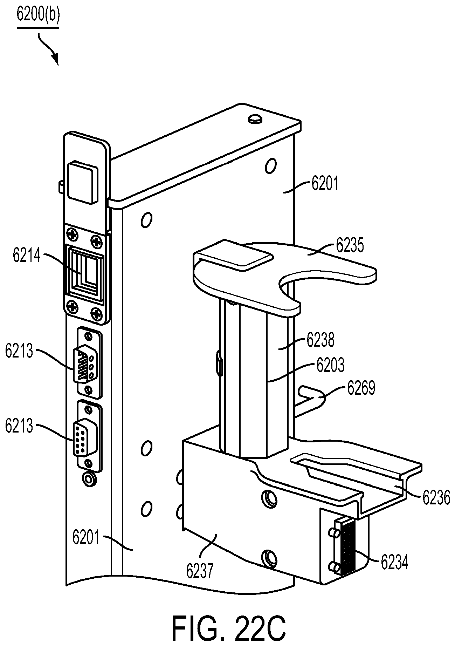

FIG. 22C depicts a partial assembly of a clamping arrangement 6200(b) comprising a backbone structure 6201 and a holding structure 6203. The backbone structure 6201 may comprise one or more data communication ports 6213 and at least one power supply inlet 6214. The backbone structure 6201 may further comprise various sites for engaging the holding structure 6203 such that a top portion 6235 of the holding structure and a base portion 6237 of the holding structure may affix with the backbone structure 6201. The holding structure 6203 may further provide an intermediate rod portion 6238 that may be bounded by the top portion 6235 and the base portion 6237. As described earlier, the backbone structure 6201 may house an internal circuitry related to the data communication and the power supply. This circuitry may be extended to an identified component through a passage 6234 provided in the base portion 6237 of the holding structure 6203. Consequently, the holding structure 6203 may be engaged with the backbone structure 6201 in a way that an outlet for the circuitry may be aligned with the passage 6234 of the holding structure 6203.

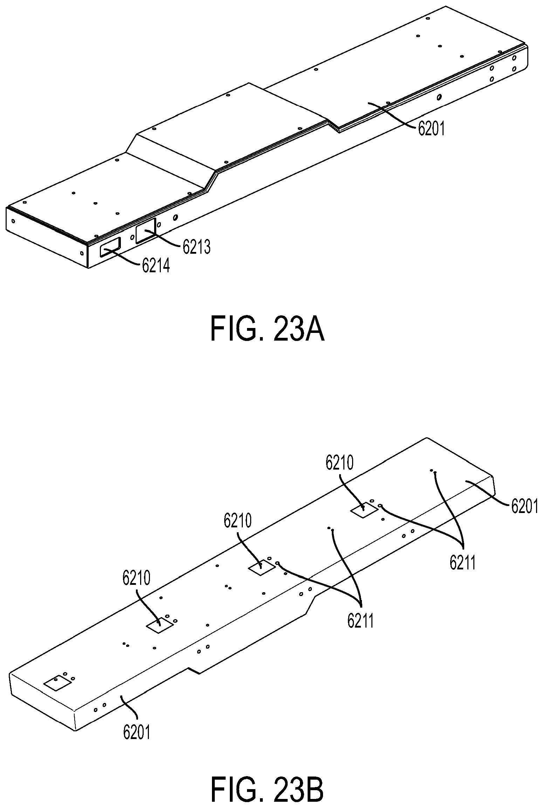

FIG. 23A - 23B depict an exemplary backbone structure 6201 that may be used in a support system. The backbone structure 6201 may generally be a planar configuration and serve as a primary support for the support system. Additionally, the backbone structure may also comprise a hollow interior which may serve to contain a communication bus or the like. The FIG. 23A depicts a back view of the backbone structure 6201 that may be held by a pole such as a rod or an IV pole. The backbone structure 6201 may be removably held by the pole through various fastening means e.g. screw clamping, bolts, pins, etc. Alternatively, the backbone structure 6201 may be permanently secured by a flat surface such as a wall, framework, etc or the like. A different embodiment of the backbone structure 6201 may be independently positioned on a flat surface such as a flat ground, floor or a table top. The backbone structure 6201 may also provided with at least one power supply inlet 6214 and one or more data communication ports 6213. A front face of the backbone structure 6201, as depicted by FIG. 23B may provide various engaging means for attaching a pre-identified component. FIG. 23B further depicts a power supply outlet 6210 that may serve to extend circuitry housed in the backbone structure 6201 to the pre-identified component. A plurality of fixation points 6211 may be provided to facilitate this engagement.

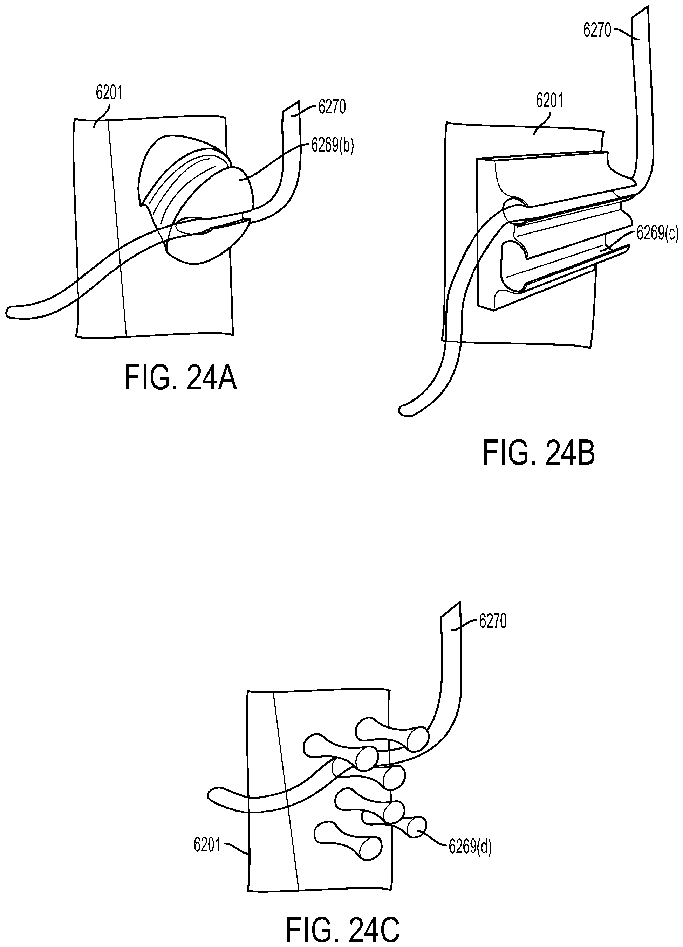

FIG. 24A to FIG. 24C depict exemplary embodiments of tube managing members 6269(b), 6269(c) and 6269(d) on a backbone structure 6201. The depicted embodiments share similar functional aspects. However, one of the embodiments may be preferred over the other in case of specific dimensions or material of the tubes. Structurally, the tube managing feature may be varieties of protrusions that may serve to provide an organized handling of the tubes, conduits or channels, etc, attached to a clamped component. The tube management features 6269 (b), (c) and (d), may be designed to engage or receive the freely suspending tubes. A single backbone structure 6201 may provide a plurality of tube managing features 6269. FIG. 24A and FIG 24B illustrate that a single protrusion may provide tube management for one or more tubes attached to the clamped device. Additionally, the tube management features 6269 may be provided at any suitable site on the backbone structure 6201. Alternatively, other resources for managing tubes or conduits may be placed on any suitable component of a support system to provide further convenience or comfort to the user of the clamping arrangement. FIG. 24C depicts a plurality of protrusions 6269(b) to provide a multitude of tube management options.

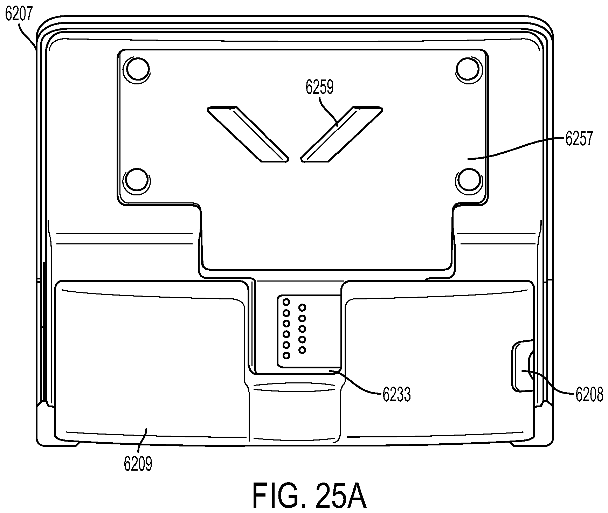

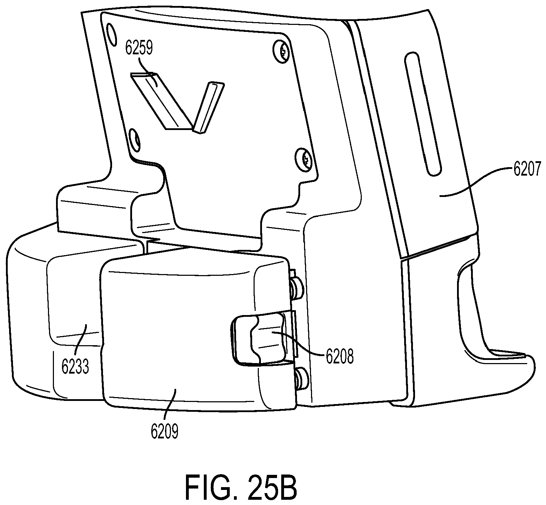

FIG. 25A and FIG. 25B illustrate an exemplary embodiment of a device 6207 to be clamped and a detachable power-supply pack 6209. The components of FIG. 25A and FIG. 25B are described with simultaneous reference to FIG. 21A. A pairing plate 6257 is also depicted by FIG. 25A which may be disposed on the back of the device 6207. The pairing plate 6257 may be fixed to the device 6207 via screws, bolts, or the like or may be permanently fastened to the back of the device 6207. Considering FIG. 21A wherein an exploded view of the clamp assembly 6205 illustrates the positional relationship between the pairing plate 6257 and the device 6207. Thus, the pairing plate 6257 may serve as an intermediate coupling member to attach the device 6207 to the clamp assembly 6205. The pairing plate 6257 may provide one or more paring members 6259 that may serve to engage the device 6207 to the clamp assembly 6205. The pairing members 6259 may be outward extensions that may be trapped in a suitable engaging means that may be provided in a clamp assembly 6205. Further, the pairing members 6259 may be configured to endure the weight of the device 6207 along with one or more additional components they engage with the clamp assembly.

A detachable power supply pack 6209 may be mechanically fastened to the device 6207. The detachable power supply pack 6209 may be configured to avoid interference to the data communication and power supply connection that is extended to the clamping device 6207. Additionally, no separate mechanism may be involved in clamping of the detachable power supply pack 6209 along with the device 6207. A snap button 6208 may be provided on the detachable power supply pack 6209. The snap button 6208 may serve to engage and/ or disengage the detachable power supply pack 6209 with the device 6207. The detachable power supply pack 6209 may continue to be engaged to the device 6207 irrespective of the device being clamped on any other clamp-able structure such as a pole or a vertical rod. The detachable power supply pack 6209 may serve to charge a battery of the device 6207 when it is not clamped to a clamp-able structure that may provide the necessary power supply to the device 6207.

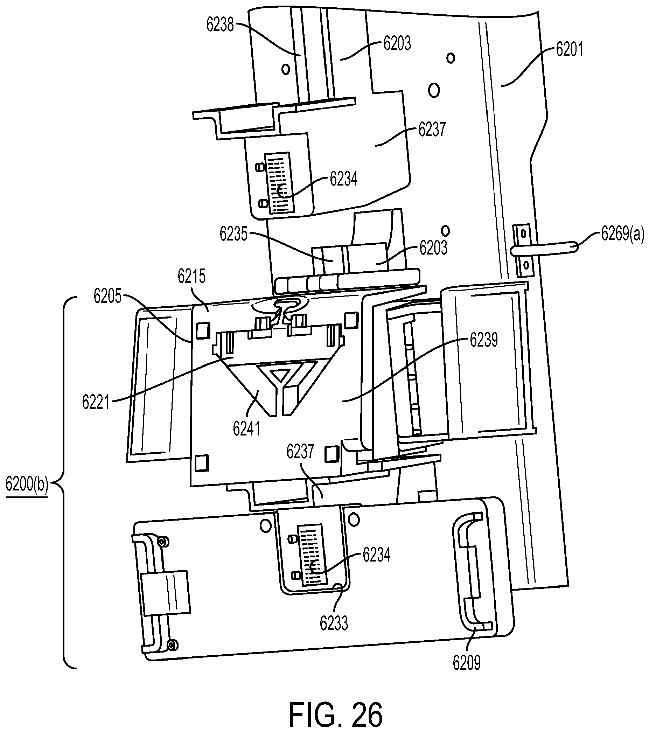

FIG. 26 depicts a back, right-side view of a partially assembled clamping arrangement 6200(b), specifically illustrating a backbone structure 6201, holding structures 6203, clamp assembly 6205 and a detachable power supply pack 6209. FIG. 26 further depicts two holding structures 6203, one of which may be provided with a partial clamping arrangement 6200(b). A similar or different clamping arrangement 6200(b), depending upon the purpose, may be achieved with the other holding structure 6203. The base portion 6237 of the holding structure 6203 may provide a passage 6234 for an electrical contact that may serve as a data communication and power supply source to the clamping device 6207. This base portion 6237 of the holding structure 6203 may be received by a depression or housing 6233 provided on the detachable power supply pack 6209. Such an arrangement may ensure that the detachable power supply pack 6209 does not obstruct the electrical contact between the holding structure 6203 and the device 6207. FIG. 26 further provides a conceptual view of a back planar surface 6239 of a frame structure 6215. The back planar surface 6239 may provide a socket 6241 for receiving a pre-determined clamping device and a latch 6221. The socket 6241 may serve to provide an engagement location for coupling the clamp assembly 6205 with the pre-determined clamping device. The latch 6221 may enable a locking mechanism for coupling the clamp assembly 6205 and the clamping device. As the device is received in the socket 6241, the latch 6221 may pivotally displace to obstruct the detaching of the clamping device with the clamp assembly 6205. A mechanical force from a user on the latch 6221 may allow detaching of the clamping device from the clamp structure 6205.

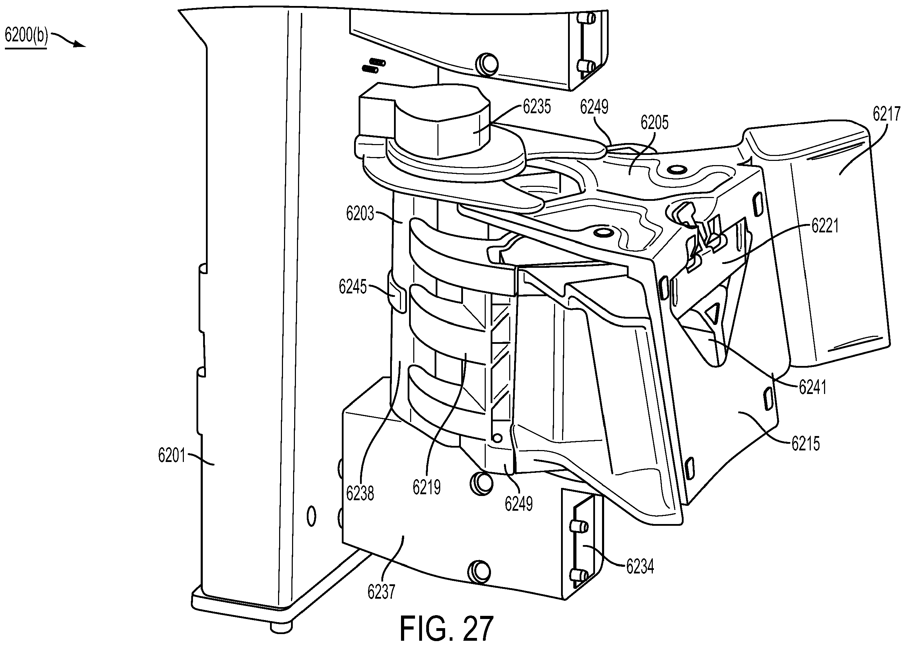

FIG. 27 depicts an exemplary embodiment of a partial clamp arrangement 6200(b), specifically illustrating a clamping engagement between a clamp assembly 6205 and a holding structure 6203. A partial gripping of the holding structure 6203 by the clamp assembly 6205 is depicted through FIG. 27. The clamp assembly 6205 is illustrated in a second position. This second position may be described as opening of a mouth of the clamp assembly 6205, such that a first jaw shaped end 6219 of a gear plate 6249 may travel away from an opposing jaw shaped end 6219 of an opposing gear plate 6249. Thereby the clamp assembly 6205 in its second position may be configured to secure an intermediate rod shaped portion 6238 of the holding structure 6203. In the first position of the clamp assembly 6205 the jaw shaped ends 6219 of the two gear plates 6249 in a clamp assembly 6205 may be interlocked. An actuator or handle 6217 may be provided on the exemplary clamp assembly 6205. The embodiment of the partial clamp arrangement 6200(b) shown in Fig. 27 depicts the use of two actuators 6217 in the example clamp assembly 6205. The actuators 6217 may be user operated components of the clamp assembly 6205. A displacement of the actuators 6217 may cause resultant displacement of the gear plates 6249, consequently shifting the clamp assembly 6205 from the first position or closed position to the second position or open position. The intermediate rod shaped portion 6238 may further provide a rib portion 6245 that may be a member that serves in enhancing the grip between the clamp assembly 6205 and the holding structure 6203 by causing the jaw shaped ends to slide over the rib portion 6245 to securely grasp onto the holding structure 6203. That is, the jaw shaped end 6219 of the gear plate 6249 may grip the intermediate rod portion 6238 such that a part of one or more jaws of the clamp assembly 6205 may slide over the rib portion 6245 and grip the intermediate holding structure 6238 via the spring biasing of the jaw shaped ends 6219 toward each other. The rib portion 6245 may be configured to provide a minimum friction or resistance to the incoming jaw shaped end 6219 of the plate 6249. It must be noted, that different embodiments of a clamping arrangement 6200(b) may provide an alternative grip enhancing feature on the holding structure 6203 or the clamp assembly 6205.

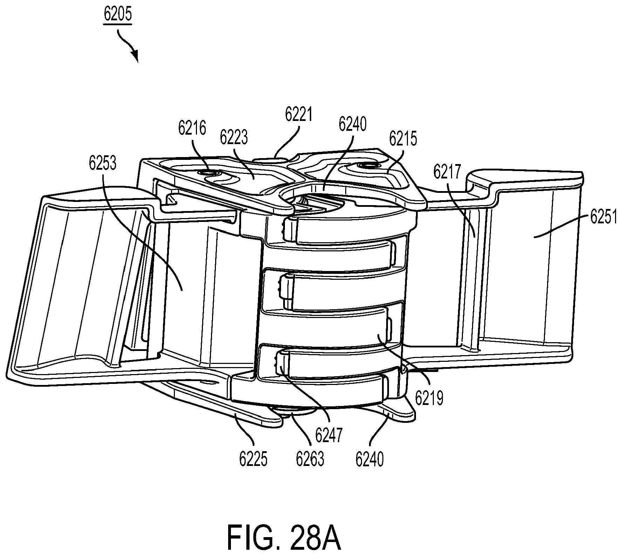

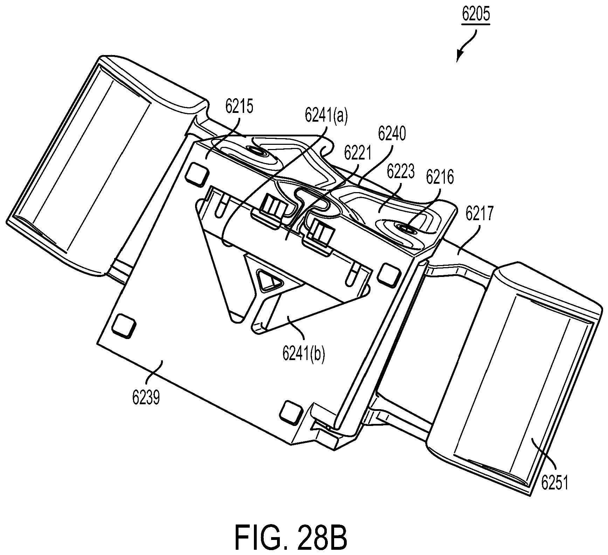

FIGS. 28A through 28E depict various isometric views of a specific embodiment of a clamp assembly 6205. Additionally, the subsequent views further depict the clamp assembly 6205 in a first position which may be a closed position and a second position which may be an open position. The exemplary clamp assembly 6205 may comprise a frame 6215 which may be similar to three face housing which when viewed in a profile that may resemble a "C" like structure. The frame 6215 may provide a top surface 6223, a bottom surface 6225 and a back surface 6239. The back surface 6239 may be more clearly visible through FIG. 28B. Additionally, the frame structure 6215 may further provide attachment points 6216 for engaging and retaining various supplementary components of the clamp assembly 6205.

A top surface 6223 and a bottom surface 6225 may be generally parallel to each other. Further, a back surface 6239 may be generally perpendicular to the top surface 6223 and the bottom surface 6225. The top and bottom surfaces 6223 and 6225, respectively, may further include an indentation 6240 each, of substantially identical dimensions. The indentations 6240 may be curved or crescent shaped and recessed into the outward projecting edges of the top and bottom surfaces 6223 and 6225, respectively. The curved or crescent shaped indentation 6240 is depicted on the top surface 6235 of the frame 6215; this unique shape of the indentations 6240 may guide a user about appropriately engaging the clamp assembly 6205 with a clamp-able structure. As the jaw shaped ends 6219 grip the clamp-able structure, the curved or crescent shaped indentations 6240 may receive the clamp-able structure without any interference with the gripping mechanism between the clamp assembly 6205 and the holding structure 6203.

As best shown in FIG. 28B, a back surface 6239 of a frame structure 6215 may provide a socket 6241. The socket 6241 may be configured to serve a dual purpose. It may serve as an attach site for a pre-determined clamping device. Further, the socket 6241 may be divided into two sections. A first section may be a latch receiving section 6241(a) and a second section may be a device receiving section 6241(b). As the device receiving section 6241(b) engages the pre-determined clamping device, a latch 6221 retained in the latch receiving section 6241(a) may provide a locking mechanism to fasten the pre-determined clamping device with the clamp assembly 6205. The latch 6221 may require user interference in order to disengage the pre-determined clamping device and the clamp assembly 6205.

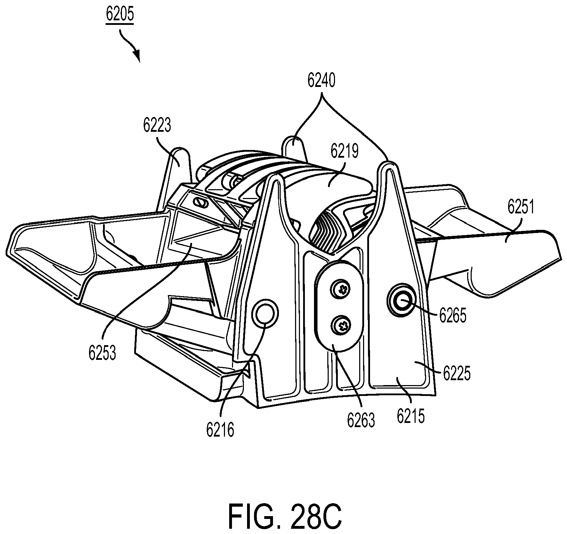

Referring to FIG. 28C, a bottom surface 6225 of the frame structure 6215 comprises an alignment component 6263. The alignment component 6263 may be made of a material other than the material used for making the frame structure 6215. The material used for the alignment component 6263 may be comprised of a metal, a metal alloy or other suitable material. The alignment component 6263 may be fastened to the frame structure 6215 by one or more fastening screws, bolts, nuts, etc. Moreover, the alignment component 6263 is configured to cooperate with a complementary alignment component that is provided on a graspable structure that receives the clamp assembly 6205. The engagement between the alignment component 6263 and the complementary alignment component may be useful in maintaining the stability of the clamp assembly 6205 as it engages with the clamp-able structure. Further, this engagement may be an additional engagement mechanism between the clamp assembly 6205 and the clamp-able structure, ensuring that no alterations occur to the complete clamping system during any allied unexpected occurrence. In other words, the interaction of the alignment component 6263 and the complementary alignment component 6236 in the holding structure 6203 may serve to inhibit relative movement between the clamp assembly 6205 and the clamp-able structure. The alignment component 6263 and the cooperating feature on the clamp-able structure may interact in a variety of ways, for example, they may interact with a tongue in groove or dovetailed type mating arrangement.

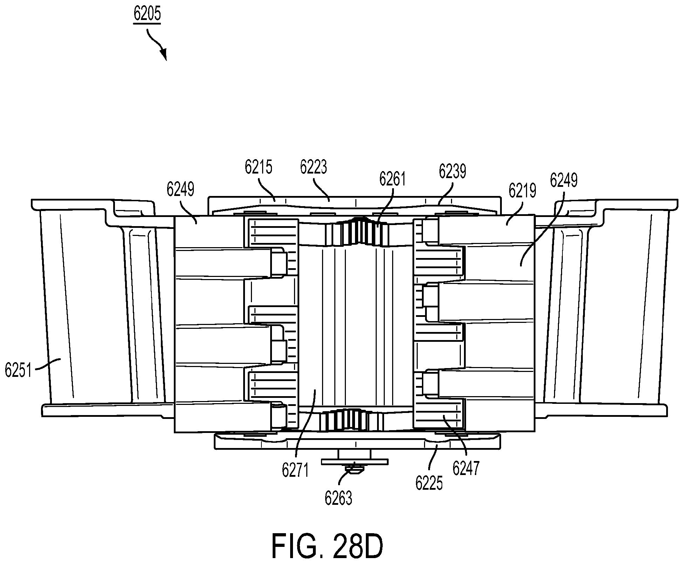

Referring to FIG. 28D, a clamp assembly 6205 may further comprise one or more gear plates 6249. An exemplary embodiment of the present disclosure exhibits a clamp assembly 6205 with two gear plates 6249. The material of the gear plates 6249 may be the same as the material of the frame structure 6215. The gear plates 6249 may further provide a gear shaped or toothed end 6261 and a jaw shaped end or portion 6219. The gear plates 6249 may be disposed in the frame structure 6215 in a way such that the gear shaped ends 6261 are proximal to the back surface 6239 of the frame structure and the jaw shaped ends 6219 are distal from the back surface 6239.

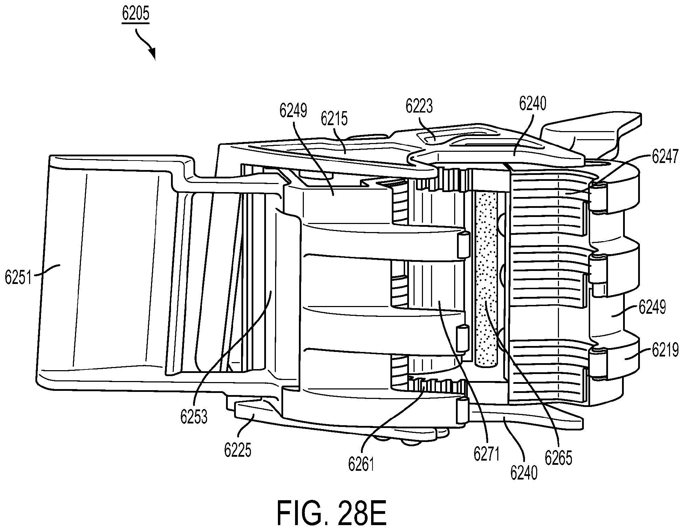

Referring now to FIG. 28E, gear plates 6249 may be engaged with a frame structure 6215 through various connectors 6265 such as a hinge pin or the like. Further, FIG. 28E depicts the clamp assembly 6205 in a second position i.e., an open position, wherein the jaw shaped ends 6219 of the opposing gear plates 6249 may separate from each other. A comparative viewing of FIG. 28A and FIG. 28E, easily explains the displacement of the clamp assembly 6205 from a first position (as shown in FIG. 28A) to a second position (as shown in FIG. 28E). Besides the opening and closing of a mouth, formed by the jaw shaped ends 6219 of the clamp assembly 6205, a displacement of the actuators 6217 when the clamp assembly 6205 shifts from the first position to the second position, may also be perceived from FIG. 28A and FIG. 28E.

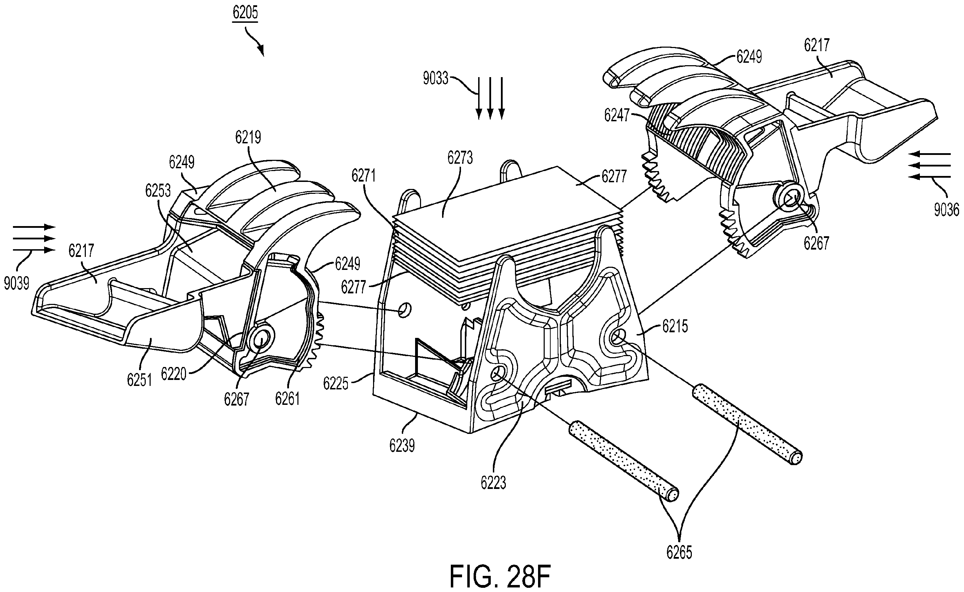

FIG. 28F depicts an exploded view of a clamp assembly 6205 and displays the positional relationship between the sub-components of the clamp assembly 6205. An interior of a frame structure 6215 may be configured to accept a stack 6271 of bias members 6273 (e.g., planar leaf springs) inside the frame structure 6215. The jawed components or gear plates 6249 may provide structural means for retaining the stack 6271 of bias members 6273. The stack 6271 of bias members 6273 may be retained in the interior of the frame structure 6215 in a way such that the edges 6277 of the stack 6271 of the bias members 6273 may be held by the one or more retainers provided in the gear plates 6249. A bias member 6273 may be a flexible, elastomeric object with an ability to store mechanical energy, when deformed, or any suitable spring. When the bias members 6273 are exposed to a force in a pre-determined manner, their reflex action may cause the desired movement of the clamp assembly 6205. The exemplary embodiment illustrated in the FIGS. 28A-28F may provide a spring steel sheet which may exert restorative force when deformed. Various other embodiments may employ other types of springs, for example, a compression spring or torsion spring, as a bias member 6273.

For the present embodiment, a bias member 6273 may be disposed in the interior of a frame structure 6215, with its edges received by one or more retaining means provided in a gear plate 6249. Such assembly of the bias member 6273 may not require a pre-load. However, this may depend on the material and type of respective bias members 6273, chosen for a specific embodiment. The stack 6271 of the bias members 6273 in the present embodiment may provide sheet-metal springs that may be initially bent in order to allow them to buckle as they are placed in the interior of the frame 6215. A mechanical force on the edges 6277 of the stack of the bias members 6271 may further axially compress or buckle the stack of the bias members 6273. Such a method of deforming the bias members 6273 may result in a relatively constant force that may be required for further compressing the bias members 6273. Additionally, the relatively constant force may be comfortable for the user and also provide a nearly consistent clamping force on various size poles.

An exploded view of a clamp assembly 6205 in FIG. 28F further depicts the assembling of the components in the clamp assembly 6205. A frame structure 6215 may be placed on its back surface such that the edges of a top surface 6223 and a bottom planar surface 6225 of the frame 6215 point away from the level at which the frame 6215 is placed. The top surface 6223 and the bottom surface 6225 may be generally parallel to each other and the back surface 6239 may be generally perpendicular to the top surface 6223 and the bottom surface 6225. Such an orientation may provide three ways of entering an interior of the frame 6215. One of the three ways may be a top entrance 9033 such that an incoming component may be opposite to the back surface 6239 of the frame 6215. The other two entrances may be from the sides adjacent to the back surface 6239. These may be depicted as a first side entrance 9036 and a second side entrance 9039. The gear plates 6249 may be received by the frame 6215 from the side entrances 9036 and 9039. The stack 6271 of bias members 6273 may be received by the interior of the frame 6215 from the top entrance 9033. The gear plates 6249 may be pivotally fastened when placed in the interior of the frame 6215. A pivotal fastening may facilitate the gear plates 6249 to move from a first position with closed jaw shaped ends 6219 to a second position with open jaw shaped ends 6219. Such pivotal motion may occur around a connector 6265 such as a hinge-pin that may also serve to attach the gear plates 6249 with the frame 6215. A different embodiment of the clamp assembly 6205 may provide a varied fastening means for facilitating the pivotal motion of the gear plate 6249. Alternatively, two separate mechanisms may be used for fastening the gear plates 6249 with the frame 6215 and facilitating the pivotal motion of the gear plate 6215.

An actuator 6217 is depicted by the FIG.28F, whose one end may be configured to be received by a gear plate 6249 and the other end may be operated by a user. The first end of the actuator 6217 may be an inserting member 6253 and the second end of the actuator may be a paddle member 6251. The inserting member 6253 may be received by a section 6220 of the gear plate 6249, thus attaching the actuator 6217 with the gear plate 6249. The engagement of the actuator 6217 and the gear plate 6249 may be configured to facilitate the operation of the gear plates 6249 via the actuators 6217. Consequently, when a user applies a downward mechanical force on the actuator 6217, displacement of the gear plates 6249 may be achieved by the actuator's 6217 rotation around the connectors 6265. Such displacement may cause the jaw shaped ends 6219 to open or close, as desired. This enables the jaw-shaped end 6219 of the gear plates 6249 to grip or un-grip a graspable component.

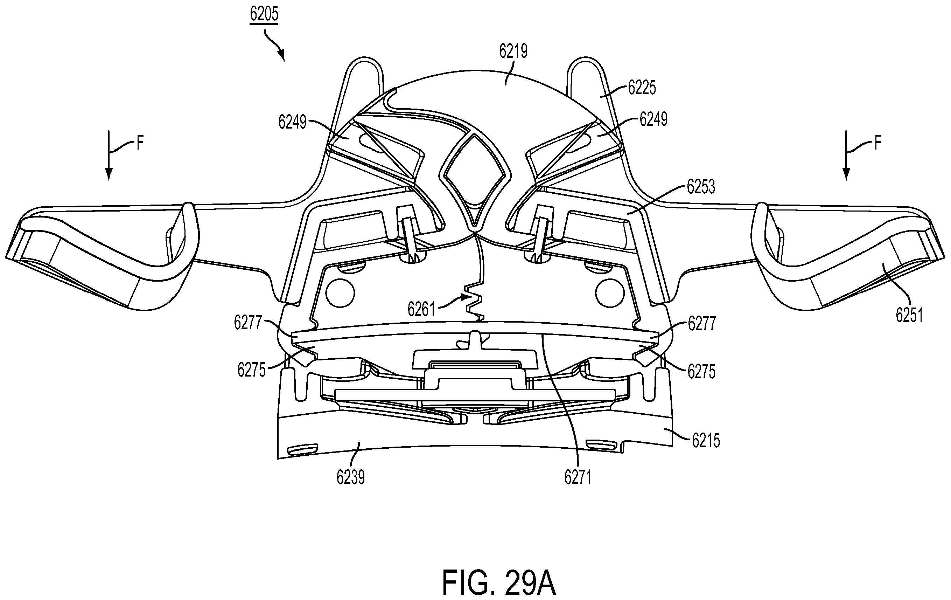

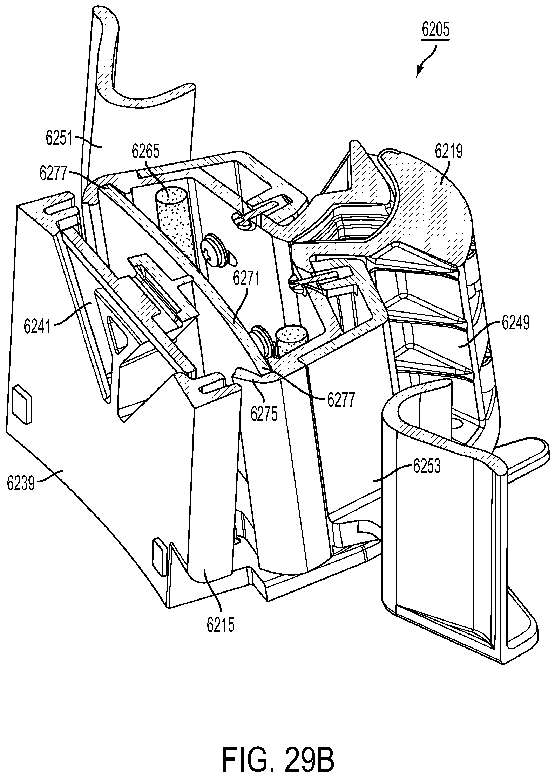

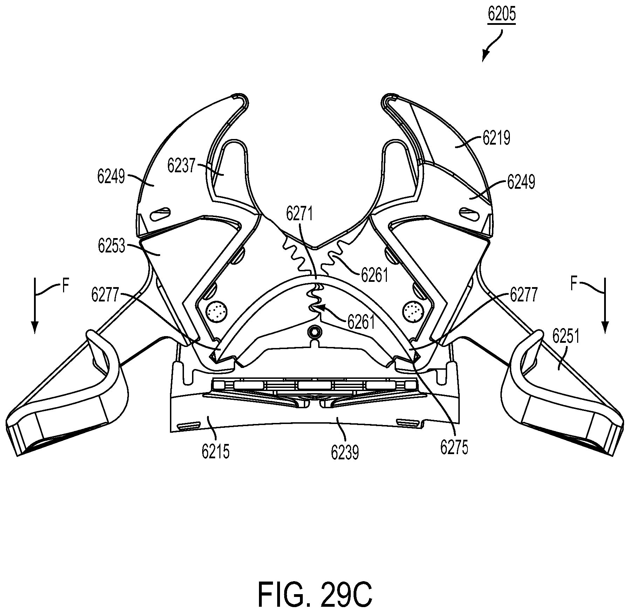

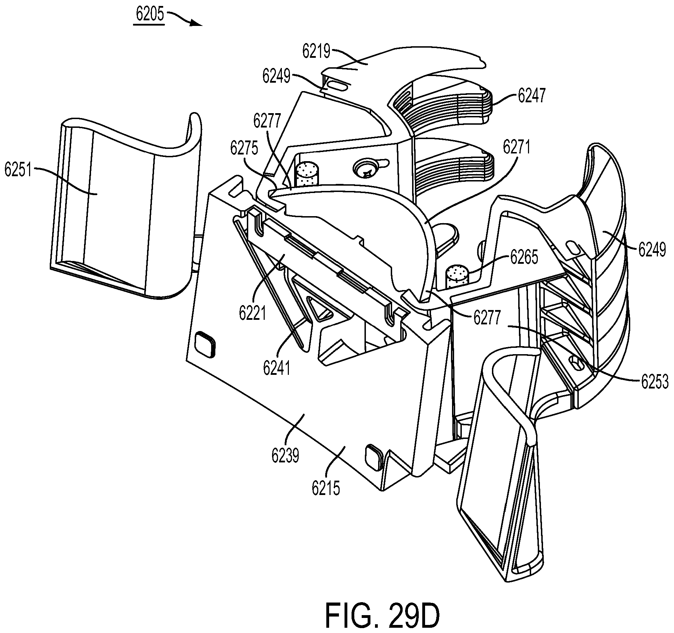

FIG. 29A to 29D depict cross-section views of an exemplary embodiment of a clamp assembly 6205. The cross-section views in FIG. 29A to 29D , best explain the motion of the clamp assembly 6205 as the gear plates 6249 displace from a first position to a second position. The exemplary clamp assembly disclosed herein employs two gear plates 6249. The gear plates 6249 may be displaced such that the jaw shaped ends 6219 and the gear shaped ends 6261 of both the gear plates 6249 may face each other. A stack of bias members 6271 may be disposed in the interior of the partially depicted frame 6215. The stack of the bias members 6271 may be disposed such that the edges 6277 of the stack of the bias member 6271 may occupy the pockets 6275 of both the opposing gear plates 6249. As the stack of the bias members 6271 is engaged on its edges 6277, any force on the bias members may be applied only on the engaged portion. The stack of bias members 6271 employed in present embodiment may be a stack of sheet of spring steel. A pre-determined number of spring metal sheets may be stacked to produce a desired force on additional compression or buckling of the stack of spring metal sheets. FIG. 29A & FIG. 29B depicts the positioning of the stack of bias members 6271 between the gear plates 6249 when the clamp assembly 6205 is in the first position, while FIG. 29C and FIG. 29D depict the positioning of the stack of bias members 6271 between the gear plates 6249 when the clamp assembly 6205 is in the second position.

In a second position, the jaw shaped ends 6219 of the two opposing gear plates 6249 are distant from each other to facilitate griping of a graspable structure. A downward force on a paddle portion 6251 of an actuator 6217 may cause displacement of the opposing gear plates 6249 each of which may be engaged with the respective actuators 6217. The resultant displacement of the gear plates 6249 may cause deformation of a stack 6271 of bias members 6273, engaged in the pockets 6275 of the opposing gear plates 6249. The deformation of the stack of bias members 6271 is depicted in FIG. 29C and FIG. 29D. As a result, an opposing mechanical force may be generated and stored in the stack of bias members 6271. The stored force in the stack of bias members 6271 may react when the force on the actuators 6217, is released. Releasing the actuators 6217 may cause the stack of bias members 6271 to return to its earlier position, as depicted in FIG. 29A and FIG. 29B. The gear shaped ends 6261 of the opposing gear plates 6249 may be in conjunction such that the tooth of the opposing gear shaped ends 6261 may mesh and un-mesh as the clamp assembly 6205 displaces from the first position to the second position, respectively.

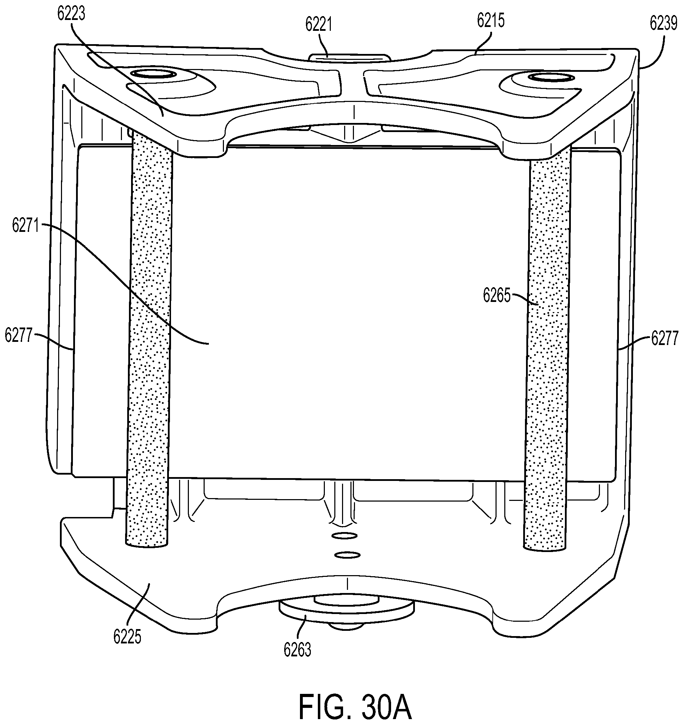

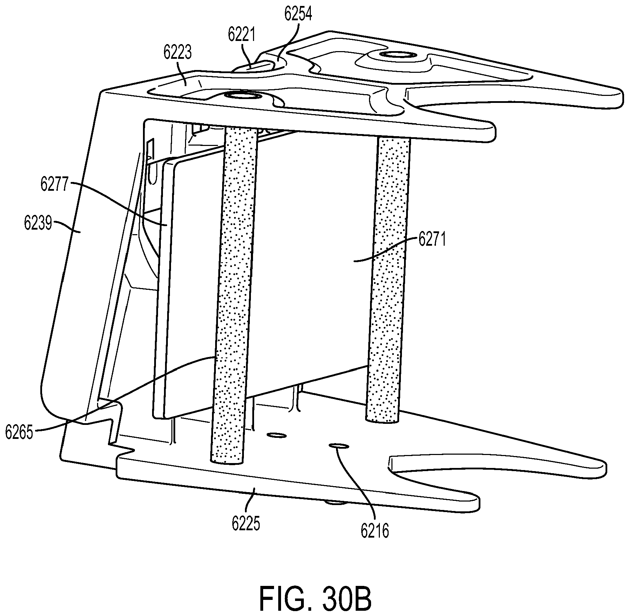

FIGS. 30A and FIG. 30B depict an exemplary embodiment of the positional relationship between a frame 6215, a stack of bias members 6271, one or more connectors 6265 and a latch 6221. The connectors 6265 may serve as attachment components between the frame 6215 and other components to form a clamp assembly. However, the connectors 6265 may be additionally configured to control the deformation of the stack of bias members 6271 disposed in the interior of the frame 6215. FIG. 30 A and FIG. 30B further depict, the positional relationship between the connectors 6265 and the stack of bias members 6271, to serve the purpose of controlling the deformation of the stack of the bias members 6271. As explained through earlier figures, the stack of bias members 6271 may be a number of spring steel sheets that may be engaged from its edges 6277 extending away from an interior of the frame 6215. A force on the edges 6277 may cause a deflection of the stack of bias members 6271. Such deflection may aid in appropriately retaining the stack of bias members 6271 inside the frame 6215. Additionally, such retainment may require further support as the stack of the bias members 6271 may undergo increased deflection when a clamp assembly 6205 displaces from a first position to a second position.

A connector 6265 may serve to provide a required additional support for retaining the stack of bias members 6271when a clamp assembly 6205 displaces to a second position. To provide such additional support, the connectors 6265 may be placed substantially closer to the edges 6277 of the stack of the bias members 6271 and in an interior of the frame 6215. The connectors 6265 may be configured to be parallel to the edges 6277 of the stack of bias members 6271. Furthermore, the connectors 6265 may extend from a top surface 6223 of the frame 6215 to a bottom surface 6225 of the frame 6215 thus covering the entire length of the stack of bias members 6271. The present embodiment may allow the connectors 6265 to fulfill a dual purpose of engaging additional components with the frame 6215 and also providing a required support for retaining the edges 6277 of the stack of bias members 6271A. Another embodiment of the clamp assembly 6205 may provide separate components for accomplishing the respective purposes. Alternatively, the mechanism of engaging the stack bias members 6271 may be different in different embodiments of the clamp assembly 6205.

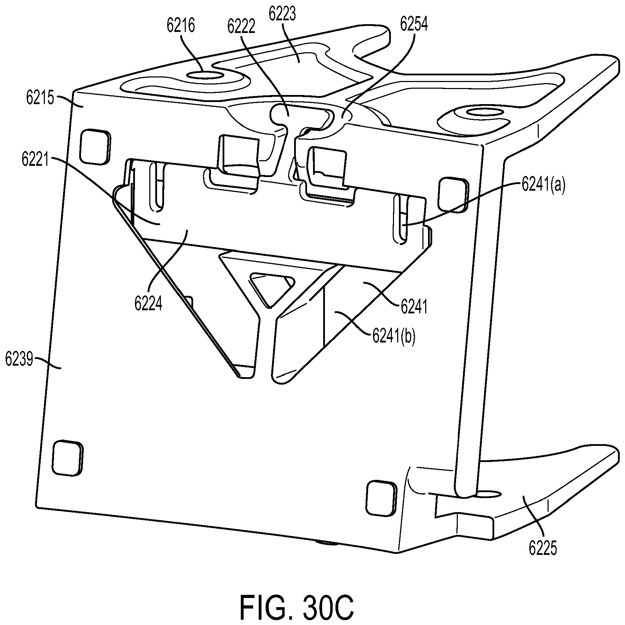

FIG. 30C depicts a latch 6221 engaged to a back planar surface 6239 of an exemplary embodiment of a frame 6215 of a clamp assembly 6205. A socket 6241 may be provided on the back surface 6239 of the exemplary frame 6215. The socket 6241 may be divided into two parts viz. 6241(a) and 6241(b). A first part 6241(a) may be configured to receive the latch 6221 and a second part 6241(b) may be configured to receive an additional pairing member to engage a clamping device. FIG 30C also depicts the engagement of the latch 6221 with back surface 6239 of the frame 6215, via the socket 6241. The socket 6241 may define a boundary in the back surface 6239 of the frame 6215. The boundary may be such as to show an inverted triangular shaped socket 6241. The latch 6221 may occupy the first part 6241(a) of the socket 6241 and may engage so as to pivot about a side of the inverted triangular shaped socket 6241. The side of the socket 6241 that engages the latch 6221 may further include an obstruction that may restrict the pivoting motion of the latch 6221.

A latch 6221 may provide a flap portion 6224 and a lever portion 6222. The latch 6221 may serve to connect a clamping device with the frame 6215. The latch 6221 may be received by a socket 6241 such that the flap portion 6224 and the lever portion 6222 may pivot about a point of attachment between the latch 6221 and the socket 6241. One or more paring members (not shown in this figure) may be received by the socket 6241 to engage the clamping device and may push the flap portion 6224 of the latch 6221 towards an interior of the frame 6215. The flap portion 6224 may pivot towards the interior of the frame 6215 and return to its original position when the paring members are completely received by a portion of the socket 6241. Consequently, by returning to the original position, the flap portion 6224 may lock the pairing members inside the socket 6241. The lever portion 6222 of the latch 6221 may be a user operative part of the latch 6221. The lever portion 6222 may originally rest on a groove 6254 provided on a connecting rim between the top surface 6223 and the back surface 6239 of the frame 6215. Forcing the lever portion 6222 away from the groove 6254 may cause the flap portion 6224 to pivot towards the interior of the frame 6215 thus releasing the pairing members from the socket 6241.

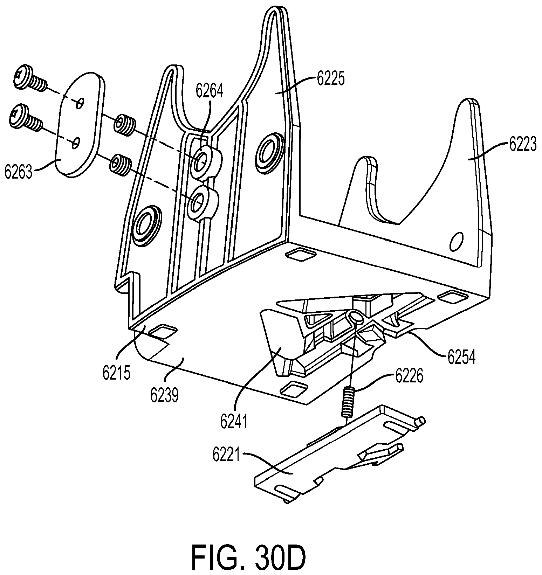

The representational view in FIG. 30D illustrates the assembling of components depicted in FIG. 30A, FIG. 30B and FIG. 30C. A bottom surface 6225 of the frame 6215 may provide an additional engagement component 6263. The additional engagement component 6263 may be a plate configured to couple with additional component The component 6263 may be attached to the bottom surface 6225 by fastening elements such as, but not limited to screws, bolts, nuts, etc. Additionally, a plurality of plate attaching points 6264 may be provided on the bottom surface 6225 of the frame 6215. Furthermore, FIG. 30D also depicts the assembling of a latch 6221 with a back planar surface 6239 of the exemplary frame 6215. The latch 6221 may be retained in a socket 6241 in the back planar surface 6239 of the frame 6215. A flexible member 6226 may be provided to pivotally engage the latch 6221 with the socket 6241. The examples of the flexible member 6226 may include, but not limited to, a coil spring or an elastomeric component. The latch 6221 may be substantially retained in the socket 6241. However, a part of the latch 6221 may also rest on the groove 6254, provided on an edge between the back planar surface 6239 and the top planar surface 6223. The latch 6221 may serve the purpose of engaging additional components to the back planar surface 6239 of the frame 6215.It must be noted, that the present embodiment of the clamp assembly discloses the component 6263 as an additional engagement component. Other embodiments of the clamp assembly may provide varied engagement components which may function differently to provide a dissimilar engagement mechanism.

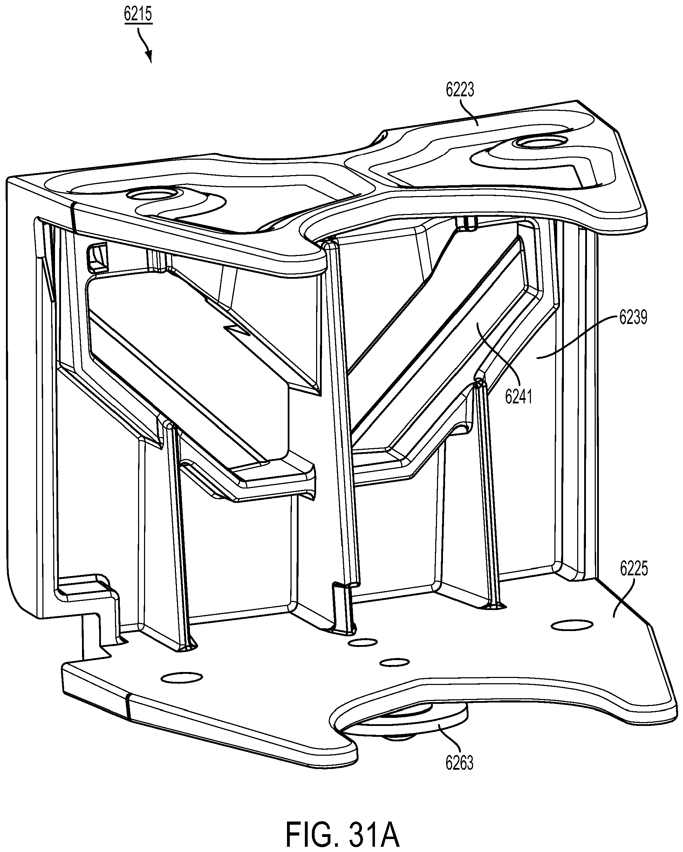

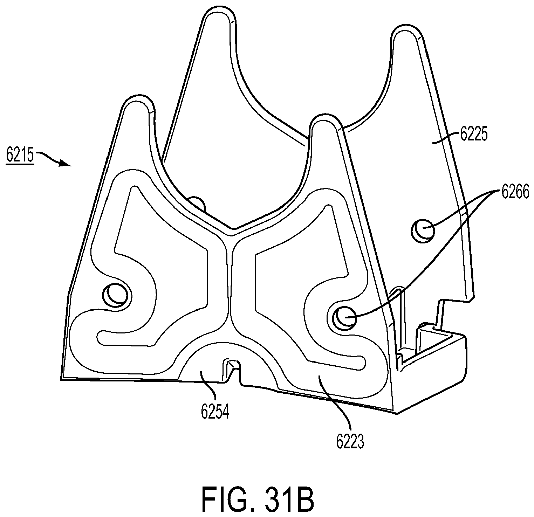

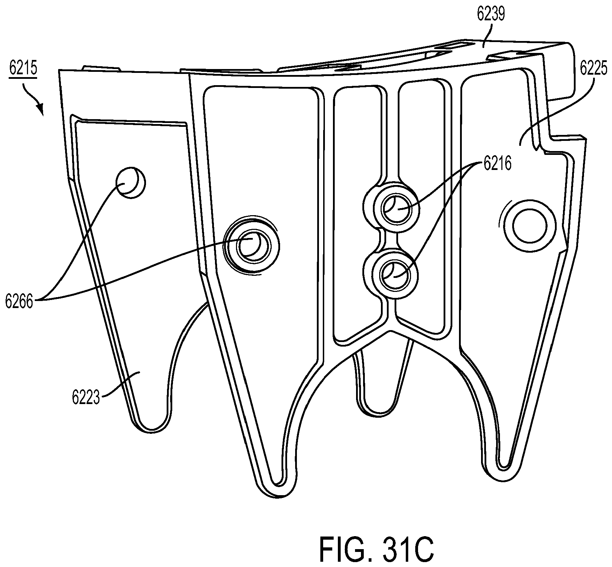

FIG. 31A to FIG. 31C depict representational views of an embodiment frame 6215 of an exemplary clamp assembly. The frame 6215 may serve as a primary base component configured to receive the other components of a clamp assembly. FIG. 31A depicts a front view of the example frame 6215, illustrating the interior of the frame 6215. A socket 6241 may be provided on a back surface 6239 of the frame 6215. The front view of the frame 6215 represents the socket 6241 as a generally V-shaped groove in the back surface of 6239 of the frame 6215. The functional and structural features of the socket 6241 have been described earlier through FIG. 28B. FIG. 31B depicts a top surface 6223 of the frame 6215. FIG. 31C depicts a bottom surface 6225 of the frame 6215. Various connecting points that receive the other components of the clamp assembly have been illustrated in FIG. 31B and FIG. 31C. Connector receiving points 6266 are depicted on the top surface 6223 and the bottom surface 6225 of the frame 6215. Attaching points 6216 on the bottom surface 6225 of the frame 6215 may serve in connecting an additional engagement component 6263 as previously described in FIG.30D.

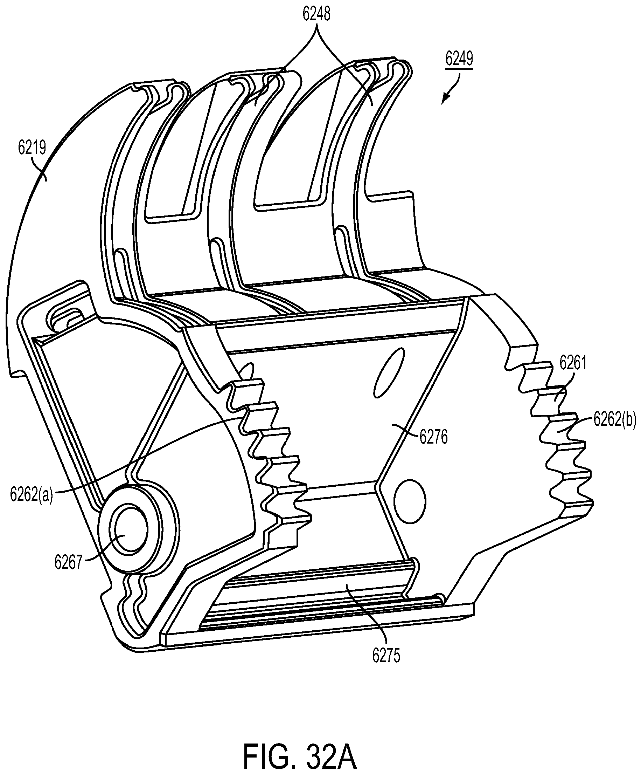

FIG. 32A to FIG. 32C depict representational views of an embodiment of a gear plate 6249 in an exemplary clamp assembly. FIG. 32A depicts a front, representational view of the gear plate 6249. FIG. 32A further illustrates a jaw shaped end 6219 and the gear shaped end 6261 of the gear plate 6249. The jaw shaped end 6219 may serve to grip a portion of a graspable structure and facilitate an engagement between the clamp assembly and the graspable structure. The jaw shaped end 6219 end may further accommodate a layer configured to enhance the grip of the jaw shaped end 6219 on a portion of the graspable structure. The layer may occupy a layer receiving surface 6248 on the jaw shaped end 6219 of the gear plate 6249. This layer may be made of an elastomeric material. Alternatively, the layer may be made of any material that maintains a high friction coefficient with the material of that portion of the graspable structure that is gripped by the jaw shaped end 6219 of the gear plate 6249. A different embodiment may provide an elastomeric layer on the gripped portion of the graspable structure. Other embodiments of the clamp assembly may provide similar or unique grip enhancing features.

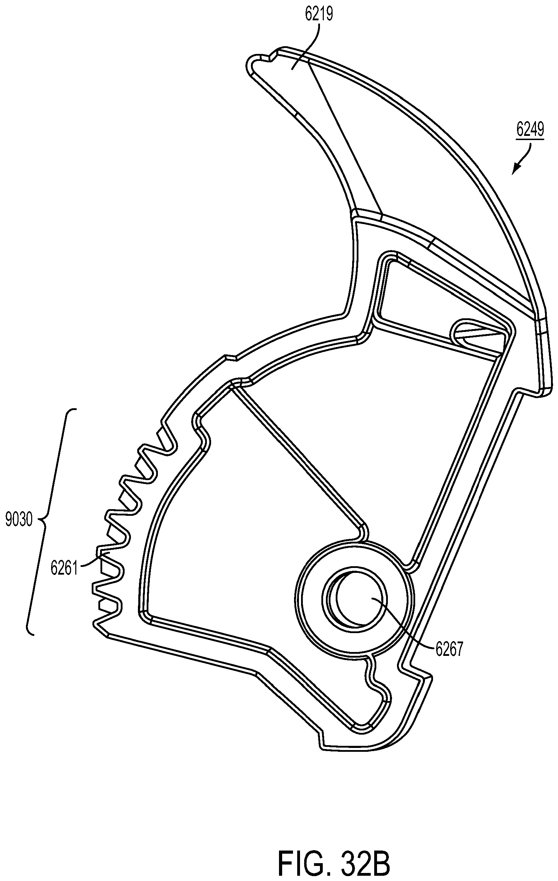

FIG. 32A, also depicts an embodiment of a gear shaped end 6261 of an exemplary gear plate 6249. The gear shaped end 6261 may be formed by a set of teeth imprinted on side edges 6262 of the gear plate 6249. A first toothed side edge 6262(a) may be substantially parallel and substantially adjacent to a second toothed side edge 6262(b). The teeth on the first toothed side edge 6262(a) of the gear shaped end 6261, may be offset by a desired tooth spacing with respect to the teeth on the second toothed side edge 6262(b) of the gear shaped end 6261. Such an embodiment may allocate an identical gear plate 6249 to be used as an opposing gear plate in the clamp assembly 6205. FIG. 32B depicts a side view of the gear plate 6249. The offset spacing 9030 between the teeth on the two toothed side edges 6262(a) and 6262(b), can be best seen through FIG. 32B. Alternatively, a different embodiment of the clamp assembly may provide two differently designed gear plates that oppose each other in a clamp assembly. FIG. 32B also depicts the connector grooves 6267 on the gear plate 6249. The connector grooves 6267 are configured to receive the connectors that may engage the gear plates 6249 with the frame 6215, as shown by FIG. 28F.

FIG. 32A further depicts a room 6276 between the adjacent toothed side edges 6262(a) and 6262(b) may include a pocket 6275. The pocket 6275 may extend from a first toothed side edge 6262(a) of the gear plate 6249 to a second toothed side edge 6262(b) of the opposing gear plate. The pocket 6275 may further serve to receive a portion of a stack of bias members 6273, as depicted in FIG. 29A and FIG. 29B. A similar pocket may be provided in an opposing gear plate 6249 of an exemplary clamp assembly. The stack of bias members may be substantially held by the two opposing pockets 6275 of the opposing gear plates 6249.

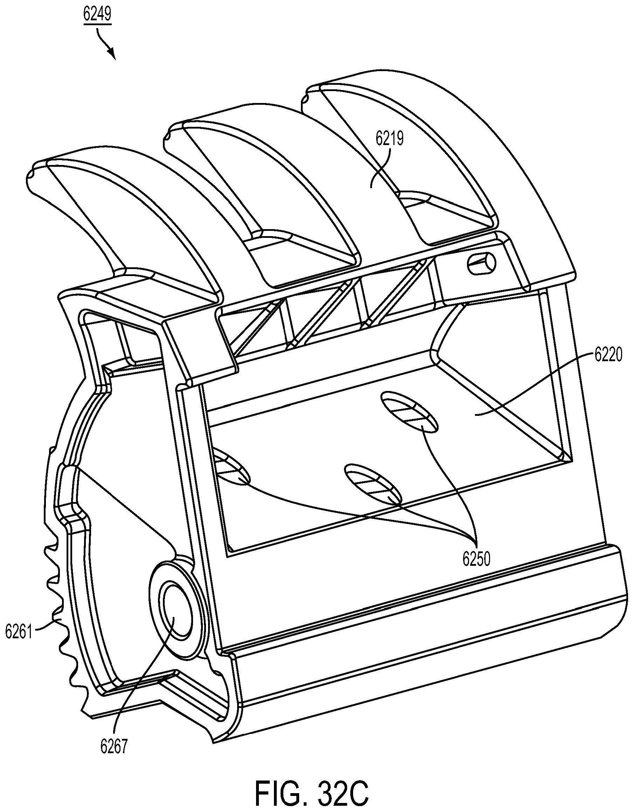

FIG. 32C depicts a representational, back view of an embodiment of a gear plate 6249 of an exemplary clamp assembly 6205. The components of FIG. 32C are hereby discussed in combination with FIG. 28F, as shown before. A section 6220 is depicted in FIG. 32C, which may serve to receive a portion of the actuator 6217. An inserting member 6253 of the actuator 6217 may be disposed in the section 6220 of the gear plate 6249, as shown in FIG. 28F. The section 6220 may also provide a set of attaching points 6250, depicted in FIG. 32C, which may serve to engage the inserting member 6253 of the actuator 6217. The inserting member 6253 may be fastened with the gear plate 6249 using screws, bolts, nuts, pins, etc.

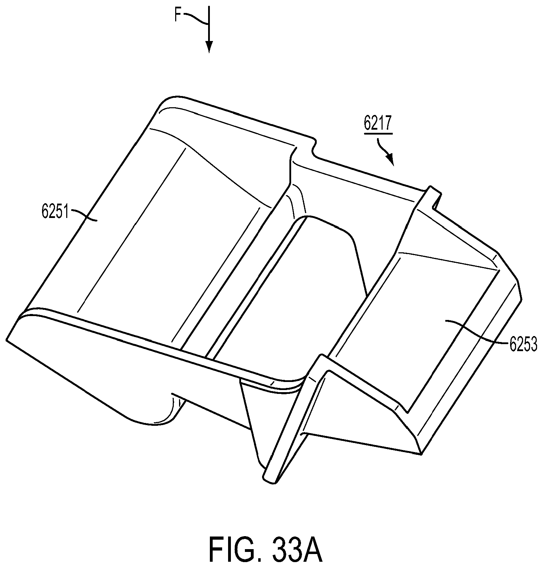

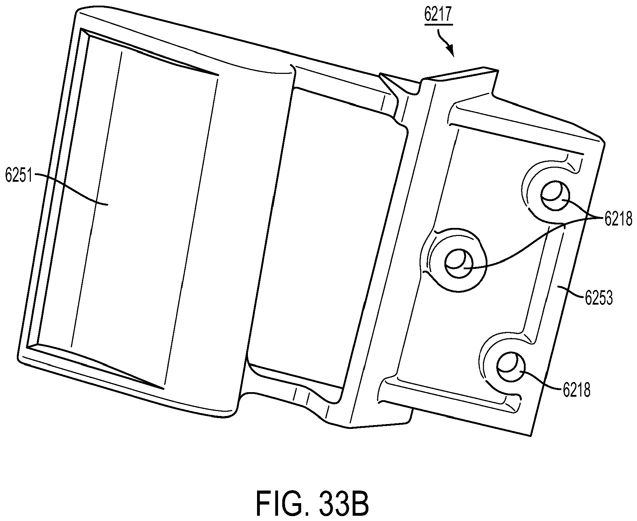

FIG. 33A and FIG. 33B respectively depict a front and back, representational view of an exemplary actuator 6217 of an example clamp assembly 6205. The components of FIG. 33A and FIG. 33B are hereby discussed in combination with FIG. 28F. The actuator 6217 may be divided into an inserting member 6253 and a paddle member 6251. The inserting member 6253 may serve as a coupling component for joining the actuator 6217 with a gear plate 6249, as shown in FIG. 28F. The inserting member 6253 may be received by a section 6220 of the gear plate 6249, as shown in FIG. 28F. FIG. 33B depicts a set of attaching points 6218 may be provided on the inserting member 6253 to engage the actuator 6217 with the gear plate 6249 as shown in FIG. 28F. These attaching points 6218 may be in conjunction with a set of attach points provided on the gear plate 6249.

A paddle member 6251 may be a user operated component and may facilitate the user to operate a clamp assembly. The paddle member 6251 may extend outward from the frame 6215 of a clamp assembly 6205, as shown in FIG. 28F. A downward force on the paddle member 6251 of the actuator 6217, may cause the clamp assembly 6205 to displace from a closed position (shown in FIG. 29A) to an open position (shown in FIG. 29C).

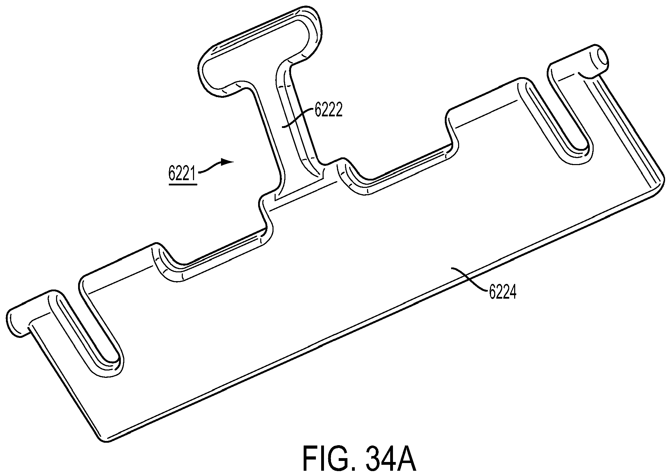

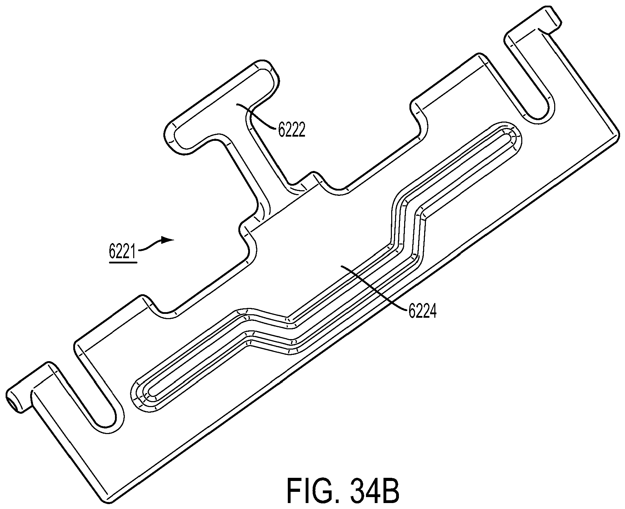

FIG 34A and FIG. 34B, respectively depict front and back representational views of an embodiment of a latch 6221 in an exemplary clamp assembly. The components of FIG. 34A and FIG. 34B may be explained with reference to FIG. 30C. As shown earlier through FIG. 30C, the latch 6221 may be configured to be received by a socket 6241 provided on a back surface 6239 of a frame 6215. The latch 6221 may serve as a locking mechanism for the engagement between the clamp assembly 6205 and a clamping device. The latch 6221 may further comprise a flap portion 6224 and a lever portion 6222. Continuing reference to FIG. 30C the latch 6221 may be coupled with the socket 6241 in a way that the flap portion 6224 and the lever portion 6222 may restrictively pivot around a rim of the socket 6241.

A lever portion 6222 of the latch 6221 may be a user operated portion. As shown in FIG. 30C, the lever portion 6222 may rest on a groove 6254 provided on the connecting edge between a top surface 6223 and a back surface 6239 of a frame 6215. The latch 6221 may be attached with the frame 6215 via a flexible member 6223, as shown in FIG. 30C. The flexible member 6223 may be glued to the frame 6215 or mechanically fastened using screws, bolts, nuts, pins, etc.

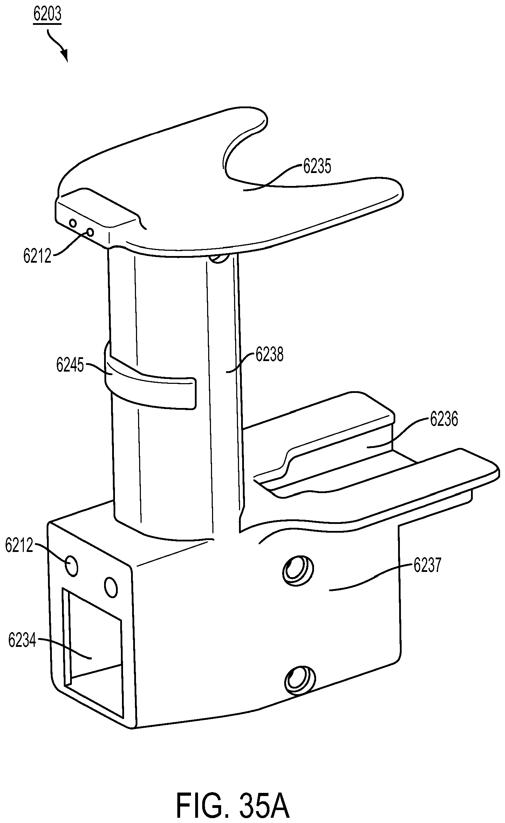

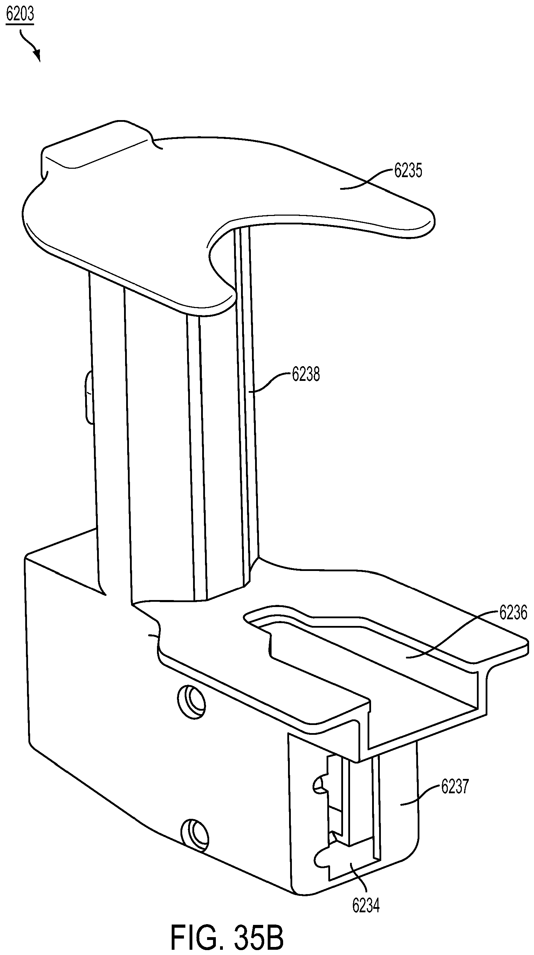

FIG. 35A and FIG. 35B depict back and front, representational views, respectively, of an embodiment of a holding structure 6203, in an exemplary clamp assembly 6205. The components of FIG. 35A and 35B can be better explained in combination with FIG. 21A. The holding structure may be divided into a top portion 6235, an intermediate rod shaped portion 6238 and a base portion 6237. The intermediate rod shaped portion 6238 may serve as a grasping portion that may be received by a jaw shaped end 6219 of the clamp assembly 6205, as shown in FIG. 21A. The intermediate rod portion 6238 may further provide a rib portion 6245. The rib portion 6245 may serve as a slide-able component as the jaw shaped end 6219 of the clamp assembly 6205, begins to grip the intermediate rod shaped portion 6238.

As shown in FIG. 35B and FIG. 21A, a base portion 6237 of the holding structure 6203 may provide a passage 6234 for extending a data and power supply circuitry from a backbone 6201 to a clamped device 6207 of an exemplary clamping arrangement 6200(b). The base portion 6237 may further comprise an alignment component 6236 configured to receive a complementary alignment component 6263 provided on the clamp assembly 6205. The engagement between the alignment component 6236 and the complementary alignment component 6263 may provide an additional engagement between the holding structure 6203 and the clamp assembly 6205, as shown in FIG. 21A. Furthermore, the base portion 6237 of the holding structure 6203 may be received by a depression or a housing 6233 in a detachable data and power supply pack 6209. Such an engagement may facilitate an unobstructed electrical communication between the base portion 6237 of the holding structure 6203 and the device 6207.

## Claims

1. A clamp assembly (6200(b)) configured for clamping a medical device onto a graspable structure, said clamp assembly (6200(b)) comprising:
a frame (6215) with a top surface (6223) and a bottom surface (6225) spaced from the top surface (6223) so as to form a gap between the top surface (6223) and the bottom surface (6225), the top surface (6223) comprising a first indentation (6240) and the bottom surface (6225) comprising a second indentation (6240), the first and second indentations (6240) being configured to receive a graspable structure, the top surface (6223) and the bottom surface (6225) being connected using a back surface (6239), the back surface (6239) further comprising an interior face and an opposing exterior face;
a first jawed component (6249) and a second jawed component (6249), the first and second jawed components (6249) each being pivotally retained in the frame (6215) such that at least part of the first and second jawed components (6249) are disposed between the top surface (6223) and the bottom surface (6225) in the gap and so as to have an axis of rotation substantially parallel to the back surface (6239), the first and second jawed components (6249) each comprising a jawed end (6219) and a geared end (6261) opposing the jawed end (6219), wherein the geared end (6261) of the first jawed component (6249) and the geared end (6216) of the second jawed component (6249) interdigitate;
a first actuator (6217) and a second actuator (6217) coupled to the first and second jawed components (6249), respectively, the first and second actuators (6217) each comprising an end (6251), the ends (6251) of the first end second actuators (6217) being distal to the first and second jawed components (6249), respectively; and
at least one bias member (6273) biasing the first and second jawed components (6249) to first positions in which there is no interference between the first and second indentations and the graspable structure wherein displacement of the first and second actuators (6217) causes the displacement of the first and second jawed components (6249) to pivot from their first positions to second positions in which the jawed ends (6219) of the first and second jawed components (6249) are spread apart from each other whereas the geared ends (6261) ensure substantially equal and opposite pivotal displacement of the first and second jawed components (6249),
**characterized in that** the bottom surface (6225) further comprises an alignment component (6263) configured to be received by complementary alignment component (6236) of the graspable structure.

2. The clamp assembly (6200(b)) of claim 1, wherein the frame (6215) further comprises a plurality of sections disposed on the interior face of the back surface (6239), the plurality of sections are configured to accept the first and second jawed components (6249).

3. The clamp assembly (6200(b)) of claim 1, wherein the first and second jawed components (6249) are pivotally retained via first and second connectors (6265) disposed between the top surface (6223) and the bottom surface (6225) of the frame (6215), wherein optionally the first and second jawed components (6249) pivot around first and second axes of rotation passing through the connectors (6265), and wherein optionally the connectors (6265) are hinge pins.

4. The clamp assembly (6200(b)) of claim 1, wherein the first and second jawed components (6249) further comprise one or more pockets (6275) configured to substantially receive the at least one bias member (6273).

5. The clamp assembly (6200(b)) of claim 4, wherein the at least one bias member (6273) is partially compressed in order to be placed in the one or more pockets (6275) of the first and second jawed components (6249) and/or is substantially disposed in opposing one or more pockets (6275) of the first and second jawed components (6249).

6. The clamp assembly of claim 1, wherein the displacement of the first and second actuators (6217) causes displacement of the first and second jawed components (6249) which further compresses the at least one bias member (6273).

7. The clamp assembly of claim 6, wherein the at least one bias member (6273) is configured to hold the first and second jawed components (6249) in their first positions and/or allow the first and second jawed components (6249) to be displaced into their second positions and/or allow the first and second jawed components (6249) to be displaced, which causes the jawed ends (6219) to grip the graspable structure.

8. The clamp assembly (6200(b)) of claim 1, wherein the jawed ends (6219) of the first and second jawed components (6249), are substantially covered with a layer.

9. The clamp assembly (6200(b)) of claim 8, wherein the layer is made of a first material and the first material is an elastomeric material.

10. The clamp assembly (6200(b)) of claim 9, wherein the layer is made of a first material having a high friction coefficient with a second material of a holding structure (6203) and/or a material having high friction coefficient with the material of a one or more slide-able members disposed on the holding structure (6203), such as rubber.

11. The clamp assembly (6200(b)) of claim 1, wherein the clamp assembly further comprises a latch, wherein the frame (6215) further comprises a socket (6241) on the back surface (6239), the socket (6241) is configured to pivotally retain the latch (6221) via a latch retaining bias member (6273), wherein optionally the latch (6221) is configured to couple the clamp assembly (6200(b)) with a clamping device (6207) when the latch (6221) pivots to a locking position and/or release the clamping device (6207) when the latch (6221) pivots to an unlocking position.

12. The clamp assembly (6200(b)) of claim 11, wherein the latch (6221) further comprises a flap portion (6224) and a lever portion (6222), wherein optionally the flap portion (6224) of the latch (6221) is pivoted towards an interior of the frame (6215) to receive one or more pairing members (6259) of a clamping device (6207), in the socket (6241) and/or the flap portion (6224) of the latch (6221) is pivoted back after completely receiving the one or more pairing members (6259) of the clamping device (6207), in the socket (6241) and/or the lever portion (6222) is configured to operatively allow releasing the pairing members (6259) from the socket (6241) in the back surface (6239) of the frame (6215).

13. The clamp assembly (6200(b)) of claim 1, wherein the frame (6215) further comprises a plurality of tracks configured to receive the first and second jawed components (6249).

14. The clamp assembly (6200(b)) of claim 1, wherein the jawed ends (6219) of the first and second jawed components (6249) are coupled with the frame (6215) through first and second connectors (6265), respectively, and wherein optionally the first and second jawed components (6249) are coupled with the frame (6215) through hinge pins.

15. The clamp assembly (6200(b)) of claim 1, wherein the first and second jawed components (6249) further comprise sections configured to partially receive the first and second actuators (6217).

16. The clamp assembly (6200(b)) of claim 15, wherein each of the first and second actuators (6217) further comprise a pairing member and a paddle member (6251), wherein optionally the sections of the first and second jawed components (6249) are configured to receive the pairing members of the first and second actuators (6217).

17. The clamp assembly (6200(b)) of claim 1, wherein the displacement of the first and second actuators (6217) causes a desired displacement of the first and second jawed components (6249).

## Patentansprüche

1. Klemmanordnung (6200(b)), die zum Festklemmen einer medizinischen Vorrichtung an einer greifbaren Struktur ausgelegt ist, wobei die Klemmanordnung (6200(b)) Folgendes umfasst:
einen Rahmen (6215) mit einer oberen Fläche (6223) und einer unteren Fläche (6225), die von der oberen Fläche (6223) beabstandet ist, sodass ein Spalt zwischen der oberen Fläche (6223) und der unteren Fläche (6225) gebildet wird, wobei die obere Fläche (6223) eine erste Vertiefung (6240) umfasst und die untere Fläche (6225) eine zweite Vertiefung (6240) umfasst, wobei die erste und die zweite Vertiefung (6240) dazu ausgelegt sind, eine greifbare Struktur zu empfangen, wobei die obere Fläche (6223) und die untere Fläche (6225) unter Verwendung einer hinteren Fläche (6239) verbunden sind, wobei die hintere Fläche (6239) ferner eine Innenfläche und eine gegenüberliegende Außenfläche umfasst;
eine erste Backenkomponente (6249) und eine zweite Backenkomponente (6249), wobei die erste und die zweite Backenkomponente (6249) jeweils schwenkbar in dem Rahmen (6215) gehalten werden, sodass mindestens ein Teil der ersten und der zweiten Backenkomponente (6249) zwischen der oberen Fläche (6223) und der unteren Fläche (6225) in dem Spalt angeordnet sind und so eine Drehachse im Wesentlichen parallel zu der hinteren Fläche (6239) aufweisen, wobei die erste und die zweite Backenkomponente (6249) jeweils ein Backenende (6219) und ein dem Backenende (6219) gegenüberliegendes verzahntes Ende (6261) umfassen, wobei das verzahnte Ende (6261) der ersten Backenkomponente (6249) und das verzahnte Ende (6216) der zweiten Backenkomponente (6249) ineinandergreifen;
einen ersten Aktuator (6217) und einen zweiten Aktuator (6217), die mit der ersten bzw. der zweiten Backenkomponente (6249) gekoppelt sind, wobei der erste und der zweite Aktuator (6217) jeweils ein Ende (6251) umfassen, wobei die Enden (6251) des ersten und des zweiten Aktuators (6217) fern von der ersten bzw. der zweiten Backenkomponente (6249) gelegen sind; und
mindestens ein Vorspannelement (6273), das die erste und die zweite Backenkomponente (6249) in erste Positionen vorspannt, in denen keine Beeinträchtigung zwischen der ersten und der zweiten Vertiefung und der greifbaren Struktur besteht, wobei die Verschiebung des ersten und des zweiten Aktuators (6217) die Verschiebung der ersten und der zweiten Backenkomponente (6249) veranlasst, aus ihren ersten Positionen in zweite Positionen zu schwenken, in denen die Backenenden (6219) der ersten und zweiten Backenkomponente (6249) voneinander abgespreizt sind, während die verzahnten Enden (6261) im Wesentlichen eine gleiche und entgegengesetzte Schwenkbewegung der ersten und zweiten Komponente (6249) gewährleisten,
**dadurch gekennzeichnet, dass** die untere Fläche (6225) ferner eine Ausrichtungskomponente (6263) umfasst, die dazu ausgelegt ist, durch eine komplementäre Ausrichtungskomponente (6236) der greifbaren Struktur aufgenommen zu werden.

2. Klemmvorrichtung (6200(b)) gemäß Anspruch 1, wobei der Rahmen (6215) ferner eine Vielzahl von Abschnitten umfasst, die auf der Innenseite der hinteren Fläche (6239) angeordnet sind, wobei die Vielzahl von Abschnitten dazu ausgelegt ist, die erste und die zweite Backenkomponente (6249) aufzunehmen.

3. Klemmanordnung (6200(b)) gemäß Anspruch 1, wobei die erste und die zweite Backenkomponente (6249) über einen ersten und einen zweiten Verbinder (6265), die zwischen der oberen Fläche (6223) und der unteren Fläche (6225) des Rahmens (6215) angeordnet sind, schwenkbar gehalten werden, wobei optional die erste und die zweite Backenkomponente (6249) um eine erste und eine zweite Drehachse schwenken, die durch die Verbinder (6265) verlaufen, und wobei optional die Verbinder (6265) Scharnierstifte sind.

4. Klemmanordnung (6200(b)) gemäß Anspruch 1, wobei die erste und die zweite Backenkomponente (6249) ferner eine oder mehrere Taschen (6275) umfassen, die dazu ausgelegt sind, im Wesentlichen das mindestens eine Vorspannelement (6273) zu aufzunehmen.

5. Klemmanordnung (6200(b)) gemäß Anspruch 4, wobei das mindestens eine Vorspannelement (6273) teilweise zusammengedrückt wird, um in der einen oder den mehreren Taschen (6275) der ersten und der zweiten Backenkomponente (6249) angeordnet zu werden, und/oder im Wesentlichen in der einen oder den mehreren Taschen (6275) der ersten und der zweiten Backenkomponente (6249) gegenüberliegend angeordnet ist.

6. Klemmanordnung gemäß Anspruch 1, wobei die Verschiebung des ersten und des zweiten Aktuators (6217) eine Verschiebung der ersten und der zweiten Backenkomponente (6249) bewirkt, wodurch ferner das mindestens eine Vorspannelement (6273) zusammengedrückt wird.

7. Klemmanordnung gemäß Anspruch 6, wobei das mindestens eine Vorspannelement (6273) dazu ausgelegt ist, die erste und die zweite Backenkomponente (6249) in ihrer ersten Position zu halten und/oder zuzulassen, dass die erste und die zweite Backenkomponente (6249) in ihre zweite Position verschoben werden und/oder zuzulassen, dass die erste und die zweite Backenkomponente (6249) verschoben werden, was die Backenenden (6219) veranlasst, die greifbare Struktur zu greifen.

8. Klemmanordnung (6200(b)) gemäß Anspruch 1, wobei die Backenenden (6219) der ersten und der zweiten Backenkomponente (6249) im Wesentlichen mit einer Schicht bedeckt sind.

9. Klemmanordnung (6200(b)) gemäß Anspruch 8, wobei die Schicht aus einem ersten Material besteht und das erste Material ein elastomeres Material ist.

10. Klemmanordnung (6200(b)) gemäß Anspruch 9, wobei die Schicht aus einem ersten Material mit einem hohen Reibungskoeffizienten mit einem zweiten Material einer Haltestruktur (6203) und/oder einem Material mit einem hohen Reibungskoeffizienten mit dem Material eines oder mehrerer gleitfähiger Elemente, die auf der Haltestruktur (6203) angeordnet sind, wie beispielsweise Gummi, besteht.

11. Klemmanordnung (6200(b)) gemäß Anspruch 1, wobei die Klemmanordnung ferner einen Riegel umfasst, wobei der Rahmen (6215) ferner eine Buchse (6241) auf der hinteren Fläche (6239) umfasst, wobei die Buchse (6241) dazu ausgelegt ist, den Riegel (6221) über ein Riegelhaltevorspannelement (6273) schwenkbar zu halten, wobei optional der Riegel (6221) dazu ausgelegt ist, die Klemmanordnung (6200(b)) mit einer Klemmvorrichtung (6207) zu koppeln, wenn der Riegel (6221) in eine Verriegelungsposition schwenkt, und/oder die Klemmvorrichtung (6207) freizugeben, wenn der Riegel (6221) in eine Entriegelungsposition schwenkt.

12. Klemmanordnung (6200(b)) gemäß Anspruch 11, wobei der Riegel (6221) ferner einen Klappenabschnitt (6224) und einen Hebelabschnitt (6222) umfasst, wobei optional der Klappenabschnitt (6224) des Riegels (6221) in Richtung eines Inneren des Rahmens (6215) geschwenkt wird, um ein oder mehrere Paarungselemente (6259) einer Klemmvorrichtung (6207) in der Buchse (6241) aufzunehmen, und/oder der Klappenabschnitt (6224) des Riegels (6221) nach vollständigem Aufnehmen des einen oder der mehreren Paarungselemente (6259) der Klemmvorrichtung (6207) in der Buchse (6241) zurückgeschwenkt wird, und/oder der Hebelabschnitt (6222) dazu ausgelegt ist, das Lösen der Paarungselemente (6259) aus der Buchse (6241) in der hinteren Fläche (6239) des Rahmens (6215) betriebsfähig zu ermöglichen.

13. Klemmanordnung (6200(b)) gemäß Anspruch 1, wobei der Rahmen (6215) ferner eine Vielzahl von Schienen umfasst, die dazu ausgelegt sind, die erste und die zweite Backenkomponente (6249) aufzunehmen.

14. Klemmanordnung (6200(b)) gemäß Anspruch 1, wobei die Backenenden (6219) der ersten und der zweiten Backenkomponente (6249) mit dem Rahmen (6215) über einen ersten bzw. einen zweiten Verbinder (6265) gekoppelt sind, und wobei optional die erste und die zweite Backenkomponente (6249) mit dem Rahmen (6215) über Scharnierstifte gekoppelt sind.

15. Klemmanordnung (6200(b)) gemäß Anspruch 1, wobei die erste und die zweite Backenkomponente (6249) ferner Abschnitte umfassen, die dazu ausgelegt sind, den ersten und den zweiten Aktuator (6217) teilweise aufzunehmen.

16. Klemmanordnung (6200(b)) gemäß Anspruch 15, wobei jeder des ersten und des zweiten Aktuators (6217) ferner ein Paarungselement und ein Wippelement (6251) umfasst, wobei optional die Abschnitte der ersten und der zweiten Backenkomponente (6249) dazu ausgelegt sind, die Paarungselemente des ersten und des zweiten Aktuators (6217) aufzunehmen.

17. Klemmanordnung (6200(b)) gemäß Anspruch 1, wobei die Verschiebung des ersten und des zweiten Aktuators (6217) eine Sollverschiebung der ersten und der zweiten Backenkomponente (6249) bewirkt.

## Revendications

1. Ensemble de fixation par serrage (6200(b)) conçu pour fixer un dispositif médical par serrage sur une structure préhensible, ledit ensemble de fixation par serrage (6200(b)) comprenant :
un étrier (6215) comportant une surface supérieure (6223) et une surface inférieure (6225) espacée de la surface supérieure (6223) de façon à former un écart entre la surface supérieure (6223) et la surface inférieure (6225), la surface supérieure (6223) comprenant une première échancrure (6240) et la surface inférieure (6225) comprenant une seconde échancrure (6240), les première et seconde échancrures (6240) étant conçues pour recevoir une structure préhensible, la surface supérieure (6223) et la surface inférieure (6225) étant raccordées par une surface arrière (6239), la surface arrière (6239) comprenant, en outre, une face intérieure et une face extérieure opposée ;
un premier composant formant mâchoire (6249) et un second composant formant mâchoire (6249), les premier et second composants formant mâchoires (6249) étant chacun retenus de manière pivotante dans l'étrier (6215) de telle sorte qu'au moins une partie des premier et second composants formant mâchoires (6249) est disposée entre la surface supérieure (6223) et la surface inférieure (6225) dans l'écart et de façon à avoir un axe de rotation sensiblement parallèle à la surface arrière (6239), les premier et second composants formant mâchoires (6249) comprenant chacun une extrémité formant mâchoire (6219) et une extrémité dentée (6261) à l'opposé de l'extrémité formant mâchoire (6219), l'extrémité dentée (6261) du premier composant formant mâchoire (6249) et l'extrémité dentée (6216) du second composant formant mâchoire (6249) étant engrenées ;
un premier actionneur (6217) et un second actionneur (6217) respectivement accouplés aux premier et second composants formant mâchoires (6249), les premier et second actionneurs (6217) comprenant chacun une extrémité (6251), les extrémités (6251) des premier et second actionneurs (6217) étant respectivement distales relativement aux premier et second composants formant mâchoires (6249) ; et
au moins un organe de sollicitation (6273) sollicitant les premier et second composants formant mâchoires (6249) vers des premières positions dans lesquelles il n'y a pas d'obstruction entre les première et seconde échancrures et la structure préhensible, un déplacement des premier et second actionneurs (6217) provoquant un déplacement des premier et second composants formant mâchoires (6249) de sorte qu'ils pivotent de leurs premières positions à des secondes positions dans lesquelles les extrémités formant mâchoires (6219) des premier et second composants formant mâchoires (6249) sont écartées l'une de l'autre tandis que les extrémités dentées (6261) assurent un déplacement par pivotement sensiblement égal et opposé des premier et second composants formant mâchoires (6249),
**caractérisé en ce que** la surface inférieure (6225) comprend, en outre, un composant d'alignement (6263) conçu pour être reçu par un composant d'alignement complémentaire (6236) de la structure préhensible.

2. Ensemble de fixation par serrage (6200(b)) selon la revendication 1, dans lequel l'étrier (6215) comprend, en outre, une pluralité de sections disposées sur la face intérieure de la surface arrière (6239), la pluralité de sections étant conçues pour accueillir les premier et second composants formant mâchoires (6249).

3. Ensemble de fixation par serrage (6200(b)) selon la revendication 1, dans lequel les premier et second composants formant mâchoires (6249) sont retenus de manière pivotante par le biais de premier et second éléments de raccordement (6265) disposés entre la surface supérieure (6223) et la surface inférieure (6225) de l'étrier (6215), dans lequel, éventuellement, les premier et second composants formant mâchoires (6249) pivotent autour de premier et second axes de rotation passant à travers les éléments de raccordement (6265), et dans lequel, éventuellement, les éléments de raccordement (6265) sont des broches d'articulation.

4. Ensemble de fixation par serrage (6200(b)) selon la revendication 1, dans lequel les premier et second composants formant mâchoires (6249) comprennent, en outre, une ou plusieurs cavités (6275) conçues pour recevoir sensiblement l'au moins un organe de sollicitation (6273).

5. Ensemble de fixation par serrage (6200(b)) selon la revendication 4, dans lequel l'au moins un organe de sollicitation (6273) est partiellement comprimé pour être placé dans la ou les cavités (6275) des premier et second composants formant mâchoires (6249) et/ou est sensiblement disposé dans une ou plusieurs cavités (6275) en regard des premier et second composants formant mâchoires (6249).

6. Ensemble de fixation par serrage selon la revendication 1, dans lequel le déplacement des premier et second actionneurs (6217) provoque le déplacement des premier et second composants formant mâchoires (6249), ce qui comprime, d'autre part, l'au moins un organe de sollicitation (6273).

7. Ensemble de fixation par serrage selon la revendication 6, dans lequel l'au moins un organe de sollicitation (6273) est conçu pour maintenir les premier et second composants formant mâchoires (6249) dans leurs premières positions et/ou permettre un déplacement des premier et second composants formant mâchoires (6249) jusqu'à leurs secondes positions et/ou permettre un déplacement des premier et second composants formant mâchoires (6249) de sorte que les extrémités formant mâchoires (6219) saisissent la structure préhensible.

8. Ensemble de fixation par serrage (6200(b)) selon la revendication 1, dans lequel les extrémités formant mâchoires (6219) des premier et second composants formant mâchoires (6249) sont sensiblement recouvertes d'une couche.

9. Ensemble de fixation par serrage (6200(b)) selon la revendication 8, dans lequel la couche est faite d'un premier matériau et le premier matériau est un matériau élastomère.

10. Ensemble de fixation par serrage (6200(b)) selon la revendication 9, dans lequel la couche est faite d'un premier matériau présentant un coefficient de frottement élevé avec un second matériau d'une structure de maintien (6203) et/ou d'un matériau présentant un coefficient de frottement élevé avec le matériau d'un ou de plusieurs organes coulissants disposés sur la structure de maintien (6203), tel que le caoutchouc.

11. Ensemble de fixation par serrage (6200(b)) selon la revendication 1, l'ensemble de fixation par serrage comprenant, en outre, un organe de verrouillage, dans lequel l'étrier (6215) comprend, en outre, un logement (6241) sur la surface arrière (6239), le logement (6241) étant conçu pour retenir de manière pivotante l'organe de verrouillage (6221) par le biais d'un organe de sollicitation de retenue d'organe de verrouillage (6273), dans lequel, éventuellement, l'organe de verrouillage (6221) est conçu pour accoupler l'ensemble de fixation par serrage (6200(b)) avec un dispositif à fixer (6207) lorsque l'organe de verrouillage (6221) pivote jusqu'à une position de verrouillage et/ou libérer le dispositif à fixer (6207) lorsque l'organe de verrouillage (6221) pivote jusqu'à une position de déverrouillage.

12. Ensemble de fixation par serrage (6200(b)) selon la revendication 11, dans lequel l'organe de verrouillage (6221) comprend, en outre, une partie ailette (6224) et une partie levier (6222), dans lequel, éventuellement, la partie ailette (6224) de l'organe de verrouillage (6221) est amenée à pivoter vers l'intérieur de l'étrier (6215) pour recevoir un ou plusieurs organes d'accouplement (6259) d'un dispositif à fixer (6207) dans le logement (6241) et/ou la partie ailette (6224) de l'organe de verrouillage (6221) est amenée à pivoter dans le sens inverse après avoir complètement reçu l'organe ou les organes d'accouplement (6259) du dispositif à fixer (6207) dans le logement (6241) et/ou la partie levier (6222) est conçue pour permettre fonctionnellement aux organes d'accouplement (6259) d'être libérés du logement (6241) dans la surface arrière (6239) de l'étrier (6215).

13. Ensemble de fixation par serrage (6200(b)) selon la revendication 1, dans lequel l'étrier (6215) comprend, en outre, une pluralité de voies conçues pour recevoir les premier et second composants formant mâchoires (6249).

14. Ensemble de fixation par serrage (6200(b)) selon la revendication 1, dans lequel les extrémités formant mâchoires (6219) des premier et second composants formant mâchoires (6249) sont respectivement accouplées avec l'étrier (6215) par le biais de premier et second éléments de raccordement (6265), et dans lequel, éventuellement, les premier et second composants formant mâchoires (6249) sont accouplés avec l'étrier (6215) par le biais de broches d'articulation.

15. Ensemble de fixation par serrage (6200(b)) selon la revendication 1, dans lequel les premier et second composants formant mâchoires (6249) comprennent, en outre, des sections conçues pour recevoir partiellement les premier et second actionneurs (6217).

16. Ensemble de fixation par serrage (6200(b)) selon la revendication 15, dans lequel chacun des premier et second actionneurs (6217) comprend, en outre, un organe d'accouplement et un organe formant palette (6251), dans lequel, éventuellement, les sections des premier et second composants formant mâchoires (6249) sont conçues pour recevoir les organes d'accouplement des premier et second actionneurs (6217).

17. Ensemble de fixation par serrage (6200(b)) selon la revendication 1, dans lequel le déplacement des premier et second actionneurs (6217) provoque un déplacement souhaité des premier et second composants formant mâchoires (6249).
